# EUROPEAN PATENT APPLICATION

(11) **EP 2 489 663 A1**
(43) Date of publication of application: **22.08.2012**
(21) Application number: 11382043.5
(22) Date of filing: 16.02.2011
(51) Int. Cl.: C07D 401/04, C07D 403/04, C07D 471/04, A61K 31/416, A61K 31/437, A61P 3/10, A61P 9/00, A61P 11/00, A61P 13/00, A61P 19/00, A61P 25/00, A61P 27/00, A61P 35/00, A61P 37/00

(54) **Compounds as syk kinase inhibitors**

(71) Applicant: Almirall, S.A., 08022 Barcelona (ES)
(72) Inventor: Vidal Juan, Bernat, 08980, Sant Feliu de Llobregat (ES); Fernandez Collado, Juan Carlos, 08980, Sant Feliu de Llobregat (ES); Erra Sola, Montserrat, 08980, Sant Feliu de Llobregat (ES); Aguilar Izquierdo, Nuria, 08980, Sant Feliu de Llobregat (ES); Mir Cepeda, Marta, 08980, Sant Feliu de Llobregat (ES); Carranco Moruno, Inés, 08980, Sant Feliu de Llobregat (ES)
(74) Representative: Elzaburu Marquez, Alberto

(57) **Abstract**

The present invention relates to a compound of formula (I), to the process for preparing such compounds and to their use in the treatment of a pathological condition or disease susceptible to amelioration by inhibition of Syk kinase.

## Description

### FIELD OF THE INVENTION

The present invention is directed to novel compounds having Syk inhibitory activity. The invention is also directed to pharmaceutical compositions comprising such compounds, methods of using such compounds to treat diseases associated with Syk activity and processes and intermediates useful for preparing such compounds.

### BACKGROUND OF THE INVENTION

Spleen tyrosine kinase (Syk) is a novel pharmaceutical target for treatment of allergic, autoimmune and neoplastic disorders. Syk is a non-receptor protein tyrosine kinase that plays an essential role in the signaling pathways of immune receptors on hematopoietic cells, mainly B-cells, mast cells, macrophages and neutrophils. These immunoreceptors, including Fc receptors and B-cell receptor, are important for both allergic diseases and antibody-mediated autoimmune diseases. Thus, pharmacological modulation of Syk activity can modulate the inflammatory response in immune-mediated disorders such as allergic disorders and autoimmune diseases. Syk belongs to the Syk/ZAP-70 family of tyrosine kinases predominantly expressed in hematopoietic cells. Syk is expressed in B cells, mast cells, basophils, eosinophils, neutrophils, macrophages, monocytes, NK cells, dendritic cells, platelets, erythrocytes and immature T cells. The contribution of Syk dependent processes to the pathology of allergic disorders, autoimmune disorders and oncology has been reviewed in Singh, R. and Matsuda, E.S.,Annu. Rep. Med. Chem., 2007, 42, 379-391.
a) Asthma and allergic rhinitis: Allergic responses of asthma and rhinitis are characterized by hyperproduction of IgE antibodies directed to common environmental antigens (allergens). Allergen-specific IgE binds to the surface of mast cells and basophils through high affinity FcεRI receptors for IgE. Following subsequent allergen exposure, activation of mast cells and basophils through FcεRI occurs and causes mast cell degranulation and release of inflammatory mediators, either preformed or synthesized *de novo.* Upon FcεRI engagement Syk is rapidly recruited and activated following the initiation of FcεRI-mediated signaling resulting in cellular degranulation and mast cell mediators release. Thus, Syk mediates signaling pathways initiated by stimulation of FcεRI, leading to allergic inflammation. Similar mechanisms are involved in signaling mediated by the engagement of Fc receptors for IgG (FcγRI, FcγRIIA and FcγRIIIA) and IgA (FcαRI) that also recruit Syk to the proximal part of their signaling pathways. Syk is critically involved in the regulation of many cellular functions initiated by Fcγ receptor engagement. For example, in macrophages Syk is relevant for phagocytosis and in neutrophils Syk activation mediates cellular spreading and respiratory burst. Reviewed in Mócsai, A. et al. Nat. Rev. Immunol. 2010, 10, 387-402 and Pamuk, N. and Tsokos G.C. Arthritis Res. Ther. 2010, 12, 222-232.
b) Rheumatoid arthritis (RA) and autoimmune diseases: The B cell receptor (BCR) controls a range of B cell responses including proliferation and differentiation into mature antibody producing cells. The BCR is a key regulator point for B cell activity and aberrant signaling can cause unregulated B cell proliferation and formation of pathogenic auto-antibodies that lead to multiple auto-immune and/or inflammatory diseases. Syk is immediately downstream BCR and is essential for BCR signaling. Inhibition of Syk is likely to block the BCR signaling and therefore may have a therapeutic benefit blocking B-cell mediated diseases processes. Moreover, Syk through FcγR-signaling plays a critical role in the activation of neutrophils and macrophages elicited by these auto-antiboides and their immune complexes. Thus, Syk inhibitors will also block the FcγR signaling through neutrophils and macrophages present in the synovial membrane (reviewed in Mócsai, A. et al. Nat. Rev. Immunol. 2010, 10, 387-402 and Pamuk, N. and Tsokos G.C. Arthritis Res. Ther. 2010, 12, 222-232). In addition, Syk inhibition has an additional benefit of inhibiting osteoclast maturation, thereby attenuating the bone erosion, joint destruction and osteopoenia associated with RA (Bajpai, M. et al .Expert Opin. Investig. Drugs 2008, 17(5), 641-659). It has also been reported that Syk deletion in mice dendritic cells abrogates FcgR-mediated cross priming of diabetogenic T cells in RIP-mOVA mice protecting from induction of diabetes (Colonna, L. et al. J. Immunol. 2010, 185, 1532-1534). Additionally, the same authors have shown that Syk inhibition, blocking BCR-antigen presentation, delays autoimmune diabetes development and prolongs survival in NOD mice. Thus targeting Syk could modulate autoimmune diseases such as type I diabetes.
c) Lymphoma: Besides the role of Syk in BCR cascade, Syk is over-expressed in a many B cell malignancies and is constitutively activated in several lymphomas, playing an essential role in BCR-mediated survival signals (Lesex, L. et al. Blood 2006, 108, 4156-4162). Research at Harvard Medical School and the Max Planck Institute has shown that over activity of Syk kinase is an essential mechanism of survival in several types of B cell lymphoma. Preclinical studies showed that inhibition of Syk kinase inhibits the growth of B cell lymphoma driven by Syk kinase over activity (Young R. M. et al. Blood 2009, 113, 2508-2516). A combination of genomic and expression profiling has identified Syk as a therapeutic target in lymphoma. Overall, Syk appears to function as a proto-oncogene in lymphocytes and, therefore offers a potential target in the treatment of lymphoma. Additionally Syk inhibitor P-505-15 has been reported to be a selective Syk inhibitor that inhibits primary chronic lymphocytic leukemia cells and in combination with fluradabine shows synergistic effects at the range of nM, confirming the potential of Syk inhibitors for treatment of chronic lymphocytic leukemia.
d) Immune Thrombocytopenic Purpura (ITP): ITP is a disorder defined as a subnormal number of platelets in the circulating blood attributed to an immune-mediated platelet destruction. Once coated with IgG autoantibodies, platelets are prematured cleared through Fcγ receptors that are expressed by tissue macrophages, predominantly in the spleen and liver. Activation of Fcγ receptors and subsequent Syk-mediated signaling has been implicated in the immune destruction of platelets, a hallmark of ITP. The immune system attack and platelet destruction leads to an abnormally low platelet count with resulting bruising and bleeding. As Syk kinase is involved in macrophage Fcγ receptor function and antibody production, its inhibition should abrogate platelet destruction. For a reference see Podolanczuk, A. et al. Blood 2009, 113, 3154-3160.
e) Ocular disorders: Syk inhibitors have been reported to be useful for the treatment of ocular related disorders such as macular degeneration, diabetic retinopathy, and diabetic macular edema (WO2009114373, WO2008033798) as well as allergic and inflammatory ophthalmic diseases (US7799778 and US7713975).
f) Renal disorders: Syk inhibitors have been reported to be useful for the prophylaxis or treatment of diabetic nephropathy and glomerulonephritis (J. Am. Soc. Nephrol. 2010, 21, 231-236 and WO2010065571).
h) Systemic lupus erythematosus (SLE): the pathogenesis of SLE has been associated with B-cell activation in which Syk plays an important role. Therefore, Syk inhibition therapy was used in lupus animal models (Arthritis Rheum. 2008, 58, 1433-1444; Arthritis Rheum. 2010, 62, 2086-2092). The fact that Syk inhibition suppresses SLE in at least three lupus-prone mice suggests that Syk inhibition in patients with SLE may be of clinical value.
i) Ischemia reperfusion injury: evidences have been published that support the therapeutic value of Syk inhibitors to conditions involving ischemia reperfusion injury, such as organ transplant and coronary and carotid revascularization (Am. J. Physiol. Gastrointest. Liver Physiol. 2010, 299, G391-399).

Patents and patent applications related to modulation of Syk kinase include: WO200109134, WO2002096905, WO2003057695, WO2003063794, WO2003078404, WO2004014382, WO2004087698, WO2004087699, WO2004080463, WO2005012294, WO2005016893, WO2005013996, WO2005026158, WO2005056547, WO2005118544, WO2006055528, WO2006055561, WO2006068770, WO2006078846, WO2006093247, WO2006129100, WO2007009681, WO2007028445, WO2007070872, WO2007042298, WO2007042299, WO2007085540, WO2007107469, WO2008033798, WO2008081928, WO2009026107, WO2009084695, WO2009086495, WO2009106441, WO2009106445, WO2009114373, WO2009131687, WO2009145856, WO2009136995, WO20100065571, WO2010015518, WO2010015520, WO2010097248, WO2010027500, WO2010147898, WO2010146133, WO2011014795.

While progress has been made in this field, there remains the need to identify further compounds which are inhibitors of Syk kinase.

The present invention relates to novel indazoles which are inhibitors of Syk kinase activity. Such derivatives have potential therapeutic benefit in the treatment of disorders associated with inappropriate Syk activity, in particular in the treatment and prevention of allergic, autoimmune and neoplastic disorders including asthma, chronic obstructive pulmonary disease, allergic rhinitis, allergic conjunctivitis, allergic dermatitis, atopic dermatitis, seasonal allergies, allergic and inflammatory ophthalmic diseases, rheumatoid arthritis, multiple sclerosis, systemic lupus erythematosus, type I diabetes, B cell lymphoma, chronic lymphocytic leukemia, immune thrombocytopenic purpura, macular degeneration, diabetic retinopathy, diabetic macular edema, diabetic nephropathy, glomerulonephritis, and ischemia reperfusion injury.

### SUMMARY OF THE INVENTION

Thus, the present invention is directed to new compounds of formula (I), or pharmaceutically acceptable salt or N-oxide or solvate or deuterated derivative thereof: 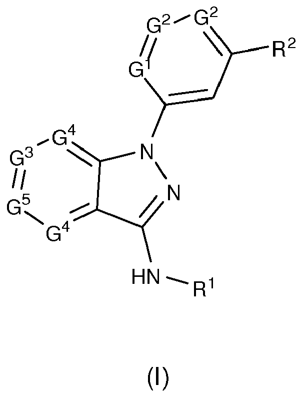 wherein:
- G¹ is selected from the group consisting of a N atom and a -CH group;
- each G² is the same or different and is independently selected from the group consisting of a N atom and a -CR³ group;
- G³ is selected from the group consisting of a N atom and a -CR⁴ group;
- each G⁴ is the same or different and is independently selected from the group consisting of a N atom and a-CH group;
- G⁵ is selected from the group consisting of a N atom and a -CR⁵ group;
- R¹ is a 5 to 6-membered monocyclic heteroaryl group comprising at least one N atom, wherein said heteroaryl group is optionally substituted with one or more substituents selected from the group consisting of a linear or branched C₁₋₆ alkyl group and a -CF₃ group;
- R² is selected from the group consisting of a -NR^{a}R^{b} group, a N-containing saturated or unsaturated 5 to 10-membered heterocyclyl group and a 5 to 6-membered monocyclic heteroaryl group comprising at least one N atom, wherein
   o R^{a} represents a hydrogen atom, a linear or branched C₁₋₆ alkyl group or a linear or branched C₁₋₆ alkoxy group, and R^{b} represents a monocyclic or polycyclic C₃₋₁₀ cycloalkyl group or a N-containing saturated or unsaturated 4 to 10-membered heterocyclyl group; or
   o R^{a} and R^{b} together with the nitrogen atom to which they are attached form a N-containing saturated 5 to 10-membered monocyclic or bicyclic heterocyclyl group optionally containing one additional N atom, and wherein
   the heterocyclyl, heteroaryl, and cycloalkyl groups are optionally substituted with one or more substituents selected from the group consisting of a halogen atom, a -CN group, a -CF₃ group, a hydroxy group, a C₁₋₆ alkyl group, a C₁₋₆ alkoxy-C₁₋₆ alkyl group, a C₁₋₆ hydroxyalkyl group, an amino group and a carbamoyl group;
- R³ is selected from the group consisting of a hydrogen atom, a -CF₃ group, a phenyl group, a C₁₋₆ alkyl group, a hydroxy group and a C₁₋₆ alkoxy group; wherein the alkyl and phenyl groups are optionally substituted with one or more substituents selected from a hydroxy group, a C₁₋₆ alkoxy group and a halogen atom;
- R⁴ is selected from the group consisting of a hydrogen atom, a halogen atom, a -CN group, a hydroxy group, a linear or branched C₁₋₆ alkyl group, a linear or branched C₁₋₆ alkylthio group, a linear or branched C₁₋₆ alkoxy group, a (C₁₋₆ alkoxy)-(C₁₋₆ alkoxy) group, a C₆₋₁₀ aryl group, a (C₆₋₁₀ aryl)-(C₁₋₆ alkyl) group, a C₆₋₁₀ aryl-C₁₋₆ alkoxy group, a 5 to 10-membered heteroaryl group, a C₃₋₁₀ cycloalkyl group, a -(CH₂)₀₋₁COOR^{d} group, a -CONR^{c}R^{d} group, a -NR^{c}COR^{d} group, a -NHSO₂R^{d} group and a -NR^{c}R^{d} group; wherein
   o R^{c} represents a hydrogen atom or a linear or branched C₁₋₆ alkyl group, and R^{d} represents a hydrogen atom, a linear or branched C₁₋₆ alkyl group, a C₃₋₁₀ cycloalkyl group, a -(CH₂)₀₋₁C₆₋₁₀ aryl group, a 5 to 6-membered heteroaryl group containing at least one heteroatom selected from N, S and O; or
   o R^{c} and R^{d} together with the nitrogen atom to which they are attached form a N-containing 4 to 6-membered heterocyclyl group;
   wherein the alkyl, aryl, heteroaryl and heterocyclyl groups are optionally substituted with one or more substituents selected from a halogen atom, a hydroxy group, a linear or branched C₁₋₆ alkyl group, a linear or branched C₁₋₆ alkoxy group, a carboxy group and a benzyl group; and
- R⁵ is selected from the group consisting of a hydrogen atom, a halogen atom and a cyano group.

The invention provides synthetic processes and intermediates described herein, which are useful for preparing compounds of the invention.

The invention also provides a pharmaceutical composition comprising a compound of the invention and a pharmaceutically-acceptable carrier. The invention further provides combinations comprising a compound of the invention and one or more additional therapeutic agents, and pharmaceutical compositions comprising such combinations.

The invention also provides a compound of the invention for use in the treatment of the human or animal body by therapy.

The invention also provides a method of treating a disease or condition susceptible to amelioration by inhibition of Syk kinase in a mammal, in particular wherein the disease or condition is selected from a pulmonary disease, such as asthma or chronic obstructive pulmonary disease, allergic rhinitis, allergic conjunctivitis, allergic dermatitis, atopic dermatitis, seasonal allergies, allergic and inflammatory ophthalmic diseases, rheumatoid arthritis, multiple sclerosis, systemic lupus erythematosus, type I diabetes, B cell lymphoma, chronic lymphocytic leukemia, immune thrombocytopenic purpura, macular degeneration, diabetic retinopathy, diabetic macular edema, diabetic nephropathy, glomerulonephritis, and ischemia reperfusion injury, comprising such method administering to the mammal, a therapeutically effective amount of a compound of the invention. The invention further provides a method of treatment comprising administering a therapeutically effective amount of a combination of a compound of the invention together with one or more additional therapeutic agents.

The invention also provides a compound as described herein for use in the treatment of a disease or condition susceptible to amelioration by inhibition of Syk kinase in a mammal, in particular wherein the disease or condition is selected from a pulmonary disease, such as asthma or chronic obstructive pulmonary disease, allergic rhinitis, allergic conjunctivitis, allergic dermatitis, atopic dermatitis, seasonal allergies, allergic and inflammatory ophthalmic diseases, rheumatoid arthritis, multiple sclerosis, systemic lupus erythematosus, type I diabetes, B cell lymphoma, chronic lymphocytic leukemia, immune thrombocytopenic purpura, macular degeneration, diabetic retinopathy, diabetic macular edema, diabetic nephropathy, glomerulonephritis, and ischemia reperfusion injury.

The invention also provides the use of a compound of the invention in the manufacture of a formulation or medicament for treating a disease or condition susceptible to amelioration by inhibition of Syk kinase in a mammal, in particular wherein the condition or disease is selected from a pulmonary disease, such as asthma or chronic obstructive pulmonary disease, allergic rhinitis, allergic conjunctivitis, allergic dermatitis, atopic dermatitis, seasonal allergies, allergic and inflammatory ophthalmic diseases, rheumatoid arthritis, multiple sclerosis, systemic lupus erythematosus, type I diabetes, B cell lymphoma, chronic lymphocytic leukemia, immune thrombocytopenic purpura, macular degeneration, diabetic retinopathy, diabetic macular edema, diabetic nephropathy, glomerulonephritis, and ischemia reperfusion injury.

The invention also provides a combination product comprising (i) a compound of the invention as described herein; and (ii) one or more active ingredients selected from the group consisting of a β2-adrenergic agonist, a corticosteroid, an anti-cholinergic agent, an anti-allergic agent, an anti-inflammatory agent, an immunosuppressant, a PDE4 inhibitor, a topically acting p38 inhibitor, an IKK2 inhibitor, an A_{2A} agonist, a PI3Kδγ inhibitor and an anti-infective agent, for simultaneous, separate or sequential use in the treatment of the human or animal body.

### DETAILED DESCRIPTION OF THE INVENTION.

When describing the compounds, compositions and methods of the invention, the following terms have the following meanings, unless otherwise indicated.

As used herein the term C₁₋₆ alkyl embraces linear or branched radicals having 1 to 6 carbon atoms. Examples include methyl, ethyl, n-propyl, i-propyl, n-butyl, sec-butyl, t-butyl, n-pentyl, neopentyl or n-hexyl.

As used herein, the term C₁₋₆ alkoxy (or alkyloxy) embraces linear or branched oxy-containing radicals each having alkyl portions of 1 to 6 carbon atoms. Examples of C₁₋₆ alkoxy radicals include methoxy, ethoxy, n-propoxy, i-propoxy, n-butoxy, sec-butoxy or t-butoxy or n-pentoxy.

As used herein, the term C₁₋₆ alkylthio embraces linear or branched alkyl radicals of 1 to 6 carbon atoms attached to a divalent -S- radical. Exemples include methylthio, ethylthio, n-propylthio, i-propylthio, n-butylthio, sec-butylthio or t-butylthio.

As used herein, the term C₁₋₆ hydroxyalkyl embraces linear or branched alkyl radicals having 1 to 6 carbon atoms, any one of which may be substituted with one or more hydroxyl radicals.
Examples of such radicals include hydroxymethyl, hydroxyethyl, hydroxypropyl, and hydroxybutyl.

As used herein, the term C₆₋₁₀ aryl radical embraces typically a C₆-C₁₀ monocyclic or polycyclic aryl radical such as phenyl or naphthyl.

As used herein, the term 5 to 10- membered heteroaryl radical embraces typically a 5 to 10- membered ring system comprising at least one heteroaromatic ring and containing at least one heteroatom, and preferably one or two heteroatoms, selected from O, S and N. A 5 to 10- membered heteroaryl radical may be a single ring or two or more fused rings wherein at least one ring contains a heteroatom.
Examples include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, pyridinyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2*H*-pyrazolo[3,4-d]pyrimidinyl, 1*H*-pyrazolo[3,4-d]pyrimidinyl, thieno[2,3-d] pyrimidinyl and the various pyrrolopyridyl radicals.

As used herein, the term 5 to 6-membered monocyclic heteroaryl group comprising at least one N atom embraces typically a 5 to 6- membered ring system comprising a single heteroaromatic ring and containing at least one, and preferably one or two, N atoms. Examples include pyridyl, pyrazinyl, pyrazolyl and pyrimidinyl.

As used herein, the term C₃₋₁₀ cycloalkyl embraces saturated carbocyclic radicals having from 3 to 10 carbon atoms. A C₃₋₁₀ cycloalkyl moiety may be monocylic or polycyclic. Examples include cyclopropyl, cyclobutyl, cyclopentyl, cyclohexyl, cycloheptyl, cyclooctyl and bicyclo[2.2.1]heptane.

As used herein, the term N-containing saturated or unsaturated 4 to 10-membered heterocyclyl group embraces typically a non-aromatic, saturated or unsaturated C₄-C₁₀ carbocyclic ring system in which one or more, preferably one or two, carbon atoms is replaced by a nitrogen atom and optionally an oxygen atom. Examples include azetidinly, piperidyl, pyrrolidyl, pyrrolinyl, piperazinyl, 1,2,3,6-tetrahydropyridinyl or morpholinyl.

As used herein, the term N-containing saturated or unsaturated 5 to 10-membered heterocyclyl group embraces typically a non-aromatic, saturated or unsaturated C₅-C₁₀ carbocyclic ring system in which one or more, preferably one or two, carbon atoms is replaced by a nitrogen atom and optionally an oxygen atom. Examples include piperidyl, pyrrolidyl, pyrrolinyl, piperazinyl, 1,2,3,6-tetrahydropyridinyl or morpholinyl.

As used herein, the term N-containing 4 to 6-membered heterocyclyl group embraces typically a non-aromatic, saturated or unsaturated C₄-C₆ carbocyclic ring system in which one or more, preferably one or two, carbon atoms is replaced by a nitrogen atom and optionally an oxygen atom. Examples include azetidinly, piperidyl, pyrrolidyl, pyrrolinyl, piperazinyl, 1,2,3,6-tetrahydropyridinyl or morpholinyl.

As used herein, some of the atoms, radicals, moieties, chains and cycles present in the general structures of the invention are "optionally substituted". This means that these atoms, radicals, moieties, chains and cycles can be either unsubstituted or substituted in any position by one or more, for example 1, 2, 3 or 4, substituents, whereby the hydrogen atoms bound to the unsubstituted atoms, radicals, moieties, chains and cycles are replaced by chemically acceptable atoms, radicals, moieties, chains and cycles. When two or more substituents are present, each substituent may be the same or different. The substituents are typically themselves unsubstituted.

As used herein, the term halogen atom embraces chlorine, fluorine, bromine or iodine atoms typically a fluorine, chlorine or bromine atom. The term halo when used as a prefix has the same meaning.

Compounds containing one or more chiral centre may be used in enantiomerically or diastereoisomerically pure form, in the form of racemic mixtures and in the form of mixtures enriched in one or more stereoisomer. The scope of the invention as described and claimed encompasses the racemic forms of the compounds as well as the individual enantiomers, diastereomers, and stereoisomer-enriched mixtures.

Conventional techniques for the preparation/isolation of individual enantiomers include chiral synthesis from a suitable optically pure precursor or resolution of the racemate using, for example, chiral high pressure liquid chromatography (HPLC). Alternatively, the racemate (or a racemic precursor) may be reacted with a suitable optically active compound, for example, an alcohol, or, in the case where the compound contains an acidic or basic moiety, an acid or base such as tartaric acid or 1-phenylethylamine. The resulting diastereomehc mixture may be separated by chromatography and/or fractional crystallization and one or both of the diastereoisomers converted to the corresponding pure enantiomer(s) by means well known to one skilled in the art. Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% isopropanol, typically from 2 to 20%, and from 0 to 5% of an alkylamine, typically 0.1 % diethylamine. Concentration of the eluate affords the enriched mixture. Stereoisomer conglomerates may be separated by conventional techniques known to those skilled in the art. See, e.g. "Stereochemistry of Organic Compounds" by Ernest L. Eliel (Wiley, New York, 1994).

The term "pharmaceutically-acceptable salt" refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid; and organic acids, for example citric, fumaric, gluconic, glutamic, lactic, maleic, malic, mandelic, mucic, ascorbic, oxalic, pantothenic, succinic, tartaric, benzoic, acetic, methanesulphonic, ethanesulphonic, benzenesulphonic, p-toluenesulphonic, xinafoic (1-hydroxy-2-naphthoic acid), napadisilic (1,5-naphthalenedisulfonic acid), triphenylacetic and the like. Particularly preferred are salts derived from fumaric, hydrobromic, hydrochloric, acetic, sulfuric, methanesulfonic, xinafoic, and tartaric acids.

Salts derived from pharmaceutically-acceptable inorganic bases include aluminum, ammonium, calcium, copper, ferric, ferrous, lithium, magnesium, manganic, manganous, potassium, sodium, zinc and the like. Particularly preferred are ammonium, calcium, magnesium, potassium and sodium salts.

Salts derived from pharmaceutically-acceptable organic bases include salts of primary, secondary and tertiary amines, including alkyl amines, arylalkyl amines, heterocyclyl amines, cyclic amines, naturally-occurring amines and the like, such as arginine, betaine, caffeine, choline, *N*,*N'*-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Other preferred salts according to the invention are quaternary ammonium compounds wherein an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and *p*-toluenesulphonate. X⁻ is preferably an anion selected from chloride, bromide, iodide, sulphate, nitrate, acetate, maleate, oxalate, succinate or trifluoroacetate. More preferably X⁻ is chloride, bromide, trifluoroacetate or methanesulphonate.

As used herein, an N-oxide is formed from the tertiary basic amines or imines present in the molecule, using a convenient oxidising agent.

The compounds of the invention may exist in both unsolvated and solvated forms. The term solvate is used herein to describe a molecular complex comprising a compound of the invention and an amount of one or more pharmaceutically acceptable solvent molecules. The term hydrate is employed when said solvent is water. Examples of solvate forms include, but are not limited to, compounds of the invention in association with water, acetone, dichloromethane, 2-propanol, ethanol, methanol, dimethylsulfoxide (DMSO), ethyl acetate, acetic acid, ethanolamine, or mixtures thereof. It is specifically contemplated that in the present invention one solvent molecule can be associated with one molecule of the compounds of the present invention, such as a hydrate.

Furthermore, it is specifically contemplated that in the present invention, more than one solvent molecule may be associated with one molecule of the compounds of the present invention, such as a dihydrate. Additionally, it is specifically contemplated that in the present invention less than one solvent molecule may be associated with one molecule of the compounds of the present invention, such as a hemihydrate. Furthermore, solvates of the present invention are contemplated as solvates of compounds of the present invention that retain the biological effectiveness of the non-solvate form of the compounds.

The invention also includes isotopically-labeled compounds of the invention, wherein one or more atoms is replaced by an atom having the same atomic number, but an atomic mass or mass number different from the atomic mass or mass number usually found in nature. Examples of isotopes suitable for inclusion in the compounds of the invention include isotopes of hydrogen, such as ²H and ³H, carbon, such as ¹¹C, ¹³C and ¹⁴C, chlorine, such as ³⁶CH, fluorine, such as ¹⁸F, iodine, such as ¹²³I and ¹²⁵I nitrogen, such as ¹³N and ¹⁵N, oxygen, such as ¹⁵O, ¹⁷O and ¹⁸O, phosphorus, such as ³²P, and sulfur, such as ³⁵S. Certain isotopically-labeled compounds of the invention, for example, those incorporating a radioactive isotope, are useful in drug and/or substrate tissue distribution studies. The radioactive isotopes tritium, ³H, and carbon-14, ¹⁴C, are particularly useful for this purpose in view of their ease of incorporation and ready means of detection. Substitution with heavier isotopes such as deuterium, ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of the invention can generally be prepared by conventional techniques known to those skilled in the art or by processes analogous to those described herein, using an appropriate isotopically-labeled reagent in place of the non-labeled reagent otherwise employed.

Preferred isotopically-labeled compounds include deuterated derivatives of the compounds of the invention. As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

Hydrogen deuterium exchange (deuterium incorporation) is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

Prodrugs of the compounds described herein are also within the scope of the invention. Thus certain derivatives of the compounds of the present invention, which derivatives may have little or no pharmacological activity themselves, when administered into or onto the body may be converted into compounds of the present invention having the desired activity, for example, by hydrolytic cleavage. Such derivatives are referred to as 'prodrugs'. Further information on the use of prodrugs may be found in Pro-drugs as Novel Delivery Systems, Vol. 14, ACS Symposium Series (T. Higuchi and W. Stella) and Bioreversible Carriers in Drug Design, Pergamon Press, 1987 (ed. E. B. Roche, American Pharmaceutical Association).

Prodrugs in accordance with the invention can, for example, be produced by replacing appropriate functionalities present in the compounds of the present invention with certain moieties known to those skilled in the art as 'pro-moieties' as described, for example, in Design of Prodrugs by H. Bundgaard (Elsevier, 1985).

In the case of compounds that are solids, it is understood by those skilled in the art that the inventive compounds and salts may exist in different crystalline or polymorphic forms, or in an amorphous form, all of which are intended to be within the scope of the present invention.

Typically, R¹ represents a pyrazinyl group or a pyridyl group, wherein the pyridyl group is optionally substituted by a methyl group or a CF₃ group.

Typically, R^{a} and R^{b} together with the nitrogen atom to which they are attached form a saturated 5 to 10-membered monocyclic or bicyclic heterocyclyl group containing one additional N atom, wherein the heterocyclyl is optionally substituted with one or more C₁₋₆ alkyl groups.

Typically, R² represents a -NR^{a}R^{b} group, wherein R^{a} and R^{b} together with the nitrogen atom to which they are attached form a saturated 5 to 10-membered monocyclic or bicyclic heterocyclyl group containing one additional N atom, wherein the heterocyclyl is optionally substituted with one or more C₁₋₆ alkyl groups.

Typically, R³ is selected from the group consisting of a hydrogen atom, a C₁₋₆ alkyl group and a phenyl group, wherein the phenyl group is optionally substituted by a halogen atom.

Typically, R^{c} represents a hydrogen atom or methyl group, and R^{d} represents a linear or branched C₁₋₆ alkyl group, a C₃₋₁₀ cycloalkyl group or a C₆ aryl group; or R^{c} and R^{d} together with the nitrogen atom to which they are attached form N-containing 4 to 6-membered heterocyclic group, wherein the alkyl, aryl and heteroaryl groups are optionally substituted with a methyl group or a hydroxy group.

Typically, R⁴ is selected from the group consisting of a hydrogen atom, a hydroxy group, an amino group, a linear or branched C₁₋₆ alkyl group, a linear or branched C₁₋₆ alkylthio group, a linear or branched C₁₋₆ alkoxy group, a (C₁₋₆ alkoxy)-(C₁₋₆ alkoxy) group, a C₃₋₁₀ cycloalkyl group, a 5 to 6-membered heteroaryl group, a C₆ aryl group, a morpholinyl group, a -CONR^{c}R^{d} group, a -NR^{c}COR^{d} group, -NHSO₂R^{d} group; wherein
o R^{c} represents a hydrogen atom or methyl group, and R^{d} represents a linear or branched C₁₋₆ alkyl group, a C₃₋₁₀ cycloalkyl group or a C₆ aryl group; or
o R^{c} and R^{d} together with the nitrogen atom to which they are attached form N-containing 4 to 6-membered heterocyclic group,
wherein the alkyl, aryl and heteroaryl groups are optionally substituted with a methyl group or a hydroxy group.

Typically, when R⁴ or R^{d} represents a linear or branched C₁₋₆ alkyl group, that alkyl group is not itself substituted with a C₁₋₆ alkyl group.

Typically, G⁵ represents a CH group.

Typically, G¹ and the two G² moieties do not all represent N.

Typically, the two G² moieties do not both represent N. Typically, one of the two G² moieties represents N.

Typically, G³, G⁵ and the two G⁴ moieties do not all represent N.

Typically, G³ and G⁵ do not both represent N.

Typically, the two G⁴ moieties do not both represent N. Typically, the two G⁴ moieties both represent -CH.

Typically, when either of the two G⁴ moieties represents N, G³ and G⁵ do not represent N.

When an R² moiety is optionally substituted with a C₁₋₆alkoxyC₁₋₆ alkyl group, it is to be understood that the C₁₋₆ alkyl portion is bonded to the R² moiety.

When R⁴ represents a C₆₋₁₀ aryl-C₁₋₆ alkyl moietiy, it is to be understood that the d₁₋₆ alkyl portion is bonded to the C atom of the -CR⁴ moiety that G³ represents.

When R⁴ represents a C₆₋₁₀ aryl-C₁₋₆ alkoxy moietiy, it is to be understood that the C₁₋₆ alkoxy portion is bonded to the C atom of the -CR⁴ moiety that G₃ represents.

In a preferred embodiment of the present invention, compounds of the present invention are those of formula (I) wherein:
- R¹ represents a pyrazinyl group or a pyridyl group, wherein the pyridyl group is optionally substituted by a methyl group or a CF₃ group;
- R² represents a -NR^{a}R^{b} group, wherein R^{a} and R^{b} together with the nitrogen atom to which they are attached form a saturated 5 to 10-membered monocyclic or bicyclic heterocyclyl group containing one additional N atom, wherein the heterocyclyl is optionally substituted with one or more C₁₋₆ alkyl groups;

- R³ is selected from the group consisting of a hydrogen atom, a C₁₋₆ alkyl group and a phenyl group, wherein the phenyl group is optionally substituted by a halogen atom;
- R⁴ is selected from the group consisting of a hydrogen atom, a hydroxy group, an amino group, a linear or branched C₁₋₆ alkyl group, a linear or branched C₁₋₆ alkylthio group, a linear or branched C₁₋₆ alkoxy group, a (C₁₋₆ alkoxy)-(C₁₋₆ alkoxy) group, a C₃₋₁₀ cycloalkyl group, a 5 to 6-membered heteroaryl group, a C₆ aryl group, a morpohlinyl group, a -CONR^{c}R^{d} group, a -NR^{c}COR^{d} group, a -NHSO₂R^{d} group; wherein
   o R^{c} represents a hydrogen atom or a methyl group, and R^{d} represents a linear or branched C₁₋₆ alkyl group, a C₃₋₁₀ cycloalkyl group, a C₆ aryl group, or
   o R^{c} and R^{d} together with the nitrogen atom to which they are attached form a N-containing 4- to 6-membered heterocyclic group,
   wherein the alkyl, aryl and heteroaryl groups are optionally substituted with a methyl group or a hydroxy group; and
- G⁵ represents a CH group.

In one embodiment of the present invention, compounds of formula (I) are those wherein:
- R¹ represents a pyrazinyl group or a pyridyl group, wherein the pyridyl group is optionally substituted with a methyl group or a -CF₃ group;
- R² is selected from the group consisting of -NR^{a}R^{b}, a piperidinyl group and a tetrahydropyridinyl group, wherein
   o R^{a} represents a hydrogen atom and R^{b} represents a cyclohexyl group, an azetidinyl group or an adamantyl group; or
   o R^{a} and R^{b} together with the nitrogen atom to which they are attached form a piperidinyl group, a piperazinyl group, a diazepanyl group, a 2,5-diazabicyclo[2.2.1]heptanyl group, an octahydropyrrolo[3,4-c]pyrrolyl group or a 3,6-diazabicyclo[3.2.1]octanyl group,
   wherein:
   the piperidinyl, piperazinyl, diazepanyl, cyclohexyl and adamantyl groups are optionally substituted with one or more substituents selected from the group consisting of a fluorine atom, a -CN group, a hydroxy group, a methyl group, a methoxymethyl group, a hydroxymethyl group, a carbamoyl group and an amino group;
- R³ is selected from the group consisting of a hydrogen atom, a methyl group, an isopropyl group, a hydroxy group, a methoxy group, a hydroxymethyl group, a methoxymethyl group and a phenyl substitued with a flourine atom;
- R⁴ is selected from the group consisting of a hydrogen atom, a fluorine atom, a bromine atom, a hydroxy group, an amino group, a 2-hydroxypropan-2-yl group, a -CN group, a methyl group, a butyl group, an isopentyl group, a cyclopropyl group, a phenyl group, a hydroxyphenyl group, a methoxy group, an ethoxy group, an isopropoxy group, an isobutoxy group, a butoxy group, a methoxyethoxy group, a benzyloxy group, a pyridyl group, a methylthio group, a pyrazolyl group substituted with a methyl group, a morpholinyl group, a -COOR^{d} group, a -CONR^{c}R^{d} group, a -NR^{c}COR^{d} group, and a -NHSO₂R^{d} group; wherein
   o R^{c} represents a hydrogen atom, a methyl group , and R^{d} represents a hydrogen atom, a methyl group, a cyclohexyl group, a phenyl group, a benzyl group or a carboxymethyl group; or
   o R^{c} and R^{d} together with the nitrogen atom to which they are attached form a piperidinyl group optionally substituted with one substituent selected from a carboxy group and a benzyl group; and
- R⁵ is selected from the group consisting of a hydrogen atom, a fluorine atom and a cyano group.

Particular individual compounds of the invention include:
*N*-Pyrazin-2-yl-1-[3-(1,2,3,6-tetrahydropyridin-4-yl)phenyl]-1*H*-indazol-3-amine;
1-[3-(4-Fluoropiperidin-4-yl)phenyl]-*N*-pyrazin-2-yl-1*H*-indazol-3-amine;
1-(3-Piperazin-1-ylphenyl)-*N*-pyrazin-2-yl-1*H*-indazol-3-amine;
1-(5-Piperazin-1-ylpyridin-3-yl)-*N*-pyrazin-2-yl-1*H*-indazol-3-amine;
*N*-(4-Methylpyridin-2-yl)-1-(2-piperazin-1-ylpyridin-4-yl)-1*H*-indazol-3-amine;
1-(2-Piperazin-1-ylpyridin-4-yl)-*N*-pyrazin-2-ly-1*H*-indazol-3-amine;
6-Methoxy-1-(2-piperazin-1-ylpyridin-4-yl)-*N*-pyrazin-2-yl-1*H*-indazol-3-amine;
1-[2-(1,4-Diazepan-1-yl)pyridin-4-yl]-*N*-pyrazin-2-yl-1*H*-indazol-3-amine;
1-(2-((1S,4S)-2,5-diazabicyclo[2.2.1]heptan-2-yl)pyridin-4-yl)-*N*-(4-methylpyridin-2-yl)-1*H*-indazol-3-amine;
1-{2-[(1S,4S)-2,5-Diazabicyclo[2.2.1]hept-2-yl]pyridin-4-yl}-N-pyrazin-2-yl-1*H*-indazol-3-amine;
1-{2-[(1S,4S)-2,5-Diazabicyclo[2.2.1]hept-2-yl]pyridin-4-yl}-3-(pyrazin-2-ylamino)-1*H-*indazole-5-carbonitrile;
1-{2-[(1S,4S)-2,5-Diazabicyclo[2.2.1]hept-2-yl]pyridin-4-yl}-*N*³-pyrazin-2-yl-1*H*-indazole-3,6-diamine;
6-Bromo-1-{2-[(1S,4S)-2,5-diazabicyclo[2.2.1]hept-2-yl]pyridin-4-yl}-*N*-pyrazin-2-yl-1*H-*indazol-3-amine;
1-{2-[(1S,4S)-2,5-Diazabicyclo[2.2.1]hept-2-yl]pyridin-4-yl}-6-phenyl-*N*-pyrazin-2-yl-1*H-*indazol-3-amine;
Methyl 1-12-[(1S,4S)-2,5-diazabicyclo[2.2.1]hept-2-yl]pyridin-4-yl}-3-(pyrazin-2-ylamino)-1*H*-indazole-6-carboxylate;
1-{2-[(1S,45)-2,5-Diazabicydo[2.2.]hept-2-yl]pyridin-4-yl}-3-(pyrazin-2-ylamino)-1*H-*indazole-6-carboxamide;
1-{2-[(1S,4S)-2,5-Diazabicyclo[2.2.1]hept-2-yl]pyridin-4-yl}-3-(pyrazin-2-ylamino)-1*H-*indazole-6-carboxylic acid;
*N*-Cyclohexyl-1-{2-[(1S,4S)-2,5-diazabicyclo[2.2.1]hept-2-yl]pyridin-4-yl}-3-(pyrazin-2-ylamino)-1*H*-indazole-6-carboxamide;
*N*-Benzyl-1-{2-[(1S,4S)-2,5-diazabicyclo[2.2.1]hept-2-yl]pyridin-4-yl}-3-(pyrazin-2-ylamino)-1*H*-indazole-6-carboxamide;
1-{[1-{2-[(1S,4S)-2,5-Diazabicyclo[2.2.1]hept-2-yl]pyridin-4-yl}-3-(pyrazin-2-ylamino)-1*H*-indazol-6-yl]carbonyl}piperidine-4-carboxylic acid;
1-{2-[(1S,4S)-2,5-Diazabicyclo[2.2.1]hept-2-yl]pyridin-4-yl}-6-(piperidin-1-ylcarbonyl)-*N-*pyrazin-2-yl-1*H*-indazol-3-amine;
6-[(4-Benzylpiperidin-1-yl)carbonyl]-1-{2-[(1S,4S)-2,5-diazabicyclo[2.2.1]hept-2-yl]pyridin-4-yl}-*N*-pyrazin-2-yl-1*H*-indazol-3-amine;
*N*-{[1-{2-[(1S,4S)-2,5-Diazabicyclo[2.2.1]hept-2-yl]pyridin-4-yl}-3-(pyrazin-2-ylamino)-1*H*-indazol-6-yl]carbonyl}-*N*-methylglycine;
1-{2-[(1S,4S)-2,5-Diazabicyclo[2.2.1]hept-2-yl]pyridin-4-yl}-3-(pyrazin-2-ylamino)-1*H-*indazole-6-carbonitrile;
1-{2-[(1S,4S)-2,5-Diazabicyclo[2.2.1]hept-2-yl]pyridin-4-yl}-6-fluoro-*N*-pyrazin-2-yl-1*H-*indazol-3-amine;
*N*-[1-{2-[(1S,4S)-2,5-Diazabicyclo[2.2.1]hept-2-yl]pyridin-4-yl}-3-(pyrazin-2-ylamino)-1 H-indazol-6-yl]acetamide;
*N*-[1-{2-[(1S,4S)-2,5-Diazabicyclo[2.2.1]hept-2-yl]pyridin-4-yl}-3-(pyrazin-2-ylamino)-1H-indazol-6-yl]benzamide;
*N*-[1-{2-[(1S,4S)-2,5-Diazabicyclo[2.2.1]hept-2-yl]pyridin-4-yl}-3-(pyrazin-2-ylamino)-1*H*-indazol-6-yl]-*N*-methylacetamide;
*N*-[1-{2-[(1S,4S)-2,5-Diazabicyclo[2.2.1]hept-2-yl]pyridin-4-yl}-3-(pyrazin-2-ylamino)-1H-indazol-6-yl]methanesulfonamide;
*N*-[1-{2-[(1S,4S)-2,5-Diazabicyclo[2.2.1]hept-2-yl]pyridin-4-yl}-3-(pyrazin-2-ylamino)-1 H-indazol-6-yl]benzenesulfonamide;
1-{2-[(1S,4S)-2,5-Diazabicyclo[2.2.1]hept-2-yl]pyridin-4-yl}-6-methoxy-*N*-pyrazin-2-yl-1*H*-indazol-3-amine;
1-{2-[(1S,4S)-2,5-Diazabicyclo[2.2.1]hept-2-yl]pyridin-4-yl}-6-ethoxy-*N*-pyrazin-2-yl-1*H-*indazol-3-amine;
4-{4-[3-(Pyrazin-2-ylamino)-1*H*-indazol-1-yl]pyridin-2-yl}piperidine-4-carbonitrile;
1-{2-[(2S,5R)-2,5-dimethylpiperazin-1-yl]pyhdin-4-yl}-*N*-pyrazin-2-yl-1*H*-indazol-3-amine;
(1S,2R)-*N*-{4-[3-(pyrazin-2-ylamlno)-1*H*-indazol-1-yl]pyridin-2-yl}cyclohexane-1,2-diamine;
(1R,2R)-*N*-{4-[3-(pyrazin-2-ylamino)-1*H*-indazol-1-yl]pyridin-2-yl}cyclohexane-1,2-diamine;
1-{4-[3-(pyrazin-2-ylamino)-1*H*-indazol-1-yl]pyridin-2-yl}piperidine-4-carboxamide;
1-{4-[3-(Pyrazin-2-ylamino)-1*H*-indazol-1-yl]pyridin-2-yl}piperidin-4-ol;
trans-4-({4-[3-(Pyrazin-2-ylamino)-1*H*-indazol-1-yl]pyridin-2-yl}amino)cyclohexanol;
(4R,7R)-4-({4-[3-(Pyrazin-2-ylamino)-1*H*-indazol-1-yl]pyridin-2-yl}amino)adamantan-1-ol;
1-(2-Methyl-6-piperazin-1-ylpyrimidin-4-yl)-N-pyrazin-2-yl-1*H*-indazol-3-amine;
1-{6-[(1S,4S)-2,5-Diazabicyclo[2.2.1]hept-2-yl]-2-methylpyrimindin-4-yl}-*N*-pyrazin-2-yl-1*H*-indazol-3-amine;
1-[2-(Methoxymethyl)-6-piperazin-1-ylpyrimidin-4-yl]-*N*-pyrazin-2-yl-1*H*-indazol-3-amine;
1-{2-[(1S,4S)-2,5-Diazabicyclo[2.2.1]hept-2-yl]pyridin-4-yl}-*N*-pyrazin-2-yl-1*H-*pyrazolo[3,4-c]pyridin-3-amine;
1-{2-[(1S,4S)-2,5-Diazabicyclo [2.2.1]hept-2-yl]pyridin-4-yl}-*N*-pyrazin-2-yl-1*H-*pyrazolo[4,3-b]pyridin-3-amine;
1-{2-[(1S,4S)-2,5-Diazabicyclo[2.2.1]hept-2-yl]pyridin-4-yl}-*N*-pyrazin-2-yl-1*H-*pyrazolo[4,3-c]pyridin-3-amine;
1-{2-[(1S,4S)-2,5-Diazabicyclo[2.2.1]hept-2-yl]pyridin-4-yl}-*N*-(4-methylpyridin-2-yl)-1*H-*pyrazolo[3,4-b]pyridin-3-amine;
1-{2-[(1S,4S)-2,5-Diazabicyclo[2.2.1]hept-2-yl]pyridin-4-yl}-*N*-[4-(trifluoromethyl) pyridin-2-yl]-1*H*-pyrazolo[3,4-b]pyridin-3-amine;
1-{2-[(1S,4S)-2,5-Diazabicyclo[2.2.1]hept-2-yl]pyridin-4-yl}-*N*-pyrazin-2-yl-1*H-*pyrazolo[3,4-b]pyridin-3-amine;
1-{2-[(2S)-2-methylpiperazin-1-yl]pyridin-4-yl}-*N*-pyrazin-2-yl-1*H*-indazol-3-amine;
1-{5-[(1S,4S)-2,5-diazabicyclo[2.2.1]hept-2-yl]pyridin-3-yl}-*N*-pyrazin-2-yl-1*H*-indazol-3-amine;
{4-piperazin-1-yl-6-[3-(pyrazin-2-ylamino)-1*H*-indazol-1-yl]pyrimidin-2-yl}methanol;
1-{2-[(1S,4S)-2,5-diazabicyclo[2.2.1]hept-2-yl]pyridin-4-yl}-3-(pyrazin-2-ylamino)-1*H-*indazol-6-ol;
1-{5-[(1S,4S)-2,5-diazabicyclo[2.2.1]hept-2-yl]pyridin-3-yl}-6-methoxy-*N*-pyrazin-2-yl-1*H*-indazol-3-amine;
1-{2-[(1S,4S)-2,5-diazabicyclo[2.2.1]hept-2-yl]pyridin-4-yl}-6-methyl-*N*-pyrazin-2-yl-1*H-*indazol-3-amine;
1-{6-[(1S,4S)-2,5-diazabicyclo[2.2.1]hept-2-yl]pyrimidin-4-yl}-6-methoxy-*N*-pyrazin-2-yl-1*H*-indazol-3-amine;
1-{6-[(1S,4S)-2,5-diazabicyclo[2.2.1]hept-2-yl]pyrimidin-4-yl}-*N*-pyrazin-2-yl-1*H-*pyrazolo[3,4-b]pyridin-3-amine;
6-cyclopropyl-1-{2-[(1S,4S)-2,5-diazabicyclo[2.2.1]hept-2-y]pyridin-4-yl}-*N*-pyrazin-2-yl-1*H*-indazol-3-amine;
1-[2-(6-methyl-1,4-diazepan-1-yl)pyridin-4-yl]-*N*-pyrazin-2-yl-1*H*-indazol-3-amine;
1-(4-piperazin-1-ylpyridin-2-yl)-*N*-pyrazin-2-yl-1*H*-indazol-3-amine;
1-{2-[(1S,4S)-2,5-diazabicyclo[2.2.1]hept-2-yl]pyridin-4-yl}-6-(2-methoxyethoxy)-*N-*pyrazin-2-yl-1*H*-indazol-3-amine;
1-[2-(1,4-diazepan-1-yl)pyridin-4-yl]-6-methoxy-*N*-pyrazin-2-yl-1*H*-indazol-3-amine;
1-{5-[(1S,4S)-2,5-Diazabicyclo[2.2.1]hept-2-yl]pyridin-3-yl}-3-(pyrazin-2-ylamino)-1*H-*indazol-6-ol;
1-{2-[(1S,4S)-2,5-Diazabicyclo[2.2.1]hept-2-yl]pyridin-4-yl}-6-methyl-*N*-pyrazin-2-yl-1*H-*pyrazolo[3,4-b]pyridin-3-amine;
1-{5-[(2S)-2-methylpiperazin-1-yl]pyridin-3-yl}-*N*-pyrazin-2-yl-1*H*-indazol-3-amine;
1-{2-[(2R)-2-methylpiperazin-1-yl]pyridin-4-yl}-*N*-pyrazin-2-yl-1*H*-indazol-3-amine;
1-[2-(3,6-diazabicyclo[3.2.1]oct-3-yl)pyridin-4-yl]-*N*-pyrazin-2-yl-1*H*-indazol-3-amine;
4-{4-[6-Methoxy-3-(pyrazin-2-ylamino)-1*H*-indazol-1-yl]pyridin-2-yl}piperidine-4-carbonitrile;
1-{4-[(1S,4S)-2,5-Diazabicyclo[2.2.1]hept-2-yl]pyridin-2-yl}-pyrazin-2-yl}-*N*-1*H*-indazol-3-amine;
1-{2-[(1S,4S)-2,5-diazabicyclo[2.2.1]hept-2-yl]pyridin-4-yl}-6-isobutoxy-*N*-pyrazin-2-yl-1*H*-indazol-3-amine;
1-{5-[(1S,4S)-2,5-diazabicyclo[2.2.1]hept-2-yl]pyridin-3-yl}-6-methyl-*N*-pyrazin-2-yl-1*H-*indazol-3-amine;
6-butyl-1-{2-[(1S,4S)-2,5-diazabicyclo[2.2.1]hept-2-yl]pyridin-4-yl}-*N*-pyrazin-2-yl-1*H-*indazol-3-amine;
1-{2-[(1S,4S)-2,5-diazabicyclo[2.2.1]hept-2-yl]pyridin-4-yl}-6-(3-methylbutyl)-*N*-pyrazin-2-yl-1*H*-indazol-3-amine;
1-{2-[4-(methoxymethyl)piperidin-4-yl]pyridin-4-yl}-*N*-pyrazin-2-yl-1*H*-indazol-3-amine;
6-butoxy-1-{2-[(1S,4S)-2,5-diazabicyclo[2.2.1]hept-2-yl]pyridin-4-yl}-*N*-pyrazin-2-yl-1*H-*indazol-3-amine;
6-Methoxy-1-{2-[(2S)-2-methylpiperazin-1-yl]pyridin-4-yl}-*N*-pyrazin-2-yl-1*H*-indazol-3-amine;
1-{2-[(1S,4S)-2,5-diazabicyclo[2.2.1]hept-2-yl]pyridin-4-yl}-6-isopropoxy-*N*-pyrazin-2-yl-1*H*-indazol-3-amine;
6-(benzyloxy)-1-{2-[(2S)-2-methylpiperazin-1-yl]pyridin-4-yl}-*N*-pyrazin-2-yl-1*H*-indazol-3-amine;
6-(Benzyloxy)-1-[2-(1,4-diazepan-1-yl)pyridin-4-yl]-*N*-pyrazin-2-yl-1*H*-indazol-3-amine;
1-(5-Piperazin-1-ylpyridin-3-yl)-*N*-pyhdin-2-yl-1*H*-indazol-3-amine;
1-(5-((1S,4S)-2,5-diazabicyclo[2.2.1]heptan-2-yl)pyridin-3-yl)-5-fluoro-6-methoxy-*N-*(pyrazin-2-yl)-1*H*-indazol-3-amine;
1-{5-[(1S,4S)-2,5-diazabicyclo[2.2.1]hept-2-yl]pyridin-3-yl}-5-fluoro-3-(pyrazin-2-ylamino)-1*H*-indazol-6-ol;
(4-{4-[3-(Pyrazin-2-ylamino)-1*H*-indazol-1-yl]pyridin-2-yl}piperidin-4-yl)methanol;
6-methyl-1-{2-[(2S)-2-methylpiperazin-1-yl]pyridin-4-yl)-*N*-pyrazin-2-yl-1*H*-indazol-3-amine;
1-[6-[(1S,4S)-2,5-diazabicyclo[2.2.1]hept-2-yl]-2-(3-fluorophenyl)pyrimidin-4-yl]-6-methoxy-*N*-pyrazin-2-yl-1*H*-indazol-3-amine;
1-[2-(1,4-diazepan-1-yl)pyridin-4-yl]-3-(pyrazin-2-ylamino)-1*H*-indazol-6-ol;
1-{2-[(2S)-2-methylpiperazin-1-yl]pyridin-4-yl}-3-(pyrazin-2-ylamino)-1*H*-indazol-6-ol;
1-{2-[(1S,4S)-2,5-diazabicyclo[2.2.1]hept-2-yl]pyridin-4-yl}-3-[(4-methylpyridin-2-yl)amino]-1*H*-indazol-6-ol;
1-{3-[(1S,4S)-2,5-diazabicyclo[2.2.1]hept-2-yl]phenyl}-6-methoxy-*N*-pyrazin-2-yl-1*H-*indazol-3-amine;
4-[1-{2-[(1S,4S)-2,5-diazabicyclo[2.2.1]hept-2-yl]pyridin-4-yl}-3-(pyrazin-2-ylamino)-1*H-*indazol-6-yl]phenol;
1-{2-[(1S,4S)-2,5-Diazabicyclo[2.2.1]hept-2-yl]pyridin-4-yl}-6-morpholin-4-yl-*N*-pyrazin-2-yl-1*H*-pyrazolo[3,4-b]pyridin-3-amine;
1-{2-[(1S,4S)-2,5-diazabicyclo[2.2.1]hept-2-yl]pyhdin-4-yl}-3-{[4-(trifluoromethyl)pyridin-2-yl]amino}-1*H*-indazol-6-ol;
Benzyl 1-{2-[(1S,4S)-2,5-diazabicyclo[2.2.1]hept-2-yl]pyridin-4-yl}-3-(pyrazin-2-ylamino)-1*H*-indazole-6-carboxylate;
1-{5-[(1S,4S)-2,5-diazabicyclo[2.2.1]hept-2-yl]-6-methoxypyhdin-3-yl}-*N*-pyrazin-2-yl-1*H*-indazol-3-amine;
1-{2-[(1S,4S)-2,5-diazabicyclo[2.2.1]hept-2-yl]pyridin-4-yl}-6-(methylthio)-*N*-pyrazin-2-yl-1*H*-indazol-3-amine;
1-{2-[(2S,5S)-2,5-Dimethylpiperazin-1-yl]pyridin-4-yl}-*N*-pyrazin-2-yl-1*H*-indazol-3-amine;
1-{6-[(1S,4S)-2,5-diazabicyclo[2.2.1]hept-2-yl]-2-isopropylpyrimidin-4-yl}-3-(pyrazin-2-ylamino)-1*H*-indazol-6-ol;
1-{2-[(1S,4S)-2,5-diazabicyclo[2.2.1]hept-2-yl]pyridin-4-yl}-*N*-pyrazin-2-yl-6-pyridin-3-yl-1*H*-indazol-3-amine;
1-{2-[(1S,4S)-2,5-diazabicyclo[2.2.1]hept-2-yl]pyridin-4-yl}-6-(1-methyl-1*H*-pyrazol-4-yl)-*N*-pyrazin-2-yl-1*H*-indazol-3-amine;
1-{5-[(2S)-2-Methylpiperazin-1-yl]pyridin-3-yl}-3-(pyrazin-2-ylamino)-1*H*-indazol-6-ol;
2-[1-{2-[(1S,4S)-2,5-Diazabicyclo[2.2.1]hept-2-yl]pyridin-4-yl}-3-(pyrazin-2-ylamino)-1*H-*indazol-6-yl]propan-2-ol;
1-[2-(Azetidin-3-ylamino)pyridin-4-yl]-*N*-pyrazin-2-yl-1*H*-indazol-3-amine;
1-{2-[(3aR,6aS)-Hexahydropyrrolo[3,4-c]pyrrol-2(1*H*)-yl]pyridin-4-yl}-N-pyrazin-2-yl-1*H-*indazol-3-amine;
1-{6-[(1S,4S)-2,5-Diazabicyclo[2.2.1]hept-2-yl]-2-methylpyrimidin-4-yl}-3-(pyrazin-2-ylamino)-1*H*-indazol-6-ol;
1-{2-Methyl-6-[(25)-2-methylpiperazin-1-yl]pyrimidin-4-yl}-3-(pyrazin-2-ylamino)-1*H-*indazol-6-ol;
3-[(1S,4S)-2,5-Diazabicyclo[2.2.1]hept-2-yl]-5-[3-(pyrazin-2-ylamino)-1*H*-indazol-1-yl]pyridin-2-ol; or
1-{3-[(2S)-2-Methylpiperazin-1-yl]phenyl}-3-(pyrazin-2-ylamino)-1*H*-indazol-6-ol.

Of particular interest are the compounds:
*N*-(4-Methylpyridin-2-yl)-1-(2-piperazin-1-ylpyridin-4-yl)-1*H*-indazol-3-amine;
1-[2-(1,4-Diazepan-1-yl)pyridin-4-yl]-*N*-pyrazin-2-yl-1*H*-indazol-3-amine;
1-(2-((1S,4S)-2,5-diazabicyclo[2.2.1]heptan-2-yl)pyridin-4-yl)-*N*-(4-methylpyridin-2-yl)-1*H*-indazol-3-amine;
1-{2-[(1S,4S)-2,5-Diazabicyclo[2.2.1]hept-2-yl]pyridin-4-yl}-*N*-pyrazin-2-yl-1*H*-indazol-3-amine;
1-{2-[(1S,4S)-2,5-Diazabicyclo[2.2.1]hept-2-yl]pyridin-4-yl}-*N*³-pyrazin-2-yl-1*H*-indazole-3,6-diamine;
1-{2-[(1S,4S)-2,5-Diazabicyclo[2.2.1]hept-2-yl]pyridin-4-yl}-6-phenyl-*N*-pyrazin-2-yl-1*H-*indazol-3-amine;
*N*-Cyclohexyl-1-{2-[(1S,4S)-2,5-diazabicyclo[2.2.1]hept-2-yl]pyhdin-4-yl}-3-(pyrazin-2-ylamino)-1*H*-indazole-6-carboxamide;
1-{2-[(1S,4S)-2,5-Diazabicyclo[2.2.1]hept-2-yl]pyhdin-4-yl}-6-(piperidin-1-ylcarbonyl)-*N-*pyrazin-2-yl-1*H*-indazol-3-amine;
*N*-[1-{2-[(1S,4S)-2,5-Diazabicycio[2.2.1]hept-2-yl]pyhdin-4-yl}-3-(pyrazin-2-ylamino)-1*H*-indazol-6-yl]acetamide;
*N*-[1-{2-[(1S,4S)-2,5-Diazabicyclo[2.2.1]hept-2-yl]pyhdin-4-yl}-3-(pyrazin-2-ylamino)-1*H*-indazol-6-yl]benzamide;
*N*-[1-{2-[(1S,4S)-2,5-Diazabicyclo[2.2.1]hept-2-yl]pyhdin-4-yl}-3-(pyrazin-2-ylamino)-1*H*-indazol-6-yl]-*N*-methylacetamide;
*N*-[1-{2-[(1S,4S)-2,5-Diazabicyclo[2.2.1]hept-2-yl]pyhdin-4-yl}-3-(pyrazin-2-ylamino)-1*H*-indazol-6-yl]methanesulfonamide;
*N*-[1-{2-[(1S,4S)-2,5-Diazabicyclo[2.2.1]hept-2-yl]pyhdin-4-yl}-3-(pyrazin-2-ylamino)-1*H*-indazol-6-yl]benzenesulfonamide;
1-{2-[(1S,4S)-2,5-Diazabicyclo[2.2.1]hept-2-yl]pyridin-4-yl}-6-methoxy-*N*-pyrazin-2-yl-1*H*-indazol-3-amine;
1-{2-[(1S,4S)-2,5-Diazabicyclo[2.2.1]hept-2-yl]pyridin-4-yl}-6-ethoxy-*N*-pyrazin-2-yl-1*H-*indazol-3-amine;
1-{6-[(1S,4S)-2,5-Diazabicyclo[2.2.1]hept-2-yl]-2-methylpyrimidin-4-yl}-*N*-pyrazin-2-yl-1*H*-indazol-3-amine;
1-{2-[(1S,4S)-2,5-Diazabicyclo[2.2.1]hept-2-yl]pyridin-4-yl}-*N*-[4-(trifluoromethyl) pyridin-2-yl]-1*H*-pyrazolo[3,4-b]pyridin-3-amine;
1-{2-[(2S)-2-methylplperazin-1-yl]pyhdin-4-yl}-*N*-pyrazin-2-yl-1*H*-indazol-3-amine;
1-{5-[(1S,4S)-2,5-diazabicyclo[2.2.1]hept-2-yl]pyridin-3-yl}-*N*-pyrazin-2-yl-1*H*-indazol-3-amine;
1-{2-[(1S,4S)-2,5-diazabicyclo[2.2.1]hept-2-yl]pyridin-4-yl}-3-(pyrazin-2-ylamino)-1*H-*indazol-6-ol;
1-{5-[(1S,4S)-2,5-diazabicyclo[2.2.1]hept-2-yl]pyhdin-3-yl}-6-methoxy-*N*-pyrazin-2-yl-1*H*-indazol-3-amine;
1-{2-[(1S,4S)-2,5-diazabicyclo[2.2.1]hept-2-yl]pyridin-4-yl}-6-methyl-*N*-pyrazin-2-yl-1*H-*indazol-3-amine;
1-{6-[(1S,4S)-2,5-diazabicyclo[2.2.1]hept-2-yl]pyrimidin-4-yl}-6-methoxy-*N*-pyrazin-2-yl-1*H*-indazol-3-amine;
6-cyclopropyl-1-{2-[(1S,4S)-2,5-diazabicyclo[2.2.1]hept-2-yl]pyridin-4-yl}-*N*-pyrazin-2-yl-1*H*-indazol-3-amine;
1-12-[(1S,4S)-2,5-diazabicyclo[2.2.1]hept-2-yl]pyridin-4-yl}-6-(2-methoxyethoxy)-*N-*pyrazin-2-yl-1*H*-indazol-3-amine;
1-[2-(1,4-diazepan-1-yl)pyridin-4-yl]-6-methoxy-*N*-pyrazin-2-yl-1*H*-indazol-3-amine;
1-{5-[(1S,4S)-2,5-Diazabicyclo[2.2.1]hept-2-yl]pyridin-3-yl}-3-(pyrazin-2-ylamino)-1*H-*indazol-6-ol;
1-{2-[(1S,4S)-2,5-Diazabicyclo[2.2.1]hept-2-yl]pyridin-4-yl}-6-methyl-*N*-pyrazin-2-yl-1*H-*pyrazolo[3,4-b]pyridin-3-amine;
1-{4-[(1S,4S)-2,5-Diazabicyclo[2.2.1]hept-2-yl]pyridin-2-yl}-*N*-pyrazin-2-yl-1*H*-indazol-3-amine;
1-{2-[(1S,4S)-2,5-diazabicyclo[2.2.1]hept-2-yl]pyridin-4-yl}-6-isobutoxy-*N*-pyrazin-2-yl-1*H*-indazol-3-amine;
1-15-[(1S,4S)-2,5-diazabicyclo[2.2.1]hept-2-yl]pyridin-3-yl}-6-methyl-*N*-pyrazin-2-yl-1*H-*indazol-3-amine;
6-butyl-1-{2-[(1S,4S)-2,5-diazabicyclo[2.2.1]hept-2-yl]pyridin-4-yl}-*N*-pyrazin-2-yl-1*H-*indazol-3-amine;
1-{2-[(1S,4S)-2,5-diazabicyclo[2.2.1]hept-2-yl]pyridin-4-yl}-6-(3-methylbutyl)-*N*-pyrazin-2-yl-1*H*-indazol-3-amine;
6-butoxy-1-{2-[(1S,4S)-2,5-diazabicyclo[2.2.1]hept-2-yl]pyridin-4-yl}-*N*-pyrazin-2-yl-1*H-*indazol-3-amine;
6-Methoxy-1-{2-[(2S)-2-methylpiperazin-1-yl]pyridin-4-yl}-*N*-pyrazin-2-yl-1*H*-indazol-3-amine;
1-{12-[(1S,4S)-2,5-diazabicyclo[2.2.1]hept-2-yl]pyridin-4-yl}-6-isopropoxy-*N*-pyrazin-2-yl-1*H*-indazol-3-amine;
6-methyl-1-{2-[(2S)-2-methylpiperazin-1-yl]pyridin-4-yl}-*N*-pyrazin-2-yl-1*H*-indazol-3-amine;
1-[6-[(1S,4S)-2,5-diazabicyclo[2.2.1]hept-2-yl]-2-(3-fluorophenyl)pyrimidin-4-yl]-6-methoxy-*N*-pyrazin-2-yl-1*H*-indazol-3-amine;
1-[2-(1,4-diazepan-1-yl)pyridin-4-yl]-3-(pyrazin-2-ylamino)-1*H*-indazol-6-ol;
1-{2-[(2S)-2-methylpiperazin-1-yl]pyridin-4-yl}-3-(pyrazin-2-ylamino)-1*H*-indazol-6-ol;
1-{2-[(1S,4S)-2,5-diazabicyclo[2.2.1]hept-2-yl]pyridin-4-yl}-3-[(4-methylpyridin-2-yl)amino]-1*H*-indazol-6-ol;
1-{3-[(1S,4S)-2,5-diazabicyclo[2.2.1]hept-2-yl]phenyl}-6-methoxy-*N*-pyrazin-2-yl-1*H-*indazol-3-amine;
4-[1-{2-[(1S,4S)-2,5-diazabicyclo[2.2.1]hept-2-yl]pyridin-4-yl}-3-(pyrazin-2-ylamino)-1*H-*indazol-6-yl]phenol;
1-{2-[(1S,4S)-2,5-Diazabicyclo[2.2.1]hept-2-yl]pyridin-4-yl}-6-morpholin-4-yl-*N*-pyrazin-2-yl-1*H*-pyrazolo[3,4-b]pyridin-3-amine;
1-{2-[(1S,4S)-2,5-diazabicyclo[2.2.1-]hept-2-yl]pyridin-4-yl}-3-{[4-(trifluoromethyl)pyridin 2-yl]amino}-1*H*-indazol-6-ol;
1-{2-[(1S,4S)-2,5-diazabicyclo[2.2.1]hept-2-yl]pyridin-4-yl}-6-(methylthio)-*N*-pyrazin-2-yl-1*H*-indazol-3-amine;
1-{2-[(2S,5S)-2,5-Dimethylpiperazin-1-yl]pyridin-4-yl}-*N*-pyrazin-2-yl-1*H*-indazol-3-amine;
1-{6-[(1S,4S)-2,5-diazabicyclo[2.2.1]hept-2-yl]-2-isopropylpyrimidin-4-yl}-3-(pyrazin-2-ylamino)-1*H*-indazol-6-ol;
1-{2-[(1S,4S)-2,5-diazabicyclo[2.2.1]hept-2-yl]pyridin-4-yl}-*N*-pyrazin-2-yl-6-pyridin-3-yl-1*H*-indazol-3-amine;
1-{2-[(1S,4S)-2,5-diazabicyclo[2.2.1]hept-2-yl]pyridin-4-yl}-6-(1-methyl-1*H*-pyrazol-4-yl)-*N*-pyrazin-2-yl-1*H*-indazol-3-amine;
2-[1-{2-[(1S,4S)-2,5-Diazabicyclo[2.2.1]hept-2-yl]pyridin-4-yl}-3-(pyrazin-2-ylamino)-1*H-*indazol-6-yl]propan-2-ol;
1-{6-[(1S,4S)-2,5-Diazabicyclo[2.2.1]hept-2-yl]-2-methylpyrimidin-4-yl}-3-(pyrazin-2-ylamino)-1*H*-indazol-6-ol; or
1-{3-[(2*S*)-2-Methylpiperazin-1-yl]phenyl}-3-(pyrazin-2-ylamino)-1*H*-indazol-6-ol.

The term "therapeutically effective amount" refers to an amount sufficient to effect treatment when administered to a patient in need of treatment.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The phrase "disease or condition susceptible to amelioration by inhibition of Syk kinase" includes all disease states and/or conditions that are acknowledged now, or that are found in the future, to be associated with Syk kinase. Such disease states include, but are not limited to, pulmonary diseases, such as asthma and chronic obstructive pulmonary disease (including chronic bronchitis and emphysema), as well as allergic rhinitis, allergic conjunctivitis, allergic dermatitis, atopic dermatitis, seasonal allergies, allergic and inflammatory ophthalmic diseases, rheumatoid arthritis, multiple sclerosis, systemic lupus erythematosus, type I diabetes, B cell lymphoma, chronic lymphocytic leukemia, immune thrombocytopenic purpura, macular degeneration, diabetic retinopathy, diabetic macular edema, diabetic nephropathy, glomerulonephritis, and ischemia reperfusion injury.

### GENERAL SYNTHETIC PROCEDURES

The compounds of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given; other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and deprotection, are well known in the art. For example, numerous protecting groups, and their introduction and removal are described in T. W. Greene and G. M. Wuts, Protecting Groups in Organic Synthesis, Third Edition, Wiley, New York, 1999, and references cited therein.

Processes for preparing compounds of the invention are provided as further embodiments of the invention and are illustrated by the procedures below.

Compounds of general formula (I) may be prepared following the synthetic scheme depicted in Scheme 1 A and Scheme 1 B.

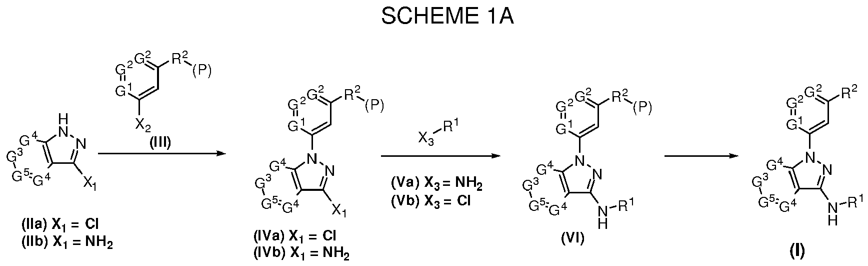

The compounds of formula (IVa) and (IVb) may be obtained by *N*-Arylation of the derivatives of formula (IIa) and (IIb) with the corresponding haloderivative of formula (III), optionally protected (P) with a group such as tert-butoxycarbonyl (BOC) . These reactions may be catalyzed by copper iodide and a ligand such as *trans-N,N'-*dimethylcyclohexane-1,2-diamine in an organic solvent such as toluene, *N-*methylpyrrolidone or dioxane and in the presence of a base such as potassium phosphate or cessium carbonate at a temperature from 60ºC to 150ºC, using conventional heating or microwave reactor.

Compounds of formula (VI) may be obtained by *N*-Arylation of the haloderivative of formula (IVa) with an aminoderivative of formula (Va) or by *N*-arylation of compounds of general formula (IVb) with the haloderivative (Vb). These reactions may be catalyzed by a palladium catalyst such as tris(dibenzylideneacetone)-dipalladium(0) or palladium(II) acetate and a ligand such as 4,5-bis(diphenylphosphino)-9,9-dimethylxanthene or (±)-2,2'-bis(diphenylphosphino)-1,1'-binaphthalene in an organic solvent such as dioxane, toluene, DMF, THF, benzene or 1,2-dimethoxyethane and in the presence of a base such as cesium carbonate, sodium carbonate, potassium phosphate or potassium tert-butoxide at a temperature from 60°C to 150°C, using conventional heating or microwave reactor.

The deprotection of intermediates of formula (VI) giving the target compounds (I) may be carried out by treating intermediates of formula (VI) with an acid such as trifluoroacetic acid or hydrochloric acid in a solvent such as dichloromethane, water or dioxane, at a temperature from room temperature to 90°C.

In the particular case wherein R² represents a -NR^{a}R^{b} group, compounds of general formula (Ia) can be prepared following the sequence described in Scheme 1B using the fluoro derivative of formula (VIII) and by final displacement of the fluorine group by a conveniently substituted amine (HNR^{a}R^{b}) in the presence of a base such as potassium carbonate in polar organic solvents such as DMSO or DMF and at temperatures ranging between 100°C and 200°C.

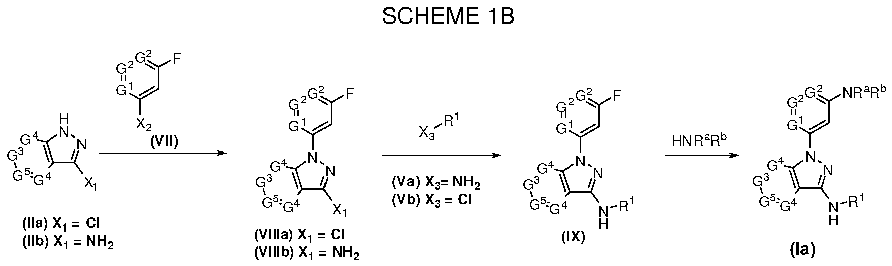

Building blocks of general formula (IIIa), wherein R² represents a -NR^{a}R^{b} group optionally protected (P) with a group such as BOC, may be prepared following the synthetic scheme illustrated in Scheme 2A. Nucleophilic addition of an amine to the intermediate (X) in the presence of a base such as potassium carbonate and in a solvent such as DMSO at temperatures ranging between 60-200°C provides compounds of general formula (IIIa).
Alternatively, building blocks of general formula (IIIa) can be obtained by *N*-arylation of the haloderivative of formula (X) with the corresponding amine (HNR^{a}R^{b}) optionally protected (P) with a group such as BOC and catalized by a palladium catalyst such as tris(dibenzylideneacetone)-dipalladium(0) or palladium(II) acetate and a ligand such as 4,5-bis(diphenylphosphino)-9,9-dimethylxanthene or (±)-2,2'-bis(diphenylphosphino)-1,1'-binaphthalene in an organic solvent such as dioxane, toluene, DMF, THF, benzene or 1,2-dimethoxyethane and in the presence of a base such as cesium carbonate, sodium carbonate, potassium phosphate or potassium tert-butoxide at a temperature from 90°C to 150ºC, using conventional heating or microwave reactor.

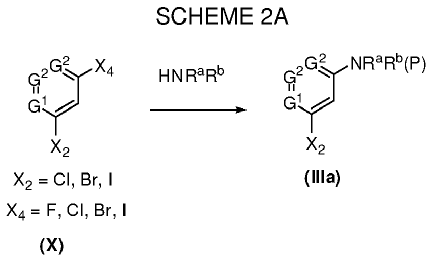

Building blocks of general formula (IIIb) wherein R² is a *N*-containing saturated or unsaturated 5 to 10-membered heterocyclic group or a 5 to 6-membered monocyclic heteroaryl group comprising at least one N atom can be obtained following the synthetic scheme illustrated in Scheme 2B. Suzuky coupling of the haloderivative of formula (XI) with the corresponding boronic or boronate derivative (XII) (wherein R^{e} and R^{f} together with the carbon atom to which they are attached form a N-containing saturated or unsaturated 5 to 10-membered heterocyclyl group or a 5 to 6-membered monocyclic heteroaryl group comprising at least one N atom) optionally protected (P) with a group such as BOC provides compounds of general formula (IIIb). Suzuki reactions (Miyaura, N.; Suzuki, A. Chem. Rev. 1995, 95, 2457) are performed typically using in R₂(P) and may be catalized by a palladium catalyst such as [1,1'-bis(diphenylphosphino)-ferrocene] dichloropalladium (II) complex with dichloromethane (1:1), tetrakis(triphenylphosphine)-palladium(0), bis(triphenylphosphine)palladium(II) chloride or tris(dibenzylideneacetone)-dipalladium(0) in an organic solvent such as dioxane, toluene, dimethylformamide, ethanol, 1,2-dimethoxyethane or mixtures of them and in the presence of a base such as cesium carbonate, sodium carbonate or potassium phosphate at a temperature from 60°C to 140ºC, using conventional heating or microwave reactor.

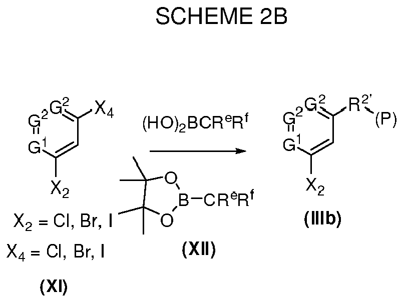

Alternatively, building blocks of general formula (IIIb) where R² is a *N*-containing saturated or unsaturated 5 to 10-membered heterocyclic group or a 5 to 6-membered monocyclic heteroaryl group comprising at least one N atom can be obtained following the synthetic scheme illustrated in Scheme 2C by nucleophilic addition of the enolate of the intermediate (XIII) (wherein R^{e} and R^{f} are as defined above) generated in the presence of a base such as potassium hexamethyldisilazane and in a solvent such as THF at temperatures ranging between -70ºC to 50ºC to provide compounds of general formula (IIIb).

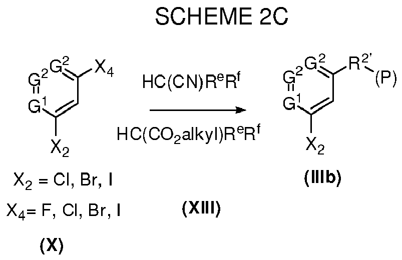

Additionally, building blocks of general formula (IIIb) wherein R² is as *N*-containing saturated or unsaturated 5 to 10-membered heterocyclic group or a 5 to 6-membered monocyclic heteroaryl group comprising at least one N atom can be obtained following the synthetic scheme illustrated in Scheme 2D. Nucleophilic addition of the organomagnesium derivative (XIV) to the ketone (XV) (wherein R^{e} and R^{f} are as defined above) in a solvent such as THF at temperatures ranging between -70ºC to 50ºC to provides compounds of general formula (IIIb).

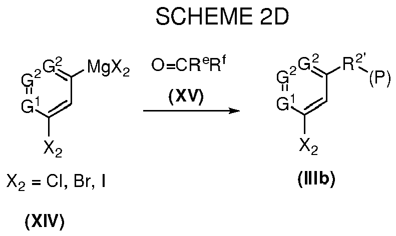

### EXAMPLES

The syntheses of the compounds of the invention are illustrated by the following Examples (1 to 107) including Preparations (1 to 21) which do not limit the scope of the invention in any way.

### General

Reagents, starting materials, and solvents were purchased from commercial suppliers and used as received. Concentration or evaporation refers to evaporation under vacuum using a Büchi rotatory evaporator.

Reaction products were purified, when necessary, by flash chromatography on silica gel (40-63 µm) with the solvent system indicated. Purifications in reverse phase were made in a Biotage SP1® automated purification system equipped with a C₁₈ column and using a gradient of water/acetonitrile/MeOH (1:1) (0.1% v/v ammonium formate both phases) from 0% to 100% acetonitrile/MeOH (1:1) in 40 column volumes. The appropriate fractions were collected and the solvents evaporated under reduced pressure and/or liofilized.

Preparative HPLC-MS were performed on a Waters instrument equipped with a 2767 injector/collector, a 2525 binary gradient pump, a 2996 PDA detector, a 515 pump as a make-up pump and a ZQ4000 Mass spectrometer detector.

The chromatographic separations were obtained using a Waters 2795 system equipped with a Symmetry C₁₈ (2.1 x 50 mm, 3.5 µm) column for methods A , B and C and a Symmetry C₁₈ (2.1 x 100 mm, 3.5 µm) for method D. The mobile phase was formic acid (0.4 mL), ammonia (0.1 mL), methanol (500 mL) and acetonitrile (500 mL) (B) and formic acid (0.5 mL), ammonia (0.125 mL) and water (1000 mL) (A), the gradients are specified in the following table for each method used.

| Method | Run time | 0% B | 0 to 95% B | 95% B |
|---|---|---|---|---|
| A | 5 min | 0.2 min | 3 min | 0.8 min |
| B | 9 min | 0.5 min | 6.5 min | 1 min |
| C | 15 min | 0 min | 10.5 min | 1.5 min |
| D | 30 min | 0 min | 20 min | 4 min |

The flow rate was 0.8 mL/min for method A and 0.4 mL/min for method B, C and D. The injection volume was 5 microliter. A Waters 2996 diode array was used as a UV detector. Chromatograms were processed at 210 nM or 254 nM. Mass spectra of the chromatograms were acquired using positive and negative electrospray ionization in a Micromass ZMD or in a Waters ZQ detectors coupled to the HPLC.

¹H Nuclear Magnetic Resonance Spectra were recorded on a Varian Gemini-2000 spectrometer operating at a frequency of 300 MHz for the ¹H spectra or in a Varian Mercury plus operating at a frequency of 400 MHz for the ¹H spectra. Samples were dissolved in the specified deuterated solvent. Tetramethylsilane was used as reference.

Mass Spectra (m/z) were recorded on a Micromass ZMD or in a Waters ZQ mass spectrometer using ESI ionization.

### Abbreviations:

- DMF: Dimethylformamide
- DMSO: Dimethylsulfoxide
- CDCl₃: Deuterated chloroform
- NMR: Nuclear magnetic resonance
- s: Singlet
- d: Doublet
- dd: Doublet doublet
- td: Triple doublet
- br: Broad
- q: Quarted
- t: Triplet
- m: Multiplet
- LRMS: Low resolution mass spectrometry
- h: hour
- min: minutes

### PREPARATION 1

### tert-Butyl 4-(3-bromophenyl)-3,6-dihydropyridine-1(2H)-carboxylate

An oven dried resealable Schlenk tube was charged with 1-bromo-3-iodobenzene (1.83 g, 6.00 mmol), *tert*-butyl 4-(4,4,5,5-tetramethyl-1,3,2-dioxaborolan-2-yl)-3,6-dihydropyridine-1(2*H*)-carboxylate (2.00 g, 6.00 mmol), sodium carbonate (2M, 7.18 mL, 14.00 mmol), ethanol (1 mL) and toluene (11 mL). The Schlenk tube was subjected to three cycles of evacuation-backfilling with argon, and 1,1'-bis(diphenylphosphino)ferrocene-palladium(II) dichloride dichloromethane complex (0.26 g, 0.30 mmol) was added. After three further cycles of evacuation-backfilling with argon, the Schlenk tube was capped and placed in an oil bath at 90 ºC. After 16 h, the mixture was cooled and filtered. Ethyl acetate and water were added to the mixture and the organic layer was washed with saturated aqueous sodium carbonate solution and brine, dried (Na₂SO₄) and evaporated to give the desired compound (1.49 g, 68%) as a yellow solid.

¹H NMR (300 MHz, , CDCl₃) δ ppm 1.49 (s, 9 H), 2.49 (bs, 2 H), 3.63 (t, *J*=5.77 Hz, 2 H), 4.08 (d, *J*=2.75 Hz, 2 H), 6.05 (bs, 1 H), 7.21 (t, *J*=7.69 Hz, 1 H), 7.27 - 7.31 (m, 1 H), 7.36 - 7.40 (m, 1 H), 7.51 (t, *J*=1.79 Hz, 1 H).

### PREPARATION 2

### tert-Butyl 4-(4-bromopyridin-2-yl)piperazine-1-carboxylate

A suspension of 4-bromo-2-fluoropyridine (1.55 g, 8.81 mmol), *tert-*butyl piperazine-1-carboxylate (2.46 g, 13.22 mmol) and potassium carbonate (3.65 g, 26.41 mmol) in dimethylsulphoxide (10 mL) was heated in a sealed tube at 100 ºC with stirring. After stirring overnight the mixture was cooled and ethyl acetate and water were added. The organic layer was washed with brine, dried (MgSO₄) and evaporated. Purification of the residue by flash chromatography (100% hexanes to 1:4 hexanes /ethyl acetate) gave the title compound (2.33 g, 78%) as a white solid.

LRMS (m/z): 342/344 (M+1)⁺.

¹H NMR (300 MHz, , CDCl₃) δ ppm 1.49 (s, 9 H), 2.49 (bs, 2 H), 3.63 (t, *J*=5.77 Hz, 2 H), 4.08 (d, *J*=2.75 Hz, 2 H), 6.05 (br s, 1 H), 7.21 (t, *J*=7.69 Hz, 1 H), 7.27 - 7.31 (m, 1 H), 7.36 - 7.40 (m, 1 H), 7.51 (t, *J*=1.79 Hz, 1 H).

### PREPARATION 3

### (1S,4S)-tert-Butyl 5-(4-bromopyridin-2-yl)-2,5-diazabicyclo[2.2.1]heptane-2-carboxylate

Obtained (72%) from 4-bromo-2-fluoropyridine and (*1S*,*4S*)-tert-butyl 2,5-diazabicyclo[2.2.1]heptane-2-carboxylate following the experimental procedure as described in preparation 2 followed by purification by flash chromatography.

LRMS (m/z): 354/356 (M+1)⁺.

### PREPARATION 4

### tert-Butyl 4-(4-bromopyridin-2-yl)-4-cyanopiperidine-1-carboxylate

A solution of *tert-*butyl 4-cyanopiperidine-1-carboxylate (1 g, 4.75 mmol) and 4-bromo-2-fluoropyridine (1 g, 5.7 mmol) in anhydrous toluene (18 mL) was cooled at 0°C and potassium hexamethyldisilazane (KHMDS, 13.3 mL, 0.5M in toluene, 6.65 mmol) was added over a period of 30 minutes. The reaction mixture was stirred at room temperature overnight. Then aqueous solution of ammonium chloride (100 mL) was added and the mixture was extracted with dichloromethane (5x20 mL), dried over Na₂SO₄ and concentrated in vacuum. The crude mixture was purified by flash chromatography (dichloromethane-methanol up to 10%) to provide the title compound as a white solid (0.81 g, 47%)

LRMS (m/z): 366/368 (M, M+2)⁺.

### PREPARATION 5

### tert-Butyl 4-(3-chlorophenyl)-4-fluoropiperidine-1-carboxylate

### Step a

### tert-Butyl 4-(3-chlorophenyl)-4-hydroxypiperidine-1-carboxylate

An oven dried resealable Schlenk tube was charged with a solution of *tert*-butyl 4-oxopiperidine-1-carboxylate (1 g, 5.02 mmol) in THF (20mL). The Schlenk tube was subjected to three cycles of evacuation-backfilling with argon and was cooled at 0ºC. Then bromo-(3-chlorophenyl)magnesium 0.5M (15 mL, 7.05 mmol) was added over a period of 30 minutes. The reaction was stirred at room temperature overnight. Then aqueous solution of ammonium chloride (20 mL) was added and the mixture was extrated with ethyl acetate. The organic layer was washed with water and dried over MgSO₄ and concentrated under vaccum. The crude mixture was purified by flash chromatography (hexane-ethyl acetate) to provide the title compound (890 mg, 52%) as a colorless oil.

LRMS (m/z): 312 (M+1)⁺

¹H NMR (400 MHz, CHLOROFORM-D) δ ppm 1.5 (s, 9 H), 1.7 (d, *J*=12.1 Hz, 2 H), 2.0 (m, 2 H), 3.2 (t, *J*=10.9 Hz, 2 H), 4.0 (m, 2 H), 7.3 (m, 3 H), 7.5 (m, *J*=2.0, 2.0 Hz, 1 H).

### Step b

### tert-Butyl 4-(3-chlorophenyl)-4-fluoropiperidine-1-carboxylate

A solution of *tert*-butyl 4-(3-chlorophenyl)-4-hydroxypiperidine-1-carboxylate (890 mg, 2.85 mmol) in anhydrous dichloromethane under argon atmosphere was cooled at -78°C. (Diethylamino)sulfur trifluoride (0.8 mL, 5.7 mmol) was added and the reaction mixture was stirred 2 h at -78ºC. Then water was added and stirred for 1 h. The mixture was extracted with dichloromethane, dried over MgSO₄ and concentrated under vaccum to give the title compound (550 mg, 57%) as a colorless oil.

LRMS (m/z): 314 (M+1)⁺

¹H-NMR (400 MHz, CHLOROFORM-D) δ ppm 1.5 (m, 9 H), 1.9 (m, 4 H), 3.1 (m, 2 H), 4.1 (m, 2 H), 7.3 (m, 3 H), 7.4 (s, 1 H).

### PREPARATION 6

### tert-Butyl-4-(3-bromophenyl)piperazine-1-carboxylate

An oven dried resealable Schlenk tube was charged with 1,3-dibromobenzene (3 g, 12.7 mmol), *tert*-butyl piperazine-1-carboxylate (2.8 g, 15 mmol), cesium carbonate (6 g, 18.4 mmol) and dioxane (50 mL). The Schlenk tube was subjected to three cycles of evacuation-backfilling with argon and tris(dibenzylideneacetone)dipalladium(0) (1.2 g, 1.3 mmol) and 4,5-bis(diphenylphosphino)-9,9-dimethylxanthene (0.75 g, 1.3 mmol) were added. After three further cycles of evacuation-backfilling with argon, the Schlenk tube was capped and placed in an oil bath at 85 ºC. After 16 h, the mixture was cooled and filtered. Ethyl acetate and water were added to the mixture and the organic layer was washed with saturated aqueous sodium carbonate solution and brine, dried (Na₂SO₄) and evaporated. The crude was purified by flash chromatography (1:1 hexanes/ethyl acetate to 100% ethyl acetate) to give the title compound (2.23 g, 50%) as a yellow solid.

LRMS (m/z): 342 (M+1)⁺.

### PREPARATION 7

### tert-Butyl 4-(5-bromopyridin-3-yl)piperazine-1-carboxylate

An oven dried resealable Schlenk tube was charged with 3,5-dibromopyridine (2 g, 8.4 mmol), *tert*-butyl piperazine-1-carboxylate (1.6 g, 8.44 mmol), sodium *tert*-butoxide (0.94 g, 9.8 mmol), tris(dibenzylideneacetone)dipalladium(0) (116 mg, 0.13 mmol) and 4,5-bis(diphenylphosphino)-9,9-dimethylxanthene (244 mg, 0.42 mmol) and toluene (60 mL) were added. After three cycles of evacuation-backfilling with argon, the Schlenk tube was capped and placed in an oil bath at 100 ºC. After 6 h, the mixture was cooled and filtered. Ethyl acetate and water were added to the mixture and the organic layer was washed with saturated aqueous sodium carbonate solution and brine, dried (Na₂SO₄) and evaporated. The crude was purified by reverse phase chromatography (C-18 silica from Waters©, water/methanol as elects [0.1% v/v formic acid buffered] 0% to 100%) to give the title compound (1.98 g, 65%) as a yellow solid.

LRMS (m/z): 343 (M+1)⁺.

### PREPARATION 8

### 6-Methoxy-1H-indazol-3-amine

Hydrazine hydrate (4.7 mL, 97 mmol) was added to a solution of 2-fluoro-4-methoxy benzonitrile (2.9 g, 19.2 mmol) in *n*-butanol (120 mL). The resulting mixture was heated at 110ºC overnight. After cooling the reaction mixture in an ice-water bath, the resulting precipitate was filtered, washed with diethyl ether and dried to provide the title compound (2.88 g, 93%) as a white solid.

LRMS (m/z): 164 (M+1)⁺.

¹H NMR (400 MHz, DMSO-*d*₆) δ ppm 3.76 (s, 3 H), 5.20 (s, 2 H), 6.51 (dd, *J*=8.99, 1.95 Hz, 1 H), 6.62 (s, 1 H), 7.52 (d, *J*=8.60 Hz, 1 H), 11.13 (bs, 1 H).

### PREPARATION 9

### 1-(2-fluoropyridin-4-yl)-N-(pyrazin-2-yl)-1H-indazol-3-amine

### a) 3-Chloro-1-(2-fluoropyridin-4-yl)-1H-indazole

3-Chloro-1*H*-indazole (3.87 g, 25.36 mmol) was added to a stirred suspension of 4-bromo-2-fluoropyridine (2.61 mL, 25.40 mmol), copper iodide (0.48 g, 2.52 mmol), *trans-N,N*'-dimethylcyclohexane-1,2-diamine (0.36 g, 2.53 mmol) and potassium phosphate (11.00 g, 51.82 mmol) in dioxane (120 mL). The mixture was degassed by argon for 5 min and heated at 110 ºC. After 64 h, the reaction mixture was let to cool down and filtered over Celite. Solvent was removed in vaccuo and crude thus obtained was purified by flash chromatography (hexanes/ diethyl ether). 1.55 g of the title compound (25% yield) were obtained.

LRMS (m/z): 248 (M+1)⁺, 246 (M-1)⁻.

¹H NMR (300 MHz, dmso) δ 7.36 (d, *J* = 19.1 Hz, 1H), 7.52 (t, *J* = 7.6 Hz, 1H), 7.65 (s, 1H), 7.72 - 7.79 (m, 1H), 7.87 - 7.93 (m, 1H), 8.25 (d, *J* = 8.7 Hz, 1H), 8.41 (d, *J* = 5.7 Hz, 1H).

### b) 1-(2-Fluoropyridin-4-yl)-N-(pyrazin-2-yl)-1H-indazol-3-amine

An oven dried resealable Schlenk tube was charged with 3-chloro-1-(2-fluoropyridin-4-yl)-1*H*-indazole (700 mg, 2.83 mmol), pyrazine-2-amine (2.50 g, 26.29 mmol), cesium carbonate (2.77g, 8.50 mmol) and dioxane (20 mL). The Schlenk tube was subjected to three cycles of evacuation-backfilling with argon and tris(dibenzylideneacetone) dipalladium(0) (259 mg, 0.28 mmol) and 4,5-bis(diphenylphosphino)-9,9-dimethylxanthene (327 mg, 0.57 mmol) were added. After three further cycles of evacuation-backfilling with argon, the Schlenk tube was capped and placed in an oil bath at 110 ºC. After 16 h, the mixture was then cooled and filtered, and the filtrate was concentrated in vacuum. Purification of the residue by flash chromatography (gradient of hexanes/ethyl acetate) gave the title compound (0.32 g, 37%).

LRMS (m/z): 307 (M+1)⁺, 305 (M-1)⁻.

### PREPARATION 10

### (S)-tert-Butyl 4-(4-bromopyridin-2-yl)-3-methylpiperazine-1-carboxylate

Obtained (67%) from 4-bromo-2-fluoropyridine and (*S*)-*tert*-butyl 3-methylpiperazine-1-carboxylate using the same experimental procedure as described in preparation 2 followed by purification by flash chromatography.

LRMS (m/z): 356 (M+1)⁺.

### PREPARATION 11

### (1S,4S)-tert-Butyl 5-(5-bromopyridin-3-yl)-2,5-diazabicyclo[2.2.1]heptane-2-carboxylate

Obtained (78%) from 3,5-dibromopyridine and (1*S*,4*S*)-*tert*-butyl 2,5-diazabicyclo[2.2.1]heptane-2-carboxylate using the same experimental procedure as described in preparation 7 followed by purification by flash chromatography.

LRMS (m/z): 356 (M+1)⁺.

### PREPARATION 12

### (1S,4S)-tert-Butyl 5-(6-chloropyrimidin-4-yl)-2,5-diazabicyclo[2.2.1]heptane-2-carboxylate

Obtained (91%) from 4,6-dichloropyrimidine and (*1S,4S*)*-tert*-butyl 2,5-diazabicyclo[2.2.1]heptane-2-carboxylate using the same experimental procedure as described in preparation 2.

LRMS (m/z): 312 (M+1)⁺.

### PREPARATION 13

Obtained (98%) from 2-chloro-3-cyanopyridine and hydrazine using the same experimental procedure as described in preparation 8.

LRMS (m/z): 135 (M+1)⁺.

### PREPARATION 14

### tert-Butyl 4-(4-bromopyridin-2-yl)-1,4-diazepane-1-carboxylate

Obtained (78%) from 4-bromo-2-fluoropyridine and *tert*-butyl 1,4-diazepane-1-carboxylate using the same experimental procedure as described in preparation 2.

LRMS (m/z): 356 (M+1)⁺.

### PREPARATION 15

### (S)-tert-Butyl 4-(5-bromopyridin-3-yl)-3-methylpiperazine-1-carboxylate

Obtained (81%) from 3,5-dibromopyridine and (*S*)-*tert*-butyl 3-methylpiperazine-1-carboxylate using the same experimental procedure as described in preparation 7 followed by purification by flash chromatography.

LRMS (m/z): 356 (M+1)⁺.

### PREPARATION 16

### 6-Methyl-1H-indazol-3-amine

Obtained (85%) from 2-fluoro-4-methylbenzonitrile and hydrazine using the same experimental procedure as described in preparation 8.

LRMS (m/z): 147 (M+1)⁺.

### PREPARATION 17

### tert-Butyl 4-(4-bromopyridin-2-yl)-4-(hydroxymethyl)piperidine-1-carboxylate

### Step a

### 1-tert-Butyl 4-methyl 4-(4-bromopyridin-2-yl)piperidine-1,4-dicarboxylate

A solution of 4-bromo-2-fluoropyridine (0.65 mL, 6.3 mmol) and 1-*tert*-butyl 4-ethyl piperidine-1,4-dicarboxylate (1.55 mL, 6.3 mmol) in THF (18 mL) was cooled at -78ºC and potassium hexamethyldisilazane (KHMDS, 18 mL, 0.5M in THF, 9 mmol) was added over a period of 30 minutes. The reaction mixture was stirred at room temperature overnight. Then aqueous solution of ammonium chloride (100 mL) was added and the mixture was extracted with dichloromethane (5x20 mL), dried over Na₂SO₄ and concentrated in vacuo. The crude mixture was purified by flash chromatography (dichloromethane-methanol up to 10%) to provide the title compound as a white solid (1 g, 31%)

LRMS (m/z): 399 (M+1)⁺.

### Step b

### tert-Butyl 4-(4-bromopyridin-2-yl)-4-(hydroxymethyl)piperidine-1-carboxylate

To a solution of 1-*tert*-butyl 4-methyl 4-(4-bromopyridin-2-yl)piperidine-1,4-dicarboxylate (1 g, 2.43 mmol) in THF (10 mL) at 0ºC was slowly added diisobutylaluminum hydride (9.72 mL, 1M solution in THF, 9.72 mmol) and the mixture was stirred at room temperature for 2 hours. Then water (4 mL) and NaOH 2N (1 mL) were added, the suspension was filtered, washed with methanol, the filtrates were extracted with ethyl acetate (3x20 mL), the organic extracts were combined dried (Na₂SO₄) and concentrated in vacuo. Purification was achieved using flash silica chromatography on Biotage equipment, eluting with an ethyl acetate/hexanes gradient. Concentration of the relevant fractions afforded the title compound (341 mg, 31%)

LRMS (m/z): 359 (M+1)⁺.

### PREPARATION 18

### 6-(Benzyloxy)-1H-indazol-3-amine

### Step a.

### 4-(Benzyloxy)-2-fluorobenzonitrile

To a solution of 2-fluoro-4-hydroxybenzonitrile (2 g, 14.6 mmol) in DMF (15 mL) was added potassium carbonate (3 g, 21.7 mmol) and (bromomethyl)benzene (2.7 g, 15.8 mmol). The mixture was heated at 75ºC for 2 hours, poured into water (100 mL), extracted with AcOEt, dried (Na₂SO₄) and concentrated in vacuo. The resulting solid was filtered and washed with diethyl ether to give the title compound as a white solid (3.7 g, 89%).

LRMS (m/z): 228 (M+1)⁺.

### Step b.

### 6-(Benzyloxy)-1H-indazol-3-amine

Obtained (99%) from 4-(benzyloxy)-2-fluorobenzonitrile and hydrazine using the same experimental procedure as described in preparation 8.

LRMS (m/z): 240 (M+1)⁺.

### PREPARATION 19

### tert-Butyl-(1S,4S)-5-(3-bromophenyl)-2,5-diazabicyclo[2.2.1]heptane-2-carboxylate

Obtained (45%) from 1,3-dibromobenzene and *tert*-butyl (1*S*,4*S*)-2,5-diazabicyclo [2.2.1]heptane-2-carboxylate following the procedure as described in preparation 6 to give the desired compound.

LRMS (m/z): 353-355 (M+1)⁺.

### PREPARATION 20

### tert-Butyl 4-(6-chloro-2-methylpyrimidin-4-yl)piperazine-1-carboxylate

Ethyldiisopropylamine (0.641 ml, 3.68 mmol) was added to a stirred solution of 4,6-dichloro-2-methylpyrimidine (500 mg, 3.07 mmol) and tert-butyl piperazine-1-carboxylate (514 mg, 2076 mmol) in THF (15 ml). The mixture was stirred at rt for 3 days and then solvent was removed. The reaction crude was partitioned between dichloromethane and NaHCO₃ 4% aquous solution. The organic layer was then dried and solvent removed to yield a crude product that was purified by column cromathography using hexane/ethylacetate to give the title compound (715 mg, 83% yield).

LRMS (m/z): 313 (M+1)⁺.

### PREPARATION 21

### tert-Butyl (1S,4S)-5-(6-chloro-2-methylpyrimidin-4-yl)-2,5-diazabicyclo[2.2.1] heptane-2-carboxylate

Obtained (97% yield) from 4,6-dichloro-2-methylpyrimidine and *tert-butyl* (1 S,4S)-2,5-diazabicyclo[2.2.1]heptane-2-carboxylate following the experimental procedure of Preparation 20.

LRMS (m/z): 325 (M+1)⁺.

### EXAMPLE 1

### N-Pyrazin-2-yl-1-[3-(1,2,3,6-tetrahydropyridin-4-yl)phenyl]-1H-indazol-3-amine

### a) tert-Butyl 4-[3-(3-chloro-1H-indazol-1-yl)phenyl]-3,6-dihydropyridine-1(2H)-carboxylate

3-Chloro-1*H*-indazole (0.15 g, 0.98 mmol) was added to a stirred suspension of *tert-*butyl 4-(3-bromophenyl)-3,6-dihydropyridine-1(2*H*)-carboxylate (preparation 1, 0.40 g, 1.18 mmol), copper iodide (0.01 g, 0.05 mmol), trans-*N,N'*-dimethylcyclohexane-1,2-diamine (0.03 mL, 0.20 mmol) and potassium phosphate (0.44 g, 2.06 mmol) in toluene (5 mL). The mixture was degassed by argon for 5 minutes and heated at 110 ºC. After 16 hours, ethyl acetate and water were added to the reaction mixture and the organic layer was washed with brine, dried (MgSO₄) and evaporated. The crude was purified by reverse phase chromatography (C-18 silica from Waters©, water/methanol as elects [0.1% v/v formic acid buffered] 0% to 100%) to provide the title compound (0.36 g, 75%).

LRMS (m/z): 410 (M+1)⁺.

### b) tert-Butyl 4-{3-[3-(pyrazin-2-ylamino)-1H-indazol-1-yl]phenyl}-3,6-dihydropyridine-1 (2H)-carboxylate

A mixture of *tert*-butyl 4-[3-(3-chloro-1*H*-indazol-1-yl)phenyl]-3,6-dihydropyridine-1(2*H*)-carboxylate (example 1 a, 0.10 g, 0.24 mmol), pyrazin-2-amine (0.12 g, 1.22 mmol), sodium tert-butoxide (0.04 g, 0.37 mmol), palladium(II) acetate (0.01 g, catalytic amount) and 4,5-bis(diphenylphosphino)-9,9-dimethylxanthene (0.01 g, catalytic amount) in toluene (4 mL) was heated at 120 °C for 20 minutes in Biotage Initiator Microwave Synthesizer. The mixture was then cooled and filtered, and the filtrate was concentrated in vacuum. Purification of the residue by flash chromatography (3:2 hexanes/ethyl acetate) gave the title compound (0.04 g, 32%).

LRMS (m/z): 469 (M+1)⁺.

¹H NMR (400 MHz, , CDCl₃) δ ppm 1.50 (s, 9 H), 2.59 (brs, 2 H), 3.68 (t, *J*=5.20 Hz, 2 H), 4.12 (bs, 2 H), 6.14 (bs, 1 H), 7.21 - 7.32 (m, 2 H), 7.48 (t, *J*=8.00 Hz, 2 H), 7.57 (bs, 1 H), 7.16 (dd, *J*=7.60 Hz, 1 H), 7.72 - 7.76 (m, 3 H), 8.18 (d, *J*=3.20 Hz, 2 H), 9.52 (s, 1 H).

### c) N-Pyrazin-2-yl-1-[3-(1,2,3,6-tetrahydropyridin-4-yl)phenyl]-1H-indazol-3-amine

2,2,2-Trifluoroacetic acid (1.5 mL) was added to a stirred solution of *tert*-butyl 4-[6-(pyrazin-2-ylamino)-4-({[3-(trifluoromethoxy)phenyl]sulfonyl}amino)-2,4'-bipyridin-2'-yl]piperazine-1-carboxylate (example 1b, 0.04 g, 0.09 mmol) in dichloromethane (1.5 mL) and the mixture was stirred at room temperature. After 1 hour, saturated aqueous sodium carbonate solution was added and the mixture was extracted with more dichloromethane. The organic layer was dried (MgSO₄) and evaporated. Purification of the residue by reverse phase chromatography (C-18 silica from Waters@, water/methanol as elects [0.1% v/v formic acid buffered] 0% to 100%) gave the title compound (0.01 g, 69%) as a monoformate salt.

LRMS (m/z): 369 (M+1)⁺.

¹H NMR (400 MHz, METHANOL-*d*₄) δ ppm 2.90 (bs, 2 H), 3.50 (t, *J*=6.06 Hz, 2 H), 3.89 (d, *J*=2.34 Hz, 2 H), 6.30 (bs, 1 H), 7.25 (t, *J*=7.33 Hz, 1 H), 7.45 (d, *J*=7.82 Hz, 1 H), 7.53 (ddd, *J*=8.45, 7.18, 0.98 Hz, 1 H), 7.58 (t, *J*=8.01 Hz, 1 H), 7.75 (dd, *J*=7.82, 1.17 Hz, 1 H), 7.80 (d, *J*=8.60 Hz, 1 H), 7.87 (t, *J*=1.76 Hz, 1 H), 8.01 (d, *J*=8.21 Hz, 1 H), 8.07 (d, *J*=2.54 Hz, 1 H), 8.26 (dd, *J*=2.54, 1.37 Hz, 1 H) 8.49 (bs, 1 H), 9.33 (d, *J*=1.37 Hz, 1 H).

### EXAMPLE 2

### 1-[3-(4-Fluoropiperidin-4-yl)phenyl]-N-pyrazin-2-yl-1H-indazol-3-amine

### a) tert-Butyl 4-[3-(3-amino-1H-indazol-1-yl)phenyl]-4-fluoropiperidine-1-carboxylate

1*H*-Indazol-3-amine (0.1 g, 0.75 mmol) was added to a stirred suspension of *tert*-butyl 4-(3-chlorophenyl)-4-fluoropiperidine-1-carboxylate (preparation 5, 0.17 g, 0.53 mmol), copper iodide (72 mg, 0.38 mmol), (1*S*,2*S*)-*N,N'*-dimethylcyclohexane-1,2-diamine (100 mg, 0.70 mmol) and potassium carbonate (218 mg, 1.58 mmol) in *N*-methylpyrrolidone (10 mL). The mixture was degassed by argon for 5 minutes and heated at 120 ºC. After 16 hours, ethyl acetate and water were added to the reaction mixture and the organic layer was washed with brine, dried (MgSO₄) and evaporated. Purification was achieved as described in the general methods. Concentration of the relevant fractions afforded the title compound (20 mg, 13%)

LRMS (m/z): 411 (M+1)⁺.

### b) tert-Butyl 4-fluoro-4-{3-[3-(pyrazin-2-ylamino)-1H-indazol-1-yl]phenyl}piperidine-1-carboxylate

An oven dried resealable Schlenk tube was charged with *tert*-butyl 4-[3-(3-amino-1*H-*indazol-1-yl)phenyl]-4-fluoropiperidine-1-carboxylate (20 mg, 0.05 mmol), 2-chloropyrazine (6 mg, 0.05 mmol), cesium carbonate (23 mg, 0.07 mmol) and toluene (2 mL). The Schlenk tube was subjected to three cycles of evacuation-backfilling with argon and tris(dibenzylideneacetone)dipalladium(0) (3 mg, 0.07 mmol) and 2,2'-bis(diphenylphosphino)-1,1'-binaphthyl (BINAP, 2 mg, 0.07 mmol) were added. After three further cycles of evacuation-backfilling with argon, the Schlenk tube was capped and placed in an oil bath at 100 ºC. After 16h, the mixture was cooled and the solvent was removed under vacuum. The crude was purified as described in the general methods to give the title compound (15 mg, 57%) as a yellow solid.

LRMS (m/z): 489 (M+1)⁺.

### c) 1-[3-(4-Fluoropiperidin-4-yl)phenyl]-N-pyrazin-2-yl-1H-indazol-3-amine

*tert*-Butyl 4-fluoro-4-{3-[3-(pyrazin-2-ylamino)-1*H*-indazol-1-yl]phenyl}piperidine-1-carboxylate (15 mg, 0.03 mmol) was dissolved in a dioxane solution of hydrochloric acid (0.12 mL, 4M solution, 0.48 mmol) and the mixture was stirred at room temperature for 4 h. The solvent was evaporated under reduced pressure and the crude was purified by reverse phase chromatography (C-18 silica from Waters@, water/methanol as eluents [0.1 % v/v formic acid buffered] 0% to 100%) to give the title compound (3 mg, 23%) as a yellow solid.

LRMS (m/z): 389 (M+1)⁺.

### EXAMPLE 3

### 1-(3-Piperazin-1-ylphenyl)-N-pyrazin-2-yl-1H-indazol-3-amine

### a) tert-Butyl 4-[3-(3-chloro-1H-indazol-1-yl)phenyl]piperazinecarboxylate

Obtained (92% yield) from 3-chloro-1*H*-indazole and the title compound of Preparation 6 following the experimental procedure described in Example 1a. The crude material was purified by normal phase chromatography using mixtures of hexanes/ethyl acetate as mobile phase.

LRMS (m/z): 421 (M+1)⁺.

### b) tert-Butyl 4-{3-[3-(pyrazin-2-ylamino)-1H-indazol-1-yl]phenyl}-piperazinencarboxylate

A mixture of the title compound of Example 3a (0.20 g, 0.48 mmol), pyrazin-2-amine (0.46 g, 4.84 mmol), cesium carbonate (0.47 g, 1.44mmol), Pd2(dba)3 (44 mg, 0.05 mmol) and 4,5-bis(diphenylphosphino)-9,9-dimethylxanthene (55 mg, 0.10 mmol) in dioxane (8 mL) was heated at 120 °C overnight under nitrogen. Then, Pd₂(dba)₃ (44 mg, 0.05 mmol) and 4,5-bis(diphenylphosphino)-9,9-dimethylxanthene (55 mg, 0.10 mmol) were further added and the mixture was heated at 120ºC overnight, again. The mixture was then cooled and filtered, and the filtrate was concentrated in vacuum. Purification of the residue by flash chromatography (gradient of hexanes/ethyl acetate) gave the title compound (0.14 g, 61%).

LRMS (m/z): 472 (M+1)⁺.

### c) 1-(3-Piperazin-1-ylphenyl)-N-pyrazin-2-yl-1H-indazol-3-amine

To a stirred solution of the title compound of Example 3b (140 mg, 0.3 mmol) dioxane it was added a 4M solution of HCl in dioxane (1 mL) and the mixture was sitirred at rt for 3 days. Solvent was removed *in vaccuo* and the solid thus obtained was suspended in acetonitrile, washed with ethyl ether and dried to yield the title compound (80 mg, 96% yeild) as chlorohydrate.

LRMS (m/z): 372 (M+1)⁺.

¹H NMR (400 MHz, DMSO) δ 10.38 (s, 1H), 9.37 (d, *J* = 1.5 Hz, 1H), 9.18 (s, 2H), 8.30 (dd, *J* = 2.6, 1.5 Hz, 1H), 8.21 (d, *J* = 8.1 Hz, 1H), 8.15 (d, *J* = 2.6 Hz, 1H), 7.81 (d, *J* = 8.6 Hz, 1H), 7.58 - 7.49 (m, 1H), 7.46 (t, *J* = 8.1 Hz, 1H), 7.32 (t, *J* = 2.1 Hz, 1H), 7.29 - 7.19 (m, 2H), 7.00 (dd, *J* = 8.4, 1.9 Hz, 1H), 5.39 (s, 1H), 3.60-3.42 (m, 4H), 3.26 (s, 4H).

### EXAMPLE 4

### 1-(5-Piperazin-1-ylpyridin-3-yl)-N-pyrazin-2-yl-1H-indazol-3-amine

### a) tert-Butyl 4-[5-(3-chloro-1H-indazol-1-yl)pyridin-3-yl]piperazinecarboxylate

Obtained (90% yield) from 3-chloro-1*H*-indazole and the title compound of Preparation 7 following the experimental procedure described in Example 1a. The crude material was purified by normal phase chromatography using mixtures of hexane/ethyl acetate as mobil phase.

LRMS (m/z): 413 (M+1)⁺.

¹H NMR (400 MHz, CDCl₃) δ 8.51 (s, 1H), 8.31 (s, 1H), 7.76 (m, 2H), 7.56 - 7.48 (m, 2H), 7.36 - 7.29 (m, 1H), 3.66 - 3.60 (m, 4H), 3.33 - 3.25 (m, 4H), 1.49 (s, 9H).

### b) tert-Butyl 4-{3-[3-(pyrazin-2-ylamino)-1H-indazol-1-yl]phenyl}-piperidincarboxylate

Obtained (75% yield) from the title compound of Example 4a and pyrazine-2amine following the experimental procedure described in Example 3b. The crude material was purified by normal phase chromatography using dichloromethane:methanol (95:5) as mobile phase.

LRMS (m/z): 473 (M+1)⁺.

### c) N-Pyrazin-2-yl-1-[3-(piperidin-4-yl)phenyl]-1H-indazol-3-amine

Obtained (88% yield) from the title compound of Example 4b following the experimental procedure described in Example 3c.

LRMS (m/z): 373 (M+1)⁺.

1H NMR (400 MHz, dmso) δ 10.59 (s, 1H), 9.49 (d, J = 17.5 Hz, 3H), 8.67 (s, 1H), 8.43 (s, 1H), 8.33 (d, J = 8.7 Hz, 2H), 8.23 (s, 1H), 8.13 (s, 1H), 7.99 (d, J = 8.5 Hz, 1H), 7.63 (t, J = 7.7 Hz, 1H), 7.35 (t, J = 7.7 Hz, 1H), 3.76 (s, 4H), 3.28 (s, 4H).

### EXAMPLE 5

### N-(4-Methylpyridin-2-yl)-1-(2-piperazin-1-ylpyridin-4-yl)-1H-indazol-3-amine

### a) tert-Butyl 4-[4-(3-chloro-1H-indazol-1-yl)pyridin-2-yl]piperazine-1-carboxylate

Obtained (89%) from 3-chloro-1*H*-indazole and *tert-*butyl 4-(4-bromopyridin-2-yl)piperazine-1-carboxylate (preparation 2) following the experimental procedure as described in example 1a, followed by purification by flash chromatography (9:1 hexanes/ethyl acetate to 1:4 hexanes/ethyl acetate).

LRMS (m/z): 414 (M+1)⁺.

¹H NMR (400 MHz, CHLOROFORM-*d*) δ ppm 1.50 (s, 9 H), 3.59 (d, *J*=5.60 Hz, 4 H), 3.65 (d, *J*=5.20 Hz, 4 H), 7.03 (s, 1 H), 7.07 (d, *J*=4.80 Hz, 1 H), 7.35 (t, *J*=7.60 Hz, 1 H), 7.56 (t, *J*=7.60 Hz, 1 H), 7.78 (d, *J*=8.00 Hz, 1 H), 7.84 (d, *J*=8.40 Hz, 1 H), 8.30 (d, *J*=5.60 Hz, 1 H).

### b) tert-Butyl 4-(4-{3-[(4-methylpyridin-2-yl)amino]-1H-indazol-1-yl}pyridin-2-yl)piperazine-1-carboxylate

A mixture of *tert*-butyl 4-[4-(3-chloro-1*H*-indazol-1-yl)pyridin-2-yl]piperazine-1-carboxylate (example 5a, 0.05 g, 0.12 mmol), 4-methylpyridin-2-amine (0.07 g, 0.60 mmol), cesium carbonate (0.06 g, 0.17 mmol), tris(dibenzylideneacetone)dipalladium(0) (0.01 g, catalytic amount) and 4,5-bis(diphenylphosphino)-9,9-dimethylxanthene (0.01 g, catalytic amount) in dioxane (2 mL) was heated at 120 °C for 90 minutes in Biotage Initiator Microwave Synthesizer. The mixture was then cooled and the solvent was removed in vacuum. The residue was purified by flash chromatography (95:5 hexanes/ethyl acetate to 100% ethyl acetate) to give the title compound (0.02 g, 36%).

LRMS (m/z): 486 (M+1)⁺.

¹H NMR (400 MHz, CHLOROFORM-*d*) δ ppm 1.50 (s, 9 H), 2.41 (s, 3 H), 3.60 - 3.66 (m, 8 H), 6.77 (d, *J*=4.80 Hz, 1 H), 7.11 (d, *J*=1.60 Hz, 1 H), 7.14 (dd, *J*=5.20, 1.60 Hz, 1 H), 7.24 - 7.27 (m, 1 H), 7.53 (t, *J*=7.20 Hz, 1 H), 7.67 (br. s, 1 H), 7.74 (t, *J*=8.00 Hz, 1 H), 7.86 (d, *J*=8.40 Hz, 1 H), 8.05 (s, 1 H), 8.13 (d, *J*=5.20 Hz, 1 H), 8.28 (d, *J*=5.20 Hz, 1 H).

### c) N-(4-Methylpyridin-2-yl)-1-(2-piperazin-1-ylpyridin-4-yl)-1H-indazol-3-amine

Obtained as a solid (94%) from *tert*-butyl 4-(4-{3-[(4-methylpyridin-2-yl)amino]-1*H-*indazol-1-yl}pyridin-2-yl)piperazine-1-carboxylate (example 5b) following the experimental procedure as described in example 1 c.

LRMS (m/z): 386 (M+1)⁺.

¹H NMR (400 MHz, CHLOROFORM-*d*) δ ppm 2.41 (s, 3 H), 3.09 (t, *J*=4.88 Hz, 4 H), 3.68 (t, *J*=4.69 Hz, 4 H), 6.76 (d, *J*=4.69 Hz, 1 H), 7.09 - 7.16 (m, 2 H), 7.23 - 7.30 (m, 1 H), 7.53 (ddd, *J*=8.35, 7.18, 0.88 Hz, 1 H), 7.74 (d, *J*=8.01 Hz, 1 H), 7.86 (d, *J*=8.60 Hz, 1 H), 8.05 (s, 1 H), 8.14 (d, *J*=5.08 Hz, 1 H), 8.29 (d, *J*=5.67 Hz, 1 H)

### EXAMPLE 6

### 1-(2-Piperazin-1-ylpyridin-4-yl)-N-pyrazin-2-yl-1H-indazol-3-amine

### a) tert-Butyl 4-{4-[3-(pyrazin-2-ylamino)-1H-indazol-1-yl]pyridin-2-yl}piperazine-1-carboxylate

Obtained (36%) from *tert*-butyl 4-[4-(3-chloro-1*H*-indazol-1-yl)pyridin-2-yl]piperazine-1-carboxylate (example 5a) and pyrazin-2-amine following the experimental procedure as described in example 5b, followed by purification by flash chromatography (1:1 hexanes/ethyl acetate).

LRMS (m/z): 473 (M+1)⁺.

¹H NMR (400 MHz, CHLOROFORM-*d*) δ ppm 1.50 (s, 9 H), 3.63 (br. s, 4 H), 3.65 (br. s, 4 H), 7.08 (br. s, 1 H), 7.16 (d, *J*=4.40 Hz, 1 H), 7.31 - 7.34 (m, 2 H), 7.57 (t, *J*=7.60 Hz, 1 H), 7.75 (d, *J*=8.00 Hz, 1 H), 7.88 (d, *J*=8.80 Hz, 1 H), 8.24 (s, 2 H), 8.29 (d, *J*=5.60 Hz, 1 H), 9.57 (s, 1 H).

### b) 1-(2-Piperazin-1-ylpyridin-4-yl)-N-pyrazin-2-yl-1H-indazol-3-amine

Obtained as a solid (68%) from *tert*-butyl 4-{4-[3-(pyrazin-2-ylamino)-1*H*-indazol-1-yl]pyridin-2-yl}piperazine-1-carboxylate (example 6a) following the experimental procedure as described in example 1 c.

LRMS (m/z): 373 (M+1)⁺.

¹H NMR (400 MHz, CHLOROFORM-*d*) δ ppm 3.07 (d, *J*=5.08 Hz, 4 H), 3.65 (d, *J*=5.08 Hz, 4 H), 7.06 (d, *J*=1.76 Hz, 1 H), 7.12 (dd, *J*=5.47, 1.76 Hz, 1 H), 7.29 (ddd, *J*=7.96, 7.08, 0.78 Hz, 1 H), 7.36 (bs, 1 H), 7.55 (ddd, *J=*8.45, 7.18, 0.98 Hz, 1 H), 7.74 (d, *J*=8.01 Hz, 1 H), 7.88 (d, *J*=8.60 Hz, 1 H), 8.22 (s, 2 H), 8.29 (d, *J*=5.47 Hz, 1 H), 9.57 (s, 1 H).

### EXAMPLE 7

### 6-Methoxy-1-(2-piperazin-1-ylpyridin-4-yl)-N-pyrazin-2-yl-1H-indazol-3-amine

### a) tert-Butyl 4-(4-(3-amino-6-methoxy-1H-indazol-1-yl)pyridin-2-yl)piperazine-1-carboxylate

Obtained (40%) from 6-Methoxy-1*H*-indazol-3-amine (preparation 8) and *tert*-butyl 4-(4-bromopyridin-2-yl)piperazine-1-carboxylate (preparation 2) using the same experimental procedure as described in example 2a, followed by purification by reverse phase chromatography as described in the general methods.

LRMS (m/z): 425 (M+1)⁺.

¹H NMR (400 MHz, DMSO-*d*6) δ ppm 1.43 (s, 9 H), 3.43 - 3.49 (m, 4 H), 3.49 - 3.59 (m, 4 H), 3.88 (s, 3 H), 6.05 (s, 2 H), 6.80 (dd, *J*=8.60, 1.95 Hz, 1 H), 6.98 (d, *J*=1.56 Hz, 1 H), 7.07 (dd, *J*=5.67, 1.76 Hz, 1 H), 7.23 (d, *J*=1.95 Hz, 1 H), 7.74 (d, *J*=8.60 Hz, 1 H), 8.13 (d, *J*=5.86 Hz, 1 H).

### b) tert-Butyl 4-(4-(6-methoxy-3-(pyrazin-2-ylamino)-1H-indazol-1-yl)pyridin-2-yl)piperazine-1-carboxylate

Obtained as a colorless oil (36%) from *tert*-butyl 4-(4-(3-amino-6-methoxy-1*H*-indazol-1-yl)pyridin-2-yl)piperazine-1-carboxylate (example 7a) and 2-chloropyrazine using the same experimental procedure as described in example 2b, followed by purification by reverse phase chromatography in the general methods.

LRMS (m/z): 503 (M+1)⁺.

### c) 6-Methoxy-1-(2-piperazin-1-ylpyridin-4-yl)-N-pyrazin-2-yl-1H-indazol-3-amine

Obtained as a hydrochloride salt (68%) from *tert*-Butyl 4-(4-(6-methoxy-3-(pyrazin-2-ylamino)-1*H*-indazol-1-yl)pyridin-2-yl)piperazine-1-carboxylate (example 7b) using the same experimental procedure as described in example 2c. The solid was filtered off and washed several times with diethyl ether and dried in the vaccum oven overnight.

LRMS (m/z): 403 (M+1)⁺.

¹H NMR (400 MHz, DMSO-*d*₆) δ ppm 3.31 (s, 4 H), 3.95 (s, 3 H), 4.06 (s, 4 H), 7.06 (dd, *J*=8.79, 1.76 Hz, 1 H), 7.43 (s, 1 H), 7.50 (d, *J*=1.17 Hz, 1 H), 7.58 (d, *J*=5.86 Hz, 1 H), 8.16 (d, *J*=7.03 Hz, 1 H), 8.26 - 8.32 (m, 2 H), 8.37 - 8.41 (m, 1 H), 9.49 (s, 1 H),9.68(s,2H), 10.72 (s, 1 H).

### EXAMPLE 8

### 1-[2-(1,4-Diazepan-1-yl)pyridin-4-yl]-N-pyrazin-2-yl-1H-indazol-3-amine

Obtained (19%) from 1,4-diazepane and 1-(2-fluoropyridin-4-yl)-*N*-(pyrazin-2-yl)-1*H-*indazol-3-amine (preparation 9) following the experimental procedure as described in preparation 2, followed by purification by reverse phase chromatography as described in the general methods.

LRMS (m/z): 387 (M+1)⁺.

¹*H* NMR (400 MHz, DMSO-*d*₆) δ ppm 1.89 - 1.98 (m, 2 *H*), 2.83 - 2.90 (m, 2 *H*), 3.01 - 3.09 (m, 2 *H*), 3.73 - 3.79 (m, 2 *H*), 3.79 - 3.86 (m, 2 *H*), 6.94 (s, 1 *H*), 7.08 (d, *J*=4.30 *H*z, 1 *H*), 7.30 (t, *J*=7.62 *H*z, 1 *H*), 7.59 (t, *J*=7.82 *H*z, 1 *H*), 8.00 (d, *J*=8.60 *H*z, 1 *H*), 8.20 (m, 2 *H*), 8.25 - 8.37 (m, 2 *H*), 9.45 (s, 1 *H*), 10.50 (s, 1 *H*).

### EXAMPLE 9

### 1-(2-((1S,4S)-2,5-diazabicyclo[2.2.1]heptan-2-yl)pyridin-4-yl)-N-(4-methylpyridin-2-yl)-1H-indazol-3-amine

### a) (1S,4S)-tert-butyl 5-(4-(3-chloro-1H-indazol-1-yl)pyridin-2-yl)-2,5-diazabicyclo[2.2.1]heptane-2-carboxylate

Obtained (82%) from 3-chloro-1*H*-indazole and (1*S*,4*S*)-*tert*-butyl 5-(4-bromopyridin-2-yl)-2,5-diazabicyclo[2.2.1]heptane-2-carboxylate (preparation 3) following the experimental procedure as described in example 1 a, followed by purification by flash chromatography (9:1 hexanes/ethyl acetate to 100% ethyl acetate).

LRMS (m/z): 426 (M+1)⁺.

### b) (1S,4S)-tert-Butyl 5-(4-{3-[(4-methylpyridin-2-yl)amino]-1H-indazol-1-yl}pyridin-2-yl)-2,5-diazabicyclo[2.2.1]heptane-2-carboxylate

Obtained (25%) from (1*S*,4*S*)-*tert*-butyl 5-[4-(3-chloro-1*H*-indazol-1-yl)pyridin-2-yl]-2,5-diazabicyclo[2.2.1]heptane-2-carboxylate (example 9a) and 4-methylpyridin-2-amine following the experimental procedure as described in example 5b, followed by purification by flash chromatography (9:1 hexanes/ethyl acetate to 100% ethyl acetate).

LRMS (m/z): 498 (M+1)⁺.

### c) 1-(2-((1S,4S)-2,5-diazabicyclo[2.2.1]heptan-2-yl)pyridin-4-yl)-N-(4-methylpyridin-2-yl)-1H-indazol-3-amine

Obtained as a solid (80%) from (1*S*,4*S*)-*tert*-butyl 5-(4-{3-[(4-methylpyridin-2-yl)amino]-1*H*-indazol-1-yl}pyridin-2-yl)-2,5-diazabicyclo[2.2.1]heptane-2-carboxylate (example 9b) following the experimental procedure as described in example 1c.

LRMS (m/z): 398 (M+1)⁺.

¹H NMR (400 MHz, CHLOROFORM-*d*) δ ppm 1.86 - 2.04 (m, 3 H), 2.41 (s, 3 H), 3.15 - 3.27 (m, 2 H), 3.39 (d, *J*=9.38 Hz, 1 H), 3.71 (dd, *J*=9.28, 1.86 Hz, 1 H), 3.95 (s, 1 H), 4.87 (bs, 1 H), 6.72 - 6.80 (m, 2 H), 7.06 (dd, *J*=5.67, 1.95 Hz, 1 H), 7.23 - 7.29 (m, 1 H), 7.51 (ddd, *J*=8.45, 7.18, 0.98 Hz, 1 H), 7.74 (d, *J*=8.01 Hz, 1 H), 7.87 (d, *J*=8.60 Hz, 1 H), 8.05 (s, 1 H), 8.13 (d, *J*=5.08 Hz, 1 H), 8.23 (d, *J*=5.67 Hz, 1 H).

### EXAMPLE 10

### 1-{2-[(1S,4S)-2,5-Diazabicyclo[2.2.1]hept-2-yl]pyridi n-4-yl}-N-pyrazin-2-yl-1H-indazol-3-amine

### a) (1S,4S)-tert-Butyl 5-{4-[3-(pyrazin-2-ylamino)-1H-indazol-1-yl]pyridin-2-yl}-2,5-diazabicyclo[2.2.1]heptane-2-carboxylate

Obtained (25%) from (1*S*,4*S*)-*tert*-butyl 5-[4-(3-chloro-1*H*-indazol-1-yl)pyridin-2-yl]-2,5-diazabicyclo[2.2.1]heptane-2-carboxylate (example 9a) and pyrazin-2-amine following the experimental procedure as described in example 5b, followed by purification by flash chromatography (9:1 hexanes/ethyl acetate to 100% ethyl acetate).

LRMS (m/z): 485 (M+1)⁺.

### b) 1-{2-[(1S,4S)-2,5-Diazabicyclo[2.2.1]hept-2-yl]pyridin-4-yl}-N-pyrazin-2-yl-1H-indazol-3-amine

Obtained as a solid (65%) from (1*S*,4*S*)-*tert*-butyl 5-{4-[3-(pyrazin-2-yiamino)-1*H-*indazol-1-yl]pyridin-2-yl}-2,5-diazabicyclo[2.2.1]heptane-2-carboxylate (example 10a) following the experimental procedure as described in example 1c.

LRMS (m/z): 385 (M+1)⁺.

¹H NMR (400 MHz, CHLOROFORM-*d*) δ ppm 1.84 - 2.00 (m, 2 H), 3.15 (s, 2 H), 3.32 (d, *J*=9.57 Hz, 1 H), 3.70 (dd, *J*=9.38, 1.95 Hz, 1 H), 3.88 (s, 1 H), 4.85 (bs, 1 H), 6.74 (d, *J*=1.56 Hz, 1 H), 7.03 (dd, *J*=5.67, 1.76 Hz, 1 H), 7.24 - 7.31 (m, 2 H), 7.50 (bs, 1 H), 7.54 (ddd, *J*=8.45, 7.18, 0.98 Hz, 1 H), 7.73 (d, *J*=8.21 Hz, 1 H), 7.88 (d, *J*=8.60 Hz, 1 H), 8.16 - 8.29 (m, 3 H), 9.57 (s, 1 H).

### EXAMPLE 11

### 1-{2-[(1S,4S)-2,5-Diazabicyclo[2.2.1]hept-2-yl]pyridin-4-yl}-3-(pyrazin-2-ylamino)-1H-indazole-5-carbonitrile

### a) 3-Bromo-1H-indazole-5-carbonitrile

1*H*-Indazole-5-carbonitrile (90 mg, 0.62 mmol) was dissolved in dry DMF (1 mL) and *N-*bromosuccinimide (134 mg, 0.75 mmol) was added at room temperature and stirred at this temperature for 2 hours. After removal of the solvent under vacuum, the residue was treated with ethyl acetate, washed successively with saturated aqueous solution of sodium hydrogen carbonate, water and brine. The organic extracts were dried over anhydrous sodium sulphate and the solvent was removed under vacuum to afford the title compound (128 mg, 93%).

LRMS (m/z): 222/224 (Br).

### b) tert-Butyl (1S,4S)-5-[4-(3-bromo-5-cyano-1H-indazol-1-yl)pyridin-2-yl]-2,5-diazabicyclo[2.2.1]heptane-2-carboxylate

Obtained (55%) from 3-bromo-1*H*-indazole-5-carbonitrile and (1*S*,4*S*)-*tert*-butyl 5-(4-bromopyridin-2-yl)-2,5-diazabicyclo[2.2.1]heptane-2-carboxylate (preparation 3) following the experimental procedure as described in example 1a, followed by purification by flash chromatography (9:1 hexanes/ethyl acetate to 100% ethyl acetate).

LRMS (m/z): 495/497 (Br).

### c) tert-Butyl (1S,4S)-5-{4-[5-cyano-3-(pyrazin-2-ylamino)-1H-indazol-1-yl]pyridin-2-yl}-2,5-diazabicyclo[2.2.1]heptane-2-carboxylate

Obtained (61%) from *tert*-butyl (1*S*,4*S*)-5-[4-(3-bromo-5-cyano-1*H*-indazol-1-yl)pyridin-2-yl]-2,5-diazabicyclo[2.2.1]heptane-2-carboxylate (example 11b) and 2-aminopyrazine following a similar experimental procedure as described in example 5b but heating at 110ºC for 16 h instead of using microwaves. Purification by flash chromatography (95:5 dichloromethane-methanol) provided the title compound (62 mg, 61 %) as a white solid.

LRMS (m/z): 510 (M+1)⁺.

### d) 1-{2-[(1S,4S)-2,5-Diazabicyclo[2.2.1]hept-2-yl]pyridin-4-yl}-3-(pyrazin-2-ylamino)-1H-indazole-5-carbonitrile

Obtained as a solid (98%) from *tert*-butyl (1*S*,4*S*)-5-{4-[5-cyano-3-(pyrazin-2-ylamino)-1*H*-indazol-1-yl]pyridin-2-yl}-2,5-diazabicyclo[2.2.1]heptane-2-carboxylate following the experimental procedure described in example 1c.

LRMS (m/z): 410 (M+1)⁺.

¹H-NMR (400 MHz, METHANOL-*d*₄) δ ppm 1.94 (d, *J*=10.16 Hz, 1 H), 2.10 (d, *J*=10.84 Hz, 1 H), 3.19 (s, 2 H), 3.46 (d, *J*=10.16 Hz, 1 H), 3.72 (dd, *J*=10.06, 2.25 Hz, 1 H), 4.09 (s, 1 H), 4.89 (s, 1 H), 6.85 (d, *J*=1.76 Hz, 1 H), 7.10 (dd, *J*=5.76, 1.86 Hz, 1 H), 7.77 (dd, *J*=8.99, 1.56 Hz, 1 H), 8.02 (dd, *J*=8.79, 0.59 Hz, 1 H), 8.14 (d, *J*=2.54 Hz, 1 H), 8.17 (d, *J*=5.86 Hz, 1 H), 8.31 (dd, *J*=2.64, 1.47 Hz, 1 H), 8.54 (s, 1 H), 9.38 (d, *J*=1.37 Hz, 1 H)

### EXAMPLE 12

### 1-{2-[(1S,4S)-2,5-Diazabicyclo[2.2.1]hept-2-yl]pyridin-4-yl}-N³-pyrazin-2-yl-1H-indazole-3,6-diamine

### a) 3-Bromo-6-nitro-1H-indazole

Obtained as a brown solid (90%) from 6-nitro-1*H*-indazole following the experimental procedure described in example 11 a.

LRMS (m/z): 240/242.

### b) tert-Butyl (1S,4S)-5-[4-(3-bromo-6-nitro-1H-indazol-1-yl)pyridin-2-yl]-2,5-diazabicyclo[2.2.1]heptane-2-carboxylate

Obtained (30%) from 3-bromo-6-nitro-1*H*-indazole and (1*S*,4*S*)-*tert*-butyl 5-(4-bromopyridin-2-yl)-2,5-diazabicyclo[2.2.1]heptane-2-carboxylate (preparation 3) following the experimental procedure described in example 1a but heating at 120ºC for 24 h instead of using microwaves. Purification by flash chromatography (9:1 hexanes/ethyl acetate to 100% ethyl acetate) provided the title compound.

LRMS (m/z): 515/517.

### c) tert-Butyl (1S,4S)-5-{4-[6-nitro-3-(pyrazin-2-ylamino)-1H-indazol-1-yl]pyridin-2-yl}-2,5-diazabicyclo[2.2.1]heptane-2-carboxylate

Obtained (36%) from *tert*-Butyl (1*S*,4*S*)-5-[4-(3-bromo-6-nitro-1*H*-indazol-1-yl)pyridin-2-yl]-2,5-diazabicyclo[2.2.1]heptane-2-carboxylate (example 12b) and 2-aminopyrazine following a similar experimental procedure as described in example 11c followed by reverse phase chromatography (C-18 silica from Waters©, water/methanol [0.1% v/v formic acid buffered] 0% to 100%).

LRMS (m/z): 530 (M+1)⁺.

### d) tert-Butyl (1S,4S)-5-{4-[6-amino-3-(pyrazin-2-ylamino)-1H-indazol-1-yl]pyridin-2-yl}-2,5-diazabicyclo[2.2.1]heptane-2-carboxylate

A resealable Schlenk tube was charged with *tert*-butyl (1*S*,4*S*)-5-{4-[6-amino-3-(pyrazin-2-ylamino)-1*H*-indazol-1-yl]pyridin-2-yl}-2,5-diazabicyclo[2.2.1]heptane-2-carboxylate (170 mg, 0.321 mmol) in ethanol (6 mL). Then ammonium chloride (0.73 mL) and Fe (71.5 mg, 1.28 mmol) were added, the Schlenk tube was capped and placed in an oil bath at 70ºC. After 2 h, the mixture was cooled, filtered and the solvent was removed under vacuum. The crude was dissolved in ethyl acetate, washed with saturated aqueous sodium carbonate solution, the organic extracts dried over Na₂SO₄ and concentrated under vacuum to provide the title compound (110 mg, 69%).

LRMS (m/z): 500 (M+1)⁺.

### e) 1-{2-[(1S,4S)-2,5-Diazabicyclo[2.2.1]hept-2-yl]pyridin-4-yl}-N³-pyrazin-2-yl-1H-indazole-3,6-diamine

Obtained as a solid (89%) from *tert*-butyl (1*S*,4*S*)-5-{4-[6-amino-3-(pyrazin-2-ylamino)-1*H*-indazol-1-yl]pyridin-2-yl}-2,5-diazabicyclo[2.2.1]heptane-2-carboxylate following the experimental procedure described in example 1c.

LRMS (m/z): 400 (M+1)+.

¹H-NMR (400 MHz, METHANOL-*d*₄) δ ppm 1.85 (d, *J*=10 Hz, 1 H), 2.01 (d, *J*=8.8 Hz, 1 H), 3.06 (s, 2 H), 3.38 (d, *J*=10 Hz, 1 H), 3.67 (dd, *J*=9.8, 2 Hz, 1 H), 3.87 (s, 1 H), 4.77 (s, 1 H), 6.68 (dd, *J*=8.8, 1.6 Hz, 1 H), 6.79 (d, *J*=1.6 Hz, 1 H), 7.06 (dd, *J*=5.6, 2 Hz, 1 H), 7.10 (d, *J*=1.2 Hz, 1 H), 7.69 (d, *J*=8.4 Hz, 1 H), 8.03 (d, *J*=6 Hz, 1 H), 8.07 (d, *J*=2.4 Hz, 1 H), 8.25 (dd, *J*= 2.4, 1.6 Hz 1 H), 9.38 (d, *J*=1.6 Hz, 1 H).

### EXAMPLE 13

### 6-Bromo-1-{2-[(1S,4S)-2,5-diazabicyclo[2.2.1]hept-2-yl]pyridin-4-yl}-N-pyrazin-2-yl-1H-indazol-3-amine

### a) tert-Butyl (1S,4S)-5-{4-[6-bromo-3-(pyrazin-2-ylamino)-1H-indazol-1-yl]pyridin-2-yl}-2,5-diazabicyclo[2.2.1]heptane-2-carboxylate

To a 60ºC solution of the title compound of Example 12d (485 mg, 0.97 mmol) in acetonitrile (5 mL), copper (II) bromide (213 mg, 0.95 mmol) was added. The mixture was purged with nitrogen and a solution of *tert*-butyl nitrite (194 µl, 1.63 mmol) in acetonitirle (2.5 ml) was added. The mixture was stirred at 60ºC for 1.5 h. It was then let to cool down and filtered over Celite and washed with warm methanol. Solvent was removed in vaccuo and crude thus obtained was purified by reverse phase chromatography (C-18 silica from Waters@, water/methanol [0.1% v/v formic acid buffered] 0% to 100%). 90 mg (16% yield) were obtained.

LRMS (m/z): 565 (M+1)+.

### b) 6-Bromo-1-{2-[(1S,4S)-2,5-diazabicyclo[2.2.1]hept-2-yl]pyridin-4-yl}-N-pyrazin-2-yl-1H-indazol-3-amine

To a stirred solution of the title compound of Example 13a (6 mg, 0.01 mmol) in methanol (0.5 ml), a solution of HCl 4M in dioxane (200 µl) was added and the mixture was stirred at rt overnight. Solvent was removed in vaccou to yield the title compound (4.3 mg, 85% yield).

LRMS (m/z): 464 (M+1)+.

### EXAMPLE 14

### 1-{2-[(1S,4S)-2,5-Diazabicyclo[2.2.1]hept-2-yl]pyridin-4-yl}-6-phenyl-N-pyrazin-2-yl-1H-indazol-3-amine

### a) tert-Butyl (1S,4S)-5-{4-[6-phenyl-3-(pyrazin-2-ylamino)-1H-indazol-1-yl]pyridin-2-yl}-2,5-diazabicyclo[2.2.1]heptane-2-carboxylate

To a stirred solution of the title compound of Example 13a (88.6 mg, 0.16 mmol) in toluene/metanol (6:4, 4 mL) phenylboronic acid (40 mg, 0.33 mmol), Pd(PPh₃)₄ (20 mg, 0.02 mmol) and potassium carbonate (69 mg, 0.5 mmol) were added and the mixture was sitrred at 120ºC overnight. It was let to cool down and filtered over Celite. Solvent was removed to yield a crude product that was purified by flash chromatography (hexanes/ethyl acetate). 73 mg of the title compound (78% yield) were obtained.

LRMS (m/z): 561 (M+1)⁺.

### b) 1-{2-[(1S,4S)-2,5-Diazabicyclo[2.2.1]hept-2-yl]pyridin-4-yl}-6-phenyl-N-pyrazin-2-yl-1H-indazol-3-amine.

Obtained (56%) yield from the title compound of Example 14a following the experimental procedure of Example 13b.

LRMS (m/z): 461 (M+1)+.

¹H NMR (400 MHz, dmso) δ 10.84 (s, 1H), 9.91 (s, 1H), 9.51 (d, *J* = 18.3 Hz, 2H), 8.49 (d, *J* = 8.4 Hz, 1H), 8.46 - 8.37 (m, 1H), 8.35 - 8.23 (m, 2H), 8.14 (d, *J* = 7.1 Hz, 1H), 7.94 - 7.85 (m, 2H), 7.75 (dd, *J* = 8.5, 1.1 Hz, 1H), 7.65 (d, *J* = 6.9 Hz, 1H), 7.56 (dd, *J* = 10.2, 4.8 Hz, 2H), 7.50 - 7.43 (m, 1H), 7.36 (s, 1H), 5.33 (s, 1H), 4.66 (s, 1H), 4.06 (d, *J* = 10.5 Hz, 1H), 3.89 (d, *J* = 10.5 Hz, 1H), 3.47 (m, 1H), 3.34 (m, 1H), 2.26 (d, *J* = 10.8 Hz, 1H), 2.13 (d, *J* = 10.8 Hz, 1H).

### EXAMPLE 15

### Methyl 1-{2-[(1S,4S)-2,5-diazabicyclo[2.2.1]hept-2-yl]pyridin-4-yl}-3-(pyrazin-2-ylamino)-1H-indazole-6-carboxylate

### a) Methyl 3-bromo-1H-indazole-6-carboxylate

To a solution of methyl 1*H*-indazole-6-carboxylate (5.8 g, 32.8 mmol) in dimethylformamide (45 mL) *N*-bromosuccinimide (6.4 g, 36.1 mmol) was slowly added and reaction was stirred at room temperature for 1 h. The mixture was poured into water and product was extracted with ethyl acetate, then organic layer was washed with water, dried (MgSO₄) and solvent was removed in vacuo to give the title compound (8.3 g, 96%) without any further purification.

LRMS (m/z): 254-256 (M+1)⁺.

¹H NMR (300 MHz, CHLOROFORM-*d*) δ ppm 3.98 (s, 3 H) 7.70 (d, *J*=8.79 Hz, 1 H) 7.91 (dd, *J*=8.38, 0.96 Hz, 1 H) 8.04 (s, 1 H) 8.26 (d, *J*=0.55 Hz, 1 H) 10.62 (br. s., 1 H).

### b) Methyl 3-bromo-1-{2-[(1S,4S)-5-(tert-butoxycarbonyl)-2,5-diazabicyclo[2.2.1] hept-2-yl]pyridin-4-yl}-1H-indazole-6-carboxylate

Obtained (24%) from methyl 3-bromo-1*H*-indazole-6-carboxylate (example 15a) and *(1S,4S)*-*tert*-butyl 5-(4-bromopyridin-2-yl)-2,5-diazabicyclo[2.2.1]heptane-2-carboxylate (preparation 3) following the experimental procedure as described in example 1 a, using dioxane as solvent. The mixture thus obtained was filtered through celite and solvent was removed. Crude was purified by reverse phase chromatography (C-18 from Merck, water/methanol-acetonitrile (1:1) as eluents, 20-80%) to give the desired compound.

LRMS (m/z): 528-530 (M+1)⁺.

### c) Methyl 1-{2-[(1S,4S)-5-(tert-butoxycarbonyl)-2,5-diazabicyclo[2.2.1]hept-2-yl]pyridin-4-yl}-3-(pyrazin-2-ylamino)-1H-indazole-6-carboxylate

Obtained (47%) from methyl 3-bromo-1-{2-[(1*S*,4*S*)-5-(*tert*-butoxycarbonyl)-2,5-diazabicyclo[2.2.1]hept-2-yl]pyridin-4-yl}-1*H*-indazole-6-carboxylate (example 15b) and pyrazin-2-amine following the experimental procedure as described in preparation 6, heating at 110ºC for 30 h. The mixture thus obtained was filtered through celite and solvent was removed. Crude was purified by reverse phase chromatography (C-18 from Merck, water/methanol-acetonitrile (1:1) as eluents, 30-100%) to give the desired compound.

LRMS (m/z): 543 (M+1)⁺.

### d) Methyl 1-{2-[(1S,4S)-2,5-diazabicyclo[2.2.1]hept-2-yl]pyridin-4-yl}-3-(pyrazin-2-ylamino)-1H-indazole-6-carboxylate

Obtained as a solid (85%) from methyl 1-{2-[(1*S*,4*S*)-5-(*tert*-butoxycarbonyl)-2,5-diazabicyclo[2.2.1]hept-2-yl]pyridin-4-yl}-3-(pyrazin-2-ylamino)-1*H*-indazole-6-carboxylate following the experimental procedure described in example 1 c.

LRMS (m/z): 443 (M+1)+.

¹H-NMR (400 MHz, CDCl₃) δ ppm 1.90 (d, *J*=10.00 Hz, 1 H), 1.98 (d, *J*=9.60 Hz, 1 H), 3.18 (s, 2 H), 3.35 (d, *J*=9.20 Hz, 1 H), 3.71 (dd, *J*=9.60, 2.00 Hz, 1 H), 3.92 (s, 1 H), 4.00 (s, 3 H), 4.86 (s, 1 H), 6.73 (d, *J*=1.60 Hz, 1 H), 7.03 (dd, *J*=5.60, 2.00 Hz, 1 H), 7.47 (bs, 1 H), 7.78 (d, *J*=8.40 Hz, 1 H), 7.92 (dd, *J*=9.60, 1.20 Hz, 1 H), 8.22-8.23 (m, 2 H), 8.26 (d, *J*=5.60 Hz, 1 H), 8.59 (s, 1 H), 9.51 (d, *J=*1.20 Hz, 1 H).

### EXAMPLE 16

### 1-{2-[(1S,4S)-2,5-Diazabicyclo[2.2.1]hept-2-yl]pyridin-4-yl}-3-(pyrazin-2-ylamino)-1H-indazole-6-carboxamide

### a) tert-Butyl (1S,4S)-5-{4-[6-(aminocarbonyl)-3-(pyrazin-2-ylamino)-1H-indazol-1-yl]pyridin-2-yl}-2,5-diazabicyclo[2.2.1]heptane-2-carboxylate

Methyl 1-{2-[(1*S*,4*S*)-5-(*tert*-butoxycarbonyl)-2,5-diazabicyclo[2.2.1]hept-2-yl]pyridin-4-yl}-3-(pyrazin-2-ylamino)-1*H*-indazole-6-carboxylate (example 15c, 20 mg, 0.04 mmol) was dissolved in a solution of ammonia in methanol (1.7 mL, 7N) and heated in a sealed tube at 110ºC for 96 hours. Ammonia was removed and displaced using argon, and purification by flash chromatography (85:15 dichloromethane-methanol) provided the title compound (8 mg, 42%) as a white solid.

LRMS (m/z): 528 (M+1)⁺.

### b)1-{2-[(1S,4S)-2,5-Diazabicyclo[2.2.1]hept-2-yl]pyridin-4-yl}-3-(pyrazin-2-ylamino)-1H-indazole-6-carboxamide

Obtained as a solid (90%) from *tert*-butyl (1*S*,4*S*)-5-{4-[6-(aminocarbonyl)-3-(pyrazin-2-ylamino)-1*H*-indazol-1-yl]pyridin-2-yl}-2,5-diazabicyclo[2.2.1]heptane-2-carboxylate following the experimental procedure described in example 1 c.

LRMS (m/z): 428 (M+1)⁺.

¹H-NMR (400 MHz, METHANOL-*d*₄) δ ppm 1.93 (d, *J*=10.00 Hz, 1 H), 2.08 (d, *J*=9.60 Hz, 1 H), 3.16 (s, 2 H), 3.45 (d, *J*=9.60 Hz, 1 H), 3.73 (d, *J*=8.40, 1 H), 4.00 (s, 1 H), 4.87 (s, 1 H), 6.89 (d, *J*=1.60 Hz, 1 H), 7.19 (dd, *J*=5.60, 2.00 Hz, 1 H), 7.78 (dd, *J*=8.40, 0.80 Hz, 1 H), 8.10-8.13 (m, 2 H), 8.13 (d, *J*=6.00 Hz, 1 H), 8.30 (s, 1 H), 8.45 (s, 1 H), 9.40 (s, 1 H).

### EXAMPLE 17

### 1-{2-[(1S,4S)-2,5-Diazabicyclo[2.2.1]hept-2-yl]pyridin-4-yl}-3-(pyrazin-2-ylamino)-1H-indazole-6-carboxylic acid

### a) 1-{2-[(1S,4S)-5-(tert-Butoxycarbonyl)-2,5-diazabicyclo[2.2.1]hept-2-yl]pyridin-4-yl}-3-(pyrazin-2-ylamino)-1H-indazole-6-carboxylic acid

To a solution of methyl 1-{2-[(1*S*,4*S*)-5-(*tert*-butoxycarbonyl)-2,5-diazabicyclo [2.2.1]hept-2-yl]pyridin-4-yl}-3-(pyrazin-2-ylamino)-1*H*-indazole-6-carboxylate (example 15c, 90 mg, 0.17 mmol) in tetrahydrofurane (3 mL) and water (0.5 mL) litium hydroxide hydrate (41.76 mg, 1 mmol) was added and mixture was stirred overnight at room temperature. Solvent was removed, water was additioned and saturated aqueous solution of potassium bisulfate was added until precipitation of the title compound. Solid was filtrated, washed with water and dried in the vacuum oven to give the desired product (87 mg, 100%) as a yellow solid.

LRMS (m/z): 529 (M+1)⁺.

### b) 1-{2-[(1S,4S)-2,5-Diazabicyclo[2.2.1]hept-2-yl]pyridin-4-yl}-3-(pyrazin-2-ylamino)-1H-indazole-6-carboxylic acid

Obtained as a solid (93%) from 1-{2-[(1*S*,4*S*)-5-(*tert*-butoxycarbonyl)-2,5-diazabicyclo[2.2.1]hept-2-yl]pyridin-4-yl}-3-(pyrazin-2-ylamino)-1*H*-indazole-6-carboxylic acid following the experimental procedure described in example 1 c.

LRMS (m/z): 429 (M+1)⁺.

¹H-NMR (400 MHz, DMSO-*d*₆) δ ppm 1.98 (d, *J*=9.60 Hz, 1 H), 2.21 (d, *J*=10.40 Hz, 1 H), 3.32 (s, 2 H), 3.64 (d, *J*=10.00 Hz, 1 H), 3.74 (d, *J*=8.80, 1 H), 4.56 (s, 1 H), 5.01 (s, 1 H), 7.01 (s, 1 H), 7.27 (s, 1 H), 7.85 (d, *J*=8.00 Hz, 1 H), 8.22-8.40 (m, 3 H), 8.47 (s, 1 H), 8.63 (bs, 1 H), 9.17 (brs, 1 H), 9.41 (s, 1 H), 10.67 (s, 1 H).

### EXAMPLE 18

### N-Cyclohexyl-1-{2-[(1S,4S)-2,5-diazabicyclo[2.2.1]hept-2-yl]pyridin-4-yl}-3-(pyrazin-2-ylamino)-1H-indazole-6-carboxamide

### a) tert-Butyl (1S,4S)-5-{4-[6-[(cyclohexylamino)carbonyl]-3-(pyrazin-2-ylamino)-1H-indazol-1-yl]pyridin-2-yl}-2,5-diazabicyclo[2.2.1]heptane-2-carboxylate

To a solution of 1-{2-[(1*S*,4*S*)-5-(*tert*-butoxycarbonyl)-2,5-diazabicyclo[2.2.1]hept-2-yl]pyridin-4-yl}-3-(pyrazin-2-ylamino)-1*H*-indazole-6-carboxylic acid (example 17a, 300 mg, 0.57 mmol) in DMF (6 mL), 1-(3-dimethylaminopropyl)-3-ethylcarbodiimide (EDC, 142 mg, 0.74 mmol) and hydroxybenzotriazole (HOBt, 91 mg, 0.67 mmol) and cyclohexanamine (0.07 mL, 0.61 mmol) were added and the mixture was stirred at room temperature overnight. Water (20 mL) and ethyl acetate (20 mL) were added, the organic phase was washed with sodium biocarbonate and brine, dried (Na₂SO₄) and concentrated under vacuum to provide the title compound (244 mg, 69%).

LRMS (m/z): 610 (M+1)+.

### b) N-Cyclohexyl-1-{2-[(1S,4S)-2,5-diazabicyclo[2.2.1]hept-2-yl]pyridin-4-yl}-3-(pyrazin-2-ylamino)-1H-indazole-6-carboxamide

Obtained as a formate salt (10%) from *tert*-Butyl (1*S*,4*S*)-5-{4-[6-[(cyclohexylamino)carbonyl]-3-(pyrazin-2-ylamino)-1*H*-indazol-1-yl]pyridin-2-yl}-2,5-diazabicyclo[2.2.1]heptane-2-carboxylate using the same experimental procedure as described in example 7c .

LRMS (m/z): 510 (M+1)⁺.

### EXAMPLE 19

### N-Benzyl-1-{2-[(1S,4S)-2,5-diazabicyclo[2.2.1]hept-2-yl]pyridin-4-yl}-3-(pyrazin-2-ylamino)-1H-indazole-6-carboxamide

### a) tert-Butyl (1S,4S)-5-{4-[6-[(benzylamino)carbonyl]-3-(pyrazin-2-ylamino)-1H-indazol-1-yl]pyridin-2-yl}-2,5-diazabicyclo[2.2.1]heptane-2-carboxylate

Obtained as a solid (70%) from 1-{2-[(1*S*,4*S*)-5-(*tert*-butoxycarbonyl)-2,5-diazabicyclo[2.2.1]hept-2-yl]pyridin-4-yl}-3-(pyrazin-2-ylamino)-1*H*-indazole-6-carboxylic acid (example 17a) and benzylamine following the experimental procedure described in example 18a.

LRMS (m/z): 618 (M+1)⁺.

### b) N-Benzyl-1-{2-[(1S,4S)-2,5-diazabicyclo[2.2.1]hept-2-yl]pyridin-4-yl}-3-(pyrazin-2-ylamino)-1H-indazole-6-carboxamide

Obtained as a formate salt (10%) from *tert*-butyl (1*S*,4*S*)-5-{4-[6-[(benzylamino)carbonyl]-3-(pyrazin-2-ylamino)-1*H*-indazol-1-yl]pyridin-2-yl}-2,5-diazabicyclo[2.2.1]heptane-2-carboxylate using the same experimental procedure as described in example 7c .

LRMS (m/z): 518 (M+1)⁺.

### EXAMPLE 20

### 1-{[1-{2-[(1S,4S)-2,5-Diazabicyclo[2.2.1]hept-2-yl]pyridin-4-yl}-3-(pyrazin-2-ylamino)-1H-indazol-6-yl]carbonyl}piperidine-4-carboxylic acid

### a) 1-{[1-{2-[(1S,4S)-5-(tert-Butoxycarbonyl)-2,5-diazabicyclo[2.2.1]hept-2-yl]pyridin-4-yl}-3-(pyrazin-2-ylamino)-1H-indazol-6-yl]carbonyl}piperidine-4-carboxylic acid

Obtained as a solid (78%) from 1-{2-[(1*S*,4*S*)-5-(*tert*-butoxycarbonyl)-2,5-diazabicyclo[2.2.1]hept-2-yl]pyridin-4-yl}-3-(pyrazin-2-ylamino)-1*H*-indazole-6-carboxylic acid (example 17a) and piperidine-4-carboxylic acid following the experimental procedure described in example 18a.

LRMS (m/z): 640 (M+1)⁺.

### b) 1-{[1-{2-[(1S,4S)-2,5-Diazabicyclo[2.2.1]hept-2-yl]pyridin-4-yl}-3-(pyrazin-2-ylamino)-1H-indazol-6-yl]carbonyl}piperidine-4-carboxylic acid

Obtained as a formate salt (10%) from 1-{[1-{2-[(1*S*,4*S*)-5-(*tert*-butoxycarbonyl)-2,5-diazabicyclo[2.2.1]hept-2-yl]pyridin-4-yl}-3-(pyrazin-2-ylamino)-1*H*-indazol-6-yl]carbonyl}piperidine-4-carboxylic acid using the same experimental procedure as described in example 7c .

LRMS (m/z): 540 (M+1)⁺.

### EXAMPLE 21

### 1-{2-[(1S,4S)-2,5-Diazabicyclo[2.2.1]hept-2-yl]pyridin-4-yl}-6-(piperidin-1-ylcarbonyl)-N-pyrazin-2-yl-1H-indazol-3-amine

### a) tert-Butyl (1S,4S)-5-{4-[6-(piperidin-1-ylcarbonyl)-3-(pyrazin-2-ylamino)-1H-indazol-1-yl]pyridin-2-yl}-2,5-diazabicyclo[2.2.1]heptane-2-carboxylate

Obtained as a solid (59%) from 1-{2-[(1*S*,4*S*)-5-(*tert*-butoxycarbonyl)-2,5-diazabicyclo[2.2.1]hept-2-yl]pyridin-4-yl}-3-(pyrazin-2-ylamino)-1*H*-indazole-6-carboxylic acid (example 17a) and piperidine following the experimental procedure described in example 18a.

LRMS (m/z): 596 (M+1)⁺.

### b) 1-{2-[(1S,4S)-2,5-Diazabicyclo[2.2.1]hept-2-yl]pyridin-4-yl}-6-(piperidin-1-ylcarbonyl)-N-pyrazin-2-yl-1H-indazol-3-amine

Obtained as a formate salt (10%) from *tert*-butyl (1*S*,4*S*)-5-{4-[6-(piperidin-1-ylcarbonyl)-3-(pyrazin-2-ylamino)-1*H*-indazol-1-yl]pyridin-2-yl}-2,5-diazabicyclo[2.2.1]heptane-2-carboxylate using the same experimental procedure as described in example 1c .

LRMS (m/z): 496 (M+1)⁺.

### EXAMPLE 22

### 6-[(4-Benzylpiperidin-1-yl)carbonyl]-1-{2-[(1S,4S)-2,5-diazabicyclo[2.2.1]hept-2-yl]pyridin-4-yl}-N-pyrazin-2-yl-1H-indazol-3-amine

### a) tert-butyl (1S,4S)-5-{4-[6-[(4-benzylpiperidin-1-yl)carbonyl]-3-(pyrazin-2-ylamino)-1H-indazol-1-yl]pyridin-2-yl}-2,5-diazabicyclo[2.2.1]heptane-2-carboxylate

Obtained as a solid (42%) from 1-{2-[(1 S,4S)-5-(*tert*-butoxycarbonyl)-2,5-diazabicyclo[2.2.1]hept-2-yl]pyridin-4-yl}-3-(pyrazin-2-ylamino)-1*H*-indazole-6-carboxylic acid (example 17a) and 4-benzylpiperidine following the experimental procedure described in example 18a.

LRMS (m/z): 686 (M+1)⁺.

### b) 6-[(4-Benzylpiperidin-1-yl)carbonyl]-1-{2-[(1S,4S)-2,5-diazabicyclo[2.2.1]hept-2-yl]pyridin-4-yl}-N-pyrazin-2-yl-1H-indazol-3-amine

Obtained as a formate salt (8%) from *tert*-butyl (1*S*,4*S*)-5-{4-[6-[(4-benzylpiperidin-1-yl)carbonyl]-3-(pyrazin-2-ylamino)-1*H*-indazol-1-yl]pyridin-2-yl}-2,5-diazabicyclo[2.2.1]heptane-2-carboxylate using the same experimental procedure as described in example 1c.

LRMS (m/z): 586 (M+1)⁺.

### EXAMPLE 23

### N-{[1-{2-[(1S,4S)-2,5-Diazabicyclo[2.2.1]hept-2-yl]pyridin-4-yl}-3-(pyrazin-2-ylamino)-1H-indazol-6-yl]carbonyl}-N-methylglycine

### a) Ethyl N-{[1-{2-[(1S,4S)-2,5-diazabicyclo[2.2.1]hept-2-yl]pyridin-4-yl}-3-(pyrazin-2-ylamino)-1H-indazol-6-yl]carbonyl}-N-methylglycinate

To a solution of 1-{2-[(1*S*,4*S*)-5-(*tert*-Butoxycarbonyl)-2,5-diazabicyclo[2.2.1]hept-2-yl]pyridin-4-yl}-3-(pyrazin-2-ylamino)-1*H*-indazole-6-carboxylic acid (example 17a, 42 mg, 0.08 mmol) in DMF (2 mL), 1-(3-dimethylaminopropyl)-3-ethylcarbodiimide (EDC, 20 mg, 0.10 mmol) and hydroxybenzotriazole (HOBt, 14 mg, 0.10 mmol) were added and the mixture was stirred at room temperature for 3 h. Then ethyl *N*-methylglycinate (19.1 mg, 0.16 mmol) was added and the mixture was stirred at room temperature overnight. Water and ethyl acetate (10 mL) were added, the organic phase was washed with sodium biocarbonate and brine, dried (Na₂SO₄) and concentrated under vacuum to provide the title compound (50 mg, 94%).

LRMS (m/z): 628 (M+1)+.

### b) N-{[1-{2-[(1S,4S)-5-(tert-Butoxycarbonyl)-2,5-diazabicyclo[2.2.1]hept-2-yl]pyridin-4-yl}-3-(pyrazin-2-ylamino)-1H-indazol-6-yl]carbonyl}-N-methylglycine

Obtained (70%) from ethyl *N*-{[1-{2-[(1S,4S)-2,5-diazabicyclo[2.2.1]hept-2-yl]pyridin-4-yl}-3-(pyrazin-2-ylamino)-1*H*-indazol-6-yl]carbonyl}-N-methylglycinate (example 23a) using the same experimental procedure as described in example 17a.

LRMS (m/z): 600 (M+1)⁺.

### c) N-{[1-{2-[(1S,4S)-2,5-Diazabicyclo[2.2.1]hept-2-yl]pyridin-4-yl}-3-(pyrazin-2-ylamino)-1H-indazol-6-yl]carbonyl}-N-methylglycine

Obtained as a formate salt (23%) from *N*-{[1-{2-[(1*S*,4*S*)-5-(*tert*-butoxycarbonyl)-2,5-diazabicyclo[2.2.1]hept-2-yl]pyridin-4-yl}-3-(pyrazin-2-ylamino)-1*H*-indazol-6-yl]carbonyl}-*N*-methylglycine (example 23b) using the same experimental procedure as described in example 7c .

LRMS (m/z): 500 (M+1)⁺.

¹H NMR (400 MHz, DMSO-D6) δ ppm 1.9 (d, *J*=11.3 Hz, 2 H), 2.2 (d, *J*=10.6 Hz, 2 H), 3.0 (m, 5 H), 3.7 (d, *J*=17.6 Hz, 2 H), 4.1 (d, *J*=10.6 Hz, 2 H), 4.4 (s, 1 H), 5.1 (s, 1 H), 6.8 (s, 1 H), 7.0 (d, *J*=5.5 Hz, 1 H), 7.4 (d, *J*=8.2 Hz, 1 H), 8.1 (s, 1 H), 8.2 (m, 3 H), 9.4 (m, *J*=7.0 Hz, 1 H), 10.5 (s, 1 H).

### EXAMPLE 24

### 1-{2-[(1S,4S)-2,5-Diazabicyclo[2.2.1]hept-2-yl]pyridin-4-yl}-3-(pyrazin-2-ylamino)-1H-indazole-6-carbonitrile

### a) 3-Bromo-1H-indazole-6-carbonitrile

Obtained as a brown solid (98%) from 1*H*-indazole-5-carbonitrile following the experimental procedure described in example 11a.

LRMS (m/z): 222/224 (Br).

### b) tert-Butyl (1S,4S)-5-[4-(3-bromo-6-cyano-1H-indazol-1-yl)pyridin-2-yl]-2,5-diazabicyclo[2.2.1]heptane-2-carboxylate

Obtained (21%) from 3-bromo-1*H*-indazole-6-carbonitrile and (1*S*,4*S*)-*tert*-butyl 5-(4-bromopyridin-2-yl)-2,5-diazabicyclo[2.2.1]heptane-2-carboxylate (preparation 3) following the experimental procedure as described in example 1a, followed by purification by flash chromatography (9:1 hexanes/ethyl acetate to 100% ethyl acetate).

LRMS (m/z): 495/497.

### c) tert-Butyl (1S,4S)-5-{4-[6-cyano-3-(pyrazin-2-ylamino)-1H-indazol-1-yl]pyridin-2-yl}-2,5-diazabicyclo[2.2.1]heptane-2-carboxylate

Obtained (52%) from *tert*-butyl (1*S*,4*S*)-5-[4-(3-bromo-6-cyano-1*H*-indazol-1-yl)pyridin-2-yl]-2,5-diazabicyclo[2.2.1]heptane-2-carboxylate (example 24b) and 2-aminopyrazine following a similar experimental procedure as described in example 11c.

LRMS (m/z): 510 (M+1)⁺.

### d)1-{2-[(1S,4S)-2,5-Diazabicyclo[2.2.1]hept-2-yl]pyridin-4-yl}-3-(pyrazin-2-ylamino)-1H-indazole-6-carbonitrile

Obtained as a solid (98%) from *tert-*butyl (1*S*,4*S*)-5-{4-[6-cyano-3-(pyrazin-2-ylamino)-1*H*-indazol-1-yl]pyridin-2-yl}-2,5-diazabicyclo[2.2.1]heptane-2-carboxylate following the experimental procedure described in example 1c.

LRMS (m/z): 410 (M+1)+.

¹H-NMR (400 MHz, METHANOL-d₄) δ ppm 1.92 (d, *J*=10.00 Hz, 1 H), 2.09 (d, *J*=9.60 Hz, 1 H), 3.14 (s, 2 H), 3.43 (d, *J*=10.00 Hz, 1 H), 3.70 (dd, *J*=8.40, 2.00 Hz, 1 H), 4.03 (s, 1 H), 4.86 (s, 1 H), 6.81 (d, *J*=1.20 Hz, 1 H), 7.07 (dd, *J*=5.60, 1.60 Hz, 1 H), 7.49 (dd, *J*=8.00, 0.8 Hz, 1 H), 8.13-8.14 (m, 2 H), 8.18 (d, *J*=8.40 Hz, 1 H), 8.28-8.30 (m, 2 H), 9.29 (d, *J*=1.20 Hz, 1 H).

### EXAMPLE 25

### 1-{2-[(1S,4S)-2,5-Diazabicyclo[2.2.1]hept-2-yl]pyridin-4-yl}-6-fluoro-N-pyrazin-2-yl-1H-indazol-3-amine

### a) 6-Fluoro-1H-indazol-3-amine

Obtained (34%) from 2,4-difluorobenzonitrile and hydrazine hydrate using the same experimental procedure as described in preparation 8 but after cooling the solvent was removed under vacuum. The crude was dissolved in ethyl acetate, washed with water and brine, the organic extracts dried over Na₂SO₄ and evaporated. The crude was purified by flash chromatography (hexane-60% ethyl acetate) to give the title compound.

LRMS (m/z): 152 (M+1)⁺.

### b) tert-Butyl (1S,4S)-5-[4-(3-amino-6-fluoro-1H-indazol-1-yl)pyridin-2-yl]-2,5-diazabicyclo[2.2.1]heptane-2-carboxylate

Obtained (5%) from 6-fluoro-1*H*-indazol-3-amine (example 25a) and *(1S,4S)-tert-*butyl 5-(4-bromopyridin-2-yl)-2,5-diazabicyclo[2.2.1]heptane-2-carboxylate (preparation 3) using the same experimental procedure as described in example 2a, followed by purification by reverse phase chromatography as described in the general methods.

LRMS (m/z): 425 (M+1)⁺.

### c) tert-Butyl (1S,4S)-5-{4-[6-fluoro-3-(pyrazin-2-ylamino)-1H-indazol-1-yl]pyridin-2-yl}-2,5-diazabicyclo[2.2.1]heptane-2-carboxylate

Obtained (10%) from *tert*-butyl (1*S*,4*S*)-5-[4-(3-amino-6-fluoro-1*H*-indazol-1-yl)pyridin-2-yl] 2,5-diazabicyclo[2.2.1]heptane-2-carboxylate (example 25b) and 2-chloropyrazine using the same experimental procedure as described in example 2b, followed by purification by flash chromatography (hexane/ethyl acetate) gave the title compound.

LRMS (m/z): 503 (M+1)⁺.

### d) 1-{2-[(1S,4S)-2,5-diazabicyclo[2.2.1]hept-2-yl]pyridin-4-yl}-6-fluoro-N-pyrazin-2-yl-1H-indazol-3-amine

Obtained as a hydrochloride salt (33% yield) from tert-butyl (1S,4S)-5-14-[6-fluoro-3-(pyrazin-2-ylamino)-1*H*-indazol-1-yl]pyridin-2-yl}-2,5-diazabicyclo[2.2.1]heptane-2-carboxylate (example 25c) using the same experimental procedure as described in example 2c.

LRMS (m/z): 403 (M+1)⁺.

¹H NMR (400 MHz, DMSO-*d*₆) δ ppm 2.09 (d, *J*=11.33 Hz, 1 H), 2.23 (d, *J*=10.94 Hz, 1 H), 3.86 (d, *J*=12.11 Hz, 1 H), 3.90 - 3.99 (m, 1 H), 4.63 (s, 1 H), 5.29 (s, 1 H), 7.15 (br. s., 1 H), 7.28 - 7.40 (m, 1 H), 7.45 - 7.52 (m, 1 H), 8.04 (d, *J*=6.64 Hz, 1 H), 8.15 (d, *J*=7.03 Hz, 1 H), 8.28 (d, *J*=2.34 Hz, 1 H), 8.39 (d, *J*=3.91 Hz, 1 H), 8.45 (t, *J*=5.67 Hz, 1 H), 9.26 (br. s., 1 H), 9.48 (s, 1 H), 9.72 (br. s., 1 H), 10.78 (s, 1 H).

### EXAMPLE 26

### N-[1-{2-[(1S,4S)-2,5-Diazabicyclo[2.2.1]hept-2-yl]pyridin-4-yl}-3-(pyrazin-2-ylamino)-1H-indazol-6-yl]acetamide

### a) tert-Butyl (1S,4S)-5-{4-[6-(acetylamino)-3-(pyrazin-2-ylamino)-1H-indazol-1-yl]pyridin-2-yl}-2,5-diazabicyclo[2.2.1]heptane-2-carboxylate

To a solution of *tert*-butyl (1*S*,4*S*)-5-{4-[6-amino-3-(pyrazin-2-ylamino)-1*H*-indazol-1-yl]pyridin-2-yl}-2,5-diazabicyclo[2.2.1]heptane-2-carboxylate (example 12d, 85 mg, 0.17 mmol) in THF (2 mL) was added triethylamine (0.047 mL, 0.34 mmol) and acetyl chloride (0.024 mL, 0.34 mmol) and the mixture was stirred at room temperature for 2 hours. After addition of water (20 mL) and ethyl acetate (30 mL), the organic phase was washed with water, dried (MgSO₄) and concentrated under vacuum to provide the title compound (85 mg, 92%).

LRMS (m/z): 542 (M+1)+.

### b) N-[1-{2-[(1S,4S)-2,5-Diazabicyclo[2.2.1]hept-2-yl]pyridin-4-yl}-3-(pyrazin-2-ylamino)-1H-indazol-6-yl]acetamide

Obtained as a solid (61%) *tert*-butyl (1*S*,4*S*)-5-{4-[6-(acetylamino)-3-(pyrazin-2-ylamino)-1*H*-indazol-1-yl]pyridin-2-yl}-2,5-diazabicyclo[2.2.1]heptane-2-carboxylate following the experimental procedure described in example 1c but stirring at 0ºC for 1 h instead of doing it at room temperature.

LRMS (m/z): 442 (M+1)⁺.

¹H-NMR (400 MHz, DMSO-*d*₆) δ ppm 1.73 (s, 1 H), 1.85 (s, 1 H), 2.12 (s, 3 H), 2.95 (bs, 2 H), 3.32 (bs, 2 H), 3.49 (bs, 1 H), 3.82 (s, 1 H), 4.76 (s, 1 H), 6.71 (s, 1 H), 6.98 (dd, *J*=5.2, 1.2 Hz, 1 H), 7.26 (d, *J*= 8.8 Hz, 1 H), 8.13 (s, 1 H), 8.15 (d, *J*= 3.2 Hz, 1 H), 8.17 (d, *J*=2.8 Hz, 1 H), 8.30 (s, 1 H), 8.66 (s, 1 H), 9.41 (s, 1 H), 10.33 (s, 1 H), 10.40 (s, 1H).

### EXAMPLE 27

### N-[1-{2-[(1S,4S)-2,5-Diazabicyclo[2.2.1]hept-2-yl]pyridin-4-yl}-3-(pyrazin-2-ylamino)-1H-indazol-6-yl]benzamide

### a) tert-Butyl (1S,4S)-5-{4-[6-(acetylamino)-3-(pyrazin-2-ylamino)-1H-indazol-1-yl]pyridin-2-yl}-2,5-diazabicyclo[2.2.1]heptane-2-carboxylate

To a solution of *tert*-butyl (1*S*,4*S*)-5-{4-[6-amino-3-(pyrazin-2-ylamino)-1*H*-indazol-1-yl]pyridin-2-yl}-2,5-diazabicyclo[2.2.1]heptane-2-carboxylate (example 12d, 70 mg, 0.14 mmol) in DMF (2 mL), benzoic acid (19.1 mg, 0.16 mmol), EDC (35 mg, 0.18 mmol) and HOBt (25 mg, 0.19 mmol) were added and the mixture was stirred at rt for two days. After addition of water and ethyl acetate (30 mL), the organic phase was washed with sodium biocarbonate and brine, dried (Na₂SO₄) and concentrated under vacuum to provide the title compound (90 mg, 61%).

LRMS (m/z): 604 (M+1)+.

### b) N-[1-{2-[(1S,4S)-2,5-Diazabicyclo[2.2.1]hept-2-yl]pyridin-4-yl}-3-(pyrazin-2-yl amino)-1H-indazol-6-yl]benzamide

Obtained as a solid (91%) from the title compound of Example 27a following the experimental procedure described in example 1c.

LRMS (m/z): 504 (M+1)⁺.

¹H NMR (400 MHz, dmso) δ 10.61 (s, 1H), 10.48 (s, 1H), 9.45 (d, *J* = 1.4 Hz, 1H), 8.81 (s, 1H), 8.34 (dd, *J* = 2.5, 1.5 Hz, 1H), 8.29 - 8.14 (m, 3H), 8.05 - 7.96 (m, 2H), 7.72 - 7.52 (m, 3H), 7.14 (dd, *J* = 5.6, 1.7 Hz, 1H), 6.88(s, 1H), 4.94(s, 1H), 4.94 (s, 1H), 4.39 (s, 1H), 4.39 (s, 1H), 3.69 (d, *J* = 8.5 Hz, 1H), 3.64 (dd, *J* = 38.9, 9.9 Hz, 23H), 3.59 (d, *J* = 11.3 Hz, 1H), 3.35 (d, *J* = 27.8 Hz, 18H), 3.25 (m, 2H), 2.14 (d, *J* = 10.4 Hz, 1H), 1.92 (d, *J* = 10.5 Hz, 1H).

### EXAMPLE 28

### N-[1-{2-[(1S,4S)-2,5-Diazabicyclo[2.2.1]hept-2-yl]pyridin-4-yl}-3-(pyrazin-2-ylamino)-1H-indazol-6-yl]-N-methylacetamide

### a) tert-Butyl (1S,4S)-5-{4-[6-(methylamino)-3-(pyrazin-2-ylamino)-1H-indazol-1-yl]pyridin-2-yl}-2,5-diazabicyclo[2.2.1]heptane-2-carboxylate

To a solution of *tert*-butyl (1*S*,4*S*)-5-{4-[6-amino-3-(pyrazin-2-ylamino)-1*H*-indazol-1-yl]pyridin-2-yl}-2,5-diazabicyclo[2.2.1]heptane-2-carboxylate (exemple 12d) (50mg, 0.10mmol) and acetaldehyde (1.5mg, 0.21mmol) in methanol (2mL) was added acetic acid (12µL, 0.21mmol) and the reaction was stirred at room temperature overnight under nitrogen atmosphere.Then sodium cianoborohydride (50 mg) was added and stirred at room temperature for 1 hour. The crude was evaporated and extracted with ethyl acetate and water. The organic extract was dried (MgSO₄) and concentrated in vacuum. Purification was achieved using reverse phase as described in the general methods (15mg, 58%).

LRMS (m/z): 514 (M+1)⁺.

### b) tert-Bbutyl (1S,4S)-5-{4-[6-[acetyl(methyl)amino]-3-(pyrazin-2-ylamino)-1H-indazol-1-yl]pyridin-2-yl}-2,5-diazabicyclo[2.2.1]heptane-2-carboxylate

To a solution of *tert*-butyl (1*S*,4*S*)-5-{4-[6-(methylamino)-3-(pyrazin-2-ylamino)-1*H-*indazol-1-yl]pyridin-2-yl}-2,5-diazabicyclo[2.2.1]heptane-2-carboxylate (exemple 28a) (15 mg, 0.03mmol) in pyridine (1 mL) was added acetic anhydride (18 µL, 0.18 mmol). The mixture was heated at 60ºC for 1 hour. The solvent was evaporated and after addition of water (5 mL) and ethyl acetate (5 mL), the organic phase was washed with water, dried (MgSO₄) and concentrated under vacuum to provide the title compound (11 mg, 61%).

LRMS (m/z): 556 (M+1)⁺.

### c) N-[1-{2-[(1S,4S)-2,5-Diazabicyclo[2.2.1]hept-2-yl]pyridin-4-yl}-3-(pyrazin-2-ylamino)-1H-indazol-6-yl]-N-methylacetamide

Obtained as a hydrochloride salt (63% yield) from *tert*-butyl (1*S*,4*S*)-5-{4-[6-[acetyl(methyl)amino]-3-(pyrazin-2-ylamino)-1*H*-indazol-1-yl]pyridin-2-yl}-2,5-diazabicyclo[2.2.1]heptane-2-carboxylate (example 28b) using the same experimental procedure as described in example 2c.

LRMS (m/z): 456(M+1)⁺.

¹H NMR (400 MHz, DMSO-*d*₆) δ ppm 1.84 - 1.96 (m, 2 H), 1.91 (br. s., 2 H), 2.09 (d, *J*=10.55 Hz, 1 H), 2.24 (d, *J*=10.94 Hz, 1 H), 3.30 (br. s., 3 H), 3.85 (d, *J*=10.55 Hz, 1 H), 3.93 (d, *J*=5.86 Hz, 1 H), 4.63 (br. s., 1 H), 5.24 (br. s., 1 H), 7.13 (br. s., 1 H), 7.39 (d, *J*=8.99 Hz, 1 H), 7.50 (br. s., 1 H), 8.11 (s, 0 H), 8.15 (d, *J*=6.64 Hz, 1 H), 8.27 (s, 1 H), 8.34 - 8.48 (m, 2 H), 9.27 (br. s., 1 H), 9.49 (s, 1 H), 9.70 (br. s., 1 H), 10.78 (br. s., 1 H).

### EXAMPLE 29

### N-[1-{2-[(1S,4S)-2,5-Diazabicyclo[2.2.1]hept-2-yl]pyridin-4-yl}-3-(pyrazin-2-ylamino)-1H-indazol-6-yl]methanesulfonamide

Obtained (30% yield) from the title compound of example 12d and methenesulfonylchloride using the experiemtnal procedure of Example 26a.

LRMS (m/z): 578(M+1)⁺.

¹H NMR (400 MHz, dmso) δ 10.09 (s, 1H), 9.28 (s, 1H), 8.25 (s, 1H), 8.16 (m, 3H), 7.84 (s, 1H), 7.13 (d, *J* = 9.1 Hz, 1H), 7.01 (d, *J* = 6.0 Hz, 1H), 6.76 (s, 1H), 4.89 (s, 1H), 4.52 (s, 1H), 3.65 - 3.48 (m, 2H), 3.35 (dd, *J* = 38.9, 9.6 Hz, 2H), 3.07 (m, 1H), 1.94 (s, 2H), 1.39 (s, 9H).

### EXAMPLE 30

### N-[1-{2-[(1S,4S)-2,5-Diazabicyclo[2.2.1]hept-2-yl]pyridin-4-yl}-3-(pyrazin-2-ylamino)-1H-indazol-6-yl]benzenesulfonamide

### a) tert-Butyl(1S,4S)-5-{4-[6-[(phenylsulfonyl)amino]-3-(pyrazin-2-ylamino)-1H-indazol-1-yl]pyridin-2-yl}-2,5-diazabicyclo[2.2.1]heptane-2-carboxylate

Obtained (30% yield) from the title compound of example 12d and benzenensulfonyl chloride using the experiemtnal procedure of Example 26a.

LRMS (m/z): 640(M+1)⁺.

### b)N-[1-{2-[(1S,4S)-2,5-Diazabicyclo[2.2.1]hept-2-yl]pyridin-4-yl}-3-(pyrazin-2-ylamino)-1H-indazol-6-yl]benzenesulfonamide

Obtained (80% yield) from the title compound of Example 30a following the experimental procedure described in Example 13b.

LRMS (m/z): 540 (M+1)⁺.

¹H NMR (400 MHz, dmso) δ 10.98 (s, 1H), 10.63 (s, 1H), 9.59 (s, 1H), 9.43 (s, 1H), 9.10 (s, 1H), 8.35 (s, 1H), 8.27 - 8.13 (m, 2H), 7.87 (dd, *J* = 14.1, 6.9 Hz, 2H), 7.59 (dt, *J* = 14.7, 7.1 Hz, 2H), 7.26 (s, 1H), 7.12 (d, *J* = 9.1 Hz, 1H), 6.99 (s, 1H), 5.18 (s, 1H), 4.65 (s, 1H), 3.84 (s, 2H), 3.36 (s, 2H), 2.27 (d, *J* = 11.2 Hz, 1H), 2.09 (d, *J* = 11.2 Hz, 1H).

### EXAMPLE 31

### 1-{2-[(1S,4S)-2,5-Diazabicyclo[2.2.1]hept-2-yl]pyridin-4-yl}-6-methoxy-N-pyrazin-2-yl-1H-indazol-3-amine

### a) tert-Butyl (1S,4S)-5-[4-(3-amino-6-methoxy-1H-indazol-1-yl)pyridin-2-yl]-2,5 diazabicyclo[2.2.1]heptane-2-carboxylate

Obtained (51%) from 6-methoxy-1*H*-indazol-3-amine (preparation 8) and (*1S,4S*)*-tert-*butyl 5-(4-bromopyridin-2-yl)-2,5-diazabicyclo[2.2.1]heptane-2-carboxylate (preparation 3) using the same experimental procedure as described in example 2a.

LRMS (m/z): 431 (M+1)⁺.

### b) tert-Butyl (1S,4S)-5-{4-[6-methoxy-3-(pyrazin-2-ylamino)-1H-indazol-1-yl]pyridin-2-yl}-2,5-diazabicyclo[2.2.1]heptane-2-carboxylate

Obtained (70%) from *tert*-butyl (1*S*,4*S*)-5-[4-(3-amino-6-methoxy-1*H*-indazol-1-yl)pyridin-2-yl]-2,5 diazabicyclo[2.2.1]heptane-2-carboxylate (example 31a) and 2-chloropyrazine using the same experimental procedure as described in example 2b, followed by purification by reverse phase chromatography as described in the general methods.

LRMS (m/z): 515 (M+1)⁺.

### c)1-{2-[(1S,4S)-2,5-Diazabicyclo[2.2.1]hept-2-yl]pyridin-4-yl}-6-methoxy-N-pyrazin-2-yl-1H-indazol-3-amine

Obtained as a hydrochoride salt (78%) from *tert*-butyl (1*S*,4*S*)-5-{4-[6-methoxy-3-(pyrazin-2-ylamino)-1*H*-indazol-1-yl]pyridin-2-yl}-2,5-diazabicyclo[2.2.1]heptane-2-carboxylate (example 31b) using the same experimental procedure as described in example 7c.

LRMS (m/z): 415 (M+1)⁺.

¹H NMR (400 MHz, DMSO-D6) δ ppm 2.1 (d, *J*=10.9 Hz, 1 H), 2.3 (d, *J*=10.9 Hz, 1 H), 3.4 (m, 1 H), 3.5 (m, 1 H), 3.9 (m, *J*=10.6 Hz, 3 H), 4.1 (m, 2 H), 4.7 (s, 1 H), 5.3 (dd, 1 H), 7.1 (dd, *J*=9.0, 2.0 Hz, 1 H), 7.6 (m, 2 H), 8.1 (d, *J*=7.0 Hz, 1 H), 8.3 (d, *J*=4.7 Hz, 2 H), 8.3 (s, 1 H), 8.4 (s, 1 H), 9.5 (s, 2 H), 9.9 (s, 1 H), 10.7 (s, 1 H).

### EXAMPLE 32

### 1-{2-[(1S,4S)-2,5-Diazabicyclo[2.2.1]hept-2-yl]pyridin-4-yl}-6-ethoxy-N-pyrazin-2-yl-1H-indazol-3-amine

### a) 4-ethoxy-2-fluorobenzonitrile

Obtained (99%) from 2-fluoro-4-hydroxybenzonitrile and iodoethane following the experimental procedure as described in preparation 18a.

1H NMR (400 MHz, CHLOROFORM-*d*) δ ppm 1.45 (t, *J*=6.84 Hz, 3 H) 4.08 (q, *J*=7.03 Hz, 2 H) 6.69 (dd, *J*=11.33, 2.34 Hz, 1 H) 6.75 (dd, *J*=8.60, 2.34 Hz, 1 H) 7.50 (dd, *J*=8.60, 7.42 Hz, 1 H).

### b) 6-ethoxy-1H-indazol-3-amine

Obtained (59%) from 4-ethoxy-2-fluorobenzonitrile (example 32a) and hydrazine following the experimental procedure as described in preparation 18b.

LRMS (m/z): 178 (M+1)⁺.

### c) tert-butyl (1S,4S)-5-[4-(3-amino-6-ethoxy-1H-indazol-1-yl)pyridin-2-yl]-2,5-diazabicyclo[2.2.1]heptane-2-carboxylate

Obtained (59%) from 6-ethoxy-1*H*-indazol-3-amine (example 32b) and (*1S,4S*)*-tert-*Butyl 5-(4-bromopyridin-2-yl)-2,5-diazabicyclo[2.2.1]heptane-2-carboxylate (preparation 3) following the experimental procedure as described in example 2a, followed by purification by reverse phase chromatography as described in the general methods.

LRMS (m/z): 451 (M+1)⁺.

### d) tert-butyl (1S,4S)-5-{4-[6-ethoxy-3-(pyrazin-2-ylamino)-1H-indazol-1-yl]pyridin-2-yl}-2,5-diazabicyclo[2.2.1]heptane-2-carboxylate

Obtained (57%) from *tert*-butyl (1*S*,4*S*)-5-[4-(3-amino-6-ethoxy-1*H*-indazol-1-yl)pyridin-2-yl]-2,5-diazabicyclo[2.2.1]heptane-2-carboxylate (example 32c) and 2-chloropyrazine using the same experimental procedure as described in example 2b, followed by purification by flash chromatography (hexane/ethyl acetate).

LRMS (m/z): 529 (M+1)⁺.

### e) 1-{2-[(1S,4S)-2,5-Diazabicyclo[2.2.1]hept-2-yl]pyridin-4-yl}-6-ethoxy-N-pyrazin-2-yl-1H-indazol-3-amine

Obtained as a hydrochoride salt (76%) from *tert*-butyl (1*S*,4*S*)-5-{4-[6-ethoxy-3-(pyrazin-2-ylamino)-1*H*-indazol-1-yl]pyridin-2-yl}-2,5-diazabicyclo[2.2.1]heptane-2-carboxylate (example 32d) using the same experimental procedure as described in example 7c.

LRMS (m/z): 429 (M+1)⁺.

¹H NMR (400 MHz, DMSO-*d*₆) δ ppm 1.41 (t, *J*=6.45 Hz, 3 H), 2.12 (d, *J*=10.30 Hz, 1 H), 2.25 (d, *J*=10.30 Hz, 1 H), 3.29 - 3.39 (m, 1 H), 3.40 - 3.52 (m, 1 H), 4.05 (d, *J*=9.77 Hz, 2 H), 4.25 (d, *J*=6.45 Hz, 2 H), 4.66 (s, 1 H), 5.30 (s, 1 H), 7.06 (d, *J*=9.38 Hz, 1 H), 7.20 (br. s., 1 H), 7.50 (s, 1 H), 7.55 (d, *J*=6.30 Hz, 1 H), 8.11 (d, *J*=6.30 Hz, 1 H), 8.24 - 8.33 (m, 2 H), 8.39 (s, 1 H), 9.44 - 9.58 (m, 2 H), 9.87 (br. s., 1 H), 10.71 (s, 1 H).

### EXAMPLE 33

### 4-{4-[3-(Pyrazin-2-ylamino)-1H-indazol-1-yl]pyridin-2-yl}piperidine-4-carbonitrile

### a) tert-Butyl 4-[4-(3-chloro-1H-indazol-1-yl)pyridin-2-yl]-4-cyanopiperidine-1-carboxylate

Obtained (54%) from 3-chloro-1*H*-indazole and *tert*-butyl 4-(4-bromopyridin-2-yl)-4-cyanopiperidine-1-carboxylate (preparation 4) following the experimental procedure as described in example 1a, followed by purification by flash chromatography (9:1 hexanes/ethyl acetate to 1:4 hexanes/ethyl acetate).

LRMS (m/z): 438 (M+1)⁺.

### b) tert-Butyl 4-cyano-4-{4-[3-(pyrazin-2-ylamino)-1H-indazol-1-yl]pyridin-2-yl}piperidine-1-carboxylate

Obtained (40%) from *tert-*butyl 4-[4-(3-chloro-1*H*-indazol-1-yl)pyridin-2-yl]-4-cyanopiperidine-1-carboxylate and 2-aminopyrazine following the experimental procedure as described in example 1b, followed by reverse phase chromatography (C-18 silica from Waters©, water/methanol [0.1% v/v formic acid buffered] 0% to 100%).

LRMS (m/z): 497 (M+1)⁺.

### c) 4-{4-[3-(Pyrazin-2-ylamino)-1H-indazol-1-yl]pyridin-2-yl}piperidine-4-carbonitrile

Obtained as a solid (98%) from tert-butyl 4-cyano-4-(4-[3-(pyrazin-2-ylamino)-1*H-*indazol-1-yl]pyridin-2-yl}piperidine-1-carboxylate following the experimental procedure described in example 1c.

LRMS (m/z): 397 (M+1)⁺.

¹H-NMR (400 MHz, DMSO-*d*₆) δ ppm 2.25 (brs, 4 H), 2.98 (bs, 2 H), 3.27 (d, *J*= 12.4 Hz, 2 H), 7.35 (t, *J*= 7.6 Hz, 1 H), 7.64 (t, *J*= 7.8 Hz, 1 H), 7.89 (dd, *J*= 5.6, 2 Hz 1 H), 8.00 (d, *J*= 2 Hz, 1 H), 8.09 (d, *J*= 8.8 Hz, 1 H), 8.22 (d, *J*= 2.4 Hz, 1 H), 8.34 (m, 2 H), 8.69 (d, *J*= 5.6 Hz, 1 H), 9.47 (d, *J*= 1.2 Hz, 1 H), 10.58 (s, 1 H).

### EXAMPLE 34

### 1-{2-[(2S,5R)-2,5-dimethylpiperazin-1-yl]pyridin-4-yl}-N-pyrazin-2-yl-1H-indazol-3-amine

Obtained as an oil from 1-(2-fluoropyridin-4-yl)-*N*-(pyrazin-2-yl)-1*H*-indazol-3-amine (preparation 9) and (2*S*,5*R*)-2,5-dimethylpiperazine using the same experimental procedure as described in preparation 2, followed by purification by reverse phase chromatography as described in the general method. The oil obtained was dissolved in a dioxane solution of hydrochloric acid and stirred at room temperature overnight. The hydrochloride salt obtained was filtered off and dried in a vacuum oven to give the title compound (10% yield) as a yellow solid (racemic mixture).

LRMS (m/z): 401 (M+1)⁺.

1H NMR (400 MHz, DMSO-*d*₆) d ppm 3.21 (d, *J*=12.30 Hz, 1 H) 3.29 - 3.43 (m, 1 H) 3.46 - 3.58 (m, 1 H) 3.79 - 3.87 (d, *J*=12.30 Hz, 1 H) 4.21 - 4.31 (m, *J*=12.90 Hz, 1 H) 4.76 (s, 1 H) 7.37 (s, 1 H) 7.45 (t, *J*=7.42 Hz, 1 H) 7.62 (d, *J*=4.69 Hz, 1 H) 7.72 (t, *J*=7.42 Hz, 1 H) 8.11 - 8.22 (m, 2 H) 8.31 (s, 1 H) 8.39 - 8.49 (m, 2 H) 9.55 (s, 1 H) 9.79 (br. s., 1 H) 10.01 (br. s., 1 H) 10.85 (s, 1 H).

### EXAMPLE 35

### (1S,2R)-N-{4-[3-(pyrazin-2-ylamino)-1H-indazol-1-yl]pyridin-2-yl}cyclohexane-1,2-diamine

Obtained (40%) from 1-(2-fluoropyridin-4-yl)-*N*-(pyrazin-2-yl)-1*H*-indazol-3-amine (preparation 9) and (1*R*,2*S*)-cyclohexane-1,2-diamine using the same experimental procedure as described in preparation 2, followed by purification by reverse phase chromatography as described in the general method.

LRMS (m/z): 401 (M+1)⁺.

¹H NMR (400 MHz, dmso) δ 9.49 (s, 1H), 8.40 (s, 2H), 8.37 - 8.27 (m, 3H), 8.21 (d, *J* = 2.5 Hz, 1H), 8.09 (d, *J* = 5.8 Hz, 1H), 8.03 (d, *J* = 8.6 Hz, 1H), 7.60 (ddd, *J* = 8.4, 7.0, 1.1 Hz, 1H), 7.37 - 7.26 (m, 1H), 7.16 (dd, *J* = 10.5, 4.7 Hz, 2H), 7.07 (dd, *J* = 5.8, 2.0 Hz, 1H), 4.37 - 4.25 (m, 1H), 3.34 (s, 1H), 1.81 (d, *J* = 9.3 Hz, 2H), 1.72 - 1.54 (m, 4H), 1.46-1.31 (m, 2H).

### EXAMPLE 36

### (1R,2R)-N-{4-[3-(pyrazin-2-ylamino)-1H-indazol-1-yl]pyridin-2-yl}cyclohexane-1,2-diamine

Obtained as a formate salt (18% yield) 1-(2-fluoropyridin-4-yl)-*N*-(pyrazin-2-yl)-1*H-*indazol-3-amine (preparation 9) and (1*R*,2*R*)-cyclohexane-1,2-diamine using the same experimental procedure as described in preparation 2, followed by purification by reverse phase chromatography as described in the general methods.

LRMS (m/z): 401 (M+1)⁺.

¹H NMR (400 MHz, DMSO-D6) δ ppm 1.3 (m, 4 H), 1.7 (d, *J*=2.0 Hz, 2 H), 2.0 (m, 2 H), 2.8 (m, 1 H), 3.8 (m, *J*=9.4 Hz, 1 H), 7.0 (d, *J*=8.2 Hz, 1 H), 7.0 (d, *J*=1.6 Hz, 1 H), 7.1 (dd, *J*=5.5, 2.0 Hz, 1 H), 7.3 (t, 1 H), 7.6 (t, 1 H), 8.0 (d, *J*=8.6 Hz, 1 H), 8.1 (d, *J*=5.9 Hz, 1 H), 8.2 (d, *J*=2.7 Hz, 1 H), 8.3 (d, *J*=7.8 Hz, 1 H), 8.3 (m, *J*=1.6 Hz, 1 H), 9.5 (s, 1 H), 10.5 (s, 1 H).

### EXAMPLE 37

### 1-{4-[3-(pyrazin-2-ylamino)-1H-indazol-1-yl]pyridin-2-yl}piperidine-4-carboxamide

Obtained (26%) from piperidine-4-carboxamide and 1-(2-fluoropyridin-4-yl)-*N*-(pyrazin-2-yl)-1*H*-indazol-3-amine (preparation 9) following the experimental procedure as described in preparation 2, followed by purification by reverse phase chromatography as described in the general methods.

LRMS (m/z): 415 (M+1)⁺.

1H NMR (400 MHz, DMSO-*d*₆) δ ppm 1.52 - 1.64 (m, 2 H) 1.76 - 1.84 (m, 2 H) 2.32 - 2.44 (m, 1 H) 2.86 - 2.97 (m, 2 H) 4.38 (d, *J*=13.29 Hz, 2 H) 6.80 (br. s., 1 H) 7.09 - 7.15 (m, 2 H) 7.27 - 7.33 (m, 2 H) 7.56 - 7.64 (m, 1 H) 8.00 (d, *J*=8.60 Hz, 1 H) 8.20 (d, *J*=2.34 Hz, 1 H) 8.23 (d, *J*=5.47 Hz, 1 H) 8.28 (d, *J*=8.21 Hz, 1 H) 8.33 (dd, *J*=2.34, 1.56 Hz, 1 H) 9.44 (d, *J*=1.56 Hz, 1 H) 10.48 (s, 1 H)

### EXAMPLE 38

### 1-{4-[3-(Pyrazin-2-ylamino)-1H-indazol-1-yl]pyridin-2-yl}piperidin-4-ol

### a) 1-[4-(3-chloro-1H-indazol-1-yl)pyridin-2-yl]piperidin-4-ol

Obtained (66%) from 3-Chloro-1-(2-fluoropyridin-4-yl)-1*H*-indazole (preparation 9a) and piperidin-4-ol using the same experimental procedure as described in preparation 2, followed by purification by reverse phase chromatography as described in the general method.

LRMS (m/z): 330 (M+1)⁺.

### b) 1-{4-[3-(Pyrazin-2-ylamino)-1H-indazol-1-yl]pyridin-2-yl}piperidin-4-ol

Obtained (20.4%) from 1-[4-(3-chloro-1*H*-indazol-1-yl)pyridin-2-yl]piperidin-4-ol (example 38a) and pyrazine-2-amine using the same experimental procedure as described in example 5b, followed by purification by flash chromatography (dichloromethane/methanol).

LRMS (m/z): 388 (M+1)⁺.

1H NMR (400 MHz, DMSO-*d*₆) d ppm 1.36 - 1.48 (m, 2 H) 1.79 - 1.88 (m, 2 H) 3.13 - 3.23 (m, 2 H) 3.69 - 3.78 (m, 1 H) 4.07 (t, *J*=4.40 Hz, 1 H) 4.10 (t, *J*=4.40 Hz, 1 H) 4.71 (d, *J*=4.30 Hz, 1 H) 7.09 - 7.13 (m, 2 H) 7.27 - 7.33 (m, 1 H) 7.59 (t, *J*=8.21 Hz, 1 H) 7.99 (d, *J*=8.60 Hz, 1 H) 8.20 (d, *J*=2.34 Hz, 1 H) 8.22 (d, *J*=6.25 Hz, 1 H) 8.27 (d, *J*=8.21 Hz, 1 H) 8.32 (dd, *J*=2.74, 1.56 Hz, 1 H) 9.43 (d, *J*=1.56 Hz, 1 H) 10.46 (s, 1 H)

### EXAMPLE 39

### trans-4-({4-[3-(Pyrazin-2-ylamino)-1H-indazol-1-yl]pyridin-2-yl}amino)cyclohexanol

### a) trans-4-{[4-(3-chloro-1H-indazol-1-yl)pyridin-2-yl]amino}cyclohexanol

Obtained (71 %) from 3-Chloro-1-(2-fluoropyridin-4-yl)-1*H*-indazole (preparation 9a) and *trans*-4-aminocyclohexanol using the same experimental procedure as described in preparation 2.

LRMS (m/z): 344 (M+1)⁺.

### b) trans-4-({4-[3-(Pyrazin-2-ylamino)-1H-indazol-1-yl]pyridin-2-yl}amino)cyclohexanol

Obtained (19%) from *trans*-4-{[4-(3-chloro-1*H*-indazol-1-yl)pyridin-2-yl]amino}cyclohexanol (example 39a) and pyrazine-2-amine using the same experimental procedure as described in example 5b, followed by purification by reverse phase chromatography as described in the general method.

LRMS (m/z): 402 (M+1)⁺.

¹H NMR (400 MHz, DMSO-*d*₆) δ ppm 1.20 - 1.37 (m, 4 H), 1.81 - 1.92 (m, 2 H), 1.96 - 2.04 (m, 2 H), 3.53 - 3.62 (m, 2 H), 3.73 - 3.85 (m, 2 H), 6.46 (br. s., 1 H), 6.93 (d, *J*=1.95 Hz, 1 H), 6.97 (dd, *J*=5.47, 1.95 Hz, 1 H), 7.25 - 7.32 (m, 1 H), 7.54 - 7.61 (m, 1 H), 7.97 (d, *J*=8.60 Hz, 1 H), 8.06 (d, *J*=5.86 Hz, 1 H), 8.18 (d, *J*=2.34 Hz, 1 H), 8.25 (d, *J*=8.21 Hz, 1 H), 8.28 - 8.31 (m, 1 H), 9.40 (br. s., 1 H).

### EXAMPLE 40

### (4r,7r)-4-({4-[3-(Pyrazin-2-ylamino)-1H-indazol-1-yl]pyridin-2-yl}amino)adamantan-1-ol

### a) (4R,7R)-4-{[4-(3-chloro-1H-indazol-1-yl)pyridin-2-yl]amino}adamantan-1-ol

Obtained (12.7%) from 3-Chloro-1-(2-fluoropyridin-4-yl)-1*H*-indazole (preparation 9a) and (4*R*,7*R*)-4-aminoadamantan-1-ol using the same experimental procedure as described in preparation 2, followed by purification by reverse phase chromatography as described in the general method.

LRMS (m/z): 395 (M+1)⁺.

### b) (4R,7R)-4-({4-[3-(Pyrazin-2-ylamino)-1H-indazol-1-yl]pyridin-2-yl}amino)adamantan-1-ol

Obtained (8.7%) from (4*R*,7*R*)-4-{[4-(3-chloro-1*H*-indazol-1-yl)pyridin-2-yl]amino}adamantan-1-ol (example 40a) and pyrazine-2-amine using the same experimental procedure as described in example 5b, followed by purification by reverse phase chromatography as described in the general method.

LRMS (m/z): 454 (M+1)⁺.

### EXAMPLE 41

### 1-(2-Methyl-6-piperazin-1-ylpyrimidin-4-yl)-N-pyrazin-2-yl-1H-indazol-3-amine

### a) tert-Butyl 4-[6-(3-chloro-1H-indazol-1-yl)-2-methylpyrimidin-4-yl]piperazine-1-carboxylate

Obtained (55%) from 3-chloro-1*H*-indazole and the title compound of Preparation 20 following the experimental procedure described in example 1a, followed by purification by flash chromatography (9.5:0.5 hexanes/ethyl acetate).

LRMS (m/z): 429 (M+1)⁺.

¹H NMR (400 MHz, DMSO-d₆) δ 8.83 (d, *J* = 8.6 Hz, 1H), 7.78 (d, *J* = 8.0 Hz, 1H), 7.67 (t, *J* = 7.5 Hz, 1H), 7.43 (t, *J* = 7.6 Hz, 1H), 6.89 (s, 1H), 3.67 (s, 4H), 3.42 (s, 4H), 2.49 (d, *J* = 9.1 Hz, 3H), 1.41 (s, 9H).

### b) tert-Butyl 4-[2-methyl-6-(3-pyrazin-2-yl-1H-indazol-1-yl)pyrimidin-4-yl]piperazine-1-carboxylate

Obtained (75%) from the title compound of example 41a and 2-aminopyrazine following the experimental procedure as described in example 1b, followed by reverse phase chromatography (C-18 silica from Waters©, water/methanol [0.1% v/v formic acid buffered] 0% to 100%).

LRMS (m/z): 488 (M+1)⁺.

### c) 1-(2-Methyl-6-piperazin-1-ylpyrimidin-4-yl)-N-pyrazin-2-yl-1H-indazol-3-amine

Obtained as a solid (81%) from the title compound of example 41b following the experimental procedure described in example 3c.

LRMS (m/z): 388 (M+1)⁺.

¹H NMR (400 MHz, DMSO-d₆) δ 10.69 (s, 1H), 9.50 (s, 3H), 8.82 (d, *J* = 8.5 Hz, 1H), 8.40 (s, 1H), 8.34 - 8.14 (m, 2H), 7.62 (t, *J* = 7.4 Hz, 1 H), 7.34 (t, *J* = 7.4 Hz, 1H), 6.95 (s, 1H), 3.96 (s, 4H), 3.23 (s, 4H), 2.56 (s, 3H).

### EXAMPLE 42

### 1-{6-[(1S,4S)-2,5-Diazabicyclo[2.2.1]hept-2-yl]-2-methylpyrimidin-4-yl}-N-pyrazin-2-yl-1H-indazol-3-amine

### a) (1S,4S)-tert-Butyl 5-(6-(3-chloro-1H-indazol-1-yl)-2-methylpyrimidin-4-yl)-2,5-diazabicyclo[2.2.1]heptane-2-carboxylate

Obtained (68%) from 3-chloro-1*H*-indazole and the title compound of Preparation 21 following the experimental procedure as described in example 1a, followed by purification by flash chromatography (80:20 hexanes/ethyl acetate).

LRMS (m/z): 441 (M+1)⁺.

### b) (1S,4S)-tert-Butyl 5-(2-methyl-6-(3-(pyrazin-2-ylamino)-1H-indazol-1-yl)pyrimidin-4-yl)-2,5-diazabicyclo[2.2.1]heptane-2-carboxylate

Obtained (43%) from the title compound of example 42a and 2-aminopyrazine following the experimental procedure as described in example 1b, followed by reverse phase chromatography (C-18 silica from Waters©, water/methanol [0.1% v/v formic acid buffered] 0% to 100%).

LRMS (m/z): 500 (M+1)⁺.

### c) 1-{6-[(1S,4S)-2,5-Diazabicyc)o[2.2.1]hept-2-yl]-2-methylpyrimidin-4-yl}-N-pyrazin-2-yl-1H-indazol-3-amine

Obtained as a solid (67%) from the title compound of example 42b following the experimental procedure described in example 3c.

LRMS (m/z): 400 (M+1)⁺.

¹H NMR (400 MHz, DMSO-d₆) δ 10.53 (s, 1H), 9.78 (s, 1H), 9.44 (s, 1H), 9.18 (s, 1H), 8.81 (m, 1H), 8.35 (s, 1H), 8.25 (m, 2H), 7.62 (s, 1H), 7.33 (s, 1H), 6.63 (s, 1H), 5.15 (s, 1H), 4.54 (s, 2H), 3.75 (d, *J* = 21.9 Hz, 2H), 3.31 (d, *J* = 32.8 Hz, 2H), 2.56 (s, 3H), 2.13 (s, 1H),2.02(s, 1H).

### EXAMPLE 43

### 1-[2-(Methoxymethyl)-6-piperazin-1-ylpyrimidin-4-yl]-N-pyrazin-2-yl-1H-indazol-3-amine

### a) 2-(Methoxymethyl)pyrimidine-4,6-diol

A suspension of diethyl malonate (5 g, 31.22mmol) and 2-methoxyethanimidamide (2.75 g, 31.2 mmol) in methanol (50 mL) and sodium methoxide (14 g, 64.8 mmol) was slowly added and heated at 80ºC overnight.

The reaction mixture was concentrated in vaccum and the solid was washed with etyl acetate and methanol. The filtrates were concentrated to give the title compound (5.2 g, 85%)

LRMS (m/z): 157 (M+1)⁺.

### b) 4,6-Dichloro-2-(methoxymethyl)pyrimidine

Phosphorous(V)oxychloride (5 mL, 52.2 mmol) was added to 2-(methoxymethyl) pyrimidine-4,6-diol (example 43a) and heated at 85ºC for 2 h. The mixture was concentrated under vaccum and ethyl acetate and water were added. The organic layer was washed with brine, dried MgSO₄ and evaporated.

The crude was purified by flash chromatography (hexanes/ethyl acetate) to provide the title compound (2.55 g, 40%) as a yelow oil.

LRMS (m/z): 194 (M+1)⁺.

### c) tert-Butyl 4-[6-chloro-2-(methoxymethyl)pyrimidin-4-yl]piperazine-1-carboxylate

Triethylamine (0.9 mL, 6.5 mmol) was added to a solution of 4,6-dichloro-2-(methoxymethyl)pyrimidine (example 43b 1 g, 5.2 mmol) and tert-butyl piperazine-1-carboxylate (0.98 g, 5.26 mmol) in THF (20 mL). The resulting mixture was stirred at room temperature for 24h. The crude was evaporated and dichoromethane and water were added. The organic layer was washed with brine, dried and concentrated to provide the title compound (1.94 g, 97%) as a colorless oil.

LRMS (m/z): 343 (M+1)⁺.

### d) tert-Butyl 4-[6-(3-chloro-1H-indazol-1-yl)-2-(methoxymethyl)pyrimidin-4-yl]piperazine-1-carboxylate

Obtained (42%) from *tert*-butyl 4-[6-chloro-2-(methoxymethyl)pyrimidin-4-yl]piperazine-1-carboxylate (example 43c) and 3-chloro-1*H*-indazole following the experimental procedure described in example 1a, followed by purification by flash chromatography (hexane/ethyl acetate).

LRMS (m/z): 459 (M+1)⁺.

### e) tert-Butyl 4-{2-(methoxymethyl)-6-[3-(pyrazin-2-ylamino)-1H-indazol-1-yl]pyrimidin-4-yl}piperazine-1-carboxylate

Obtained (49%) from *tert-*butyl 4-[6-(3-chloro-1*H*-indazol-1-yl)-2-(methoxymethyl)pyrimidin-4-yl]piperazine-1-carboxylate (example 43d) and aminopyrazine following a experimental procedure as described in example 5b, followed by purification by flash chromatography (hexane/ ethyl acetate).

LRMS (m/z): 518 (M+1)⁺.

### f) 1-[2-(Methoxymethyl)-6-piperazin-1-ylpyrimidin-4-yl]-N-pyrazin-2-yl-1H-indazol-3-amine

Obtained as a solid (72%) from *tert*-butyl 4-12-(methoxymethyl)-6-[3-(pyrazin-2-ylamino)-1*H*-indazol-1-yl]pyrimidin-4-yl}piperazine-1-carboxylate (example 43e) following the experimental procedure described in example 3c. The hydrochloride salt obtained was dried in vaccum oven to give the title compound.

LRMS (m/z): 418 (M+1)⁺.

¹H NMR (400 MHz, DMSO-D6) δ ppm 3.2 (m, 4 H), 3.5 (m, 3 H), 3.9 (m, *J*=3.9 Hz, 4 H), 4.5 (s, 2 H), 7.0 (s, 1 H), 7.3 (t, *J*=7.4 Hz, 1 H), 7.6 (t, *J*= 7.6 Hz, 1 H), 8.3 (m, 2 H), 8.4 (s, 1 H), 8.9 (d, *J*=8.6 Hz, 1 H), 9.3 (s, 2 H), 9.5 (s, 1 H), 10.6 (s, 1 H).

### EXAMPLE 44

### 1-{2-[(1S,4S)-2,5-Diazabicyclo[2.2.1]hept-2-yl]pyridin-4-yl}-N-pyrazin-2-yl-1H-pyrazolo[3,4-c]pyridin-3-amine

### a) tert-Butyl (1S,4S)-5-[4-(3-bromo-1H-pyrazolo[3,4-c]pyridin-1-yl)pyridin-2-yl]-2,5-diazabicyclo[2.2.1]heptane-2-carboxylate

Obtained (61%) from 3-bromo-1*H*-pyrazolo[3,4-c]pyridine and (1*S,*4*S*)-*tert*-butyl 5-(4-bromopyridin-2-yl)-2,5-diazabicyclo[2.2.1]heptane-2-carboxylate (preparation 3) following the experimental procedure as described in example 1a, followed by purification by flash chromatography (9:1 hexanes/ethyl acetate to 100% ethyl acetate).

LRMS (m/z): 471/473.

### b) tert-Butyl (1S,4S)-5-{4-[3-(pyrazin-2-ylamino)-1H-pyrazolo[3,4-c]pyridin-1-yl]pyridin-2-yl}-2,5-diazabicyclo[2.2.1]heptane-2-carboxylate

Obtained (50%) from *tert*-butyl (1*S*,4*S*)-5-[4-(3-bromo-1*H*-pyrazolo[3,4-*c*]pyridin-1-yl)pyridin-2-yl]-2,5-diazabicyclo[2.2.1]heptane-2-carboxylate (example 44a) and 2-aminopyrazine following a similar experimental procedure as described in example 11c.

LRMS (m/z): 486 (M+1)⁺.

### c) 1-{2-[(1S,4S)-2,5-Diazabicyclo[2.2.1]hept-2-yl]pyridin-4-yl}-N-pyrazin-2-yl-1H-pyrazolo[3,4-c]pyridin-3-amine

Obtained as a solid (92%) from *tert*-butyl (1*S*,4*S*)-5-{4-[3-(pyrazin-2-ylamino)-1*H-*pyrazolo[3,4-*c*]pyridin-1-yl]pyridin-2-yl]-2,5-diazabicyclo[2.2.1]heptane-2-carboxylate following the experimental procedure described in example 1c.

LRMS (m/z): 386 (M+1)⁺.

¹H-NMR (400 MHz, CDCl₃) δ ppm 1.91 (d, *J*=9.60 Hz, 1 H), 2.01 (d, *J*=10.00 Hz, 1 H), 3.15 (s, 2 H), 3.37 (d, *J*=9.20 Hz, 1 H), 3.70 (dd, *J*=9.60, 2.00 Hz, 1 H), 3.96 (s, 1 H), 4.86 (s, 1 H), 6.74 (d, *J*=1.20 Hz, 1 H), 7.05 (dd, *J*=5.60, 2.00 Hz, 1 H), 7.88 (dd, *J*=5.60, 0.80 Hz, 1 H), 8.19-8.24 (m, 3 H), 8.42 (d, *J*=6.0 Hz, 1 H), 9.34 (s, 1 H), 9.44 (d, *J*=1.20 Hz, 1 H).

### EXAMPLE 45

### 1-{2-[(1S,4S)-2,5-Diazabicyclo[2.2.1]hept-2-yl]pyridin-4-yl}-N-pyrazin-2-yl-1H-pyrazolo[4,3-b]pyridin-3-amine

### a) tert-Butyl (1S,4S)-5-[4-(3-bromo-1H-pyrazolo[4,3-b]pyridin-1-yl)pyridin-2-yl]-2,5-diazabicyclo[2.2.1]heptane-2-carboxylate

Obtained (61%) from 3-bromo-1*H*-pyrazolo[4,3-*b*]pyridine and (1*S,*4*S*)-*tert*-butyl 5-(4-bromopyridin-2-yl)-2,5-diazabicyclo[2.2.1]heptane-2-carboxylate (preparation 3) following the experimental procedure as described in example 1a.

LRMS (m/z): 471/473.

### b) tert-Butyl (1S,4S)-5-{4-[3-(pyrazin-2-ylamino)-1H-pyrazolo[4,3-b]pyridin-1-yl]pyridin-2-yl}-2,5-diazabicyclo[2.2.1]heptane-2-carboxylate

Obtained (73%) from *tert*-butyl (1*S*,4*S*)-5-[4-(3-bromo-1*H*-pyrazolo[4,3-*b*]pyridin-1-yl)pyridin-2-yl]-2,5-diazabicyclo[2.2.1]heptane-2-carboxylate (example 45a) and 2-aminopyrazine following a similar experimental procedure as described in example 11c.

LRMS (m/z): 486 (M+1)⁺.

### c) 1-{2-[(1S,4S)-2,5-Diazabicyclo[2.2.1]hept-2-yl]pyridin-4-yl}-N-pyrazin-2-yl-1H-pyrazolo[4,3-b]pyridin-3-amine

Obtained as a solid (20%) from *tert*-butyl (1*S*,4*S*)-5-{4-[3-(pyrazin-2-ylamino)-1*H-*pyrazolo[4,3-*b*]pyridin-1-yl]pyridin-2-yl}-2,5-diazabicyclo[2.2.1]heptane-2-carboxylate following the experimental procedure described in example 1c.

LRMS (m/z): 386 (M+1)+.

¹H-NMR (400 MHz, METHANOL-*d*₄) δ ppm 1.88 (d, *J*=8 Hz, 1 H), 2.00 (d, *J*=8.6 Hz, 1 H), 3.06 (s, 2 H), 3.34 (d, *J*=9.6 Hz, 1 H), 3.62 (dd, *J*=9.6, 2.00 Hz, 1 H,) 3.89 (s,

1 H), 4.75 (s, 1 H), 6.68 (d, *J*=1.60 Hz, 1 H), 6.96 (dd, *J*=5.60, 2.00 Hz, 1 H), 7.46 (dd, *J*=8.8, 4.4 Hz, 1 H), 8.00 (d, *J*= 6 Hz, 1 H), 8.12 (d, *J*=2.4 Hz, 1 H), 8.23 (d, *J*= 1.2 Hz, 1 H), 8.27 (m, 1 H), 8.46 (dd, *J*= 4, 1.2 Hz, 1 H), 9.31 (d, *J*= 1.6 Hz, 1 H).

### EXAMPLE 46

### 1-{2-[(1S,4S)-2,5-Diazabicyclo[2.2.1]hept-2-yl]pyridin-4-yl}-N-pyrazin-2-yl-1H-pyrazolo[4,3-c]pyridin-3-amine

### a) tert-Butyl (1S,4S)-5-[4-(3-bromo-1H-pyrazolo[4,3-c]pyridin-1-yl)pyridin-2-yl]-2,5-diazabicyclo[2.2.1]heptane-2-carboxylate

Obtained (25%) from 3-bromo-1*H*-pyrazolo[4,3-*c*]pyridine and (1*S,*4*S*)*-tert*-butyl 5-(4-bromopyridin-2-yl)-2,5-diazabicyclo[2.2.1]heptane-2-carboxylate (preparation 3) following the experimental procedure as described in example 1a.

LRMS (m/z): 471/473.

### b) tert-Butyl (1S,4S)-5-{4-[3-(pyrazin-2-ylamino)-1H-pyrazolo[4,3-c]pyridin-1-yl]pyridin-2-yl}-2,5-diazabicyclo[2.2.1]heptane-2-carboxylate

Obtained (69%) from *tert*-butyl (1*S*,4*S*)-5-[4-(3-bromo-1*H*-pyrazolo[4,3-*c*]pyridin-1-yl)pyridin-2-yl]-2,5-diazabicyclo[2.2.1]heptane-2-carboxylate (example 46a) and 2-aminopyrazine following a similar experimental procedure as described in example 11c.

LRMS (m/z): 486 (M+1)⁺.

### c) 1-{2-[(1S,4S)-2,5-Diazabicyclo[2.2.1]hept-2-yl]pyridin-4-yl}-N-pyrazin-2-yl-1H-pyrazolo[4,3-c]pyridin-3-amine

Obtained as a solid (41%) from *tert*-butyl (1*S*,4*S*)-5-{4-[3-(pyrazin-2-ylamino)-1*H-*pyrazolo[4,3-*c*]pyridin-1-yl]pyridin-2-yl}-2,5-diazabicyclo[2.2.1]heptane-2-carboxylate following the experimental procedure described in example 1c.

LRMS (m/z): 386 (M+1)+.

¹H-NMR (400 MHz, METHANOL-*d*₆) δ ppm 2.12 (d, *J*=10.8 Hz, 1 H), 2.33 (d, *J*=10.4 Hz, 1 H), 2.65 (s, 2 H), 3.65 (d, *J*=10.8 Hz, 1 H), 3.82 (dd, *J*=11.2, 2.00 Hz, 1 H), 4.58 (s, 1 H), 5.07 (s, 1 H), 6.92 (d, *J*=1.6 Hz, 1 H), 7.20 (dd, *J*=6, 1.6 Hz, 1 H), 7.92 (d, *J*=6 Hz, 1 H), 8.17 (d, *J*= 2.4 Hz, 1 H), 8.21 (d, *J*=5.6 Hz, 1 H), 8.33 (br s, 2 H), 8.48 (d, *J*=5.6 Hz, 1 H), 9.34 (m, 2 H).

### EXAMPLE 47

### 1-{2-[(1S,4S)-2,5-Diazabicyclo[2.2.1]hept-2-yl]pyridin-4-yl}-N-(4-methylpyridin-2-yl)-1H-pyrazolo[3,4-b]pyridin-3-amine

### a) tert-Butyl (1S,4S)-5-[4-(3-amino-1H-pyrazolo[3,4-b]pyridin-1-yl)pyridin-2-yl]-2,5-diazabicyclo[2.2.1]heptane-2-carboxylate

Obtained (26%) from 1H-pyrazolo[3,4-*b*]pyridin-3-amine and *(*1*S,*4*S*)*-tert*-butyl 5-(4-bromopyridin-2-yl)-2,5-diazabicyclo[2.2.1]heptane-2-carboxylate (preparation 3) following the experimental procedure as described in example 1a.

LRMS (m/z): 408 (M+H)⁺.

### b) tert-Butyl (1S,4S)-5-(4-{3-[(4-methylpyridin-2-yl)amino]-1H-pyrazolo[3,4-b]pyridin-1-yl}pyridin-2-yl)-2,5-diazabicyclo[2.2.1]heptane-2-carboxylate

Obtained (41%) from tert-butyl (1*S*,4*S*)-5-[4-(3-amino-1*H*-pyrazolo[3,4-*b*]pyridin-1-yl)pyridin-2-yl]-2,5-diazabicyclo[2.2.1]heptane-2-carboxylate (example 47a) and 2-bromo-4-methylpyridine following the experimental procedure described in example 5b.

LRMS (m/z): 499 (M+1)⁺.

### c) 1-{2-[(1S,4S)-2,5-Diazabicyclo[2.2.1]hept-2-yl]pyridin-4-yl}-N-(4-methylpyridin-2-yl)-1H-pyrazolo[3,4-b]pyridin-3-amine

Obtained as a solid (78%) from *tert*-butyl (1*S*,4*S*)-5-(4-{3-[(4-methylpyridin-2-yl)amino]-1*H*-pyrazolo[3,4-*b*]pyridin-1-yl]pyridin-2-yl)-2,5-diazabicyclo[2.2.1]heptane-2-carboxylate following the experimental procedure described in example 1c.

LRMS (m/z): 399 (M+1)⁺.

¹H-NMR (400 MHz, CDCl₃) δ ppm 1.89 (d, *J*=9.60 Hz, 1 H), 1.97 (d, *J*=9.20 Hz, 1 H), 2.43 (s, 3 H), 3.17 (d, *J*=10.00 Hz, 2 H), 3.37 (d, *J*=9.20 Hz, 1 H), 3.74 (d, *J*=8.40 Hz, 1 H), 3.89 (s, 1 H), 4.25 (br s, 1 H), 4.85 (s, 1 H), 6.78 (d, *J*=4.8 Hz, 1 H), 7.20 (dd, *J*=8.00, 4.80 Hz, 1 H), 7.52 (s, 1 H), 7.72 (dd, *J*=5.60, 1.20 Hz, 1 H), 7.98 (s, 1 H), 8.10-8.14 (m, 2 H), 8.22 (d, *J*=5.60 Hz, 1 H), 8.64 (dd, *J*=4.00, 0.8 Hz, 1 H).

### EXAMPLE 48

### 1-{2-[(1S,4S)-2,5-Diazabicyclo[2.2.1]hept-2-yl]pyridin-4-yl}-N-[4-(trifluoromethyl) pyridin-2-yl]-1H-pyrazolo[3,4-b]pyridin-3-amine

### a) tert-Butyl (1S,4S)-5-[4-(3-{[4-(trifluoromethyl)pyridin-2-yl]amino}-1H-pyrazolo[3,4-c]pyridin-1-yl)pyridin-2-yl]-2,5-diazabicyclo[2.2.1]heptane-2-carboxylate

Obtained (41%) from *tert*-butyl (1*S*,4*S*)-5-[4-(3-amino-1*H*-pyrazolo[3,4-*b*]pyridin-1-yl)pyridin-2-yl]-2,5-diazabicyclo[2.2.1]heptane-2-carboxylate (example 47a) and 2-bromo-4-trifluoromethylpyridine following the experimental procedure described in example 5b.

LRMS (m/z): 553 (M+1)⁺.

### b) 1-{2-[(1S,4S)-2,5-Diazabicyclo[2.2.1]hept-2-yl]pyridin-4-yl}-N-[4-(trifluoromethyl)pyridin-2-yl]-1H-pyrazolo[3,4-b]pyridin-3-amine

Obtained as a solid (97%) from *tert*-butyl (1*S*,4*S*)-5-[4-(3-{[4-(trifluoromethyl)pyridin-2-yl]amino}-1*H*-pyrazolo[3,4-*c*]pyridin-1-yl)pyridin-2-yl]-2,5-diazabicyclo[2.2.1]heptane-2-carboxylate following the experimental procedure described in example 1c.

LRMS (m/z): 453 (M+1)+.

¹H-NMR (400 MHz, CDCl₃) δ ppm 1.89 (d, *J*=9.60 Hz, 1 H) 1.98 (d, *J*=9.60 Hz, 1 H) 3.17 (q, *J*=10.00 Hz, 2 H) 3.35 (d, *J*=9.60 Hz, 1 H) 3.70 (dd, *J*=9.20, 1.6 Hz, 1 H) 3.91 (s, 1 H) 4.83 (s, 1 H) 7.13 (d, *J*=4.8 Hz, 1 H) 7.20 (dd, *J*=8.00, 4.80 Hz, 1 H) 7.27 (dd, *J*=7.60, 0.80 Hz, 1 H) 7.87 (dd, *J*=5.60, 1.60 Hz, 1 H) 8.08 (dd, *J*=8.40, 1.60 Hz, 1 H) 8.19 (d, *J*=6.00 Hz, 1 H) 8.42 (d, *J*=5.20 Hz, 1 H) 8.57 (s, 1 H) 8.64 (dd, *J*=3.6, 0.8 Hz, 1 H)

### EXAMPLE 49

### 1-{2-[(1S,4S)-2,5-Diazabicyclo[2.2.1]hept-2-yl]pyridin-4-yl)-N-pyrazin-2-yl-1H-pyrazolo[3,4-b]pyridin-3-amine

### a) tert-Butyl (1S,4S)-5-{4-[3-(pyrazin-2-ylamino)-1H-pyrazolo[3,4-b]pyridin-1-yl]pyridin-2-yl}-2,5-diazabicyclo[2.2.1]heptane-2-carboxylate

Obtained (38%) from tert-butyl (1*S*,4*S*)-5-[4-(3-amino-1*H*-pyrazolo[3,4-*b*]pyridin-1-yl)pyridin-2-yl]-2,5-diazabicyclo[2.2.1]heptane-2-carboxylate (example 47a) and 2-chloropyrazine following the experimental procedure described in example 2b.

LRMS (m/z): 486 (M+1)⁺.

### b) 1-{2-[(1S,4S)-2,5-Diazabicyclo[2.2.1]hept-2-yl]pyridin-4-yl}-N-pyrazin-2-yl-1H-pyrazolo[3,4-b]pyridin-3-amine

Obtained as a solid (92%) from tert-butyl (1*S*,4*S*)-5-{4-[3-(pyrazin-2-ylamino)-1*H-*pyrazolo[3,4-b]pyridin-1-yl]pyridin-2-yl}-2,5-diazabicyclo[2.2.1]heptane-2-carboxylate following the experimental procedure described in example 1c.

LRMS (m/z): 386 (M+1)⁺.

¹H-NMR (400 MHz, METHANOL-*d*₆) δ ppm 1.89 (d, *J*=9.60 Hz, 1 H), 2.04 (d, *J*=10.00 Hz, 1 H), 3.10 (s, 2 H), 3.40 (d, *J*=9.6 Hz, 1 H), 3.67 (dd, *J*=9.2, 2.00 Hz, 1 H), 3.94 (s, 1 H), 4.76 (s, 1 H), 7.26 (dd, *J*=8, 4.4 Hz, 1 H), 7.59 (d, *J*= 1.6 Hz, 1 H), 7.70 (dd, *J*=6, 1.6 Hz, 1 H), 8.02 (d, *J*= 6 Hz, 1 H), 8.14 (d, *J*=2.4 Hz, 1 H), 8.30 (m, 1 H), 8.45 (dd, *J*=8.20, 1.6 Hz, 1 H), 8.61 (dd, *J*= 4.4, 1.6 Hz, 1 H), 9,35 (d, *J*= 1.6 Hz, 1 H).

### EXAMPLE 50

### 1-{2-[(2S)-2-methylpiperazin-1-yl]pyridin-4-yl}-N-pyrazin-2-yl-1H-indazol-3-amine

### a) tert-Butyl (3S)-4-[4-(3-amino-1H-indazol-1-yl)pyridin-2-yl]-3-methylpiperazine-1-carboxylate

Obtained (40%) from 1*H*-indazol-3-amine and *tert*-butyl (3*S*)-4-(4-bromopyridin-2-yl)-3-methylpiperazine-1-carboxylate (preparation 10) using the same experimental procedure as described in example 2a.

LRMS (m/z): 409 (M+1)⁺.

### b) tert-Butyl (3S)-3-methyl-4-{4-[3-(pyrazin-2-ylamino)-1H-indazol-1-yl]pyridin-2-yl}piperazine-1-carboxylate

Obtained as a colorless oil (48%) from *tert*-Butyl (3*S*)-4-[4-(3-amino-1*H*-indazol-1-yl)pyridin-2-yl]-3-methylpiperazine-1-carboxylate (example 50a) and 2-chloropyrazine using the same experimental procedure as described in example 2b.

LRMS (m/z): 487 (M+1)⁺.

### c) 1-{2-[(2S)-2-methylpiperazin-1-yl]pyridin-4-yl}-N-pyrazin-2-yl-1H-indazol-3-amine

Obtained as a hydrochloride salt (44%) from *tert*-Butyl (3*S*)-3-methyl-4-{4-[3-(pyrazin-2-ylamino)-1*H*-indazol-1-yl]pyridin-2-yl}piperazine-1-carboxylate
(example 50b) using the same experimental procedure as described in example 2c.

LRMS (m/z): 387 (M+1)⁺.

1H NMR (400 MHz, DMSO-*d*₆) δ ppm 1.47 (d, *J*=7.03 Hz, 3 H) 3.10-3.24 (m, 1 H) 3.30 - 3.45 (m, 3 H) 3.64 (t, *J*=12.50 Hz, 1 H) 4.38 (d, *J*=13.68 Hz, 1 H) 4.89 (s, 1 H) 7.38 (s, 1 H) 7.45 (t, *J*=7.62 Hz, 1 H) 7.61 (d, *J*=6.25 Hz, 1 H) 7.71 (t, *J*=7.82 Hz, 1 H) 8.18 (d, *J*=7.42 Hz, 2 H) 8.29 (d, *J*=1.56 Hz, 1 H) 8.39 - 8.46 (m, 2 H) 9.51 (s, 1 H) 9.54 - 9.62 (m, 1 H) 9.89 - 10.01 (m, 1 H) 10.82 (s, 1 H).

### EXAMPLE 51

### 1-{5-[(1S,4S)-2,5-diazabicyclo[2.2.1]hept-2-yl]pyridin-3-yl)-N-pyrazin-2-yl-1H-indazol-3-amine

### a) tert-Butyl (1S,4S)-5-[5-(3-amino-1H-indazol-1-yl)pyridin-3-yl]-2,5-diazabicyclo[2.2.1]heptane-2-carboxylate

Obtained (65%) from 1*H*-indazol-3-amine and (1*S*,4*S*)-*tert*-butyl 5-(5-bromopyridin-3-yl)-2,5-diazabicyclo[2.2.1]heptane-2-carboxylate (preparation 11) using the same experimental procedure as described in example 2a.

LRMS (m/z): 407 (M+1)⁺.

### b) tert-butyl (1S,4S)-5-{5-[3-(pyrazin-2-ylamino)-1H-indazol-1-yl]pyridin-3-yl}-2,5-diazabicyclo[2.2.1]heptane-2-carboxylate

Obtained as a yellow oil (33% yield) from *tert*-butyl (1*S*,4*S*)-5-[5-(3-amino-1*H*-indazol-1-yl)pyridin-3-yl]-2,5-diazabicyclo[2.2.1]heptane-2-carboxylate (example 51a) and 2-chloropyrazine using the same experimental procedure as described in example 2b.

LRMS (m/z): 485 (M+1)⁺.

### c) 1-{5-[(1S,4S)-2,5-diazabicyclo[2.2.1]hept-2-yl]pyridin-3-yl)-N-pyrazin-2-yl-1H-indazol-3-amine

Obtained as a hydrochloride salt (83% yield) from *tert*-butyl (1*S*,4*S*)-5-{5-[3-(pyrazin-2-ylamino)-1*H*-indazol-1-yl]pyridin-3-yl}-2,5-diazabicyclo[2.2.1]heptane-2-carboxylate (example 51b) using the same experimental procedure as described in example 2c.

LRMS (m/z): 385 (M+1)⁺.

¹H NMR (400 MHz, DMSO-*d*₆) δ ppm 2.07 (d, *J*=10.3 Hz, 1 H), 2.20 (d, *J*=10.3 Hz, 1 H), 3.22 - 3.37 (m, 2 H), 3.67 - 3.83 (m, 2 H), 4.58 (s, 1 H), 5.03 (s, 1 H), 7.36 (t, *J*=7.23 Hz, 1 H), 7.63 (t, *J*=7.62 Hz, 1 H), 7.89 (s, 1 H), 8.05 (d, *J*=8.60 Hz, 1 H), 8.22 (d, *J*=7.42 Hz, 2 H), 8.30 - 8.40 (m, 2 H), 8.58 (s, 1 H), 9.48 (bs, 2 H), 9.90 (s, 1 H), 10.64(s, 1 H).

### EXAMPLE 52

### {4-piperazin-1-yl-6-[3-(pyrazin-2-ylamino)-1H-indazol-1-yl]pyrimidin-2-yl}methanol

Hydrobromic acid (0.3 mL, 48% in water, 3.7 mmol) was added to 1-[2-(methoxymethyl)-6-piperazin-1-ylpyrimidin-4-yl]-*N*-pyrazin-2-yl-1*H*-indazol-3-amine (example 43f, 20 mg, 0.05 mmol). The suspension was heated at 100ºC. After 2 h the mixture was cooled and purified by reverse phase chromatography as described in the general methods. The compound was obtained as a formate salt (10% yield).

LRMS (m/z): 404 (M+1)⁺.

### EXAMPLE 53

### 1-{2-[(1S,4S)-2,5-diazabicyclo[2.2.1]hept-2-yl]pyridin-4-yl}-3-(pyrazin-2-ylamino)-1H-indazol-6-ol

Obtained as a formate salt (13% yield) from tert-butyl (1*S*,4*S*)-5-{4-[6-methoxy-3-(pyrazin-2-ylamino)-1*H*-indazol-1-yl]pyridin-2-yl}-2,5-diazabicyclo[2.2.1]heptane-2-carboxylate (example 31b) following the experimental procedure described for the preparation of Example 52.

LRMS (m/z): 401 (M+1)⁺.

¹H NMR (400 MHz, DMSO-D6) δ ppm 1.9 (d, *J*=10.2 Hz, 1 H), 2.1 (d, *J*=10.2 Hz, 1 H), 3.1 (d, *J*=10.6 Hz, 1 H), 3.3 (d, *J*=9.8 Hz, 1 H), 3.6 (m, 2 H), 4.3 (s, 1 H), 4.9 (s, 1 H), 6.8 (m, 2 H), 7.1 (dd, *J*=5.5, 1.6 Hz, 1 H), 7.3 (d, *J*=1.6 Hz, 1 H), 8.1 (d, *J*=8.6 Hz, 1 H), 8.2 (d, 1 H), 8.3 (d, *J*=9.0 Hz, 4 H), 9.5 (d, *J*=1.6 Hz, 1 H), 10.4 (s, 1 H).

### EXAMPLE 54

### 1-{5-[(1S,4S)-2,5-diazabicyclo[2.2.1]hept-2-yl]pyridin-3-yl}-6-methoxy-N-pyrazin-2-yl-1H-indazol-3-amine

### a) tert-Butyl(1S,4S)-5-[5-(3-amino-6-methoxy-1H-indazol-1-yl)pyridin-3-yl]-2,5-diazabicyclo[2.2.1]heptane-2-carboxylate

Obtained (65%) from 6-methoxy-1*H*-indazol-3-amine (preparation 8) and (1*S,*4*S*)-*tert-*butyl 5-(5-bromopyridin-3-yl)-2,5-diazabicyclo[2.2.1]heptane-2-carboxylate (preparation 11) using the same experimental procedure as described in example 2a, followed by purification by flash chromatography (dichloromethane/methanol).

LRMS (m/z): 437 (M+1)⁺.

### b) tert-Butyl (1S,4S)-5-{5-[6-methoxy-3-(pyrazin-2-ylamino)-1H-indazol-1-yl]pyridin-3-yl}-2,5-diazabicyclo[2.2.1]heptane-2-carboxylate

Obtained (56%) from *tert-butyl* (1*S*,4*S*)-5-[5-(3-amino-6-methoxy-1*H*-indazol-1-yl)pyridin-3-yl]-2,5-diazabicyclo[2.2.1]heptane-2-carboxylate (example 54a) and 2-chloropyrazine using the same experimental procedure as described in example 2b, followed by purification by flash chromatography (dichloromethane/methanol).

LRMS (m/z): 515 (M+1)⁺.

### c) 1-{5-[(1S,4S)-2,5-Diazabicyclo[2.2.1]hept-2-yl]pyridin-3-yl}-6-methoxy-N-pyrazin-2-yl-1H-indazol-3-amine

Obtained as a hydrochloride salt (92% yield) from *tert*-butyl (1*S*,4*S*)-5-{5-[6-methoxy-3-(pyrazin-2-ylamino)-1*H*-indazol-1-yl]pyridin-3-yl]-2,5-diazabicyclo[2.2.1]heptane-2-carboxylate (example 54b) using the same experimental procedure as described in example 2c.

LRMS (m/z): 415 (M+1)⁺.

¹H NMR (400 MHz, DMSO-*d*₆) δ ppm 2.06 (d, *J*=10.16 Hz, 1 H), 2.20 (d, *J*=10.94 Hz, 1 H), 3.22 - 3.38 (m, 2 H), 3.70 - 3.84 (m, 2 H), 3.93 (s, 3 H), 4.57 (s, 1 H), 5.00 (s, 1 H), 6.96 (d, *J*=8.99 Hz, 1 H), 7.37 (s, 1 H), 7.87 (s, 1 H), 8.20 (d, *J*=8.99 Hz, 2 H), 8.35 (s, 1 H), 8.58 (s, 1 H), 9.48 (s, 1 H), 9.52 (br. s., 1 H), 9.83 (br. s., 1 H), 10.54 (s, 1 H).

### EXAMPLE 55

### 1-{2-[(1S,4S)-2,5-Diazabicyclo[2.2.1]hept-2-yl]pyridin-4-yl}-6-methyl-N-pyrazin-2-yl-1H-indazol-3-amine

### a) tert-butyl (1S,4S)-5-[4-(3-amino-6-methyl-1H-indazol-1-yl)pyridin-2-yl]-2,5-diazabicyclo[2.2.1]heptane-2-carboxylate

Obtained (19%) from 6-methyl-1*H*-indazol-3-amine (preparation 16) and (*1S,4S*)-*tert-*butyl 5-(4-bromopyridin-2-yl)-2,5-diazabicyclo[2.2.1]heptane-2-carboxylate (preparation 3) using the same experimental procedure as described in example 2a.

LRMS (m/z): 421 (M+1)⁺.

### b) tert-butyl (1S,4S)-5-{4-[6-methyl-3-(pyrazin-2-ylamino)-1H-indazol-1-yl]pyridin-2-yl}-2,5-diazabicyclo[2.2.1]heptane-2-carboxylate

Obtained (33%) from *tert*-butyl (1*S*,4*S*)-5-[4-(3-amino-6-methyl-1*H*-indazol-1-yl)pyridin-2-yl]-2,5-diazabicyclo[2.2.1]heptane-2-carboxylate (example 55a) and 2-chloropyrazine using the same experimental procedure as described in example 2b.

LRMS (m/z): 499 (M+1)⁺.

### c) 1-{2-[(1S,4S)-2,5-diazabicyclo[2.2.1]hept-2-yl]pyridin-4-yl}-6-methyl-N-pyrazin-2-yl-1H-indazol-3-amine

Obtained as a hydrochloride salt (81% yield) from tert-butyl (1*S*,4*S*)-5-{4-[6-methyl-3-(pyrazin-2-ylamino)-1*H*-indazol-1-yl]pyridin-2-yl}-2,5-diazabicyclo[2.2.1]heptane-2-carboxylate (example 55b) using the same experimental procedure as described in example 2c.

LRMS (m/z): 399 (M+1)⁺.

¹H NMR (400 MHz, dmso) δ 10.77 (s, 1H), 9.95 (bs, 1H), 9.53 (m, 2H), 8.40 (s, 1H), 8.33 - 8.26 (m, 2H), 8.11 (dd, *J* = 7.0, 2.6 Hz, 1H), 7.99 (s, 1H), 7.58 (d, *J* = 6.5 Hz, 1H), 7.28 (d, *J* = 5.9 Hz, 1H), 7.22 (bs, 1H), 5.36 (s, 1H), 4.67 (s, 1H), 3.91 (m, 2H), 3.45 (m, 1H), 3.35 (m, 1H), 2.56 (s, 3H), 2.27 (d, *J* = 10.5Hz, 1H), 2.13 (d, *J* = 10.5 Hz, 1H).

### EXAMPLE 56

### 1-{6-[(1S,4S)-2,5-diazabicyclo[2.2.1]hept-2-yl]pyrimidin-4-yl}-6-methoxy-N-pyrazin-2-yl-1H-indazol-3-amine

### a) tert-Butyl (1S,4S)-5-(6-iodopyrimidin-4-yl)-2,5-diazabicyclo[2.2.1]heptane-2-carboxylate

An oven dried resealable Schlenk tub was charged with (1*S*,4*S*)-*tert*-butyl 5-(6-chloropyrimidin-4-yl)-2,5-diazabicyclo[2.2.1]heptane-2-carboxylate (preparation 12, 350 mg, 1.14 mmol), copper iodide (10.82 mg, 0.06 mmol), sodium iodide (340 mg, 2.27 mg), trans-*N*,*N*'-dimethylcyclohexane-1,2-diamine (17 mg, 0.11 mmol), and dioxane (10 mL). The Schlenk tube was subjected to three cycles of evacuation-backfilling with argon, capped and heated at 110°C. After 48 h the mixture was cooled and concentrated. Ethyl acetate and water was added to the mixture and the organic layer was washed with aqueous ammonia, dried (Na₂SO₄) and evaporated to give the desired compound (324 mg, 69%).

LRMS (m/z): 403 (M+1)⁺.

### b) tert-Butyl (1S,4S)-5-[6-(3-amino-6-methoxy-1H-indazol-1-yl)pyrimidin-4-yl]-2,5-diazabicyclo[2.2.1]heptane-2-carboxylate

Obtained (34%) from *tert-butyl* (1*S*,4*S*)-5-(6-iodopyrimidin-4-yl)-2,5-diazabicyclo[2.2.1]heptane-2-carboxylate (example 56a) and 6-methoxy-1*H*-indazol-3-amine (preparation 8) using the same experimental procedure as described in example 2a.

LRMS (m/z): 438 (M+1)⁺.

### c) tert-Butyl (1S,4S)-5-{6-[6-methoxy-3-(pyrazin-2-ylamino)-1H-indazol-1-yl]pyrimidin-4-yl}-2,5-diazabicyclo[2.2.1]heptane-2-carboxylate

Obtained (36%) from *tert*-butyl (1*S*,4*S*)-5-[6-(3-amino-6-methoxy-1*H*-indazol-1-yl)pyrimidin-4-yl]-2,5-diazabicyclo[2.2.1]heptane-2-carboxylate (example 56b) and 2-chloropyrazine using the same experimental procedure as described in example 2b.

LRMS (m/z): 516 (M+1)⁺.

### d) 1-{6-[(1S,4S)-2,5-Diazabicyclo[2.2.1]hept-2-yl]pyrimidin-4-yl}-6-methoxy-N-pyrazin-2-yl-1H-indazol-3-amine

Obtained as a hydrochloride salt (59% yield) from tert-butyl (1*S*,4*S*)-5-{6-[6-methoxy-3-(pyrazin-2-ylamino)-1*H*-indazol-1-yl]pyrimidin-4-yl}-2,5-diazabicyclo[2.2.1]heptane-2-carboxylate (example 56c) using the same experimental procedure as described in example 7c.

LRMS (m/z): 416 (M+1)⁺.

¹H NMR (400 MHz, DMSO-D6) δ ppm 2.0 (m, 2 H), 2.2 (d, J=11.3 Hz, 2 H), 3.3 (m, 2 H), 4.6 (s, 1 H), 5.1 (s, 1 H), 7.0 (dd, *J*=8.8, 2.1 Hz, 1 H), 8.2 (d, *J*=9.0 Hz, 1 H), 8.2 (d, *J*=2.7 Hz, 1 H), 8.3 (d, *J*=2.0 Hz, 1 H), 8.4 (s, 1 H), 8.5 (s, 1 H), 9.0 (s, 1 H), 9.5 (s, 1 H), 9.6 (m, 1 H), 10.6 (s, 1 H).

### EXAMPLE 57

### 1-{6-[(1S,4S)-2,5-diazabicyclo[2.2.1]hept-2-yl]pyrimidin-4-yl)-N-pyrazin-2-yl-1H-pyrazolo[3,4-b]pyridin-3-amine

### a) tert-Butyl (1S,4S)-5-[6-(3-amino-1H-pyrazolo[3,4-b]pyridin-1-yl)pyrimidin-4-yl]-2,5-diazabicyclo[2.2.1]heptane-2-carboxylate

Obtained (12%) from *tert*-butyl (1*S*,4*S*)-5-(6-iodopyrimidin-4-yl)-2,5-diazabicyclo[2.2.1]heptane-2-carboxylate (example 56a) and 1*H*-pyrazolo[3,4-*b*]pyridin-3-amine (preparation 13) using the same experimental procedure as described in example 2a.

LRMS (m/z): 409 (M+1)⁺.

### b) tert-Butyl (1S,4S)-5-{6-[3-(pyrazin-2-ylamino)-1H-pyrazolo[3,4-b]pyridin-1-yl]pyrimidin-4-yl}-2,5-diazabicyclo[2.2.1]heptane-2-carboxylate

Obtained (62%) from *tert*-butyl (1*S*,4*S*)-5-[6-(3-amino-1*H*-pyrazolo[3,4-*b*]pyridin-1-yl)pyrimidin-4-yl]-2,5-diazabicyclo[2.2.1]heptane-2-carboxylate (example 57a) and 2-chloropyrazine using the same experimental procedure as described in example 2b, using dioxane as solvent.

LRMS (m/z): 487 (M+1)⁺.

### c) 1-{6-[(1S,4S)-2,5-Diazabicyclo[2.2.1]hept-2-yl]pyrimidin-4-yl}-N-pyrazin-2-yl-1H-pyrazolo[3,4-b]pyridin-3-amine

Obtained as a hydrochloride salt (97% yield) from *tert*-butyl (1*S*,4*S*)-5-{6-[3-(pyrazin-2-ylamino)-1*H*-pyrazolo[3,4-*b*]pyridin-1-yl]pyrimidin-4-yl}-2,5-diazabicyclo[2.2.1]heptane-2-carboxylate (example 57b) using the same experimental procedure as described in example 7c.

LRMS (m/z): 387 (M+1)⁺.

¹H NMR (300 MHz, dmso) 82.06 (d, *J*=9.61 Hz, 1 H), 2.19-2.40 (m, 1 H), 3.24 - 3.39 (m, 2H), 3.82 (br. s., 3H), 4.64 (br. s., 1 H), 7.49 - 7.59 (m, 1 H), 7.67 (br. s., 1 H), 8.34 (d, *J*=2.20 Hz, 1 H), 8.44 (d, *J*=1.37 Hz, 1 H), 8.69 (d, *J*=0.82 Hz, 1 H), 8.79 - 8.94 (m, 2H), 9.69 (d, *J*=1.65 Hz, 1 H), 10.96 (s, 1H).

### EXAMPLE 58

### 6-cyclopropyl-1-{2-[(1S,4S)-2,5-diazabicyclo[2.2.1]hept-2-yl]pyridin-4-yl}-N-pyrazin-2-yl-1H-indazol-3-amine

### a) 4-cyclopropyl-2-fluorobenzonitrile

An oven dried resealable Schlenk tube was charged with 4-bromo-2-fluorobenzonitrile (2.00 g, 10.00 mmol), cyclopropylboronic acid (1.16 g, 13.50 mmol), potassium phosphate (7.37 g, 34.70 mmol), water (2.4 mL) and toluene (48 mL). The Schlenk tube was subjected to three cycles of evacuation-backfilling with argon, palladium acetate (0.12 g, 0.53 mmol) and tricyclohexylphosphine (0.25g, 0.90 mmol) were added. After three further cycles of evacuation-backfilling with argon, the Schlenk tube was capped and placed in an oil bath at 100 ºC. After 4 h, the mixture was cooled and filtered. Ethyl acetate and water were added to the mixture and the organic layer was washed with brine, dried (Na₂SO₄) and evaporated to give the desired compound (1.60 g, 92%) as a brown solid.

¹H NMR (400 MHz, CDCl₃) δ 7.47 (dd, J = 8.0, 6.8 Hz, 1H), 6.93 (dd, J = 8.1, 1.6 Hz, 1 H), 6.84 (dd, J = 10.4, 1.6 Hz, 1 H), 1.93 (m, 1 H), 1.17 - 1.10 (m, 2H), 0.79 (m, 2H).

### b) 6-cyclopropyl-1H-indazol-3-amine

Obtained (55%) from 4-cyclopropyl-2-fluorobenzonitrile (example 58a) and hydrazine hydrate using the same experimental procedure as described in preparation 8.

LRMS (m/z): 174 (M+1)⁺.

### c) tert-butyl (1S,4S)-5-[4-(3-amino-6-cyclopropyl-1H-indazol-1-yl)pyridin-2-yl]-2,5-diazabicyclo[2.2.1]heptane-2-carboxylate

Obtained (38%) from 6-cyclopropyl-1*H*-indazol-3-amine (example 58b) and *(1S,4S)-tert-*butyl 5-(4-bromopyridin-2-yl)-2,5-diazabicyclo[2.2.1]heptane-2-carboxylate (preparation 3) using the same experimental procedure as described in example 2a, followed by purification by reverse phase chromatography as described in the general methods.

LRMS (m/z): 447 (M+1)⁺.

### d) tert-butyl (1S,4S)-5-{4-[6-cyclopropyl-3-(pyrazin-2-ylamino)-1H-indazol-1-yl]pyridin-2-yl}-2,5-diazabicyclo[2.2.1]heptane-2-carboxylate

Obtained (33%) from *tert*-butyl (1*S*,4*S*)-5-[4-(3-amino-6-cyclopropyl-1*H*-indazol-1-yl)pyridin-2-yl]-2,5-diazabicyclo[2.2.1]heptane-2-carboxylate (example 58c) and 2-chloropyrazine using the same experimental procedure as described in example 2b, followed by purification by reverse phase chromatography as described in the general methods.

LRMS (m/z): 525 (M+1)⁺.

### e) 6-Cyclopropyl-1-12-[(1S,4S)-2,5-diazabicyclo[2.2.1]hept-2-yl]pyridin-4-yl}-N-pyrazin-2-yl-1H-indazol-3-amine

Obtained as a hydrochloride salt (96% yield) from *tert*-butyl (1*S*,4*S*)-5-{4-[6-cyclopropyl-3-(pyrazin-2-ylamino)-1*H*-indazol-1-yl]pyridin-2-yl}-2,5-diazabicyclo[2.2.1]heptane-2-carboxylate (example 58d) using the same experimental procedure as described in example 2c.

LRMS (m/z): 425 (M+1)⁺.

1H NMR (400 MHz, dmso) δ 10.76 (s, 1H), 9.85 (bs, 1H), 9.52 (s, 1H), 9.42 (bs, 1H), 8.40 (s, 1H), 8.32 - 8.24 (m, 2H), 8.12 (d, J = 6.9 Hz, 1H), 7.84 (s, 1H), 7.57 (d, J = 6.9 Hz, 1H), 7.24 (bs, 1H), 7.10 (d, J = 8.4 Hz, 1H), 5.31 (s, 1H), 4.67 (s, 1H), 4.04 (d, J *=* 10.9 Hz, 1H), 3.90 (d, J = 10.7 Hz, 1H), 3.48 (m, 1H), 3.38 - 3.28 (m, 1H), 2.30 -2.17(m, 2H), 2.12 (d, J=11.4 Hz, 1H), 1.10 (d, J = 8.1 Hz, 2H), 0.93 (d, J = 4.8 Hz, 2H).

### EXAMPLE 59

### 1-[2-(6-methyl-1,4-diazepan-1-yl)pyridin-4-yl]-N-pyrazin-2-yl-1H-indazol-3-amine

Obtained as a hydrochloride salt (31% yield) from 1-(2-fluoropyridin-4-yl)-*N*-(pyrazin-2-yl)-1H-indazol-3-amine (preparation 9) and 6-methyl-1,4-diazepane using the same experimental procedure as described in preparation 2, followed by purification by reverse phase chromatography as described in the general methods. The oil obtained was dissolved in dioxane and a 4M solution of hydrochloric acid in dioxane was added.The mixture was stirred at room temperature for 2 days. The precipitate was filtered off and washed several times with dioxane and ethyl ether. The yelow solid was dried in the vacuum oven overnight to give the title compound.

LRMS (m/z): 401 (M+1)⁺.

¹H NMR (400 MHz, DMSO-D6) δ ppm 1.1 (m, *J*=6.3 Hz, 3 H), 3.1 (m, 1 H), 3.4 (m, 4 H), 3.6 (m, 1 H), 4.0 (m, 1 H), 4.1 (d, *J*=13.3 Hz, 1 H), 4.4 (m, *J*=15.6 Hz, 1 H), 7.3 (s, 1 H), 7.5 (t, *J*=7.4 Hz, 1 H), 7.6 (d, *J*=6.6 Hz, 1 H), 7.7 (t, *J*=7.8 Hz, 2 H), 8.1 (dd, *J*=11.9, 8.0 Hz, 2 H), 8.4 (m, 2 H), 9.4 (s, 1 H), 9.5 (s, 1 H), 9.8 (s, 1 H), 10.8 (s, 1 H).

### EXAMPLE 60

### 1-(4-piperazin-1-ylpyridin-2-yl)-N-pyrazin-2-yl-1H-indazol-3-amine

### a) tert-Butyl 4-(2-chloropyridin-4-yl)piperazine-1-carboxylate

Obtained (90%) from 4-bromo-2-chloropyridine and *ter*t-butyl piperazine-1-carboxylate using the same experimental procedure as described in preparation 7 followed by purification by flash chromatography.

LRMS (m/z): 298 (M+1)⁺.

¹H NMR (300 MHz, CHLOROFORM-*d*) δ ppm 1.50 (s, 9 H), 3.34 (d, *J*=4.67 Hz, 4 H), 3.57 (d, *J*=5.49 Hz, 4 H), 6.58 (dd, *J*=6.18, 2.33 Hz, 1 H), 6.66 (d, *J*=2.20 Hz, 1 H), 8.06 (d, *J*=6.04 Hz, 1 H).

### b) tert-Butyl 4-[2-(3-chloro-1H-indazol-1-yl)pyridin-4-yl]piperazine-1-carboxylate

Obtained (80%) from 3-chloro-1*H*-indazole and *tert*-butyl 4-(2-chloropyridin-4-yl)piperazine-1-carboxylate (example 60a) following the experimental procedure as described in example 1a, followed by purification by flash chromatography (hexanes 100% to 1:1 hexanes/ethyl acetate).

LRMS (m/z): 414 (M+1)⁺.

¹H NMR (300 MHz, DMSO-d₆) δ ppm 1.48 (s, 9 H), 3.51 (d, *J*=2.75 Hz, 8 H), 6.90 (dd, *J*=6.04, 2.47 Hz, 1 H), 7.29 (d, *J*=2.47 Hz, 1 H), 7.46 (ddd, *J*=7.97, 7.00, 0.96 Hz, 1 H), 7.69 (ddd, *J*=8.51, 7.14, 1.10 Hz, 1 H), 7.83 (ddd, *J*=8.10, 0.96, 0.82 Hz, 1 H), 8.23 (d, *J*=6.04 Hz, 1 H), 8.80 (dt, *J*=8.79, 0.82 Hz, 1 H).

### c) tert-Butyl 4-{2-[3-(pyrazin-2-ylamino)-1H-indazol-1-yl]pyridin-4-yl}piperazine-1-carboxylate

Obtained (11% yield) from *tert-*butyl 4-[2-(3-chloro-1*H*-indazol-1-yl)pyridin-4-yl]piperazine-1-carboxylate (example 60b) and pyrazine-2-amine following the experimental procedure described in example 3b, followed by purification by flash chromatography (hexanes 100% to 1:1 hexanes/ethyl acetate).

LRMS (m/z): 473 (M+1)⁺.

### d) 1-(4-Piperazin-1-ylpyridin-2-yl)-N-pyrazin-2-yl-1H-indazol-3-amine

Obtained as a hydrochloride salt (99% yield) from *tert*-butyl 4-{2-[3-(pyrazin-2-ylamino)-1*H*-indazol-1-yl]pyridin-4-yl}piperazine-1-carboxylate (example 60c) using the same experimental procedure as described in example 7c.

LRMS (m/z): 373 (M+1)⁺.

¹H NMR (300 MHz, DMSO-*d*₆) δ ppm 3.28 (br. s., 4 H), 3.77 (br. s., 4 H), 6.94 (d, *J*=5.77 Hz, 1 H), 7.28 - 7.40 (m, 1 H), 7.62 (t, *J*=8.24 Hz, 1 H), 8.24 (dd, *J*=4.39, 1.92 Hz, 2 H), 8.30 (d, *J*=7.97 Hz, 1 H), 8.36 (dd, *J*=2.47, 1.37 Hz, 1 H), 8.56 (d, *J*=9.61 Hz, 1 H), 9.22 (br. s., 2 H), 9.53 (s, 1 H), 10.59 (s, 1 H).

### EXAMPLE 61

### 1-{2-[(1S,4S)-2,5-diazabicyclo[2.2.1]hept-2-yl]pyridin-4-yl}-6-(2-methoxyethoxy)-N-pyrazin-2-yl-1H-indazol-3-amine

### a) 2-Fluoro-4-(2-methoxyethoxy)benzonitrile

Obtained as a colorless oil (95%) from 2-fluoro-4-hydroxybenzonitrile and 2-bromoethyl methyl ether using the same experimental procedure as described in example 32a.

LRMS (m/z): 196 (M+1)⁺

### b) 6-(2-Methoxyethoxy)-1H-indazol-3-amine

From 2-fluoro-4-(2-methoxyethoxy)benzonitrile (example 61 a) following the same experimental procedure described in preparation 8, to provide the title compound as a white solid (24%).

LRMS (m/z): 208 (M+1)⁺

### c) tert-Butyl (1S,4S)-5-{4-[3-amino-6-(2-methoxyethoxy)-1H-indazol-1-yl]pyridin-2-yl}-2,5-diazabicyclo[2.2.1]heptane-2-carboxylate

Obtained (28%) from 6-(2-methoxyethoxy)-1*H*-indazol-3-amine (example 61 b) and (1*S*,4*S*)-*tert*-butyl 5-(4-bromopyridin-2-yl)-2,5-diazabicyclo[2.2.1]heptane-2-carboxylate (preparation 3) following the same experimental procedure as described in example 7a.

LRMS (m/z) 481 (M+1)⁺

### d) tert-Butyl (1S,4S)-5-{4-[6-(2-methoxyethoxy)-3-(pyrazin-2-ylamino)-1H-indazol-1-yl]pyridin-2-yl}-2,5-diazabicyclo[2.2.1]heptane-2-carboxylate

Obtained (47%) from *tert*-butyl (1*S*,4*S*)-5-{4-[3-amino-6-(2-methoxyethoxy)-1*H*-indazol-1-yl]pyridin-2-yl}-2,5-diazabicyclo[2.2.1]heptane-2-carboxylate (example 61 c) and 2-chloropyrazine using the same experimental procedure as described in example 2b, followed by purification by reverse phase chromatography in the general methods.

LRMS (m/z) 559 (M+1)⁺

### e)1-{2-[(1S,4S)-2,5-diazabicyclo[2.2.1]hept-2-yl]pyridin-4-yl}-6-(2-methoxyethoxy)-N-pyrazin-2-yl-1H-indazol-3-amine

Obtained as a hydrochloride salt (68%) from *tert*-butyl (1*S*,4*S*)-5-{4-[6-(2-methoxyethoxy)-3-(pyrazin-2-ylamino)-1*H*-indazol-1-yl]pyridin-2-yl}-2,5-diazabicyclo[2.2.1]heptane-2-carboxylate (example 61 d) using the same experimental as described in example 7c.

¹H NMR (400 MHz, DMSO-D6) δ ppm 2.1 (d, *J*=10.9 Hz, 1 H), 2.3 (d, *J*=10.9 Hz, 1 H), 3.5 (s, 1 H), 3.7 (s, 2 H), 3.9 (d, *J*=10.2 Hz, 1 H), 4.1 (d, *J*=10.6 Hz, 1 H), 4.3 (s, 3 H), 4.7 (s, 1 H), 5.3 (s, 1 H), 7.1 (d, *J*=9.0 Hz, 1 H), 7.6 (m, 2 H), 8.1 (d, *J*=7.0 Hz, 1 H), 8.3 (m, 2 H), 8.4 (s, 1 H), 9.5 (s, 1 H), 9.6 (m, *J*=2.3 Hz, 1 H), 10.0 (s, 1 H), 10.8 (s, 1 H).

### EXAMPLE 62

### 1-[2-(1,4-diazepan-1-yl)pyridin-4-yl]-6-methoxy-N-pyrazin-2-yl-1H-indazol-3-amine

### a) tert-Butyl 4-[4-(3-amino-6-methoxy-1H-indazo)-1-yl)pyridin-2-yl]-1,4-diazepane-1-carboxylate

Obtained (45% yield) from 6-methoxy-1*H*-indazol-3-amine (preparation 8) and *tert*-butyl 4-(4-bromopyridin-2-yl)-1,4-diazepane-1-carboxylate (preparation 14) following the experimental procedure described in Example 2a using DMSO as a solvent.

LRMS (m/z): 439 (M+1)⁺

### b) tert-Butyl 4-{4-[6-methoxy-3-(pyrazin-2-ylamino)-1H-indazol-1-yl]pyridin-2-yl}-1,4-diazepane-1-carboxylate

Obtained (34% yield) from *tert*-butyl 4-[4-(3-amino-6-methoxy-1*H*-indazol-1-yl)pyridin-2-yl]-1,4-diazepane-1-carboxylate (example 62a) and and 2-chloropyrazine following the experimental procedure described in example 2b followed by purification by reverse phase chromatography in the general methods.

LRMS (m/z): 517 (M+1)⁺

### c) 1-[2-(1,4-Diazepan-1-yl)pyridin-4-yl]-6-methoxy-N-pyrazin-2-yl-1H-indazol-3-amine

Obtained (74% yield) from tert-butyl 4-{4-[6-methoxy-3-(pyrazin-2-ylamino)-1*H*-indazol-1-yl]pyridin-2-yl}-1,4-diazepane-1-carboxylate (example 62b) using the same experimental procedure as described in example 7c.

LRMS (m/z): 417 (M+1)⁺.

¹H NMR (400 MHz, DMSO-D6) δ ppm 2.2 (m, 2 H), 3.3 (m, 2 H), 3.4 (m, 2 H,) 3.9 (m, *J*=5.1 Hz, 2 H), 3.9 (s, 3 H), 4.1 (m, 2 H), 7.1 (d, *J*=8.6 Hz, 1 H), 7.3 (s, 1 H), 7.5 (s, 1 H), 7.5 (s, 1 H), 8.1 (d, *J*=6.6 Hz, 1 H), 8.3 (d, *J*=9.0 Hz, 2 H), 8.4 (s, 1 H), 9.5 (d, *J*=1.6 Hz, 3 H), 10.7 (s, 1 H).

### EXAMPLE 63

### 1-{5-[(1S,4S)-2,5-Diazabicyclo[2.2.1]hept-2-yl]pyridin-3-yl}-3-(pyrazin-2-ylamino)-1H-indazol-6-ol

Obtained (15%) from 1-{5-[(1*S*,4*S*)-2,5-diazabicyclo[2.2.1]hept-2-yl]pyridin-3-yl}-6-methoxy-*N*-pyrazin-2-yl-1*H*-indazol-3-amine (example 54c) following the same experimental procedure described for Example 52.

LRMS (m/z): 401 (M+1)⁺

### EXAMPLE 64

### 1-{2-[(1S,4S)-2,5-Diazabicyclo[2.2.1]hept-2-yl]pyridin-4-yl}-6-methyl-N-pyrazin-2-yl-1H-pyrazolo[3,4-b]pyridin-3-amine

### a) 6-Methyl-1H-pyrazolo[3,4-b]pyridin-3-amine

Obtained (96% yield) from 2-chloro-6-methylnicotinonitrile following the experimental procedure described in preparation 8.

LRMS (m/z): 149 (M+1)⁺.

¹H NMR (300 MHz, DMSO-*d*₆) δ ppm 2.53 (s, 3 H), 5.50 (s, 2 H), 6.86 (d, *J*=7.97 Hz, 1 H), 8.00 (d, *J*=7.97 Hz, 1 H), 11.75 (s, 1 H).

### b) tert-Butyl (1 S,4S)-5-[4-(3-amino-6-methyl-1H-pyrazolo[3,4-b]pyridin-1-yl)pyridin-2-yl]-2,5-diazabicyclo[2.2.1]heptane-2-carboxylate

Obtained (37%) from 6-methyl-1*H*-pyrazolo[3,4-*b*]pyridin-3-amine (example 64a) and (1*S*,4*S*)-*tert*-butyl 5-(4-bromopyridin-2-yl)-2,5-diazabicyclo[2.2.1]heptane-2-carboxylate (preparation 3) following the experimental procedure as described in example 2a, followed by purification by flash chromatography (hexanes/ethyl acetate).

LRMS (m/z): 422 (M+1)⁺.

¹H NMR (300 MHz, CHLOROFORM-*d*) δ ppm 1.44 (d, *J*=16.21 Hz, 9 H), 2.03 (d, *J*=7.14 Hz, 2 H), 2.70 (s, 3 H), 3.42 - 3.58 (m, 3 H), 3.60 - 3.69 (m, 1 H), 4.36 (br. s., 2 H), 4.51 - 4.74 (m, 1 H), 4.84 - 5.02 (m, 1 H), 7.00 (d, *J*=8.24 Hz, 1 H), 7.43 (d, *J*=15.66 Hz, 1 H), 7.72 (d, *J*=5.49 Hz, 1 H), 7.81 (d, *J*=8.24 Hz, 1 H), 8.15 (d, *J*=5.77 Hz, 1 H).

### c) tert-Butyl (1S,4S)-5-{4-[6-methyl-3-(pyrazin-2-ylamino)-1H-pyrazolo[3,4-b]pyridin-1-yl]pyridin-2-yl}-2,5-diazabicyclo[2.2.1]heptane-2-carboxylate

Obtained (34% yield) from *tert*-butyl (1*S*,4*S*)-5-[4-(3-amino-6-methyl-1*H*-pyrazolo[3,4-*b*]pyridin-1-yl)pyridin-2-yl]-2,5-diazabicyclo[2.2.1]heptane-2-carboxylate (example 64b) and 2-chloropyrazine following the experimental procedure described in example 2b, followed by purification by flash chromatography (dichloromethane 100% to 95:5 dichloromethane:methanol).

LRMS (m/z): 500 (M+1)⁺.

¹H NMR (300 MHz, DMSO-*d*₆) δ ppm 1.49 (d, *J*=18.68 Hz, 9 H), 2.10 (br. s., 2 H), 2.80 (s, 3 H), 3.32 - 3.45 (m, 3 H), 3.73 (d, *J*=6.04 Hz, 1 H), 4.62 (d, *J*=18.40 Hz, 1 H), 4.96 (d, *J*=11.54 Hz, 1 H), 7.38 (d, *J*=8.24 Hz, 1 H), 7.70 (br. s., 1 H), 7.77 (d, *J*=6.04 Hz, 1 H), 8.29 (d, *J*=5.49 Hz, 1 H), 8.34 (d, *J*=2.75 Hz, 1 H), 8.45 (dd, *J*=2.47, 1.37 Hz, 1 H), 8.68 (d, *J*=8.24 Hz, 1 H), 9.52 (s, 1 H), 10.79 (s, 1 H).

### d) 1-{2-[(1S,4S)-2,5-Diazabicyc)o[2.2.1]hept-2-yl]pyridin-4-yl}-6-methyl-N-pyrazin-2-yl-1H-pyrazolo[3,4-b]pyridin-3-amine

Obtained as a hydrochloride salt (96% yield) from *tert*-butyl (1*S*,4*S*)-5-{4-[6-methyl-3-(pyrazin-2-ylamino)-1*H*-pyrazolo[3,4-*b*]pyridin-1-yl]pyridin-2-yl}-2,5-diazabicyclo[2.2.1]heptane-2-carboxylate (example 64c) using the same experimental procedure as described in example 7c.

LRMS (m/z): 400 (M+1)⁺.

¹H NMR (300 MHz, DMSO-*d*₆) δ ppm 2.17 (d, *J*=8.24 Hz, 2 H), 2.34 (d, *J*=14.28 Hz, 2 H), 2.77 (s, 3 H), 3.83 - 4.17 (m, 2 H), 4.71 (br. s., 1 H), 7.46 (d, *J*=6.04 Hz, 1 H), 8.06 (d, *J*=3.30 Hz, 2 H), 8.21 (d, *J*=4.40 Hz, 1 H), 8.35 (br. s., 1 H), 8.46 (br. s., 1 H), 8.74 (d, *J*=6.32 Hz, 1 H), 9.35 - 9.63 (m, 2 H), 9.95 (br. s., 1 H), 11.02 (br. s., 1 H).

### EXAMPLE 65

### 1-{5-[(2S)-2-methylpiperazin-1-yl]pyridin-3-yl}-N-pyrazin-2-yl-1H-indazol-3-amine

### a) tert-Butyl (3S)-4-[5-(3-amino-1H-indazol-1-yl)pyridin-3-yl]-3-methylpiperazine-1-carboxylate

Obtained (51 %) from 1*H*-indazol-3-amine and *tert*-butyl (3*S*)-4-(5-bromopyridin-3-yl)-3-methylpiperazine-1-carboxylate (preparation 15) using the same experimental procedure as described in example 2a.

LRMS (m/z): 409 (M+1)⁺.

### b) tert-Butyl (3S)-3-methyl-4-{5-[3-(pyrazin-2-ylamino)-1H-indazol-1-yl]pyridin-3-yl}piperazine-1-carboxylate

Obtained as a yellow oil (48%) from *tert*-Butyl (3*S*)-4-[5-(3-amino-1*H*-indazol-1-yl)pyridin-3-yl]-3-methylpiperazine-1-carboxylate (example 65a) and 2-chloropyrazine using the same experimental procedure as described in example 2b.

LRMS (m/z): 487 (M+1)⁺.

### c) 1-{5-[(2S)-2-methylpiperazin-1-yl]py*ridin-3-yl}-N-pyrazin-2-yl-1H-indazol-3-amine

Obtained as a hydrochloride salt (89%) from *tert*-butyl (3*S*)-3-methyl-4-{5-[3-(pyrazin-2-ylamino)-1*H*-indazol-1-yl]pyridin-3-yl]piperazine-1-carboxylate (example 65b) using the same experimental procedure as described in example 2c.

LRMS (m/z): 387 (M+1)⁺.

1H NMR (400 MHz, dmso) δ 10.66 (s, 1H), 9.93 - 9.82 (m, 1H), 9.50 - 9.47 (m, 1H), 9.40 (m, 1H), 8.68 (d, J = 1.6 Hz, 1H), 8.42 - 8.32 (m, 3H), 8.24 (d, J = 2.5 Hz, 1H), 8.13 (s,1H), 8.00 (d, J = 8.6 Hz, 1H), 7.64 (t, J = 7.7 Hz, 1H), 7.39 - 7.32 (m, 1H), 4.58 (s, 1H), 3.98 (d, J = 13.3 Hz, 1H), 3.48 - 3.21 (m, 4H), 3.15 - 3.02 (m, 1H), 1.33 (d, J = 6.9 Hz, 3H).

### EXAMPLE 66

### 1-{2-[(2R)-2-methylpiperazin-1-yl]pyridin-4-yl}-N-pyrazin-2-yl-1H-indazol-3-amine

### a) (R)-tert-butyl 4-(4-bromopyridin-2-yl)-3-methylpiperazine-1-carboxylate

Obtained (19%) from 4-bromo-2-fluoropyridine and *(R)*-*tert*-butyl 3-methylpiperazine-1-carboxylate using the same experimental procedure as described in preparation 2, followed by purification by reverse phase chromatography as described in the general methods.

LRMS (m/z): 356/358 (M+1)⁺.

### b) tert-Butyl (3R)-4-[4-(3-amino-1H-indazol-1-yl)pyridin-2-yl]-3-methylpiperazine-1-carboxylate

Obtained (60%) from 1*H*-indazol-3-amine and *tert*-butyl (3*R*)-4-(4-bromopyridin-2-yl)-3-methylpiperazine-1-carboxylate (example 66a) using the same experimental procedure as described in example 2a.

LRMS (m/z): 409 (M+1)⁺.

### c) tert-Butyl (3R)-3-methy)-4-{4-[3-(pyrazin-2-ylamino)-1H-indazol-1-yl]pyridin-2-yl}piperazine-1-carboxylate

Obtained as a yellow oil (20%) from *tert*-Butyl (3*R*)-4-[4-(3-amino-1*H*-indazol-1-yl)pyridin-2-yl]-3-methylpiperazine-1-carboxylate (example 66b) and 2-chloropyrazine using the same experimental procedure as described in example 2b.

LRMS (m/z): 487 (M+1)⁺.

### d) 1-{2-[(2R)-2-Methylpiperazin-1-yl]pyridin-4-yl}-N-pyrazin-2-yl-1H-indazol-3-amine

Obtained as a hydrochloride salt (87%) from tert-butyl (3*R*)-3-methyl-4-{4-[3-(pyrazin-2-ylamino)-1*H*-indazol-1-yl]pyridin-2-yl}piperazine-1-carboxylate (example 66c) using the same experimental procedure as described in example 2c.

LRMS (m/z): 387 (M+1)⁺.

¹H NMR (400 MHz, DMSO-D6) δ 10.79 (s, 1H), 9.83 (bs, 1H), 9.52 (s, 1H), 9.42 (bs, 1H), 8.42 (m, 2H), 8.29 (s, 1H), 8.19 (dd, *J* = 12.7, 7.6 Hz, 2H), 7.70 (t, *J* = 7.5 Hz, 1H), 7.57 (bs, 1H), 7.44 (t, *J* = 7.5 Hz, 1H), 7.36 (s, 1 H), 4.87 (s, 1H), 4.37 (d, *J* = 13.7 Hz, 1H), 3.62 - 3.51 (m, 1H), 3.37 (m, 3H), 3.16 (m, 1H), 1.43 (d, *J* = 6.5 Hz, 3H).

### EXAMPLE 67

### 1-[2-(3,6-diazabicyclo[3.2.1]oct-3-yl)pyridin-4-yl]-N-pyrazin-2-yl-1H-indazol-3-amine

### a) tert-Butyl 3-{4-[3-(pyrazin-2-ylamino)-1H-indazol-1-yl]pyridin-2-yl}-3,6-diazabicyclo[3.2.1]octane-6-carboxylate

Obtained as an oil from 1-(2-fluoropyridin-4-yl)-N-(pyrazin-2-yl)-1H-indazol-3-amine (preparation 9) and *tert*-butyl 3,6-diazabicyclo[3.2.1]octane-6-carboxylate using the same experimental procedure as described in preparation 2, followed by purification by reverse phase chromatography as described in the general method.

LRMS (m/z): 499 (M+1)⁺.

### b) 1-[2-(3,6-diazabicyclo[3.2.1]oct-3-yl)pyridin-4-yl]-N-pyrazin-2-yl-1H-indazol-3-amine

Obtained as a hydrochloride salt (98%) from *tert*-Butyl 3-{4-[3-(pyrazin-2-ylamino)-1*H-*indazol-1-yl]pyridin-2-yl}-3,6-diazabicyclo[3.2.1]octane-6-carboxylate (example 67a) using the same experimental procedure as described in example 2c.

LRMS (m/z): 399 (M+1)⁺.

¹H NMR (400 MHz, DMSO-*d*₆) δ ppm 1.93 - 2.00 (m, 1 H), 2.03 - 2.09 (m, 1 H), 2.90 (s, 1 H), 3.22 - 3.37 (m, 2 H), 3.45 (t, *J*=10.94 Hz, 2 H), 4.20 - 4.30 (m, 2 H), 4.41 (d, *J*=12.11 Hz, 1 H), 7.39 (d, *J*=1.56 Hz, 1 H), 7.44 - 7.49 (m, 1 H), 7.65 (dd, *J*=7.23, 1.37 Hz, 1 H), 7.72 (t, *J*=7.42 Hz, 1 H), 8.19 (d, *J*=7.03 Hz, 1 H), 8.27 (d, *J*=8.99 Hz, 1 H), 8.30 (d, *J*=2.34 Hz, 1 H), 8.39 - 8.43 (m, 1 H), 8.45 (d, *J*=8.21 Hz, 1 H), 9.51 (s, 1 H), 9.80 (bs, 1 H), 10.02 (bs, 1 H), 10.84 (s, 1 H).

### EXAMPLE 68

### 4-{4-[6-Methoxy-3-(pyrazin-2-ylamino)-1H-indazol-1-yl]pyridin-2-yl}piperidine-4-carbonitrile

### a) tert-Butyl 4-[4-(3-amino-6-methoxy-1H-indazol-1-yl)pyridin-2-yl]-4-cyanopiperidine-1-carboxylate

Obtained (27%) from 6-methoxy-1*H*-indazol-3-amine (preparation 8) and *tert-*butyl 4-(4-bromopyridin-2-yl)-4-cyanopiperidine-1-carboxylate (preparation 4) following the experimental procedure as described in example 2a, followed by purification by flash chromatography (hexanes/ethyl acetate).

LRMS (m/z): 449 (M+1)⁺.

¹H NMR (300 MHz, CHLOROFORM-*d*) δ ppm 1.49 (s, 9 H), 2.10 (d, *J*=13.73 Hz, 2 H), 2.28 (dd, *J*=12.91, 4.12 Hz, 2 H), 3.21 (br. s., 2 H), 3.93 (s, 3 H), 4.32 (br. s., 4 H), 6.87 (dd, *J*=8.79, 1.92 Hz, 1 H), 7.26 (s, 1 H), 7.48 (d, *J*=8.79 Hz, 1 H), 7.58 (dd, *J*=5.63, 2.06 Hz, 1 H), 8.02 (d, *J*=2.20 Hz, 1 H), 8.58 (d, *J*=5.77 Hz, 1 H).

### b) tert-Butyl 4-cyano-4-{4-[6-methoxy-3-(pyrazin-2-ylamino)-1H-indazol-1-yl]pyridin-2-yl}piperidine-1-carboxylate

Obtained (38% yield) from *tert*-butyl 4-[4-(3-amino-6-methoxy-1*H*-indazol-1-yl)pyridin-2-yl]-4-cyanopiperidine-1-carboxylate (example 68a) and 2-chloropyrazine following the experimental procedure described in example 2b, followed by purification by reverse phase chromatography (C-18 silica from Waters©, water/methanol 0% to 100%).

LRMS (m/z): 527 (M+1)⁺

¹H NMR (300 MHz, CHLOROFORM-*d*) δ ppm 1.50 (s, 9 H), 2.11 (d, *J*=11.54 Hz, 2 H), 2.33 (dd, *J*=12.22, 3.98 Hz, 2 H), 3.22 (br. s., 2 H), 3.97 (s, 3 H), 4.34 (br. s., 2 H), 6.97 (d, *J*=8.51 Hz, 1 H), 7.29 (s, 1 H), 7.35 (s, 1 H), 7.62 (d, *J*=8.79 Hz, 1 H), 7.76 (dd, *J*=6.59, 1.92 Hz, 1 H), 8.13 (s, 1 H), 8.25 (s, 2 H), 8.67 (d, *J*=5.77 Hz, 1 H), 9.61 (s, 1 H).

### c) 4-{4-[6-Methoxy-3-(pyrazin-2-ylamino)-1H-indazol-1-yl]pyridin-2-yl}piperidine-4-carbonitrile

Obtained as a hydrochloride salt (47% yield) from *tert*-butyl 4-cyano-4-{4-[6-methoxy-3-(pyrazin-2-ylamino)-1*H*-indazol-1-yl]pyridin-2-yl}piperidine-1-carboxylate (example 68b) using the same experimental procedure as described in example 7c.

LRMS (m/z): 427 (M+1)⁺.

¹H NMR (300 MHz, METHANOL-*d*₄) δ ppm 2.50 - 2.81 (m, 4 H), 3.37 - 3.55 (m, 2 H), 3.72 (d, *J*=15.38 Hz, 2 H), 3.99 (s, 3 H), 7.08 (d, *J*=7.69 Hz, 1 H), 7.52 (br. s., 1 H), 8.00 - 8.14 (m, 2 H), 8.30 (br. s., 2 H), 8.57 (br. s., 1 H), 8.74 (d, *J*=6.04 Hz, 1 H), 9.51 (br. s., 1 H).

### EXAMPLE 69

### 1-{4-[(1S,4S)-2,5-Diazabicyclo[2.2.1]hept-2-yl]pyridin-2-yl}-N-pyrazin-2-yl-1H-indazol-3-amine

### a) tert-Butyl (1S,4S)-5-(2-chloropyridin-4-yl)-2,5-diazabicyclo[2.2.1]heptane-2-carboxylate

Obtained (61% yield) from 4-bromo-2-chloropyridine and *tert*-butyl (1*S*,4*S*)-2,5-diazabicyclo[2.2.1]heptane-2-carboxylate using the same experimental procedure as described in preparation 7, followed by purification by flash chromatography (hexanes/ethyl acetate).

LRMS (m/z): 310 (M+1)⁺.

### b) tert-Butyl (1S,4S)-5-[2-(3-chloro-1H-indazol-1-yl)pyridin-4-yl]-2,5-diazabicyclo[2.2.1]heptane-2-carboxylate

Obtained (93% yield) from 3-chloro-1*H*-indazole and *tert*-butyl (1*S*,4*S*)-5-(2-chloropyridin-4-yl)-2,5-diazabicyclo[2.2.1]heptane-2-carboxylate (example 69a) following the experimental procedure as described in example 2a, using toluene as a solvent, followed by purification by flash chromatography (hexanes/ethyl acetate).

LRMS (m/z): 426 (M+1)⁺.

### c) tert-Butyl (1S,4S)-5-{2-[3-(pyrazin-2-ylamino)-1H-indazol-1-yl]pyridin-4-yl}-2,5-diazabicyclo[2.2.1]heptane-2-carboxylate

A mixture of *tert-*butyl (1*S*,4*S*)-5-[2-(3-chloro-1*H*-indazol-1-yl)pyridin-4-yl]-2,5-diazabicyclo[2.2.1]heptane-2-carboxylate (example 69b), pyridine-2-amine, cesium carbonate, Pd₂(dba)₃ and 4,5-bis(diphenylphosphino)-9,9-dimethylxanthene in 1,2-dimethoxyethane was heated at 140 °C for 2 h under microwave conditions. The mixture was then cooled and filtered, and the filtrate was concentrated in vacuum. The crude was dissolved in ethyl acetate, washed with water, the organic extracts dried over Na₂SO₄ and concentrated under vacuum. The crude was purified by reverse phase chromatography (C-18 silica from Waters©, water/methanol 0% to 100%) to give the title compound (4%).

LRMS (m/z): 485 (M+1)⁺.

### d) 1-{4-[(1S,4S)-2,5-Diazabicyclo[2.2.1]hept-2-yl]pyridin-2-yl}-N-pyrazin-2-yl-1H-indazol-3-amine

Obtained as a hydrochloride salt (52% yield) from tert-butyl (1*S*,4*S*)-5-{2-[3-(pyrazin-2-ylamino)-1*H*-indazol-1-yl]pyridin-4-yl}-2,5-diazabicyclo[2.2.1]heptane-2-carboxylate (example 69c) using the same experimental procedure as described in example 7c.

LRMS (m/z): 385 (M+1)⁺, 383 (M-1)⁻.

¹H NMR (300 MHz, METHANOL-*d*₄) δ ppm 2.19 - 2.32 (m, 1 H), 2.43 (d, *J*=11.54 Hz, 1 H), 3.55 (br. s., 2 H), 3.98 (br. s., 2 H), 4.74 (br. s., 1 H), 5.16 - 5.39 (m, 1 H), 7.31 (br. s., 1 H), 7.52 (t, *J*=7.55 Hz, 1 H), 7.80 (t, *J*=8.24 Hz, 1 H), 8.17 (d, *J*=7.14 Hz, 2 H), 8.26 (d, *J*=3.02 Hz, 3 H), 8.43 (s, 1 H), 9.42 (s, 1 H).

### EXAMPLE 70

### 1-{2-[(1S,4S)-2,5-diazabicyclo[2.2.1]hept-2-yl]pyridin-4-yl}-6-isobutoxy-N-pyrazin-2-yl-1H-indazol-3-amine

### a) 2-Fluoro-4-isobutoxybenzonitrile

Obtained as a colorless oil (35%) from 2-fluoro-4-hydroxybenzonitrile and 1-bromo-2-methylpropane using the same experimental procedure as described in example 32a.

LRMS (m/z): 194 (M+1)⁺

### b) 6-isobutoxy-1H-indazol-3-amine

From 2-fluoro-4-isobutoxybenzonitrile (example 70a) following the same experimental procedure described in preparation 8, to provide the title compound as a white solid (67%).

LRMS (m/z): 164 (M+1)⁺

### c) tert-butyl (1S,4S)-5-[4-(3-amino-6-isobutoxy-1H-indazol-1-yl)pyridin-2-yl]-2,5-diazabicyclo[2.2.1]heptane-2-carboxylate

Obtained (58%) from 6-isobutoxy-1*H*-indazol-3-amine (example 70b) and (1*S,*4*S)-tert* butyl 5-(4-bromopyridin-2-yl)-2,5-diazabicyclo[2.2.1]heptane-2-carboxylate (preparation 3) following the same experimental procedure as described in example 2a.

LRMS (m/z) 479 (M+1)⁺

### d) tert-butyl (1S,4S)-5-{4-[6-isobutoxy-3-(pyrazin-2-ylamino)-1H-indazol-1-yl]pyridin-2-yl}-2,5-diazabicyclo[2.2.1]heptane-2-carboxylate

Obtained (10%) from *tert*-butyl(1*S*,4*S*)-5-[4-(3-amino-6-isobutoxy-1*H*-indazol-1-yl)pyridin-2-yl]-2,5-diazabicyclo[2.2.1]heptane-2-carboxylate
(example 70c) and 2-chloropyrazine using the same experimental procedure as described in example 2b, followed by purification by reverse phase chromatography in the general methods.

LRMS (m/z) 557 (M+1)⁺

### d) 1-{2-[(1S,4S)-2,5-diazabicyclo[2.2.1]hept-2-yl]pyridin-4-yl}-6-isobutoxy-N-pyrazin-2-yl-1H-indazol-3-amine

Obtained as a hydrochloride salt (95%) from *tert*-butyl (1*S*,4*S*)-5-{4-[6-isobutoxy-3-(pyrazin-2-ylamino)-1*H*-indazol-1-yl]pyridin-2-yl}-2,5-diazabicyclo[2.2.1]heptane-2-carboxylate (example 70c) using the same experimental procedure as described in example 7c.
LRMS (m/z) 457 (M+1)⁺

### EXAMPLE 71

### 1-{5-[(1S,4S)-2,5-diazabicyclo[2.2.1]hept-2-yl]pyridin-3-yl}-6-methyl-N-pyrazin-2-yl-1H-indazol-3-amine

### a) tert-Butyl (1S,4S)-5-[5-(3-amino-6-methyl-1H-indazol-1-yl)pyridin-3-yl]-2,5-diazabicyclo[2.2.1]heptane-2-carboxylate

Obtained (30%) from 6-methyl-1*H*-indazol-3-amine (preparation 16) and (*1S,4S)-tert-*butyl 5-(5-bromopyridin-3-yl)-2,5-diazabicyclo[2.2.1]heptane-2-carboxylate (preparation 11) using the same experimental procedure as described in example 2a.

LRMS (m/z): 421 (M+1)⁺.

### b) tert-Butyl (1S,4S)-5-{5-[6-methyl-3-(pyrazin-2-ylamino)-1H-indazol-1-yl]pyridin-3-yl}-2,5-diazabicyclo[2.2.1]heptane-2-carboxylate

Obtained (64%) from *tert*-butyl (1*S*,4*S*)-5-[5-(3-amino-6-methyl-1*H*-indazol-1-yl)pyridin-3-yl]-2,5-diazabicyclo[2.2.1]heptane-2-carboxylate (example 71 a) and 2-chloropyrazine using the same experimental procedure as described in example 2b.

LRMS (m/z): 499 (M+1)⁺.

### c) 1-{5-[(1S,4S)-2,5-diazabicyclo[2.2.1]hept-2-yl]pyridin-3-yl}-6-methyl-N-pyrazin-2-yl-1H-indazol-3-amine

Obtained as a hydrochloride salt (85% yield) from *tert*-butyl (1*S*,4*S*)-5-{5-[6-methyl-3-(pyrazin-2-ylamino)-1*H*-indazol-1-yl]pyridin-3-yl}-2,5-diazabicyclo[2.2.1]heptane-2-carboxylate (example 71 b) using the same experimental procedure as described in example 7c.

LRMS (m/z): 399 (M+1)⁺.

¹H NMR (400 MHz, DMSO-*d*₆) δ ppm 2.07 (d, *J*=10.3 Hz, 1 H) 2.21 (d, *J*=10.3 Hz, 1 H) 2.53 (s, 3 H) 3.24 - 3.38 (m, 2 H) 3.76 (d, *J*=10.3 Hz, 1 H) 3.80 - 3.88 (d, *J*=10.3 Hz, 1 H) 4.59 (s, 1 H) 5.04 (s, 1 H) 7.18 (d, *J*=8.21 Hz, 1 H) 7.83 - 7.95 (m, 2 H) 8.16 - 8.27 (m, 3 H) 8.36 (s, 1 H) 8.58 (s, 1 H) 9.48 (s, 1 H) 9.55 (bs, 1 H) 9.86 (bs, 1 H) 10.60 (s, 1 H)

### EXAMPLE 72

### 6-butyl-1-{2-[(1S,4S)-2,5-diazabicyclo[2.2.1]hept-2-yl]pyridin-4-yl}-N-pyrazin-2-yl-1H-indazol-3-amine

### a) 4-butyl-2-fluorobenzonitrile

Obtained (68%) from 4-bromo-2-fluorobenzonitrile and butylboronic acid using the same experimental procedure as described in example 58a, followed by purification by flash chromatography (100% hexanes to 5% ethyl acetate).

1H NMR (400 MHz, CHLOROFORM-*d*) δ ppm 0.94 (t, *J*=7.23 Hz, 3 H) 1.30 - 1.41 (m, 2 H) 1.55 - 1.65 (m, 2 H) 2.66 (t, *J*=7.82 Hz, 2 H) 7.03 (d, *J*=10.16 Hz, 1 H) 7.07 (d, *J*=7.82 Hz, 1 H) 7.49 - 7.54 (m, 1 H)

### b) 6-butyl-1H-indazol-3-amine

Obtained (44%) from 4-butyl-2-fluorobenzonitrile (example 72a) and hydrazine hydrate using the same experimental procedure as described in preparation 8.

LRMS (m/z): 190 (M+1)⁺.

### c) tert-Butyl (1S,4S)-5-[4-(3-amino-6-butyl-1H-indazol-1-yl)pyridin-2-yl]-2,5-diazabicyclo[2.2.1]heptane-2-carboxylate

Obtained (46%) from 6-butyl-1*H*-indazol-3-amine (example 72b) and *(1S,4S)-tert-*butyl 5-(4-bromopyridin-2-yl)-2,5-diazabicyclo[2.2.1]heptane-2-carboxylate (preparation 3) using the same experimental procedure as described in example 2a.

LRMS (m/z): 463 (M+1)⁺.

### d) tert-butyl (1S,4S)-5-{4-[6-butyl-3-(pyrazin-2-ylamino)-1H-indazol-1-yl]pyridin-2-yl}-2,5-diazabicyclo[2.2.1]heptane-2-carboxylate

Obtained (34%) from *tert*-butyl (1*S*,4*S*)-5-[4-(3-amino-6-butyl-1*H*-indazol-1-yl)pyridin-2-yl]-2,5-diazabicyclo[2.2.1]heptane-2-carboxylate (example 72c) and 2-chloropyrazine using the same experimental procedure as described in example 2b, followed by purification by flash chromatography (100% hexanes to 100% ethyl acetate).

LRMS (m/z): 541 (M+1)⁺.

### e) 6-butyl-1-{2-[(1S,4S)-2,5-diazabicyclo[2.2.1]hept-2-yl]pyridin-4-yl}-N-pyrazin-2-yl-1H-indazol-3-amine

Obtained as a hydrochloride salt (74% yield) from *tert*-butyl (1*S*,4*S*)-5-{4-[6-butyl-3-(pyrazin-2-ylamino)-1*H*-indazol-1-yl]pyridin-2-yl}-2,5-diazabicyclo[2.2.1]heptane-2-carboxylate (example 72d) using the same experimental procedure as described in example 7c.

LRMS (m/z): 441 (M+1)⁺.

¹H NMR (400 MHz, DMSO-*d*₆) δ ppm 0.93 (t, *J*=6.84 Hz, 3 H) 1.28 - 1.44 (m, 2 H) 1.57 - 1.73 (m, 2 H) 2.13 (d, *J*=10.16 Hz, 1 H) 2.26 (d, *J*=12.50 Hz, 1 H) 2.74 - 2.90 (m, 2 H) 3.29 - 3.39 (m, 1 H) 3.40 - 3.50 (m, 1 H) 3.89 (d, *J*=9.38 Hz, 1 H) 4.05 (d, *J*=8.60 Hz, 1 H) 4.66 (s, 1 H) 5.33 (s, 1 H) 7.18 (br. s., 1 H) 7.31 (d, *J*=8.21 Hz, 1 H) 7.57 (d, *J*=3.52 Hz, 1 H) 7.95 (s, 1 H) 8.12 (d, *J*=6.64 Hz, 1 H) 8.24 - 8.35 (m, 2 H) 8.40 (s, 1 H) 9.44 - 9.59 (m, 2 H) 9.92 (br. s., 1 H) 10.76 (s, 1 H).

### EXAMPLE 73

### 1-{2-[(1S,4S)-2,5-diazabicyclo[2.2.1]hept-2-yl]pyridin-4-yl}-6-(3-methylbutyl)-N-pyrazin-2-yl-1H-indazol-3-amine

### a) 2-fluoro-4-(3-methylbutyl)benzonitrile

Obtained (84%) from 4-bromo-2-fluorobenzonitrile and isopentylboronic acid using the same experimental procedure as described in example 58a, followed by purification by flash chromatography (100% hexanes to 5% ethyl acetate).

¹H NMR (400 MHz, CHLOROFORM-*d*) d ppm 0.94 (d, *J*=6.64 Hz, 6 H) 1.46 - 1.54 (m, 2 H) 1.55 - 1.62 (m, 1 H) 2.63 - 2.69 (m, 2 H) 6.99 - 7.09 (m, 2 H) 7.52 (t, *J*=7.23 Hz, 1 H).

### b) 6-(3-methylbutyl)-1H-indazol-3-amine

Obtained (52%) from 2-fluoro-4-(3-methylbutyl)benzonitrile (example 73a) and hydrazine hydrate using the same experimental procedure as described in preparation 8.

LRMS (m/z): 204 (M+1)⁺.

### c) tert-butyl (1S,4S)-5-{4-[3-amino-6-(3-methylbutyl)-1H-indazol-1-yl]pyridin-2-yl}-2,5-diazabicyclo[2.2.1]heptane-2-carboxylate

Obtained (46%) from) 6-(3-methylbutyl)-1*H*-indazol-3-amine (example 73b) and *(1S,4S)-tert* butyl 5-(4-bromopyridin-2-yl)-2,5-diazabicyclo[2.2.1]heptane-2-carboxylate (preparation 3) using the same experimental procedure as described in example 2a, followed by purification by flash chromatography (90% hexanes to 70% ethyl acetate).

LRMS (m/z): 477 (M+1)⁺.

### d) tert-butyl (1S,4S)-5-{4-[6-(3-methylbutyl)-3-(pyrazin-2-ylamino)-1H-indazol-1-yl]pyridin-2-yl}-2,5-diazabicyclo[2.2.1]heptane-2-carboxylate

Obtained (18%) from tert-butyl (1*S*,4*S*)-5-{4-[3-amino-6-(3-methylbutyl)-1*H*-indazol-1-yl]pyridin-2-yl}-2,5-diazabicyclo[2.2.1]heptane-2-carboxylate (example 73c) and 2-chloropyrazine using the same experimental procedure as described in example 2b.

LRMS (m/z): 555 (M+1)⁺.

### e) 1-{2-[(1S,4S)-2,5-diazabicyclo[2.2.1]hept-2-yl]pyridin-4-yl}-6-(3-methylbutyl)-N-pyrazin-2-yl-1H-indazol-3-amine

Obtained as a hydrochloride salt (87% yield) from *tert*-butyl (1*S*,4*S*)-5-{4-[6-(3-methylbutyl)-3-(pyrazin-2-ylamino)-1*H*-indazol-1-yl]pyridin-2-yl}-2,5-diazabicyclo[2.2.1]heptane-2-carboxylate (example 73d) using the same experimental procedure as described in example 7c.

LRMS (m/z): 455 (M+1)⁺.

¹H NMR (400 MHz, dmso) δ 10.76 (s, 1H), 9.97 (s, 1H), 9.55 (s, 1H), 9.50 (s, 1H), 8.39 (s, 1H), 8.31 (d, *J* = 8.3 Hz, 1H), 8.27 (s, 1H), 8.11 (d, *J* = 6.9 Hz, 1H), 7.95 (s, 1H), 7.57 (d, *J* = 6.4 Hz, 1H), 7.30 (d, *J* = 8.3 Hz, 1H), 7.17 (s, 1H), 5.34 (s, 1H), 4.66 (s, 1H), 4.06 (d, *J* = 10.3 Hz, 1H), 3.90 (d, *J* = 10.5 Hz, 1H), 3.47 (m, 1H), 3.35 (m, 1H), 2.84 (d, *J* = 7.6 Hz, 1H), 2.26 (d, *J* = 10.8 Hz, 1H), 2.13 (d, *J* = 10.6 Hz, 1H), 1.57 (m, 3H), 0.95 (d, *J* = 5.8 Hz, 6H).

### EXAMPLE 74

### 1-{2-[4-(methoxymethyl)piperidin-4-yl]pyridin-4-yl}-N-pyrazin-2-yl-1H-indazol-3-amine

### a) tert-butyl 4-(4-bromopyridin-2-yl)-4-(methoxymethyl)piperidine-1-carboxylate

To a solution of *tert*-butyl 4-(4-bromopyridin-2-yl)-4-(hydroxymethyl)piperidine-1-carboxylate (preparation 17, 150 mg, 0.40 mmol) in THF (5 mL) sodium hydride (60%) (32 mg, 0,81 mmol) was added. The mixture was stirred at room temperature for 20 min. Then dimethyl sulfate (0.061 mL, 0.65 mmol) was added and the mixture was stirred at room temperature for 12 hours. After addition of saturated aqueous ammonium chloride solution and ethyl acetate, the organic phase was washed with water, dried over MgSO₄ and concentrated in vacuum to provide the title compound (160 mg, 97%).

LRMS (m/z): 385/387 (M+1)+

¹H NMR (300 MHz, CHLOROFORM-D) δ ppm 1.4 (s, 9 H), 1.8 (m, 2 H), 2.3 (m, *J=*13.5 Hz, 2 H), 3.0 (t, *J*=11.4 Hz, 2 H), 3.2 (s, 3 H), 3.4 (s, 2 H), 3.7 (m, *J*=12.6 Hz, 2 H), 7.3 (m, *J*=5.8 Hz, 1 H), 7.5 (s, 1 H), 8.4 (d, *J*=5.8 Hz, 1 H).

### b) tert-butyl 4-[4-(3-amino-1H-indazol-1-yl)pyridin-2-yl]-4-(methoxymethyl) piperidine-1-carboxylate

Obtained (16%) from 1*H*-indazol-3-amine and *tert*-butyl 4-(4-bromopyridin-2-yl)-4-(methoxymethyl)piperidine-1-carboxylate (example 74a) following the experimental procedure as described in example 2a using toluene as solvent, followed by purification by flash chromatography (1:1 hexanes/ethyl acetate).

LRMS (m/z): 439 (M+1)⁺.

¹H NMR (300 MHz, CHLOROFORM-D) δ ppm 1.5 (s, 9 H) 1.8 (m, 2 H) 2.4 (m, *J*=12.1 Hz,2H)3.1 (t, *J*=11.3 Hz, 2 H) 3.2 (s, 3 H) 3.5 (s, 2 H) 3.8 (m, 2 H) 4.4 (t, 2 H) 7.2 (t, *J*=7.4 Hz, 1 H) 7.5 (m, 2 H) 7.6 (d, *J*=8.0 Hz, 1 H) 7.7 (m, 1 H) 7.8 (d, *J*=8.5 Hz, 1 H) 8.6 (d, *J*=5.5 Hz, 1 H)

### c) tert-Butyl 4-(methoxymethyl)-4-{4-[3-(pyrazin-2-ylamino)-1H-indazol-1-yl]pyridin-2-yl}piperidine-1-carboxylate

Obtained (61 %) from *tert-*butyl 4-[4-(3-amino-1*H*-indazol-1-yl)pyridin-2-yl]-4-(methoxymethyl)piperidine-1-carboxylate (example 74b) and 2-chloropyrazine following the experimental procedure as described in example 2b, followed by purification by flash chromatography (9:1 hexanes/ethyl acetate to 100% ethyl acetate).

LRMS (m/z): 517 (M+1)⁺.

¹H NMR (300 MHz, CHLOROFORM-D) δ ppm 1.5 (s, 9 H) 1.8 (s, 2 H) 2.4 (d, *J*=12.4 Hz, 2 H) 3.2 (t, *J*=1 0.6 Hz, 2 H) 3.3 (s, 3 H) 3.5 (s, 2 H) 3.8 (m, 2 H) 7.3 (m, 1 H) 7.6 (m, *J*=12.9 Hz, 1 H), 7.8 (m, *J*=8.0 Hz, 2 H), 7.9 (d, *J*=8.5 Hz, 1 H), 8.2 (s, 2 H), 8.7 (d, *J*=5.5 Hz, 2 H), 9.6 (s, 1 H).

### d) 1-{2-[4-(Methoxymethyl)piperidin-4-yl]pyridin-4-yl}-N-pyrazin-2-yl-1H-indazol-3-amine

Obtained as a hydrochloride salt (59%) from *tert*-butyl 4-(methoxymethyl)-4-{4-[3-(pyrazin-2-ylamino)-1*H*-indazol-1-yl]pyridin-2-yl}piperidine-1-carboxylate (example 74c) using the same experimental procedure as described in example 2c. The solid was filtered off and washed several times with diethyl ether and dried in the vacuum oven overnight.

LRMS (m/z): 416 (M+1)⁺.

¹H NMR (300 MHz, DMSO-D6) δ ppm 2.2 (m, 2 H), 2.5 (m, 2 H), 3.0 (m, 2 H), 3.2 (s, 3 H), 3.6 (s, 2 H), 3.8 (m, 2 H), 7.4 (t, *J*=7.7 Hz, 1 H), 7.7 (t, *J*=7.3 Hz, 1 H), 8.0 (s, 1 H), 8.1 (d, *J*=8.8 Hz, 1 H), 8.3 (d, *J*=2.2 Hz, 1 H), 8.4 (m, 2 H), 8.7 (d, *J*=6.0 Hz, 1 H), 8.9 (s, 1 H), 9.5 (s, 1 H), 10.8 (s, 1 H).

### EXAMPLE 75

### 6-butoxy-1-{2-[(1S,4S)-2,5-diazabicyclo[2.2.1]hept-2-yl]pyridin-4-yl}-N-pyrazin-2-yl-1H-indazol-3-amine

### a) 4-butoxy-2-fluorobenzonitrile

Obtained (99%) from 2-fluoro-4-hydroxybenzonitrile and 1-bromobutane following the experimental procedure as described in preparation 18a.

¹H NMR (400 MHz, CHLOROFORM-*d*) δ ppm 0.95 (t, *J*=7.42 Hz, 3 H), 1.40 - 1.52 (m, 2 H), 1.71 - 1.83 (m, 2 H), 3.97 (t, *J*=6.45 Hz, 2 H), 6.67 (dd, *J*=10.94, 2.54 Hz, 1 H), 6.73 (dd, *J*=8.79, 2.54 Hz, 1 H), 7.47 (dd, *J*=8.79, 7.62 Hz, 1 H).

### b) 6-butoxy-1H-indazol-3-amine

Obtained (45%) from 4-butoxy-2-fluorobenzonitrile (example 75a) and hydrazine following the experimental procedure as described in preparation 18b.

LRMS (m/z): 206 (M+1)⁺.

### c) tert-butyl (1S,4S)-5-[4-(3-amino-6-butoxy-1H-indazol-1-yl)pyridin-2-yl]-2,5-diazabicyclo[2.2.1 ]heptane-2-carboxylate

Obtained (38%) from 6-butoxy-1*H*-indazol-3-amine (example 75b) and *(1S,4S)-tert-*Butyl 5-(4-bromopyridin-2-yl)-2,5-diazabicyclo[2.2.1]heptane-2-carboxylate (preparation 3) following the experimental procedure as described in example 2a, followed by purification by reverse phase chromatography as described in the general methods.

LRMS (m/z): 479 (M+1)⁺.

### d) tert-butyl (1S,4S)-5-{4-[6-butoxy-3-(pyrazin-2-ylamino)-1H-indazol-1-yl]pyridin-2-yl}-2,5-diazabicyclo[2.2.1]heptane-2-carboxylate

Obtained (8%) from *tert*-butyl (1*S*,4*S*)-5-[4-(3-amino-6-butoxy-1*H*-indazol-1-yl)pyridin-2-yl]-2,5-diazabicyclo[2.2.1]heptane-2-carboxylate (example 75c) and 2-chloropyrazine using the same experimental procedure as described in example 2b, followed by purification by reverse phase chromatography as described in the general methods.

LRMS (m/z): 557 (M+1)⁺.

### e) 6-butoxy-1-{2-[(1S,4S)-2,5-diazabicyc)o[2.2.1]hept-2-yl]pyridin-4-y)}-N-pyrazin-2-yl-1H-indazol-3-amine

Obtained as a hydrochoride salt (97%) from *tert*-butyl (1*S*,4*S*)-5-{4-[6-butoxy-3-(pyrazin-2-ylamino)-1*H*-indazol-1-yl]pyridin-2-yl}-2,5-diazabicyclo[2.2.1]heptane-2-carboxylate (example 75d) using the same experimental procedure as described in example 7c.

LRMS (m/z): 429 (M+1)⁺.

1H NMR (400 MHz, DMSO-*d*₆) δ ppm 0.97 (t, *J*=7.42 Hz, 3 H), 1.43 - 1.56 (m, 2 H), 1.72 - 1.83 (m, 2 H), 2.07 (d, *J*=10.46 Hz, 1 H), 2.24 (d, *J*=10.46 Hz, 1 H), 3.27 - 3.37 (m, 1 H), 3.37 - 3.45 (m, 1 H), 3.80 - 3.88 (m, 2 H), 4.18 (t, *J*=6.45 Hz, 2 H), 4.62 (s, 1 H), 5.19 (s, 1 H), 7.04 (d, *J*=8.21 Hz, 1 H), 7.16 (br. s., 1 H), 7.42 - 7.50 (m, 2 H), 8.15 (d, *J*=6.64 Hz, 1 H), 8.26 (m, 2 H), 8.38 (s, 1 H), 9.19 (br. s., 1 H), 9.51 (s, 1 H), 9.59 (br. s., 1 H), 10.66 (s, 1 H).

### EXAMPLE 76

### 6-Methoxy-1-{2-[(2S)-2-methylpiperazin-1-yl] pyridin-4-yl}-N-pyrazin-2-yl-1H-indazol-3-amine

### a) tert-butyl (3S)-4-[4-(3-amino-6-methoxy-1H-indazol-1-yl)pyridin-2-yl]-3-methylpiperazine-1-carboxylate

Obtained (42%) from 6-methoxy-1*H*-indazol-3-amine (preparation 8) and *tert*-butyl (3*S*)-4-(4-bromopyridin-2-yl)-3-methylpiperazine-1-carboxylate (preparation 10) using the same experimental procedure as described in example 2a.

LRMS (m/z): 439 (M+1)⁺.

### b) tert-butyl (3S)-4-{4-[6-methoxy-3-(pyrazin-2-ylamino)-1H-indazol-1-yl]pyridin-2-yl}-3-methylpiperazine-1-carboxylate

Obtained as a brown oil (53%) from *tert*-butyl (3*S*)-4-[4-(3-amino-6-methoxy-1*H-*indazol-1-yl)pyridin-2-yl]-3-methylpiperazine-1-carboxylate (example 76a) and 2-chloropyrazine using the same experimental procedure as described in example 2b.

LRMS (m/z): 517 (M+1)⁺.

### c) 6-methoxy-1-{2-[(2S)-2-methylpiperazin-1-yl]pyridin-4-yl}-N-pyrazin-2-yl-1H indazol-3-amine

Obtained as a free base (32%) from *tert*-butyl (3*S*)-4-{4-[6-methoxy-3-(pyrazln-2-ylamino)-1*H*-indazol-1-yl]pyridin-2-yl}-3-methylpiperazine-1-carboxylate
(example 76b) using the same experimental procedure as described in example 7c. The crude was neutralized and purified by reverse phase chromatography as described in the general methods without buffer.

LRMS (m/z): 417 (M+1)⁺.

¹H NMR (400 MHz, CDCl₃) δ 9.52 (d, *J* = 0.9 Hz, 1H), 8.28 (d, *J* = 5.5 Hz, 1H), 8.20 (s, 2H), 7.58 (d, *J* = 8.8 Hz, 1H), 7.50 (s, 1H), 7.23 (d, *J* = 2.0 Hz, 1H), 7.02 (dd, *J* = 5.5, 1.8 Hz, 1H), 6.97 (d, *J* = 1.6 Hz, 1H), 6.90 (dd, *J* = 8.8, 2.0 Hz, 1H), 4.57 - 4.46 (m, 1H), 4.08 - 4.00 (m, 1H), 3.92 (s, 3H), 3.20 - 3.08 (m, 3H), 2.97 (d, *J* = 12.2 Hz, 1H), 2.94 - 2.85 (m, 1H), 1.28 (d, *J* = 6.7 Hz, 3H).

### EXAMPLE 77

### 1-{2-[(1S,4S)-2,5-diazabicyclo[2.2.1]hept-2-yl]pyridin-4-yl}-6-isopropoxy-N-pyrazin-2-yl-1H-indazol-3-amine

### a) 2-fluoro-4-isopropoxybenzonitrile

An oven dried resealable Schlenk tube was charged with 2-fluoro-4-hydroxybenzonitrile (1.00 g, 7.29 mmol), propan-2-ol (1.24 ml, 16.09 mmol), triphenylphosphine (4.20 g, 16.01 mmol), diisopropylethylamine (5.20 ml, 29.85 mmol) and THF (10 mL). The Schlenk tube was subjected to three cycles of evacuation-backfilling with nitrogen, the reaction mixture cooled with ice bath and diisopropyl (*E*)-diazene-1,2-dicarboxylate (3.50 ml, 17.78 mmol) was added dropwise. The reaction mixture was then stirred at room temperature overnight. The solid formed was filtered off and the filtrate concentrated under vacuum. Ethyl acetate and water were added to the crude and the organic layer was washed with brine, dried (Na₂SO₄) and evaporated to give the desired compound (1.43 g, 99%) as colorless oil.

1H NMR (400 MHz, CDCl₃) δ ppm 1.34 (d, *J*=5.86 Hz, 6 H), 4.51 - 4.61 (m, 1 H), 6.64 (dd, *J*=11.33, 2.34 Hz, 1 H), 6.69 (dd, *J*=8.99, 2.34 Hz, 1 H), 7.46 (dd, *J*=8.60, 7.42 Hz, 1 H).

### b) 6-isopropoxy-1H-indazol-3-amine

Obtained (20%) from 2-fluoro-4-isopropoxybenzonitrile (example 77a) and hydrazine following the experimental procedure as described in preparation 18b.

LRMS (m/z): 192 (M+1)⁺.

### c) tert-butyl (1S,4S)-5-[4-(3-amino-6-isopropoxy-1H-indazol-1-yl)pyridin-2-yl]-2,5-diazabicyclo[2.2.1]heptane-2-carboxylate

Obtained (27%) from 6-isopropoxy-1*H*-indazol-3-amine (example 77b) and *(1S,4S)-tert*-Butyl 5-(4-bromopyridin-2-yl)-2,5-diazabicyclo[2.2.1]heptane-2-carboxylate (preparation 3) following the experimental procedure as described in example 2a, followed by purification by reverse phase chromatography as described in the general methods.

LRMS (m/z): 465 (M+1)⁺.

### d) tert-butyl (1S,4S)-5-{4-[6-isopropoxy-3-(pyrazin-2-ylamino)-1H-indazol-1-yl]pyridin-2-yl}-2,5-diazabicyclo[2.2.1]heptane-2-carboxylate

Obtained (45%) from *tert*-butyl (1*S*,4*S*)-5-[4-(3-amino-6-isopropoxy-1*H*-indazol-1-yl)pyridin-2-yl]-2,5-diazabicyclo[2.2.1 ]heptane-2-carboxylate (example 77c) and 2-chloropyrazine using the same experimental procedure as described in example 2b, followed by purification by reverse phase chromatography as described in the general methods.

LRMS (m/z): 543 (M+1)⁺.

### e) 1-{2-[(1S,4S)-2,5-diazabicyclo[2.2.1]hept-2-yl]pyridin-4-yl}-6-isopropoxy-N-pyrazin-2-yl-1H-indazol-3-amine

Obtained as a hydrochoride salt (97%) from *tert*-butyl (1*S*,4*S*)-5-{4-[6-isopropoxy-3-(pyrazin-2-ylamino)-1*H*-indazol-1-yl]pyridin-2-yl}-2,5-diazabicyclo[2.2.1 ]heptane-2-carboxylate (example 77d) using the same experimental procedure as described in example 7c.

LRMS (m/z): 443 (M+1)⁺.

¹H NMR (400 MHz, DMSO-*d*₆) δ ppm 1.42 (d, *J*=5.90 Hz, 6 H), 2.07 (d, *J*=10.46 Hz, 1 H), 2.24 (d, *J*=10.46 Hz, 1 H), 3.27 - 3.37 (m, 1 H), 3.37 - 3.45 (m, 1 H), 3.91 - 4.12 (m, 2 H), 4.63 (m, 1 H), 4.92 (s, 1 H), 5.39 (s, 1 H), 7.04 - 7.58 (m, 5 H), 8.15 (d, *J*=6.64 Hz, 1 H), 8.28 (m, 2 H), 9.58 (m, 2 H), 9.98 (br. s., 1 H), 10.76 (s, 1 H).

### EXAMPLE 78

### 6-(benzyloxy)-1-{2-[(2S)-2-methylpiperazin-1-yl]pyridin-4-yl}-N-pyrazin-2-yl-1H-indazol-3-amine

### a) tert-Butyl (35)-4-{4-[3-amino-6-(benzyloxy)-1H-indazol-1-yl]pyridin-2-yl}-3-methylpiperazine-1-carboxylate

Obtained (42%) from 6-(benzyloxy)-1*H*-indazol-3-amine (preparation 18) and *(S)-tert* butyl 4-(4-bromopyridin-2-yl)-3-methylpiperazine-1-carboxylate (preparation 10) following the experimental procedure described in example 2a, but with dioxane as a solvent.

LRMS (m/z): 515 (M+1)⁺.

### b) tert-Butyl (3S)-4-{4-[6-(benzyloxy)-3-(pyrazin-2-ylamino)-1H-indazol-1-yl]pyridin-2-yl}-3-methylpiperazine-1-carboxylate

Obtained (41%) from *tert-*butyl (3*S*)*-*4-{4-[3-amino-6-(benzyloxy)-1*H*-indazol-1-yl]pyridin-2-yl}-3-methylpiperazine-1-carboxylate (example 78a) and 2-chloropirazine using the same experimental procedure as described in example 2b.

LRMS (m/z): 593 (M+1)⁺.

### c) 6-(Benzyloxy)-1-{2-[(2S)-2-methylpiperazin-1-yl]pyridin-4-yl}-N-pyrazin-2-yl-1H-indazol-3-amine

Obtained (36%) from *tert*-butyl (3S)-4-{4-[6-(benzyloxy)-3-(pyrazin-2-ylamino)-1*H-*indazol-1-yl]pyridin-2-yl}-3-methylpiperazine-1-carboxylate (example 78b) using the same experimental pocedure as described in example 7c, followed by a purification by reverse phase chromatography described in the general methods without buffer. After concentration of the relevant fractions the oil obtained was dissolved in dioxane and was added a solution 4M of hydrochloric acid. The mixture was stirred at room temperature for 2 h. The solid was filtered off and washed several times with diethyl ether to give the desired compound.

LRMS (m/z): 493 (M+1)⁺.

¹H NMR (400 MHz, DMSO-*d*₆) δ ppm 1.44 (d, *J*=6.64 Hz, 3 H), 3.05 - 3.20 (m, 1 H), 3.26 - 3.45 (m, 3 H), 3.50 - 3.64 (m, 1 H), 4.85 (s, 1 H), 5.33 (s, 2 H), 7.31 - 7.40 (m, 2 H), 7.44 (t, *J*=7.23 Hz, 2 H), 7.46 - 7.64 (m, 4 H), 8.18 (d, *J*=6.64 Hz, 1 H), 8.23 - 8.33 (m, 1 H), 8.35 - 8.46 (m, 1 H), 9.46 (s, 1 H), 9.50 (s, 1 H), 9.86 (s, 1 H), 10.72 (s, 1 H).

### EXAMPLE 79

### 6-(Benzyloxy)-1-[2-(1,4-diazepan-1-yl)pyridin-4-yl]-N-pyrazin-2-yl-1H-indazol-3-amine

### a) tert-Butyl 4-{4-[3-amino-6-(benzy)oxy)-1H-indazol-1-yl]pyridin-2-yl}-1,4-diazepane-1-carboxylate

Obtained (36%) from 6-(benzyloxy)-1*H*-indazol-3-amine (preparation 18) and *tert*-Butyl 4-(4-bromopyridin-2-yl)-1,4-diazepane-1-carboxylate (preparation 14) following the same experimental procedure as described in example 2a, but with dioxane as a solvent.

LRMS (m/z): 515 (M+1)⁺.

### b) tert-Butyl 4-{4-[6-(benzyloxy)-3-(pyrazin-2-ylamino)-1H-indazol-1-yl]pyridin-2-yl}-1,4-diazepane-1-carboxylate

Obtained as a yellow oil (67%) from *tert*-butyl 4-{4-[3-amino-6-(benzyloxy)-1*H*-indazol-1-yl]pyridin-2-yl}-1,4-diazepane-1-carboxylate (example79a) and 2-chloropirazine using the same experimental as described in example 2b, followed by purification by reverse phase chromatography in the general methods.

### c) 6-(Benzyloxy)-1-[2-(1,4-diazepan-1-yl)pyridin-4-yl]-N-pyrazin-2-yl-1H-indazol-3-amine

Obtained as a hydrochloride salt (16%) from *tert*-butyl 4-{4-[6-(benzyloxy)-3-(pyrazin-2-ylamino)-1*H*-indazol-1-yl]pyridin-2-yl}-1,4-diazepane-1-carboxylate (example 79b) using the same experimental procedure as described in example 7c.

LRMS (m/z): 493 (M+1)⁺.

¹H NMR (400 MHz, DMSO-*d*₆) δ ppm 2.20 - 2.29 (m, 2 H), 2.44 - 2.53 (m, 2 H), 3.23 - 3.36 (m, 2 H), 3.85 - 3.93 (m, 2 H), 4.12 - 4.20 (m, 2 H), 5.25 - 5.42 (m, 2 H), 7.18 (d, *J*=8.60 Hz, 1 H), 7.27 - 7.31 (m, 1 H), 7.39 - 7.52 (m, 3 H), 7.52 - 7.64 (m, 3 H), 8.19 (d, *J*=7.03 Hz, 1 H), 8.28 - 8.36 (m, 2 H), 8.43 (s, 1 H), 9.31 (br. s., 2 H), 9.55 (s, 1 H), 10.74(s, 1H).

### EXAMPLE 80

### 1-(5-Piperazin-1-ylpyridin-3-yl)-N-pyridi n-2-yl-1H-indazol-3-amine

### a) tert-Butyl 4-{5-[3-(pyridin-2-ylamino)-1H-indazol-1-yl]pyridin-3-yl}piperazine-1-carboxylate

A mixture of *tert-*butyl 4-[5-(3-chloro-1*H*-indazol-1-yl)pyridin-3-yl]piperazine-1-carboxylate (Example 4a), pyridine-2-amine, cesium carbonate, Pd₂(dba)₃ and 4,5-bis(diphenylphosphino)-9,9-dimethylxanthene in dioxane was heated at 120 °C for 4 h under microwave conditions. The mixture was then cooled and filtered, and the filtrate was concentrated in vacuum. The crude was dissolved in ethyl acetate, washed with water, the organic extracts dried over Na₂SO₄ and concentrated under vacuum. Purification of the residue by flash chromatography (gradient of dichloromethane-methanol up to 5%) gave the title compound (42%).

LRMS (m/z): 472 (M+1)⁺.

### b) 1-(5-Piperazin-1-ylpyridin-3-yl)-N-pyridin-2-yl-1H-indazol-3-amine

Obtained as a hydrochloride salt (29%) from *tert*-butyl 4-{5-[3-(pyridin-2-ylamino)-1*H-*indazol-1-yl]pyridin-3-yl]piperazine-1-carboxylate (Example 80a) using the same experimental procedure as described in example 7c.

LRMS (m/z): 372 (M+1)⁺.

¹H NMR (400 MHz, DMSO-d₆) d ppm 3.60 (s, 4 H), 3.76 - 3.84 (m, *J*=4.69 Hz, 4 H), 7.35 (t, *J*=6.64 Hz, 1 H), 7.47 (t, *J*=7.62 Hz, 1 H), 7.73 (t, *J*=7.62 Hz, 1 H), 8.06 (d, *J*=8.60 Hz, 1 H), 8.18 (d, *J*=8.99 Hz, 1 H), 8.27 - 8.34 (m, 2 H), 8.46 - 8.55 (m, 2 H), 8.63 (d, *J*=8.21 Hz, 1 H), 8.89 (s, 1 H), 9.61 (s, 2 H).

### EXAMPLE 81

### 1-(5-((1S,4S)-2,5-diazabicyclo[2.2.1]heptan-2-yl)pyridin-3-yl)-5-fluoro-6-methoxy-N-(pyrazin-2-yl)-1 H-indazol-3-amine

### a) 5-fluoro-6-methoxy-1H-indazol-3-amine

Obtained (71%) from 2,5-difluoro-4-methoxybenzonitrile and hydrazine hydrate using the same experimental procedure as described in preparation 8.

LRMS (m/z): 182 (M+1)⁺.

¹H NMR (400 MHz, dmso) δ 11.24 (s, 1H), 7.40 (d, *J* = 11.3 Hz, 1H), 6.77 (d, *J* = 7.1 Hz, 1H), 5.18 (s, 2H), 3.81 (s, 3H).

### b) tert-butyl (1S,4S)-5-[5-(3-amino-5-fluoro-6-methoxy-1H-indazol-1-yl)pyridin-3-yl]-2,5-diazabicyclo[2.2.1]heptane-2-carboxylate

Obtained (51%) from 5-fluoro-6-methoxy-1*H*-indazol-3-amine (example 81 a) and *(1S,4S)-tert-*butyl 5-(5-bromopyridin-3-yl)-2,5-diazabicyclo[2.2.1]heptane-2-carboxylate (preparation 11) using the same experimental procedure as described in example 2a, followed by purification by flash chromatography (50% hexanes to 100% ethyl acetate).

LRMS (m/z): 455 (M+1)⁺.

### c) tert-butyl (1S,4S)-5-{5-[5-fluoro-6-methoxy-3-(pyrazin-2-ylamino)-1H-indazol-1-yl]pyridin-3-yl}-2,5-diazabicyclo[2.2.1]heptane-2-carboxylate

Obtained (58%) from *tert*-butyl (1*S*,4*S*)-5-[5-(3-amino-5-fluoro-6-methoxy-1*H*-indazol-1-yl)pyridin-3-yl]-2,5-diazabicyclo[2.2.1]heptane-2-carboxylate (example 81 b) and 2-chloropyrazine using the same experimental procedure as described in example 2b, followed by purification by flash chromatography (50% hexanes to 100% ethyl acetate).

LRMS (m/z): 533 (M+1)⁺.

### d) 1-{5-[(1S,4S)-2,5-diazabicyclo[2.2.1]hept-2-yl]pyridin-3-yl}-5-fluoro-6-methoxy-N-pyrazin-2-yl-1H-indazol-3-amine

Obtained as a hydrochloride salt (98% yield) from *tert*-butyl (1*S*,4*S*)-5-{5-[5-fluoro-6-methoxy-3-(pyrazin-2-ylamino)-1*H*-indazol-1-yl]pyridin-3-yl}-2,5-diazabicyclo[2.2.1]heptane-2-carboxylate (example 81 c) using the same experimental procedure as described in example 7c.

LRMS (m/z): 433 (M+1)⁺.

¹H NMR (400 MHz, dmso) δ 10.52 (s, 1H), 9.79 (bs, 1H), 9.61 (bs, 1H), 9.44 (d, *J*= 1.4 Hz, 1H), 8.61 (d, *J* = 1.7 Hz, 1H), 8.34 (dd, *J* = 2.5, 1.5 Hz, 1H), 8.22 (m, 2H), 8.16 (d, *J*= 11.1 Hz, 1H), 7.91 (s, 1H), 7.56 (d, *J* = 6.9 Hz, 1H), 5.01 (s, 1H), 4.58 (s, 1H), 4.03 (s, 3H), 3.85 (d, *J* = 10.7 Hz, 1H), 3.74 (d, *J* = 10.2 Hz, 1H), 3.34 (m, 1H), 3.26 (m, 1H), 2.20 (d, *J*= 11.0 Hz, 1H), 2.06 (d, *J*= 10.6 Hz, 1H).

### EXAMPLE 82

### 1-{5-[(1S,4S)-2,5-diazabicyclo[2.2.1]hept-2-yl]pyridin-3-yl}-5-fluoro-3-(pyrazin-2-ylamino)-1H-indazol-6-ol

Obtained (21%) from 1-{5-[(1*S*,4*S*)-2,5-diazabicyclo[2.2.1]hept-2-yl]pyridin-3-yl}-5-fluoro-6-methoxy-*N*-pyrazin-2-yl-1*H*-indazol-3-amine (example 81) using the same experimental procedure as described in example 53, followed by purification by reverse phase chromatography as described in the general methods without buffer.

LRMS (m/z): 419 (M+1)⁺.

¹H NMR (400 MHz, dmso) δ 11.10 (s, 1H), 10.49 (s, 1H), 10.02 (s, 1H), 9.44 (m, 2H), 8.47 (d, *J* = 1.6 Hz, 1H), 8.34 (dd, *J* = 2.5, 1.6 Hz, 1H), 8.22 (d, *J* = 2.5 Hz, 1H), 8.17 (d, *J* = 0.8 Hz, 1H), 8.10 (d, *J* = 10.9 Hz, 1H), 7.78 (s, 1H), 7.69 (d, *J* = 7.0 Hz, 1H), 5.07 (s, 1H), 4.57 (s, 1H), 3.81 - 3.71 (m, 2H), 3.33 (m, 2H), 2.19 (d, *J* = 10.6 Hz, 1H), 2.07 (d, *J* = 10.6 Hz, 1H).

### EXAMPLE 83

### (4-{4-[3-(Pyrazin-2-ylamino)-1H-indazol-1-yl]pyridin-2-yl}piperidin-4-yl)methanol

### a) tert-Butyl 4-[4-(3-amino-1H-indazol-1-yl)pyridin-2-yl]-4-(hydroxymethyl)piperidine-1-carboxylate

Obtained (14%) from 1*H*-indazol-3-amine and *tert*-butyl 4-(4-bromopyridin-2-yl)-4-(hydroxymethyl)piperidine-1-carboxylate (preparation 17b) using the same experimental procedure as described in preparation 9a using toluene as a solvent.

LRMS (m/z): 424 (M+1)⁺.

### b) tert-Butyl 4-(hydroxymethyl)-4-{4-[3-(pyrazin-2-ylamino)-1H-indazol-1-yl]pyridin-2-yl}piperidine-1-carboxylate

Obtained (27% yield) from *tert*-butyl 4-[4-(3-amino-1*H*-indazol-1-yl)pyridin-2-yl]-4-(hydroxymethyl)piperidine-1-carboxylate (example 83a) and 2-chloropyrazine following the experimental procedure described in example 2b, followed by purification by flash chromatography (hexanes/ethyl acetate).

LRMS (m/z): 502 (M+1)⁺

### c) (4-{4-[3-(Pyrazin-2-ylamino)-1H-indazol-1-yl]pyridin-2-yl}piperidin-4-yl)methanol

Obtained as a hydrochloride salt (99% yield) from *tert*-butyl 4-(hydroxymethyl)-4-{4-[3-(pyrazin-2-ylamino)-1*H*-indazol-1-yl]pyridin-2-yl}piperidine-1-carboxylate (example 83b) using the same experimental procedure as described in example 7c.

LRMS (m/z): 402 (M+1)⁺.

¹H NMR (300 MHz, dmso) δ 2.20 (brs, 2H), 2.58 (d, J = 13.9 Hz, 2H), 2.99 (brs, 2H), 3.27 (brs, 2H), 3.71 (brs, 2H), 7.49 (d, J = 7.7 Hz, 1H), 7.76 (d, J = 6.7 Hz, 1H), 8.08 (s, 1H), 8.22 (d, J = 6.6 Hz, 2H), 8.32 (s, 1H), 8.53 - 8.38 (m, 2H), 8.71 (d, J = 7.2 Hz, 1H), 9.01 (s, 2H), 10.91 (s, 1H).

### EXAMPLE 84

### 6-methyl-1-{2-[(2S)-2-methylpiperazin-1-yl]pyridin-4-yl}-N-pyrazin-2-yl-1H-indazol-3-amine

### a) tert-Butyl (3S)-4-[4-(3-amino-6-methyl-1H-indazol-1-yl)pyridin-2-yl]-3-methylpiperazine-1-carboxylate

Obtained (17%) from 6-methyl-1*H*-indazol-3-amine (preparation 16) and *tert-*butyl (3*S*)-4-(4-bromopyridin-2-yl)-3-methylpiperazine-1-carboxylate (preparation 10) using the same experimental procedure as described in example 2a.

LRMS (m/z): 423 (M+1)⁺.

### b) tert-Butyl (3S)-3-methyl-4-{4-[6-methyl-3-(pyrazin-2-ylamino)-1H-indazol-1-yl]pyridin-2-yl}piperazine-1-carboxylate

Obtained (48%) from *ter*t-butyl (3*S*)-4-[4-(3-amino-6-methyl-1*H*-indazol-1-yl)pyridin-2-yl]-3-methylpiperazine-l-carboxylate (example 84a) and 2-chloropyrazine using the same experimental procedure as described in example 2b.

LRMS (m/z): 501 (M+1)⁺.

### c) 6-Methyl-1-{2-[(2S)-2-methylpiperazin-1-yl]pyridin-4-yl}-N-pyrazin-2-yl-1H-indazol-3-amine

Obtained as a hydrochloride salt (25% yield) from *tert*-butyl (3*S*)-3-methyl-4-{4-[6-methyl-3-(pyrazin-2-ylamino)-1*H*-indazol-1-yl]pyridin-2-yl}piperazine-1-carboxylate (example 84b) using the same experimental procedure as described in example 7c, followed by purification by reverse phase chromatography as described in the general method, without buffer.

LRMS (m/z): 401 (M+1)⁺.

¹H NMR (400 MHz, DMSO-*d*₆) δ ppm 1.28 (d, *J*=7.03 Hz, 3 H), 2.52 (s, 3 H), 3.08 (br. s., 2 H), 3.32 (br. s., 3 H), 4.32 (br. s., 1 H), 4.84 (br. s., 1 H), 7.16 (d, *J*=7.82 Hz, 2 H), 7.29 (d, *J*=5.08 Hz, 1 H), 7.81 (s, 1 H), 8.12 - 8.22 (m, 2 H), 8.26 (d, *J*=5.47 Hz, 1 H), 8.33 (s, 1 H), 8.76 (br. s., 1 H), 9.19 (br. s., 1 H), 9.46 (s, 1 H), 10.49 (s, 1 H).

### EXAMPLE 85

### 1-[6-[(1S,4S)-2,5-diazabicyclo[2.2.1]hept-2-yl]-2-(3-fluorophenyl)pyrimidin-4-yl]-6-methoxy-N-pyrazin-2-yl-1H-indazol-3-amine

### a) 2-(3-fluorophenyl)pyrimidine-4,6-diol

Obtained (80%) from 3-fluorobenzenecarboximidamide and diethyl malonate using the same experimental procedure as described in example 43a.

LRMS (m/z): 207 (M+1)⁺.

### b) 4,6-dichloro-2-(3-fluorophenyl)pyrimidine

Obtained (44%) from 2-(3-fluorophenyl)pyrimidine-4,6-diol using the same experimental procedure as described in example 43b.

LRMS (m/z): 243/245 (M+1)⁺.

### c) tert-butyl (1S,4S)-5-[6-chloro-2-(3-fluorophenyl)pyrimidin-4-yl]-2,5-diazabicyclo[2.2.1]heptane-2-carboxylate

Obtained (94%) from 4,6-dichloro-2-(3-fluorophenyl)pyrimidine (example 85b) and (1*S*,4*S*)-*tert*-butyl 2,5-diazabicyclo[2.2.1 ]heptane-2-carboxylate using the same experimental procedure as described in example 43c.

LRMS (m/z): 405 (M+1)⁺.

### d) tert-butyl (1S,4S)-5-[2-(3-fluorophenyl)-6-iodopyrimidin-4-yl]-2,5-diazabicyclo[2.2.1]heptane-2-carboxylate

Obtained (45%) from *tert*-butyl (1*S*,4*S*)-5-[6-chloro-2-(3-fluorophenyl)pyrimidin-4-yl]-2,5-diazabicyclo[2.2.1]heptane-2-carboxylate (example 85c) using the same experimental procedure as described in example 43d.

LRMS (m/z): 497 (M+1)⁺.

### e) tert-butyl (1S,4S)-5-[6-(3-amino-6-methoxy-1H-indazol-1-yl)-2-(3-fluorophenyl)pyrimidin-4-yl]-2,5-diazabicyclo[2.2.1]heptane-2-carboxylate

Obtained (79%) from 6-methoxy-1*H*-indazol-3-amine (preparation 8) and *tert*-butyl (1*S*,4*S*)-5-[2-(3-fluorophenyl)-6-iodopyrimidin-4-yl]-2,5-diazabicyclo[2.2.1]heptane-2-carboxylate (example 85d) using the same experimental procedure as described in example 2a, followed by purification by flash chromatography (100% hexanes to 80% ethyl acetate).

LRMS (m/z): 532 (M+1)⁺.

### f) tert-butyl (1S,4S)-5-{2-(3-fluorophenyl)-6-[6-methoxy-3-(pyrazin-2-ylamino)-1H-indazol-1-yl]pyrimidin-4-yl}-2,5-diazabicyclo[2.2.1]heptane-2-carboxylate

Obtained (52%) from *tert*-butyl (1*S*,4*S*)-5-[6-(3-amino-6-methoxy-1*H*-indazol-1-yl)-2-(3-fluorophenyl)pyrimidin-4-yl]-2,5-diazabicyclo[2.2.1]heptane-2-carboxylate (example 85e) and 2-chloropyrazine using the same experimental procedure as described in example 2b, followed by purification by flash chromatography (80% hexanes to 100% ethyl acetate).

LRMS (m/z): 610 (M+1)⁺.

### g) 1-[6-[(1S,4S)-2,5-diazabicyclo[2.2.1]hept-2-yl]-2-(3-fluorophenyl)pyrimidin-4-yl]-6-methoxy-N-pyrazin-2-yl-1H-indazol-3-amine

Obtained as a free base (66% yield) from tert-butyl (1 S,4S)-5-{2-(3-fluorophenyl)-6-[6-methoxy-3-(pyrazin-2-ylamino)-1H-indazol-1-yl]pyrimidin-4-yl}-2,5-diazabicyclo[2.2.1]heptane-2-carboxylate (example 85f) using the same experimental procedure as described in example 7c. The crude was neutralized and purified by reverse phase chromatography as described in the general methods without buffer.

LRMS (m/z): 510(M+1)⁺.

¹H NMR (400 MHz, DMSO-*d₆*) δ ppm 1.65 - 1.93 (m, 2 H), 2.86 (m, 1 H), 2.96 (d, *J*=9.38 Hz, 1H), 3.95 (s, 3 H), 5.17 (b s, 1 H), 6.50 (br s, 1 H), 6.92 (dd, *J*=8.99, 2.34 Hz, 1 H), 7.31 - 7.42 (m, 1 H), 7.53 - 7.63 (m, 1 H), 8.13 (m, 2 H), 8.20 (d, *J*=2.34 Hz, 1 H), 8.25 (d, *J*=7.82 Hz, 1 H), 8.32 (s, 1 H), 8.43 (d, *J*=1.95 Hz, 1 H), 9.45 (s, 1 H), 10.54 (br s, 1 H).

### EXAMPLE 86

### 1-[2-(1,4-diazepan-1-yl)pyridin-4-yl]-3-(pyrazin-2-ylamino)-1H-indazol-6-ol

### a) tert-butyl 4-{4-[6-hydroxy-3-(pyrazin-2-ylamino)-1H-indazol-1-yl]pyridin-2-yl}-1,4-diazepane-1-carboxylate

*Tert*-butyl 4-{4-[6-(benzyloxy)-3-(pyrazin-2-ylamino)-1*H*-indazol-1-yl]pyridin-2-yl}-1,4-diazepane-1-carboxylate (example 79b, 20 mg, 0.03 mmol) was dissolved in methanol (2 mL) and palladium on activated charcoal (10%) was added. The mixture was hydrogenated at 15 psi for 16 h. The catalyst was filtered off and the filtrate concentrated in vacuum. The crude was purified by reverse phase chromatography as described in the general method, without buffer. The title compound was obtained as a yellow solid (70% yield).

LRMS (m/z): 503 (M+1)⁺.

### b) 1-[2-(1,4-diazepan-1-yl)pyridin-4-yl]-3-(pyrazin-2-ylamino)-1H-indazol-6-ol

Obtained as a hydrochloride salt (64%) from *tert*-butyl 4-{4-[6-hydroxy-3-(pyrazin-2-ylamino)-1*H*-indazol-1-yl]pyridin-2-yl}-1,4-diazepane-1-carboxylate (example 86a) using the same experimental procedure as described in example 7c.

LRMS (m/z): 403 (M+1)⁺.

¹H NMR (400 MHz, DMSO-*d₆*) δ ppm 2.14 - 2.28 (m, 2 H), 2.53 - 2.59 (m, 2 H), 3.23 - 3.31 (m, 2 H), 3.75 - 3.88 (m, 2 H), 4.03 - 4.13 (m, 2 H), 6.89 (d, *J*=8.99 Hz, 1 H), 7.12 (br. s., 1 H), 7.39 - 7.47 (m, 1 H), 7.48 (br. s., 1 H), 8.05 - 8.20 (m, 2 H), 8.25 (s, 1 H), 8.37 (s, 1 H), 9.13 (br. s., 2 H), 9.50 (s, 1 H), 10.61 (br. s., 1 H).

### EXAMPLE 87

### 1-{2-[(2S)-2-methylpiperazin-1-yl]pyridin-4-yl}-3-(pyrazin-2-ylamino)-1H-indazol-6-ol

### a) tert-Butyl (3S)-4-{4-[6-hydroxy-3-(pyrazin-2-ylamino)-1H-indazol-1-yl]pyridin-2-yl}-3-methylpiperazine-1-carboxylate

Obtained (65%) from *tert*-butyl (3*S*)-4-{4-[6-(benzyloxy)-3-(pyrazin-2-ylamino)-1*H-*indazol-1-yl]pyridin-2-yl}-3-methylpiperazine-1-carboxylate (example 78b) using the same experimental procedure as described in example 86a.

LRMS (m/z): 503 (M+1)⁺.

### b)1-{2-[(2S)-2-methylpiperazin-1-yl]pyridin-4-yl}-3-(pyrazin-2-y)amino)-1H-indazol-6-ol

Obtained as a hydrochloride salt (59%) from *tert*-butyl (3S)-4-{4-[6-hydroxy-3-(pyrazin-2-ylamino)-1*H*-indazol-1-yl]pyridin-2-yl}-3-methylpiperazine-1-carboxylate (example 87a) using the same experimental procedure as described in example 7c.

LRMS (m/z): 403 (M+1)⁺.

¹H NMR (400 MHz, DMSO-*d₆*) δ ppm 1.46 (d, *J*=6.64 Hz, 3 H), 3.18 (d, *J*=10.55 Hz, 1 H), 3.30 - 3.45 (m, 3 H), 3.61 - 3.68 (m, 1 H), 4.40 (d, *J*=14.07 Hz, 2 H), 4.55 (br. s., 2 H), 4.90 (br. s., 1 H), 6.91 (d, *J*=8.99 Hz, 1 H), 7.26 (s, 1 H), 7.53 (d, *J*=7.03 Hz, 1 H), 7.68 (s, 1 H), 8.14 - 8.25 (m, 2 H), 8.27 (d, *J*=2.34 Hz, 1 H), 8.39 (s, 1 H), 9.45 (br. s., 1 H), 9.51 (s, 1 H), 9.82 (br. s., 1 H), 10.68 (s, 1 H).

### EXAMPLE 88

### 1-{2-[(1S,4S)-2,5-diazabicyclo[2.2.1]hept-2-yl]pyridin-4-yl}-3-[(4-methylpyridin-2-yl)amino]-1H-indazol-6-ol

### a) tert-butyl (1S,4S)-5-(4-{6-methoxy-3-[(4-methylpyridin-2-yl)amino]-1H-indazol-1-yl}pyridin-2-yl)-2,5-diazabicyclo[2.2.1]heptane-2-carboxylate

Obtained (33%) from *tert*-butyl (1*S*,4*S*)-5-[4-(3-amino-6-methoxy-1*H*-indazol-1-yl)pyridin-2-yl]-2,5 diazabicyclo[2.2.1]heptane-2-carboxylate (example 31a) and 2-chloro-4-methylpyridine using the same experimental procedure as described in example 2b.

### b) 1-{2-[(1S,4S)-2,5-diazabicyclo[2.2.1]hept-2-yl]pyridin-4-yl}-3-[(4-methylpyridin-2-yl)amino]-1H-indazol-6-ol

Obtained as a free base (16%) from *tert*-butyl (1*S*,4*S*)-5-(4-{6-methoxy-3-[(4-methylpyridin-2-yl)amino]-1*H*-indazol-1-yl}pyridin-2-yl)-2,5-diazabicyclo[2.2.1]heptane-2-carboxylate (example 88a) using the same experimental procedure as described in example 53, purified by reverse chromatography as described in the general methods without buffer.

¹H NMR (400 MHz, DMSO-D6) δ ppm 1.7 (d, *J*=8.6 Hz, 1 H), 1.8 (d, *J*=8.6 Hz, 1 H), 2.3 (m, *J*=10.2 Hz, 3 H), 2.9 (m, 2 H), 3.3 (d, *J*=10.6 Hz, 1 H), 3.6 (dd, *J*=9.2, 2.1 Hz, 1 H), 3.7 (s, 1 H), 4.7 (s, 1 H), 6.7 (s, 1 H), 6.8 (m, 2 H), 7.0 (dd, *J*=5.5, 2.0 Hz, 1 H), 7.3 (d, *J*=2.0 Hz, 1 H), 8.1 (m, 3 H), 9.9 (s, 1 H), 10.1 (s, 1 H).

### EXAMPLE 89

### 1-{3-[(1S,4S)-2,5-diazabicyclo[2.2.1]hept-2-yl]phenyl}-6-methoxy-N-pyrazin-2-yl-1H-indazol-3-amine

### a) tert-Butyl (1S,4S)-5-[3-(3-amino-6-methoxy-1H-indazol-1-yl)phenyl]-2,5-diazabicyclo[2.2.1]heptane-2-carboxylate

Obtained (15%) from *tert*-butyl (1*S*,4*S*)-5-(3-bromophenyl)-2,5-diazabicyclo[2.2.1] heptane-2-carboxylate (preparation 19) and 6-methoxy-1*H*-indazol-3-amine (preparation 8) following the procedure as described in example 2a to give the desired compound.

LRMS (m/z): 436 (M+1)⁺.

¹H NMR (300 MHz, CHLOROFORM-*d*) δ ppm 1.43 (d, *J*=14.01 Hz, 9 H), 1.83 - 2.09 (m, 2 H), 3.07 - 3.71 (m, 4 H), 3.85 (s, 3 H), 4.14 (s, 2 H), 4.35 - 4.73 (m, 2 H), 6.46 (d, *J*=8.24 Hz, 1 H), 6.69 - 6.89 (m, 2 H), 6.96 (d, *J*=7.14 Hz, 1 H), 7.03 - 7.11 (m, 1 H), 7.28 - 7.37 (m, 1 H), 7.45 (d, *J*=8.79 Hz, 1 H).

### b) tert-butyl (1S,4S)-5-{3-[6-methoxy-3-(pyrazin-2-ylamino)-1H-indazol-1-yl]phenyl}-2,5-diazabicyclo[2.2.1]heptane-2-carboxylate

Obtained (50%) from *tert-butyl* (1*S*,4*S*)-5-[3-(3-amino-6-methoxy-1*H*-indazol-1-yl)phenyl]-2,5-diazabicyclo[2.2.1]heptane-2-carboxylate (example 89a) and 2-chloropyrazine following the procedure as described in example 2b.

LRMS (m/z): 514 (M+1)⁺.

¹H NMR (300 MHz, CHLOROFORM-*d*) δ ppm 1.43 (d, *J*=14.01 Hz, 9 H), 1.83 - 2.09 (m, 2 H), 3.13 - 3.35 (m, 2 H), 3.36 - 3.60 (m, 3 H), 3.66 (d, *J*=9.34 Hz, 1 H), 3.88 (s, 3 H), 4.40 - 4.74 (m, 3 H), 6.52 (dd, *J*=8.10, 2.61 Hz, 1 H), 6.89 (d, *J*=7.97 Hz, 1 H), 7.04 (t, *J*=6.18 Hz, 1 H), 7.14 (br s, 2 H), 7.30 - 7.43 (m, 2 H), 7.55 (d, *J*=8.51 Hz, 1 H), 8.16 (dd, *J*=6.59, 1.65 Hz, 2 H), 9.41 (d, *J*=1.37 Hz, 1 H).

### c) 1-{3-[(1S,4S)-2,5-diazabicyclo[2.2.1]hept-2-yl]phenyl}-6-methoxy-N-pyrazin-2-yl-1H-indazol-3-amine

Obtained (100%) as hydrochloride salt from *tert*-butyl (1S,4*S*)-5-{3-[6-methoxy-3-(pyrazin-2-ylamino)-1*H*-indazol-1-yl]phenyl}-2,5-diazabicyclo[2.2.1]heptane-2-carboxylate (example 89b) following the procedure as described in example 2c. The solid was filtered off and washed several times with diethyl ether and dried in the vacuum oven overnight.

LRMS (m/z): 414 (M+1)⁺.

¹H NMR (300 MHz, DMSO-*d₆*) δ ppm 2.00 (d, *J*=9.89 Hz, 1 H), 2.24 (d, *J*=10.99 Hz, 1 H), 3.27 - 3.34 (m, 2 H), 3.61 (d, *J*=0.55 Hz, 3 H), 3.71 - 3.80 (m, 1 H), 3.91 (s, 3 H), 4.53 (br s, 1 H), 4.77 (s, 1 H), 6.70 (d, *J*=8.51 Hz, 1 H), 6.90 (dd, *J*=8.93, 2.06 Hz, 1 H), 7.00 (s, 1 H), 7.12 - 7.24 (m, 2 H), 7.45 (t, *J*=8.10 Hz, 1 H), 8.07 - 8.23 (m, 2 H), 8.33 (dd, *J*=2.61, 1.51 Hz, 1 H), 8.76 (bs, 1 H), 9.28 (br s, 1 H), 9.43 (s, 1 H), 10.34 (s, 1 H).

### EXAMPLE 90

### 4-[1-{2-[(1S,4S)-2,5-diazabicyclo[2.2.1]hept-2-yl]pyridin-4-yl}-3-(pyrazin-2-ylamino)-1H-indazol-6-yl]phenol

### a) 3-fluoro-4'-methoxybiphenyl-4-carbonitrile

An oven dried resealable Schlenk tube was charged with 4-bromo-2-fluorobenzonitrile (1.00 g, 5.00 mmol), 4-methoxyphenylboronic acid (0.84 g, 5.50 mmol), 2M aqueous solution of potassium carbonate (5.00 mL, 10.00 mmol) and dioxane (30 mL). The Schlenk tube was subjected to three cycles of evacuation-backfilling with argon and tetrakis(triphenylphosphine)palladium(0) (0.58 g, 0.50 mmol) was added. After three further cycles of evacuation-backfilling with argon, the Schlenk tube was capped and placed in an oil bath at 100 ºC overnight. The mixture was cooled and filtered. Ethyl acetate and water were added to the mixture and the organic layer was washed with brine, dried (Na₂SO₄) and evaporated to give the desired compound (0.91 g, 80%) as a white solid.

LRMS (m/z): 228 (M+1)⁺.

### b) 6-(4-methoxyphenyl)-1H-indazol-3-amine

Obtained (71%) from 3-fluoro-4'-methoxybiphenyl-4-carbonitrile (example 90a) and hydrazine hydrate using the same experimental procedure as described in preparation 8.

LRMS (m/z): 240 (M+1)⁺.

### c) tert-butyl (1S,4S)-5-{4-[3-amino-6-(4-methoxyphenyl)-1H-indazol-1-yl]pyridin-2-yl}-2,5-diazabicyclo[2.2.1]heptane-2-carboxylate

Obtained (50%) from 6-(4-methoxyphenyl)-1*H*-indazol-3-amine (example 90b) and (1*S*,4*S*)-*tert*-butyl 5-(4-bromopyridin-2-yl)-2,5-diazabicyclo[2.2.1]heptane-2-carboxylate (preparation 3) using the same experimental procedure as described in example 2a, followed by purification by flash chromatography (80% hexanes to 100% ethyl acetate).

LRMS (m/z): 513 (M+1)⁺.

### d) tert-butyl (1 S,4S)-5-{4-[6-(4-methoxyphenyl)-3-(pyrazin-2-ylamino)-1H-indazol-1-yl]pyridin-2-yl}-2,5-diazabicyclo[2.2.1]heptane-2-carboxylate

Obtained (70%) from *tert*-butyl (1*S*,4*S*)-5-{4-[3-amino-6-(4-methoxyphenyl)-1*H*-indazol-1-yl]pyridin-2-yl}-2,5-diazabicyclo[2.2.1]heptane-2-carboxylate (example 90c) and 2-chloropyrazine using the same experimental procedure as described in example 2b, followed by purification by flash chromatography (80% hexanes to 100% ethyl acetate).

LRMS (m/z): 610 (M+1)⁺.

### e) 4-[1-{2-[(1S,4S)-2,5-diazabicyclo[2.2.1]hept-2-yl]pyridin-4-yl}-3-(pyrazin-2-ylamino)-1H-indazol-6-yl]phenol

Obtained (10%) from *tert*-butyl (1*S*,4*S*)-5-{4-[6-(4-methoxyphenyl)-3-(pyrazin-2-ylamino)-1*H*-indazol-1-yl]pyridin-2-yl}-2,5-diazabicyclo[2.2.1]heptane-2-carboxylate (example 90d) using the same experimental procedure as described in example 53. The crude was neutralized and purified by reverse phase chromatography as described in the general methods without buffer.

LRMS (m/z): 477 (M+1)⁺.

¹H NMR (400 MHz, dmso) δ 10.50 (s, 1H), 9.68 (bs, 1H), 9.44 (s, 1H), 8.33 (s, 1H), 8.28 (d, *J* = 8.4 Hz, 1H), 8.19 (m, 2H), 7.98 (s, 1H), 7.65 (d, *J* = 8.4 Hz, 2H), 7.52 (d, *J* = 8.5 Hz, 1H), 7.13 (d, *J* = 5.2 Hz, 1H), 6.90 (d, *J* = 8.4 Hz, 2H), 6.81 (s, 1H), 4.76 (s, 1H), 3.72 (s, 1H), 3.55 (d, *J* = 8.7 Hz, 1H), 3.27 (d, *J* = 9.4 Hz, 1H), 2.92 (dd, *J* = 17.8, 9.4 Hz, 2H), 1.83 (d, *J* = 9.0 Hz, 1H), 1.70 (d, *J* = 9.0 Hz, 1H).

### EXAMPLE 91

### 1-{2-[(1S,4S)-2,5-Diazabicyclo[2.2.1]hept-2-yl]pyridin-4-yl}-6-morpholin-4-yl-N-pyrazin-2-yl-1H-pyrazolo[3,4-b]pyridin-3-amine

### a) 6-Chloro-1H-pyrazolo[3,4-b]pyridine

Obtained (40%) from 2,6-dichloronicotinaldehyde and hydrazine using the same experimental procedure as described in preparation 8, followed by purification by reverse phase chromatography (C-18 silica from Waters©, water/methanol 0% to 100%).

LRMS (m/z): 154 (M+1)⁺.

### b) 3-Bromo-6-chloro-1H-pyrazolo[3,4-b]pyridine

*N*-Chlorosuccinimide (1.05 g, 5.93 mmol) was added to a solution of 6-chloro-1*H-*pyrazolo[3,4-b]pyridine (example 91 a, 827 mg, 5.39 mmol) in DMF (19 mL). The mixture was stirred at room temperature. After 1 hour, water and ice were added to the reaction and the mixture was extracted with ethyl acetate. The organic layer was washed with brine, dried (MgSO₄) and evaporated. Purification of the residue by flash chromatography (8:2 hexanes/ethyl acetate) gave the title compound (0.86 g, 55%).

LRMS (m/z): 232/234 (M+1)⁺, 230/232 (M-1)⁻.

¹H NMR (300 MHz, CHLOROFORM-*d*) δ ppm 7.27 (d, *J*=8.51 Hz, 1 H), 7.97 (d, *J*=8.51 Hz, 1 H), 11.05 (br. s., 1 H).

### c) tert-Butyl (1S,4S)-5-[4-(3-bromo-6-chloro-1H-pyrazolo[3,4-b]pyridin-1-yl)pyridin-2-yl]-2,5-diazabicyclo[2.2.1]heptane-2-carboxylate

Obtained (4% yield) from 3-bromo-6-chloro-1*H*-pyrazolo[3,4-b]pyridine (example 91b) and (1*S,4S*)*-tert-*butyl 5-(4-bromopyridin-2-yl)-2,5-diazabicyclo[2.2.1]heptane-2-carboxylate (preparation 3) following the experimental procedure as described in example 1 a, followed by purification by flash chromatography (hexanes/ethyl acetate).

LRMS (m/z): 505/507 (M+1)⁺.

### d) tert-Butyl (1S,4S)-5-[4-(3-bromo-6-morpholin-4-yl-1H-pyrazolo[3,4-b]pyridin-1-yl)pyridin-2-yl]-2,5-diazabicyclo[2.2.1]heptane-2-carboxylate

A mixture of *tert*-butyl (1*S*,4*S*)-5-[4-(3-bromo-6-chloro-1*H*-pyrazolo[3,4-b]pyridin-1-yl)pyridin-2-yl]-2,5-diazabicyclo[2.2.1]heptane-2-carboxylate (example 91 c, 30 mg, 0.06 mmol), morpholine (52 µL, 0.60 mmol), triethylamine (82 µL, 0.60 mmol) in ethanol (3 ml) and THF (1 ml) was heated at 120 °C for 4h under microwave conditions. The mixture was then cooled, dichloromethane and 4% aqueous sodium hydrogen carbonate solution were added, and the organic extracts dried over Na₂SO₄ and concentrated under vacuum to give the title compound (70%).

LRMS (m/z): 556/558 (M/M+2)⁺.

### e) tert-Butyl (1 S,4S)-5-{4-[6-morpholin-4-yl-3-(pyrazin-2-ylamino)-1H-pyrazolo[3,4-b]pyridin-1-yl]pyridin-2-yl}-2,5-diazabicyclo[2.2.1]heptane-2-carboxylate

Obtained (27% yield) from *tert*-butyl (1*S*,4*S*)-5-[4-(3-bromo-6-morpholin-4-yl-1*H-*pyrazolo[3,4-b]pyridin-1-yl)pyridin-2-yl]-2,5-diazabicyclo[2.2.1 ]heptane-2-carboxylate (example 91 d) and pyrazine-2-amine following the experimental procedure described in example 2b, followed by purification by flash chromatography (9:1 dichloromethane:methanol).

LRMS (m/z): 570 (M+1)⁺.

### f) 1-{2-[(1S,4S)-2,5-Diazabicyclo[2.2.1]hept-2-yl]pyridin-4-yl}-6-morpholin-4-yl-N-pyrazin-2-yl-1H-pyrazolo[3,4-b]pyridin-3-amine

Obtained as a hydrochloride salt (97% yield) from *tert*-butyl (1*S*,4*S*)-5-{4-[6-morpholin-4-yl-3-(pyrazin-2-ylamino)-1*H*-pyrazolo[3,4-b]pyridin-1-yl]pyridin-2-yl}-2,5-diazabicyclo[2.2.1]heptane-2-carboxylate (example 91e) using the same experimental procedure as described in example 7c.

LRMS (m/z): 471 (M+1)⁺

### EXAMPLE 92

### 1-{2-[(1S,4S)-2,5-diazabicyclo[2.2.1]hept-2-yl]pyridin-4-yl}-3-{[4-(trifluoromethyl)pyridin-2-yl]amino}-1H-indazo1-6-ol

### a) tert-butyl (1S,4S)-5-[4-(6-methoxy-3-{[4-(trifluoromethyl)pyridin-2-yl]amino}-1H-indazol-1-yl)pyridin-2-yl]-2,5-diazabicyclo[2.2.1]heptane-2-carboxylate

Obtained (56%) from *tert-butyl* (1*S*,4*S*)-5-[4-(3-amino-6-methoxy-1*H*-indazol-1-yl)pyridin-2-yl]-2,5 diazabicyclo[2.2.1]heptane-2-carboxylate (example 31a) and 2-chloro-4-(trifluoromethyl)pyridine using the same experimental procedure as described in example 2b.

### b)1-{2-[(1S,4S)-2,5-diazabicyclo[2.2.1]hept-2-yl]pyridin-4-yl}-3-{[4-(trifluoromethyl)pyridin-2-yl]amino}-1H-indazol-6-ol

Obtained (46%) from *tert*-butyl (1*S*,4*S*)-5-[4-(6-methoxy-3-{[4-(trifluoromethyl)pyridin-2-yl]amino}-1*H*-indazol-1-yl)pyridin-2-yl]-2,5-diazabicyclo[2.2.1]heptane-2-carboxylate (example 92a) using the same experimental procedure as described in example 53. The purification was performed without buffer.

¹H NMR (400 MHz, DMSO-*d₆*) δ ppm 2.1 (d, *J*=10.6 Hz, 1 H), 2.3 (m, 1 H), 3.4 (d, *J*=16.8 Hz, 1 H), 3.5 (m, 1 H), 3.9 (m, *J*=10.9 Hz, 1 H), 4.0 (d, *J*=10.6 Hz, 1 H), 4.7 (s, 1 H), 5.3 (s, 1 H), 6.9 (m, 1 H), 7.1 (s, 1 H), 7.4 (m, 2 H), 7.6 (s, 1 H), 8.2 (d, *J*=7.4 Hz, 1 H), 8.3 (d, *J*=9.0 Hz, 1 H), 8.6 (m, 2 H), 9.4 (s, 1 H), 9.9 (s, 1 H), 10.7 (s, 1 H), 10.9 (s, 1 H).

### EXAMPLE 93

### Benzyl 1-{2-[(1S,4S)-2,5-diazabicyclo[2.2.1]hept-2-yl]pyridin-4-yl}-3-(pyrazin-2-ylamino)-1H-indazole-6-carboxylate

### a) Benzyl1-{2-[(1S,4S)-5-(tert-butoxycarbonyl)-2,5-diazabicyclo[2.2.1]hept-2-yl]pyridin-4-yl}-3-(pyrazin-2-ylamino)-1H-indazole-6-carboxylate

1-{2-[(1*S*,4*S*)-5-(*tert*-Butoxycarbonyl)-2,5-diazabicyclo[2.2.1]hept-2-yl]pyridin-4-yl}-3-(pyrazin-2-ylamino)-1*H*-indazole-6-carboxylic acid (example 17a, 30 mg, 0.06 mg), 1-ethyl-3-(3-dimethyllaminopropyl)carbodiimide hydrochloride (13.8 mg, 0.08 mmol) and 4-dimethylaminopyridine (8 mg, 0.08 mmol) were dissolved in dichloromethane (0.5 mL) and dimethylformamide (0.5 mL) and phenylmethanol (7.4 µL, 0.06 mmol) was added. Reaction was stirred overnight at room temperature. Ethyl acetate was added and organic layer was washed with water, dried (MgSO₄) and solvent was removed in vacuo. Crude was purified by reverse phase chromatography (C-18 from Merck, water/methanol-acetonitrile (1:1) as eluents, 30-100%) to give the title compound (3.4 mg,10%).

LRMS (m/z): 619 (M+1)⁺.

¹H NMR (300 MHz, CHLOROFORM-*d*) δ ppm 1.45 (d, *J*=15.38 Hz, 9 H), 1.97 (d, *J*=10.16 Hz, 2 H), 3.34 - 3.71 (m, 4 H), 4.52 - 4.74 (m, 1 H), 4.94 (d, *J*=18.95 Hz, 1 H), 5.31 (s, 1 H), 5.44 (s, 2 H), 6.71 (br. s., 1 H), 7.08 (dd, *J*=5.63, 1.79 Hz, 1 H), 7.27 - 7.55 (m, 4 H), 7.78 (d, *J*=7.69 Hz, 1 H), 7.98 (dd, *J*=8.51, 1.37 Hz, 1 H,) 8.18 - 8.30 (m, 2 H), 8.63 (s, 1 H), 9.52 (br. s., 1 H).

### b) Benzyl 1-{2-[(1S,4S)-2,5-diazabicyclo[2.2.1]hept-2-yl]pyridin-4-yl}-3-(pyrazin-2-ylamino)-1H-indazole-6-carboxylate

Obtained (100%) as hydrochloride salt from benzyl 1-{2-[(1S,4S)-5-(tert-butoxycarbonyl)-2,5-diazabicyclo[2.2.1]hept-2-yl]pyridin-4-yl}-3-(pyrazin-2-ylamino)-1*H-*indazole-6-carboxylate (example 93a) following the procedure as described in example 2c. The solid was filtered off and washed several times with diethyl ether and dried in the vacuum oven overnight.

LRMS (m/z): 519 (M+1)⁺.

### EXAMPLE 94

### 1-{5-[(1S,4S)-2,5-diazabicyclo[2.2.1]hept-2-yl]-6-methoxypyridin-3-yl}-N-pyrazin-2-yl-1H-indazol-3-amine

### a) tert-butyl (1S,4S)-5-(5-bromo-2-methoxypyridin-3-yl)-2,5-diazabicyclo[2.2.1]heptane-2-carboxylate

Obtained (95%) from 5-bromo-3-iodo-2-methoxypyridine and (1S,4S)-tert-butyl 2,5-diazabicyclo[2.2.1]heptane-2-carboxylate following the experimental procedure as described in preparation 7.

LRMS (m/z): 384/386 (M+1)⁺.

### b) tert-butyl (1S,4S)-5-[5-(3-amino-1H-indazol-1-yl)-2-methoxypyridin-3-yl]-2,5-diazabicyclo[2.2.1]heptane-2-carboxylate

Obtained (24%) from 1*H*-indazol-3-amine and *tert*-butyl (1*S*,4*S*)-5-(5-bromo-2-methoxypyridin-3-yl)-2,5-diazabicyclo[2.2.1]heptane-2-carboxylate
(example 94a) using the same experimental procedure as described in example 2a, followed by purification by reverse phase chromatography as described in the general methods.

LRMS (m/z): 437 (M+1)⁺.

### c) tert-butyl (1S,4S)-5-{2-methoxy-5-[3-(pyrazin-2-ylamino)-1H-indazol-1-yl]pyridin-3-yl}-2,5-diazabicyclo[2.2.1]heptane-2-carboxylate

Obtained (42%) from *tert-*butyl (1*S*,4*S*)-5-[5-(3-amino-1*H*-indazol-1-yl)-2-methoxypyridin-3-yl]-2,5-diazabicyclo[2.2.1]heptane-2-carboxylate
(example 94b) and 2-chloropyrazine using the same experimental procedure as described in example 2b, followed by purification by flash chromatography (100% hexanes to 50% ethyl acetate).

LRMS (m/z): 515 (M+1)⁺.

### d) 1-{5-[(1S,4S)-2,5-diazabicyclo[2.2.1]hept-2-yl]-6-methoxypyridin-3-yl}-N-pyrazin-2-yl-1H-indazol-3-amine

Obtained (19%) from tert-butyl (1*S*,4*S*)-5-{2-methoxy-5-[3-(pyrazin-2-ylamino)-1*H-*indazol-1-yl]pyridin-3-yl}-2,5-diazabicyclo[2.2.1]heptane-2-carboxylate
(example 94c) using the same experimental procedure as described in example 2c. The crude was neutralized and purified by reverse phase chromatography as described in the general methods without buffer.

LRMS (m/z): 415 (M+1)⁺.

¹H NMR (400 MHz, DMSO-*d*₆) δ ppm 1.81 (d, *J*=9.77 Hz, 1 H), 1.95 (d, *J*=9.77 Hz, 1 H), 3.10 (d, *J*=10.16 Hz, 1 H), 3.22 (d, *J*=10.16 Hz, 1 H), 3.30 (d, *J*=10.16 Hz, 1 H), 3.75 (dd, *J*=9.96, 1.76 Hz, 1 H), 3.93 (s, 3 H), 4.00 (s, 1 H), 4.65 (s, 1 H), 7.18 - 7.24 (m, 2 H), 7.47 - 7.52 (m, 1 H), 7.70 (d, *J*=8.60 Hz, 1 H), 7.91 (d, *J*=2.34 Hz, 1 H), 8.13 (d, *J*=2.74 Hz, 1 H), 8.18 (d, J=8.21 Hz, 1 H), 8.27 (dd, J=2.34, 1.56 Hz, 1 H), 8.34 (s, 2 H), 9.32 (d, *J*=1.17 Hz, 1 H), 10.34 (s, 1 H).

### EXAMPLE 95

### 1-{2-[(1S,4S)-2,5-diazabicyclo[2.2.1]hept-2-yl]pyridin-4-yl}-6-(methylthio)-N-pyrazin-2-yl-1H-indazol-3-amine

### a) 2-Fluoro-4-(methylthio)benzonitrile

4-Amino-2-fluorobenzonitrile (0.5 g, 3,67 mmol) was dissolved in dichloroethane and dimethyl disulfide (0.45 g, 478 mmol) was added. The mixture was heated at 60°C and under nitrogen conditions *tert*-butyl nitrite (0.5 g, 4.85 mmol) was added dissolved in dichloroethane (3 mL). The reaction was heated at 60°C for 2 h.

The mixture was cooled and water was added (10 mL). The organic layer was washed with brine and 2N HCl. The crude was purified by flash chromatography (hexane/ethyl acetate) to gave the title compound (0.55 g, 59%).

### b) 6-(Methylthio)-1H-indazol-3-amine

Obtained from 2-fluoro-4-(methylthio)benzonitrile (example 95a) following the same experimental procedure described in preparation 8, to provide the title compound as a yellow solid (51%).

LRMS (m/z): 180 (M+1)⁺

### c) tert-Butyl (1S,4S)-5-{4-[3-amino-6-(methylthio)-1H-indazol-1-yl]pyridin-2-yl}-2,5-diazabicyclo[2.2.1]heptane-2-carboxylate

Obtained (12%) from 6-(methylthio)-1*H*-indazol-3-amine (example 95b) and (1*S*,4*S*)-*tert*-butyl 5-(4-bromopyridin-2-yl)-2,5-diazabicyclo[2.2.1]heptane-2-carboxylate (preparation 3) following the same experimental procedure as described in example 2a.

LRMS (m/z) 453 (M+1)⁺

### d) tert-Butyl (1S,4S)-5-{4-[6-(methylthio)-3-(pyrazin-2-ylamino)-1H-indazol-1-yl]pyridin-2-yl}-2,5-diazabicyclo[2.2.1]heptane-2-carboxylate

Obtained (62%) from *tert*-butyl (1*S*,4*S*)-5-{4-[3-amino-6-(methylthio)-1*H*-indazol-1-yl]pyridin-2-yl}-2,5-diazabicyclo[2.2.1]heptane-2-carboxylate (example 95c) and 2-chloropyrazine using the same experimental procedure as described in example 2b, followed by purification by flash chromatography (hexane/ethyl acetate) to provide the title compound as a yellow oil.

LRMS (m/z) 531 (M+1)⁺

### e) 1-{2-[(1S,4S)-2,5-Diazabicyclo[2.2.1]hept-2-yl]pyridin-4-yl}-6-(methylthio)-N-pyrazin-2-yl-1H-indazol-3-amine

Obtained as a hydrochloride salt (73%) from *tert*-butyl (1*S*,4*S*)-5-{4-[6-(methylthio)-3-(pyrazin-2-ylamino)-1*H*-indazol-1-yl]pyridin-2-yl}-2,5-diazabicyclo[2.2.1]heptane-2-carboxylate (example 95d) using the same experimental procedure as described in example 7c.

LRMS (m/z) 431 (M+1)⁺

¹H NMR (400 MHz, DMSO-*d*₆) δ ppm 2.1 (d, *J*=10.2 Hz, 1 H), 2.2 (d, *J*=10.9 Hz, 1 H), 2.7 (m, *J*=10.6 Hz, 3 H), 3.9 (m, 1 H), 3.9 (m, 1 H), 4.6 (s, 1 H), 5.2 (s, 1 H), 7.3 (d, *J*=8.6 Hz, 1 H), 7.5 (s, 1 H), 7.8 (s, 1 H), 8.2 (d, *J*=6.6 Hz, 1 H), 8.3 (m, 1 H), 8.4 (s, 1 H), 9.2 (m, 1 H), 9.5 (s, 1 H), 9.7 (m, 1 H), 10.7 (s, 1 H).

### EXAMPLE 96

### 1-{2-[(2S,5S)-2,5-Dimethylpiperazin-1-yl]pyridin-4-yl}-N-pyrazin-2-yl-1H-indazol-3-amine

Obtained (13%) from 1-(2-fluoropyridin-4-yl)-*N*-(pyrazin-2-yl)-1*H*-indazol-3-amine (preparation 9) and (2*S*,5*S*)-2,5-dimethylpiperazine (*J. Am. Chem. Soc*. **2001**, *8*, 1792) using the same experimental procedure as described in preparation 2 followed by purification by reverse phase chromatography (C-18 silica from Waters©, water/methanol 0% to 60%).

LRMS (m/z): 401 (M+1)⁺, 399 (M-1)⁻.

¹H NMR (300 MHz, METHANOL-*d*₄) δ ppm 1.23 (d, *J*=6.32 Hz, 3 H), 1.27 (d, *J*=6.59 Hz, 3 H), 2.71 (t, *J*=10.85 Hz, 1 H), 2.79 - 2.94 (m, 1 H), 3.00 (d, *J*=14.28 Hz, 1 H), 3.06 - 3.17 (m, 1 H), 3.61 (q, *J*=6.96 Hz, 1 H), 4.06 (dd, *J*=11.67, 3.71 Hz, 1 H), 4.47 (br. s., 1 H), 7.15 (d, *J*=1.65 Hz, 1 H), 7.19 (dd, *J*=5.77, 1.92 Hz, 1 H), 7.32 (td, *J*=7.55, 0.82 Hz, 1 H), 7.61 (ddd, *J*=8.51, 7.14, 1.10 Hz, 1 H), 7.98 (dd, *J*=8.65, 0.69 Hz, 1 H), 8.07 (dd, *J*=8.24, 0.82 Hz, 1 H), 8.13 (d, *J*=2.47 Hz, 1 H), 8.20 (d, *J*=5.77 Hz, 1 H), 8.31 (dd, *J*=2.61, 1.51 Hz, 1 H), 9.43 (d, *J*=1.65 Hz, 1 H).

### EXAMPLE 97

### 1-{6-[(1S,4S)-2,5-diazabicyclo[2.2.1]hept-2-yl]-2-isopropylpyrimidin-4-yl}-3-(pyrazin-2-ylamino)-1H-indazol-6-ol

### a) 2-isopropylpyrimidine-4,6-diol

Obtained (80%) from isobutyrimidamide and diethyl malonate using the same experimental procedure as described in example 43a.

LRMS (m/z): 155 (M+1)⁺.

### b) 4,6-dichloro-2-isopropylpyrimidine

Obtained (15%) from 2-isopropylpyrimidine-4,6-diol using the same experimental procedure as described in example 43b.

¹H NMR (400 MHz, CHLOROFORM-*d*) δ ppm 1.34 (d, *J*=6.64 Hz, 6 H), 3.13 - 3.24 (m, 1 H), 7.23 (s, 1 H).

### c) tert-butyl (1S,4S)-5-(6-chloro-2-isopropylpyrimidin-4-yl)-2,5-diazabicyclo[2.2.1]heptane-2-carboxylate

Obtained (86%) from 4,6-dichloro-2-isopropylpyrimidine (example 97b) and (1*S,*4*S*)-tert-butyl 2,5-diazabicyclo[2.2.1]heptane-2-carboxylate using the same experimental procedure as described in example 43c.

LRMS (m/z): 353 (M+1)⁺.

### d) tert-butyl (1S,4S)-5-(6-iodo-2-isopropylpyrimidin-4-yl)-2,5-diazabicyclo[2.2.1]heptane-2-carboxylate

Obtained (45%) from *tert*-butyl (1*S*,4*S*)-5-(6-chloro-2-isopropylpyrimidin-4-yl)-2,5-diazabicyclo[2.2.1]heptane-2-carboxylate (example 97c) using the same experimental procedure as described in example 43d.

LRMS (m/z): 445 (M+1)⁺.

### e) tert-butyl (1S,4S)-5-{6-[3-amino-6-(benzyloxy)-1H-indazol-1-yl]-2-isopropylpyrimidin-4-yl}-2,5-diazabicyclo[2.2.1]heptane-2-carboxylate

Obtained (34%) from 6-(benzyloxy)-1*H*-indazol-3-amine (preparation 18) and tert-butyl (1*S*,4*S*)-5-(6-iodo-2-isopropylpyrimidin-4-yl)-2,5-diazabicyclo[2.2.1 ]heptane-2-carboxylate (example 97d) using the same experimental procedure as described in example 2a, followed by purification by flash chromatography (100% hexanes to 100% ethyl acetate).

LRMS (m/z): 556 (M+1)⁺.

### f) tert-butyl (1S,4S)-5-{6-[6-(benzyloxy)-3-(pyrazin-2-ylamino)-1H-indazol-1-yl]-2-isopropylpyrimidin-4-yl}-2,5-diazabicyclo[2.2.1]heptane-2-carboxylate

Obtained (25%) from *tert*-butyl (1 S,4S)-5-{6-[3-amino-6-(benzyloxy)-1*H*-indazol-1-yl]-2-isopropylpyrimidin-4-yl}-2,5-diazabicyclo[2.2.1]heptane-2-carboxylate (example 97e) and 2-chloropyrazine using the same experimental procedure as described in example 2b, followed by purification by flash chromatography (100% hexanes to 100% ethyl acetate).

LRMS (m/z): 634 (M+1)⁺.

### g) tert-butyl (1S,4S)-5-{6-[6-hydroxy-3-(pyrazin-2-ylamino)-1H-indazol-1-yl]-2-isopropylpyrimidin-4-yl}-2,5-diazabicyclo[2.2.1]heptane-2-carboxylate

Obtained (26%) from *tert*-butyl (1*S*,4*S*)-5-{6-[6-(benzyloxy)-3-(pyrazin-2-ylamino)-1*H-*indazol-1-yl]-2-isopropylpyrimidin-4-yl}-2,5-diazabicyclo[2.2.1]heptane-2-carboxylate
(example 97f) using the same experimental procedure as described in example 86a, followed by purification by reverse phase chromatography as described in the general methods.

LRMS (m/z): 544 (M+1)⁺.

### h) 1-{6-[(1S,4S)-2,5-diazabicyclo[2.2.1]hept-2-yl]-2-isopropylpyrimidin-4-yl}-3-(pyrazin-2-ylamino)-1H-indazol-6-ol

Obtained as a hydrochloride salt (83% yield) from *tert*-butyl (1 S,4S)-5-{6-[6-hydroxy-3-(pyrazin-2-ylamino)-1*H*-indazol-1-yl]-2-isopropylpyrimidin-4-yl}-2,5-diazabicyclo[2.2.1]heptane-2-carboxylate (example 97g) using the same experimental procedure as described in example 7c.

LRMS (m/z): 444 (M+1)⁺.

### EXAMPLE 98

### 1-{2-[(1S,4S)-2,5-diazabicyclo[2.2.1]hept-2-yl]pyridin-4-yl}-N-pyrazin-2-yl-6-pyridin-3-yl-1H-indazol-3-amine

### a) tert-butyl (1S,4S)-5-[4-(3-amino-6-bromo-1H-indazol-1-yl)pyridin-2-yl]-2,5-diazabicyclo[2.2.1]heptane-2-carboxylate

Obtained (18%) from 6-bromo-1*H*-indazol-3-amine and *(1S,4S)-tert*-butyl 5-(4-bromopyridin-2-yl)-2,5-diazabicyclo[2.2.1]heptane-2-carboxylate (preparation 3) using the same experimental procedure as described in example 2a.

LRMS (m/z): 485/487 (M+1)⁺.

### b) tert-butyl (1S,4S)-5-{4-[6-bromo-3-(pyrazin-2-ylamino)-1H-indazol-1-yl]pyridin-2-yl}-2,5-diazabicyclo[2.2.1]heptane-2-carboxylate

Obtained (50%) from *tert*-butyl (1*S*,4*S*)-5-[4-(3-amino-6-bromo-1*H*-indazol-1-yl)pyridin-2-yl]-2,5-diazabicyclo[2.2.1]heptane-2-carboxylate (example 98a) and 2-iodopyrazine using the same experimental procedure as described in example 2b, followed by purification by reverse phase chromatography as described in the general methods.

LRMS (m/z): 563/565 (M+1)⁺.

### c) tert-butyl (1S,4S)-5-{4-[3-(pyrazin-2-ylamino)-6-pyridin-3-yl-1H-indazol-1-yl]pyridin-2-yl}-2,5-diazabicyclo[2.2.1]heptane-2-carboxylate

An oven dried resealable Schlenk tube was charged with *tert*-butyl (1*S*,4*S*)-5-{4-[6-bromo-3-(pyrazin-2-ylamino)-1*H*-indazol-1-yl]pyridin-2-yl}-2,5-diazabicyclo[2.2.1]heptane-2-carboxylate (90 mg, 0.16mmol), pyridin-3-ylboronic acid (26 mg, 0.21 mmol), potassium carbonate (44 mg, 0.32 mmol), water (0.15 mL) and dioxane (1.5 mL). The Schlenk tube was subjected to three cycles of evacuation-backfilling with argon, tetrakis(triphenylphosphine)palladium(0) (11 mg, 0.06 mmol) was added. After three further cycles of evacuation-backfilling with argon, the Schlenk tube was capped and placed in an oil bath at 90 °C overnight. The mixture was cooled and filtered. Ethyl acetate and water were added to the mixture and the organic layer was washed with brine, dried (Na₂SO₄) and evaporated. The crude was purified by flash chromatography (9:1 hexanes/ethyl acetate to 100% ethyl acetate) to give the desired compound (30 mg, 33%) as an oil.

LRMS (m/z): 562 (M+1)⁺.

### d) 1-{2-[(1S,4S)-2,5-diazabicyclo[2.2.1]hept-2-yl]pyridin-4-yl}-N-pyrazin-2-yl-6-pyridin-3-yl-1H-indazol-3-amine

Obtained as a hydrochloride salt (37% yield) from *tert*-butyl (1*S*,4*S*)-5-{4-[3-(pyrazin-2-ylamino)-6-pyridin-3-yl-1*H*-indazol-1-yl]pyridin-2-yl}-2,5-diazabicyclo[2.2.1]heptane-2-carboxylate (example 98c) using the same experimental procedure as described in example 7c.

LRMS (m/z): 462 (M+1)⁺.

¹H NMR (400 MHz, DMSO-*d*₆) δ ppm 2.12 (d, *J*=10.94 Hz, 1 H), 2.26 (d, *J*=10.94 Hz, 1 H), 3.34 - 3.42 (m, 1 H), 3.44 - 3.50 (m, 1 H), 3.88 (d, *J*=10.94 Hz, 1 H), 4.11 (d, *J*=10.94 Hz, 1 H), 4.66 (s, 1 H), 5.38 (s, 1 H), 7.41 (br. s., 1 H), 7.65 (d, *J*=5.86 Hz, 1 H), 7.88 (d, *J*=8.60 Hz, 1 H), 7.96 - 8.04 (m, 1 H), 8.16 (d, *J*=6.64 Hz, 1 H), 8.30 (s, 1 H), 8.42 (s, 1 H), 8.52 (s, 1 H), 8.57 (d, *J*=8.21 Hz, 1 H), 8.80 - 8.92 (m, 2 H), 9.42 (s, 1 H), 9.52 (s, 1 H), 9.57 (br. s., 1 H), 9.94 (br. s., 1 H), 10.89 (s, 1 H).

### EXAMPLE 99

### 1-{2-[(1S,4S)-2,5-diazabicyclo[2.2.1]hept-2-yl]pyridin-4-yl}-6-(1-methyl-1H-pyrazol-4-yl)-N-pyrazin-2-yl-1H-indazol-3-amine

### a) tert-butyl (1S,4S)-5-{4-[6-(1-methyl-1H-pyrazol-4-yl)-3-(pyrazin-2-ylamino)-1H-indazol-1-yl]pyridin-2-yl}-2,5-diazabicyclo[2.2.1]heptane-2-carboxylate

Obtained (51%) from *tert*-butyl (1*S*,4*S*)-5-[4-(3-amino-6-bromo-1*H*-indazol-1-yl)pyridin-2-yl]-2,5-diazabicyclo[2.2.1]heptane-2-carboxylate and 1-methyl-4-(4,4,5,5-tetramethyl-1,3,2-dioxaborolan-2-yl)-1*H*-pyrazole using the same experimental procedure as described in example 98c.

LRMS (m/z): 565 (M+1)⁺.

### b) 1-{2-[(1S,4S)-2,5-diazabicyclo[2.2.1]hept-2-yl]pyridin-4-yl}-6-(1-methyl-1H-pyrazol-4-yl)-N-pyrazin-2-yl-1H-indazol-3-amine

Obtained as a hydrochloride salt (75% yield) from tert-butyl (1*S*,4*S*)-5-{4-[6-(1-methyl-1*H*-pyrazol-4-yl)-3-(pyrazin-2-ylamino)-1*H*-indazol-1-yl]pyridin-2-yl}-2,5-diazabicyclo[2.2.1]heptane-2-carboxylate (example 99a) using the same experimental procedure as described in example 7c.

LRMS (m/z): 465 (M+1)⁺.

¹H NMR (400 MHz, DMSO-*d*₆) δ ppm 2.04 - 2.27 (m, 2 H), 3.25 - 3.49 (m, 2 H), 3.85 (s, 3 H), 4.05 - 4.15 (m, 2 H), 4.63 (s, 1 H), 5.32 (s, 1 H), 7.23 (br. s., 1 H), 7.52 - 7.66 (m, 2 H), 8.04 - 8.16 (m, 3 H), 8.24 (s, 1 H), 8.29 - 8.45 (m, 2 H), 9.46 (s, 1 H), 9.59 (br. s., 1 H), 9.86 (br. s., 1 H), 10.71 (s, 1 H).

### EXAMPLE 100

### 1-{5-[(2S)-2-Methylpiperazin-1-yl]pyridin-3-yl}-3-(pyrazin-2-ylamino)-1H-indazol-6-ol

### a) tert-Butyl (3S)-4-{5-[3-amino-6-(benzyloxy)-1H-indazol-1-yl]pyridin-3-yl}-3-methylpiperazine-1-carboxylate

Obtained (77%) from *tert*-butyl (3*S*)-4-(5-bromopyridin-3-yl)-3-methylpiperazine-1-carboxylate (preparation 15) and 6-(benzyloxy)-1*H*-indazol-3-amine (preparation 18) following the procedure as described in example 1a, using potassium carbonate as base and dioxane as solvent. The mixture thus obtained was filtered through celite and solvent was removed. Crude was purified by normal phase chromatography using hexane and ethyl acetate as eluents (gradient 10-80%) to give the desired compound.

LRMS (m/z): 515 (M+1)⁺.

### b) tert-butyl (3S)-4-{5-[6-(benzyloxy)-3-(pyrazin-2-ylamino)-1H-indazol-1-yl]pyridin-3-yl}-3-methylpiperazine-1-carboxylate

Obtained (51%) from *tert-*butyl (3*S*)-4-{5-[3-amino-6-(benzyloxy)-1*H*-indazol-1-yl]pyridin-3-yl}-3-methylpiperazine-1-carboxylate (example 100a) and 2-chloropyrazine following the procedure as described in example 2b.

LRMS (m/z): 593 (M+1)⁺.

¹H NMR (300 MHz, CHLOROFORM-*d*) δ ppm 1.14 (d, *J*=6.32 Hz, 3 H), 1.54 (d, 9 H), 3.03 - 3.44 (m, 4 H), 3.78 - 4.30 (m, 3 H), 5.15 (s, 2 H), 6.98 (dd, *J*=8.79, 1.92 Hz, 1 H), 7.21 (s, 2 H), 7.31 - 7.52 (m, 5 H), 7.53 - 7.65 (m, 1 H), 8.11 - 8.27 (m, 3 H), 8.47 (d, *J*=1.92 Hz, 1 H), 9.45 (s, 1 H).

### c) tert-butyl (3S)-4-{5-[6-hydroxy-3-(pyrazin-2-ylamino)-1H-indazol-1-yl]pyridin-3-yl}-3-methylpiperazine-1-carboxylate

A resealable Schlenk tube was charged with *tert*-butyl (3*S*)-4-{5-[6-(benzyloxy)-3-(pyrazin-2-ylamino)-1*H*-indazol-1-yl]pyridin-3-yl}-3-methylpiperazine-1-carboxylate (example 100b, 262 mg, 0.44 mmol) and methanol (15 mL). The schlenk tube was subjected to three cycles of evacuation-backfilling with nitrogen and 10% Pd/C (70 mg, 0.66 mmol) was added. After three further cycles of evacuation-backfilling with hydrogen, the Schlenk tube was capped and reaction stirred for 4 days at room temperature. Reaction mixture was filtered through celite and solvent was removed. The crude thus obtained was purified by reverse phase chromatography (C-18 from Merck, water/methanol-acetonitrile (1:1) as eluents, 20-80%) to give the desired compound (75.6 mg, 34%).

LRMS (m/z): 503 (M+1)⁺.

¹H NMR (300 MHz, CHLOROFORM-*d*) δ ppm 1.20 (d, *J*=6.32 Hz, 3 H), 1.55 (s, 9 H), 2.99 - 3.69 (m, 4 H), 3.77 - 4.47 (m, 3 H), 6.98 (d, *J*=8.51 Hz, 1 H), 7.22 - 7.44 (m, 2 H), 7.55 - 7.79 (m, 2 H), 8.15 (s, 1 H), 8.21 - 8.36 (m, 2 H), 8.61 (s, 1 H), 9.56 (s, 1 H), 11.44 (bs, 1H).

### d) 1-{5-[(2S)-2-Methylpiperazin-1-yl]pyridin-3-yl}-3-(pyrazin-2-ylamino)-1H-indazol-6-ol

Obtained (94%) as hydrochloride salt from *tert*-butyl (3*S*)-4-{5-[6-hydroxy-3-(pyrazin-2-ylamino)-1H-indazol-1-yl]pyridin-3-yl}-3-methylpiperazine-1-carboxylate (example 100c) following the procedure as described in example 2c. The solid was filtered off and washed several times with diethyl ether and dried in the vacuum oven overnight.

LRMS (m/z): 403 (M+1)⁺.

¹H NMR (300 MHz, DMSO-*d*₆) δ ppm 1.30 (d, *J*=6.87 Hz, 3 H), 3.13 (d, *J*=13.73 Hz, 2 H), 3.23 - 3.46 (m, 4 H), 4.53 (bs, 1 H), 6.83 (d, *J*=8.51 Hz, 1 H), 7.28 (s, 1 H), 7.95 (bs, 1 H), 8.11 (d, *J*=8.79 Hz, 1 H), 8.20 (d, *J*=2.75 Hz, 1 H), 8.29 - 8.38 (m, 1 H), 8.58 (d, *J*=1.37 Hz, 1 H), 9.12 (bs, 1 H), 9.46 (d, *J*=1.10 Hz, 1 H), 9.56 (d, *J*=10.71 Hz, 1 H), 10.46 (s, 1 H).

### EXAMPLE 101

### 2-[1-{2-[(1S,4S)-2,5-Diazabicyclo[2.2.1]hept-2-yl]pyridin-4-yl}-3-(pyrazin-2-ylamino)-1H-indazol-6-yl]propan-2-ol

### a) tert-Butyl (1S,4S)-5-{4-[6-(1-hydroxy-1-methylethyl)-3-(pyrazin-2-ylamino)-1H-indazol-1-yl]pyridin-2-yl}-2,5-diazabicyclo[2.2.1]heptane-2-carboxylate

To a solution of methylmagnesium bromide (0.11 mL of a 3 M solution in diethyl eter, 0.44 mmol) in tetrahydrofurane (1.5 mL) previously cooled at 0°C, methyl 1-{2-[(1*S*,4*S*)-5-(*tert*-butoxycarbonyl)-2,5-diazabicyclo[2.2.1]hept-2-yl]pyridin-4-yl}-3-(pyrazin-2-ylamino)-1*H*-indazole-6-carboxylate (example 15c, 67.20 mg, 0.11 mmol) in tetrahydrofurane (1.5 mL) was added dropwise. The mixture was allowed to warm until room temperature and was stirred for 4 h. Reaction mixture was poured into saturated aqueous ammonium chloride solution and product was extracted with ethyl acetate. Organic layer was washed with brine, dried (MgSO₄) and concentrated. The crude was purified by reverse phase chromatography (C-18 from Merck, water/methanol-acetonitrile (1:1) as eluents, 0-80%) to give the desired compound.

LRMS (m/z): 543 (M+1)⁺.

### b) 2-[1-{2-[(1S,4S)-2,5-diazabicyclo[2.2.1]hept-2-yl]pyridin-4-yl}-3-(pyrazin-2-ylamino)-1H-indazol-6-yl]propan-2-ol

Obtained (71%) as hydrochloride salt from *tert*-butyl (1*S*,4*S*)-5-{4-[6-(1-hydroxy-1-methylethyl)-3-(pyrazin-2-ylamino)-1*H*-indazol-1-yl]pyridin-2-yl}-2,5-diazabicyclo[2.2.1]heptane-2-carboxylate (example 101a) following the procedure as described in example 2c. The solid was filtered off and washed several times with diethyl ether and dried in the vacuum oven overnight.

LRMS (m/z): 443 (M+1)⁺.

¹H NMR (400 MHz, CHLOROFORM-*d*) δ ppm 1.50 (s, 9 H), 2.41 (s, 3 H), 3.60 - 3.66 (m, 8 H), 6.77 (d, *J*=4.80 Hz, 1 H), 7.11 (d, *J*=1.60 Hz, 1 H), 7.14 (dd, *J*=5.20, 1.60 Hz, 1 H), 7.24-7.27 (m, 1 H), 7.53 (t, *J*=7.20 Hz, 1 H), 7.67 (br. s, 1 H), 7.74 (t, *J*=8.00 Hz, 1 H), 7.86 (d, *J*=8.40 Hz, 1 H), 8.05 (s, 1 H), 8.13 (d, *J*=5.20 Hz, 1 H), 8.28 (d, *J*=5.20 Hz, 1 H).

### EXAMPLE 102

### 1-[2-(Azetidin-3-ylamino)pyridin-4-yl]-N-pyrazin-2-yl-1H-indazol-3-amine

### a) tert-butyl 3-({4-[3-(pyrazin-2-ylamino)-1H-indazol-1-yl]pyridin-2-yl}amino)azetidine-1-carboxylate

Obtained (27%) as an oil from 1-(2-fluoropyridin-4-yl)-*N*-(pyrazin-2-yl)-1H-indazol-3-amine (preparation 9) and *tert*-butyl 3-aminoazetidine-1-carboxylate using the same experimental procedure as described in preparation 2, followed by purification by reverse phase chromatography as described in the general methods.

LRMS (m/z): 459 (M+1)⁺.

### b) 1-[2-(azetidin-3-ylamino)pyridin-4-yl]-N-pyrazin-2-yl-1H-indazol-3-amine

Obtained (19%) from *tert*-butyl 3-({4-[3-(pyrazin-2-ylamino)-1*H*-indazol-1-yl]pyridin-2-yl}amino)azetidine-1-carboxylate (example 102a) using the same experimental procedure as described in example 2c, followed by purification by reverse phase chromatography as described in the general method without buffer.

LRMS (m/z): 359 (M+1)⁺.

¹H NMR (400 MHz, DMSO-*d*₆) δ ppm 2.84 - 2.98 (m, 2 H), 4.34 - 4.42 (m, 1 H), 4.44 - 4.51 (m, 1 H), 4.64 - 4.74 (m, 1 H), 7.34 (d, *J*=1.95 Hz, 1 H), 7.47 (t, *J*=7.42 Hz, 1 H), 7.57 (dd, *J*=7.42, 2.34 Hz, 1 H), 7.74 (t, *J*=7.82 Hz, 1 H), 8.16 (d, *J*=8.60 Hz, 1 H), 8.28 (d, *J*=7.42 Hz, 1 H), 8.31 (d, *J*=2.34 Hz, 1 H), 8.40 - 8.43 (m, 1 H), 8.46 (d, *J*=7.82 Hz, 1 H), 9.53 (d, *J*=1.17 Hz, 1 H), 10.84 (br. s., 1 H).

### EXAMPLE 103

### 1-{2-[(3aR,6aS)-Hexahydropyrrolo[3,4-c]pyrrol-2(1H)-yl]pyridin-4-yl}-N-pyrazin-2-yl-1H-indazol-3-amine

### a) tert-Butyl (3aR,6aS)-5-{4-[3-(pyrazin-2-ylamino)-1H-indazol-1-yl]pyridin-2-yl}hexahydropyrrolo[3,4-c]pyrrole-2(1H)-carboxylate

Obtained (27%) as a solid from 1-(2-fluoropyridin-4-yl)-*N*-(pyrazin-2-yl)-1*H*-indazol-3-amine (preparation 9) and *tert*-butyl (3a*R*,6a*S*)-hexahydropyrrolo[3,4-*c*]pyrrole-2(1*H*)-carboxylate using the same experimental procedure as described in preparation 2.

LRMS (m/z): 499 (M+1)⁺.

### c) 1-{2-[(3aR,6aS)-hexahydropyrrolo[3,4-c]pyrro)-2(1H)-yl]pyridine-4-yl}-N-pyrazin-2-yl-1H-indazol-3-amine

Obtained as a hydrochloride salt (84%) from *tert*-butyl (3a*R*6a*S*)-5-{4-[3-(pyrazin-2-ylamino)-1*H*-indazol-1-yl]pyridine-2-yl}hexahydropyrrolo[3,4-*c*]pyrrole-2(1*H*)-carboxylate (example 103a) using the same experimental procedure as described in example 2c.

LRMS (m/z): 399 (M+1)⁺.

¹H NMR (400 MHz, DMSO-*d*₆) δ ppm 3.17 - 3.34 (m, 4 H), 3.39 - 3.50 (m, 2 H), 3.82 - 3.98 (m, 4 H), 7.07 (d, *J*=1.95 Hz, 1 H), 7.45 (t, *J*=7.42 Hz, 1 H), 7.56 (dd, *J*=7.23, 1.76 Hz, 1 H), 7.71 (t, *J*=7.62 Hz, 1 H), 8.08 (d, *J*=7.42 Hz, 1 H), 8.22 (d, *J*=8.60 Hz, 1 H), 8.29 (s, 1 H), 8.41 (s, 1 H), 8.45 (d, *J*=7.82 Hz, 1 H), 9.49 (s, 1 H), 9.95 (br. s., 1 H), 10.12 (br. s., 1 H), 10.84 (s, 1 H), 13.84 (br. s., 1 H).

### EXAMPLE 104

### 1-{6-[(1S,4S)-2,5-Diazabicyclo[2.2.1]hept-2-yl]-2-methylpyrimidin-4-yl}-3-(pyrazin-2-ylamino)-1H-indazol-6-ol

### a) tert-Butyl (1S,4S)-5-(6-chloro-2-methylpyrimidin-4-yl)-2,5-diazabicyclo[2.2.1]heptane-2-carboxylate

Obtained (98%) from 4,6-dichloro-2-methylpyrimidine and (*1S,4S*)-tert-butyl 2,5-diazabicyclo[2.2.1]heptane-2-carboxylate using the same experimental procedure as described in example 43c.

LRMS (m/z): 325 (M+1)⁺.

### b) tert-Butyl (1S,4S)-5-(6-iodo-2-methylpyrimidin-4-yl)-2,5-diazabicyclo[2.2.1]heptane-2-carboxylate

Obtained (27%) from *tert-butyl* (1*S*,4*S*)-5-(6-chloro-2-methylpyrimidin-4-yl)-2,5-diazabicyclo[2.2.1]heptane-2-carboxylate (example 104a) using the same experimental procedure as described in example 43d.

LRMS (m/z): 417 (M+1)⁺.

### c) tert-Butyl (1S,4S)-5-{6-[3-amino-6-(benzyloxy)-1H-indazol-1-yl]-2-methylpyrimidin-4-yl}-2,5-diazabicyclo[2.2.1]heptane-2-carboxylate

Obtained (45%) from 6-(benzyloxy)-1*H*-indazol-3-amine (preparation 18) and tert-butyl (1*S*,4*S*)-5-(6-iodo-2-methylpyrimidin-4-yl)-2,5-diazabicyclo[2.2.1]heptane-2-carboxylate (example 104b) using the same experimental procedure as described in example 2a, followed by purification by flash chromatography (100% hexanes to 90% ethyl acetate).

LRMS (m/z): 528 (M+1)⁺.

### d) tert-Butyl (1S,4S)-5-{6-[6-(benzyloxy)-3-(pyrazin-2-ylamino)-1H-indazol-1-yl]-2-methylpyrimidin-4-yl}-2,5-diazabicyclo[2.2.1]heptane-2-carboxylate

Obtained (74%) from tert-butyl (1*S*,4*S*)-5-{6-[3-amino-6-(benzyloxy)-1*H*-indazol-1-yl]-2-methylpyrimidin-4-yl}-2,5-diazabicyclo[2.2.1]heptane-2-carboxylate (example 104c) and 2-chloropyrazine using the same experimental procedure as described in example 2b, followed by purification by flash chromatography (100% hexanes to 100% ethyl acetate).

LRMS (m/z): 606 (M+1)⁺.

### e) tert-Butyl (1S,4S)-5-{6-[6-hydroxy-3-(pyrazin-2-ylamino)-1H-indazol-1-yl]-2-methylpyrimidin-4-yl}-2,5-diazabicyclo[2.2.1]heptane-2-carboxylate

Obtained (38%) from *tert*-butyl (1*S*,4*S*)-5-{6-[6-(benzyloxy)-3-(pyrazin-2-ylamino)-1*H-*indazol-1-yl]-2-methylpyrimidin-4-yl}-2,5-diazabicyclo[2.2.1 ]heptane-2-carboxylate (example 104d) using the same experimental procedure as described in example 86a, followed by purification by reverse phase chromatography as described in the general methods.

LRMS (m/z): 516 (M+1)⁺.

### f) 1-{6-[(1S,4S)-2,5-Diazabicyclo[2.2.1]hept-2-yl]-2-methylpyrimidin-4-yl}-3-(pyrazin-2-ylamino)-1H-indazol-6-ol

Obtained as a hydrochloride salt (54% yield) from *tert*-butyl (1*S*,4*S*)-5-{6-[6-hydroxy-3-(pyrazin-2-ylamino)-1*H*-indazol-1-yl]-2-methylpyrimidin-4-yl}-2,5-diazabicyclo[2.2.1]heptane-2-carboxylate (example 104e) using the same experimental procedure as described in example 7c.

LRMS (m/z): 416 (M+1)⁺.

¹H NMR (400 MHz, DMSO-*d*₆) δ ppm 1.98 (d, *J*=9.77 Hz, 1 H), 2.15 (d, *J*=8.99 Hz, 1 H), 2.55 (s, 3 H), 3.21 - 3.29 (m, 2 H), 3.30 - 3.38 (m, 1 H), 3.67 - 3.77 (m, 2 H), 4.55 (s, 1 H), 6.81 (dd, *J*=8.79, 2.15 Hz, 1 H), 8.03 - 8.10 (m, 1 H), 8.22 (s, 2 H), 8.35 (s, 1 H), 9.01 (br. s., 1 H), 9.48 (s, 1 H), 9.59 (br. s., 1 H), 10.50 (s, 1 H).

### EXAMPLE 105

### 1-{2-Methyl-6-[(2S)-2-methylpiperazin-1-yl]pyrimidin-4-yl}-3-(pyrazin-2-ylamino)-1H-indazol-6-ol

### a) tert-Butyl (3S)-4-(6-chloro-2-methylpyrimidin-4-yl)-3-methylpiperazine-1-carboxylate

Obtained (75%) from 4,6-dichloro-2-methylpyrimidine and (*S*)-tert-butyl 3-methylpiperazine-1-carboxylate using the same experimental procedure as described in example 43c.

LRMS (m/z): 327 (M+1)⁺.

### b) tert-Butyl (3S)-4-(6-iodo-2-methylpyrimidin-4-yl)-3-methylpiperazine-1-carboxylate

Obtained (13%) from *tert-*butyl (3*S*)-4-(6-chloro-2-methylpyrimidin-4-yl)-3-methylpiperazine-1-carboxylate (example 105a) using the same experimental procedure as described in example 43d.

LRMS (m/z): 419 (M+1)⁺.

### c) tert-Butyl (3S)-4-{6-[3-amino-6-(benzyloxy)-1H-indazol-1-yl]-2-methylpyrimidin-4-yl}-3-methylpiperazine-1-carboxylate

Obtained (21 %) from 6-(benzyloxy)-1*H*-indazol-3-amine (preparation 18) and *tert*-butyl (3*S*)-4-(6-iodo-2-methylpyrimidin-4-yl)-3-methylpiperazine-1-carboxylate (example 105b) using the same experimental procedure as described in example 2a, followed by purification by flash chromatography (100% hexanes to 90% ethyl acetate).

LRMS (m/z): 530 (M+1)⁺.

### d) tert-Butyl (3S)-4-{6-[6-(benzyloxy)-3-(pyrazin-2-ylamino)-1H-indazol-1-yl]-2-methylpyrimidin-4-yl}-3-methylpiperazine-1-carboxylate

Obtained (37%) *tert-*butyl (3*S*)-4-{6-[3-amino-6-(benzyloxy)-1*H*-indazol-1-yl]-2-methylpyrimidin-4-yl}-3-methylpiperazine-1-carboxylate (example 105c) and 2-chloropyrazine using the same experimental procedure as described in example 2b, followed by purification by flash chromatography (100% hexanes to 100% ethyl acetate).

LRMS (m/z): 608 (M+1)⁺.

### e) tert-Butyl (3S)-4-{6-[6-hydroxy-3-(pyrazin-2-ylamino)-1H-indazol-1-yl]-2-methylpyrimidin-4-yl}-3-methylpiperazine-1-carboxylate

Obtained (18%) from *tert*-butyl (3*S*)-4-{6-[6-(benzyloxy)-3-(pyrazin-2-ylamino)-1*H-*indazol-1-yl]-2-methylpyrimidin-4-yl}-3-methylpiperazine-1-carboxylate (example 105d) using the same experimental procedure as described in example 86a, followed by purification by reverse phase chromatography as described in the general methods.

LRMS (m/z): 518 (M+1)⁺.

### f) 1-{2-Methyl-6-[(2S)-2-methylpiperazin-1-yl]pyrimidin-4-yl}-3-(pyrazin-2-ylamino)-1H-indazol-6-ol

Obtained as a hydrochloride salt (84% yield) from *tert*-butyl (3*S*)-4-{6-[6-hydroxy-3-(pyrazin-2-ylamino)-1*H*-indazol-1-yl]-2-methylpyrimidin-4-yl}-3-methylpiperazine-1-carboxylate (example 105e) using the same experimental procedure as described in example 7c.

LRMS (m/z): 418 (M+1)⁺.

### EXAMPLE 106

### 3-[(1S,4S)-2,5-Diazabicyclo[2.2.1]hept-2-yl]-5-[3-(pyrazin-2-ylamino)-1H-indazol-1-yl]pyridin-2-ol

Obtained as hydrochloride salt (9%) from *tert*-butyl (1*S*,4*S*)-5-{2-methoxy-5-[3-(pyrazin-2-ylamino)-1*H*-indazol-1-yl]pyridin-3-yl}-2,5-diazabicyclo[2.2.1]heptane-2-carboxylate (example 94c) using the same experimental procedure as described in example 2c, followed by purification by reverse phase chromatography as described in the general methods without buffer.

LRMS (m/z): 401 (M+1)⁺.

¹H-NMR (400 MHz, DMSO-*d*₆) δ ppm 1.86 (d, *J* =10.55 Hz, 1 H), 2.07 (d, *J* =10.94 Hz, 1 H), 3.16 - 3.23 (m, 2 H), 3.46 (d, *J* =11.72 Hz, 1 H), 3.72 (d, *J* =10.55 Hz, 1 H), 4.38 (s, 1 H), 5.25 (s, 1 H), 6.68 (d, *J* =2.34 Hz, 1 H), 7.15 - 7.22 (m, 2 H), 7.44 - 7.49 (m, 1 H), 7.52 - 7.56 (m, 1 H), 8.12 (d, *J* =2.34 Hz, 1 H), 8.14 (d, *J* =8.21 Hz, 1 H), 8.26 (dd, *J* =2.34, 1.56 Hz, 1 H), 8.74 (br. s., 1 H), 9.25 - 9.31 (m, 2 H), 10.27 (s, 1 H), 11.74 (br. s., 1 H).

### EXAMPLE 107

### 1-{3-[(2S)-2-Methylpiperazin-1-yl]phenyl}-3-(pyrazin-2-ylamino)-1H-indazol-6-ol

### a) tert-Butyl (3S)-4-(3-bromophenyl)-3-methylpiperazine-1-carboxylate

Obtained (29%) from 1,3-dibromobenzene and (*S*)-*tert* butyl 3-methylpiperazine-1-carboxylate using the same experimental procedure as described in preparation 7 followed by purification by flash chromatography.

LRMS (m/z): 356 (M+1)⁺.

### b) tert-Butyl (3S)-4-{3-[3-amino-6-(benzyloxy)-1H-indazol-1-yl]phenyl}-3-methylpiperazine-1-carboxylate

Obtained (35%) from 6-(benzyloxy)-1*H*-indazol-3-amine (preparation 18) and *tert*-butyl (3*S*)-4-(3-bromophenyl)-3-methylpiperazine-1-carboxylate (example 107a) using the same experimental procedure as described in example 2a, followed by purification by flash chromatography (100% hexanes to 50% ethyl acetate).

LRMS (m/z): 514 (M+1)⁺.

### c) tert-butyl (3S)-4-{3-[6-(benzyloxy)-3-(pyrazin-2-ylamino)-1H-indazol-1-yl]phenyl}-3-methylpiperazine-1-carboxylate

Obtained (40%) *tert*-butyl (3*S*)-4-{3-[3-amino-6-(benzyloxy)-1*H*-indazol-1-yl]phenyl}-3-methylpiperazine-1-carboxylate (example 107b) and 2-chloropyrazine using the same experimental procedure as described in example 2b, followed by purification by flash chromatography (100% hexanes to 50% ethyl acetate).

LRMS (m/z): 592 (M+1)⁺.

### d) tert-Butyl (3S)-4-{3-[6-hydroxy-3-(pyrazin-2-ylamino)-1H-indazol-1-yl]phenyl}-3-methylpiperazine-1-carboxylate

Obtained (60%) from *tert*-butyl (3*S*)-4-{3-[6-(benzyloxy)-3-(pyrazin-2-ylamino)-1*H-*indazol-1-yl]phenyl}-3-methylpiperazine-1-carboxylate (example 107c) using the same experimental procedure as described in example 86a, followed by purification by reverse phase chromatography as described in the general methods.

LRMS (m/z): 502 (M+1)⁺.

### e) 1-{3-[(2S)-2-Methylpiperazin-1-yl]phenyl}-3-(pyrazin-2-ylamino)-1H-indazol-6-ol

Obtained (38%) from *tert*-butyl (3*S*)-4-{3-[6-hydroxy-3-(pyrazin-2-ylamino)-1*H*-indazol-1-yl]phenyl}-3-methylpiperazine-1-carboxylate (example 107d) using the same experimental procedure as described in example 2c, followed by purification by reverse phase chromatography as described in the general methods. The solid obtained was acidified with a dioxane solution of hydrochloric acid and stirred at room temperature overnight to give the title compound as hydrochloride salt.

LRMS (m/z): 402 (M+1)⁺.

¹H NMR (600 MHz, DMSO-*d*₆) δ ppm 1.23 (d, *J* =7.04 Hz, 3 H), 3.17 - 3.22 (m, 1 H), 3.28 - 3.43 (m, 4 H), 3.67 (d, *J* =12.91 Hz, 1 H), 4.29 - 4.34 (m, 1 H), 6.80 (dd, *J* =8.80, 1.76 Hz, 1 H), 7.03 (d, *J* =7.04 Hz, 1 H), 7.20 (s, 1 H), 7.29 - 7.36 (m, 2 H), 7.53 (t, *J* =7.92 Hz, 1 H), 8.07 (d, *J* =8.80 Hz, 1 H), 8.21 (d, *J* =2.35 Hz, 1 H), 8.35 - 8.39 (m, 1 H), 9.18 (br. s., 1 H), 9.45 (s, 1 H), 9.63 (br. s., 1 H), 10.39 (s, 1 H).

Compounds of the invention intended for pharmaceutical use may be administered as crystalline or amorphous products, or mixtures thereof. They may be obtained, for example, as solid plugs, powders, or films by methods such as precipitation, crystallization, freeze drying, spray drying, or evaporative drying.
Microwave or radio frequency drying may be used for this purpose.

### BIOLOGICAL TESTING

### Syk Enzyme assay- Radioactive filtration kinase assay

Recombinant human full-length Syk was supplied by Millipore. The activity of Syk kinase was assessed using a radioactive filtration kinase assay.

10 µL of substrate poly-GT (100 µg/mL final) and 4 µl of Syk (1 nM final) in 50 mM Tris-HCl (pH 7.5), 0.1 mM EGTA, 4 mM Mg(CH₃COO)₂, 0.1 mM Na₃VO₄, 0.1% (v/v) β-mercaptoethanol, 0.133 mg/mL BSA (bovine serum albumin) were added to 1.5 µl of various concentrations of compound or DMSO vehicle (5% final). The reaction was initiated by the addition of 14.5 µL of 0.36 µCi [γ-³³P] ATP (adenosine tri-phosphate) (10 mCi/ml, PerkinElmer). This was prepared in a half-area NBS plate (Corning) and allowed to stand at room temperature for 40 min.

15 µL of reaction mixture was transferred to a filter plate (Millipore) previously prewetted with a solution of 75 mM phosphoric acid. The reaction was terminated with the addition of 200 µL phosphoric acid. The filter plate was washed three times under vacuum on a vacuum manifold (Millipore cat# MSVMHTS00) at 8" Hg vacuum level with 200 µL phosphoric acid per wash.

30 µL Optiphase™ Supermix (PerkinElmer) was added to each test well and allowed to incubate for at least 1 h before counting. Radioactivity counting was performed in a Wallac MicroBeta^{®}, and the tyrosine phosphorylation activity by Syk was calculated.

### LAD2 cells assay

LAD2 is a human mast cell line depending on stem cell factor for its survival, maturation and differentiation. This mast cell line was established by the NIH from bone marrow aspirates from a patient with mast cell sarcoma. LAD2 cells resemble CD34+ - derived human mast cells as they have granules containing tryptase, histamine and β-hexosaminidase. They also express c-kit and FcεRI (Kirshenbaum, et al., Leukemia Research 27: 677-682, 2003).

### Sensitization of LAD2 cells

Cells were sensitized (0.2x10⁶ cells/mL) in its normal growth factor (Complete StemPro-34 SFM, Gibco 10639-011 containing StemPro-34 nutrient supplement (1/40); glutamine (2 mM) and supplemented with 100 ng/ml SCF) by adding 100 ng/mL of biotin-labeled IgE (USBiological) overnight (16-18 h, 37º, 5% CO₂) in a humidified atmosphere (90%).

### Compound preparation

Compounds were prepared from a 10⁻²M stock in 100% DMSO to give seven successive 1/3 dilutions in DMSO. From each concentration, 2 further 1/10 dilutions were prepared in release buffer (137 mM NaCl, 2.70 mM KCI, 0.4 mM NaH₂PO₄, 5.6 mM (D)-glucose, 1.8 mM CaCl₂, 1.3 mM MgSO₄, 0.025% BSA). Finally, 5 µl from each diluted concentration were transferred into the assay plate (flat 96-well Nunc). The final compound concentrations tested ranged from 1x10⁻⁵M to 4.12x10⁻⁹M with a final proportion of DMSO in the assay of 0.1%. Control wells were treated with 0.1 % DMSO.

### LAD2 cells activation

Sensitized LAD2 cells were centrifuged (300 g, 3 min, R.T.) and washed 2 times with release buffer. Cells were resuspended in release buffer and adjusted to a density of 0.2x10⁶/mL. 45 µL of the adjusted cells were transfered to the assay plate (∼10,000cells/well) containg 5 µL of the final compound concentrations. LAD2 cells were incubated with the compound for 30 min (37ºC, 5% CO₂ 90% of humidity) and then activated with 125 ng/mL of streptavidin (Pierce 21125) for 30 min (37°C, 5% CO₂, 90% of humidity). After the activation, cells were centrifuged at 1200 g, 10 min, 12ºC and supernatats were removed and transfered to another well for determination of released β-hexosaminidase. Empty wells were filled with 50 µL of release buffer for determination of intracellular β-hexosaminidase. Cells were lysed with 2 cycles of freeze-thaw.

### β-hexosaminidase assay

Cells were incubated for 90 minutes, 37ºC with 1 mM β-hexosaminidase substrate (p-nitrophenyl *N*-acetyl-D-glucosamide, in citric buffer, pH 4.5). Reaction was stopped with 0.4 M glycine solution (pH 10.0) and the plate is read immediately at 405 nm.

| **Example** | **Syk IC₅₀ (nM)** | **LAD₂ IC₅₀ (nM)** |
|---|---|---|
| Ex. 8 | 17 | - |
| Ex. 10 | 7,4 | 55 |
| Ex. 12 | 2,5 | 167 |
| Ex. 21 | 10,5 | 342 |
| Ex. 30 | 20 | - |
| Ex. 31 | 8 | 111 |
| Ex. 42 | 9 | 254 |
| Ex. 50 | 11 | 80 |
| Ex. 55 | 6 | 142 |
| Ex. 56 | 15 | 179 |
| Ex. 58 | 4 | 266 |
| Ex. 61 | 12 | 136 |
| Ex.62 | 17 | 169 |
| Ex. 63 | 11 | 363 |
| Ex. 69 | 13 | 263 |
| Ex. 71 | 5 | 111 |
| Ex. 76 | 11 | 137 |
| Ex. 86 | 15 | 263 |
| Ex. 88 | 5 | 364 |
| Ex. 89 | 16 | 160 |

As it can be seen form the table, compounds of the present invention are potent inhibitor of Syk kinases. Preferred compounds of the invention posses an IC₅₀ value for the inhibition of Syk kinase of less than 1 µM, preferably less than 100 nM, more preferably less than 20 nM, being most preferable less than 10 nM.

Compounds described herein have shown activity inhibiting LAD2 degranulation with an IC₅₀ value of <10 µM, preferably less than 1 µM and more preferably less than 100 nM.

### PHARMACEUTICAL COMPOSITIONS

### Administration routes

### i) Oral Administration

The compounds of the invention may be administered orally (peroral administration; *per os* (latin)). Oral administration involve swallowing, so that the compound is absorbed from the gut and delivered to the liver via the portal circulation (hepatic first pass metabolism) and finally enters the gastrointestinal (GI) tract.

Compositions for oral administration may take the form of tablets, retard tablets, sublingual tablets, capsules, inhalation aerosols, inhalation solutions, dry powder inhalation, or liquid preparations, such as mixtures, solutions, elixirs, syrups or suspensions, all containing the compound of the invention; such preparations may be made by methods well-known in the art. The active ingredient may also be presented as a bolus, electuary or paste.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl- substituted hydroxypropyl cellulose, starch, pregelatinized starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinized starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate. Tablets may also optionally include surface active agents, such as sodium lauryl sulfate and polysorbate 80, and glidants such as silicon dioxide and talc. When present, surface active agents are typically in amounts of from 0.2 wt% to 5 wt% of the tablet, and glidants typically from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally are present in amounts from 0.25 wt% to 10 wt%, preferably from 0.5 wt% to 3 wt% of the tablet. Other conventional ingredients include anti-oxidants, colorants, flavoring agents, preservatives and taste-masking agents.

Exemplary tablets contain up to about 80 wt% drug, from about 10 wt% to about 90 wt% binder, from about 0 wt% to about 85 wt% diluent, from about 2 wt% to about 10 wt% disintegrant, and from about 0.25 wt% to about 10 wt% lubricant. Tablet blends may be compressed directly or by roller to form tablets. Tablet blends or portions of blends may alternatively be wet-, dry-, or melt-granulated, melt congealed, or extruded before tabletting. The final formulation may include one or more layers and may be coated or uncoated; or encapsulated.

The formulation of tablets is discussed in detail in "Pharmaceutical Dosage Forms: Tablets, Vol. 1 ", by H. Lieberman and L. Lachman, Marcel Dekker, N.Y., 1980.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Solid formulations for oral administration may be formulated to be immediate and/or modified release. Modified release formulations include delayed-, sustained-, pulsed-, controlled-, targeted and programmed release.

Suitable modified release formulations are described in U.S. Patent No. 6,106,864. Details of other suitable release technologies such as high energy dispersions and osmotic and coated particles can be found in Verma et al, Pharmaceutical Technology On-line, 25(2), 1-14 (2001). The use of chewing gum to achieve controlled release is described in WO 00/35298. The disclosures of these references are incorporated herein by reference in their entireties.

Liquid formulations include suspensions, solutions, syrups and elixirs. Such formulations may be used as fillers in soft or hard capsules and typically include a carrier, for example, water, ethanol, polyethylene glycol, propylene glycol, methylcellulose, or a suitable oil, and one or more emulsifying agents and/or suspending agents. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent. Liquid formulations may also be prepared by the reconstitution of a solid, for example, from a sachet.

### ii) Oral mucosal administration

The compounds of the invention can also be administered via the oral mucosal. Within the oral mucosal cavity, delivery of drugs is classified into three categories: (a) sublingual delivery, which is systemic delivery of drugs through the mucosal membranes lining the floor of the mouth, (b) buccal delivery, which is drug administration through the mucosal membranes lining the cheeks (buccal mucosa), and (c) local delivery, which is drug delivery into the oral cavity.

Pharmaceutical products to be administered via the oral mucosal can be designed using mucoadhesive, quick dissolve tablets and solid lozenge formulations, which are formulated with one or more mucoadhesive (bioadhesive) polymers (such as hydroxy propyl cellulose, polyvinyl pyrrolidone, sodium carboxymethyl cellulose, hydroxy propyl methyl cellulose, hydroxy ethyl cellulose, polyvinyl alcohol, polyisobutylene or polyisoprene); and oral mucosal permeation enhancers (such as butanol, butyric acid, propranolol, sodium lauryl sulphate and others)

### iii) Inhaled administration

The compounds of the invention can also be administered by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1 ,1 ,1 ,2-tetrafluoroethane or 1 ,1 ,1 ,2,3,3,3-heptafluoropropane. For intranasal use, the powder may include a bioadhesive agent, for example, chitosan or cyclodextrin.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2 µg and 1000 µg of each therapeutically active ingredient. or the equivalent amount of a pharmaceutically acceptable salt thereof. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/00771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuair® (formerly known as Novolizer SD2FL), which is described the following patent applications Nos: WO97/000703, WO03/000325 and WO2006/008027.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with. Such atomiser is the Respimat® which is described, for example, in PCT Patent Applications Nos. WO 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetra-fluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants (eg oleic acid or lecithin) and cosolvens (eg ethanol). Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10 µm, preferably 2-5 µm. To achieve these particle sizes the particles of the active ingredient as produced may be size reduced by conventional means eg by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.
Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

### iv) Nasal mucosal administration

The compounds of the invention may also be administered via the nasal mucosal.

Typical compositions for nasal mucosa administration are typically applied by a metering, atomizing spray pump and are in the form of a solution or suspension in an inert vehicle such as water optionally in combination with conventional excipients such as buffers, anti-microbials, tonicity modifying agents and viscosity modifying agents.

### v) Parenteral Administration

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilization, may readily be accomplished using standard pharmaceutical techniques well known to those skilled in the art. The solubility of compounds of the invention used in the preparation of parenteral solutions may be increased by the use of appropriate formulation techniques, such as the incorporation of solubility-enhancing agents. Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

### vi) Topical Administration

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated; see, for example, J Pharm Sci, 88 (10), 955-958 by Finnin and Morgan (October 1999). Other means of topical administration include delivery by electroporation, iontophoresis, phonophoresis, sonophoresis and microneedle or needle-free injection.

Formulations for topical administration may be formulated to be immediate and/or modified release. Modified release formulations include delayed-, sustained-, pulsed-, controlled-, targeted and programmed release.

### vii) Rectal/Intravaginal Administration

Compounds of the invention may be administered rectally or vaginally, for example, in the form of a suppository, pessary, or enema. Cocoa butter is a traditional suppository base, but various alternatives may be used as appropriate. Formulations for rectal/vaginal administration may be formulated to be immediate and/or modified release. Modified release formulations include delayed-, sustained-, pulsed-, controlled-, targeted and programmed release.

### viii) Ocular Administration

Compounds of the invention may also be administered directly to the eye or ear, typically in the form of drops of a micronized suspension or solution in isotonic, pH-adjusted, sterile saline. Other formulations suitable for ocular and aural administration include ointments, biodegradable {e.g. absorbable gel sponges, collagen) and nonbiodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

### ix) Other Technologies

Compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.
The amount of the active compound administered will be dependent on the subject being treated, the severity of the disorder or condition, the rate of administration, the disposition of the compound and the discretion of the prescribing physician. However, an effective dosage is typically in the range of 0.01-3000 mg, more preferably 0.5-1000 mg of active ingredient or the equivalent amount of a pharmaceutically acceptable salt thereof per day. Daily dosage may be administered in one or more treatments, preferably from 1 to 4 treatments, per day.

The pharmaceutical formulations may conveniently be presented in unit dosage form and may be prepared by any of the methods well known in the art of pharmacy.
Preferably the composition is in unit dosage form, for example a tablet, capsule or metered aerosol dose, so that the patient may administer a single dose.
Each dosage unit contains suitably from 0.5 µg to 1000 µg.

The active substance compositions according to the invention are preferably administered in the form of compositions for inhalation delivered with the help of inhalers, especially dry powder inhalers; however, any other form of nasal, topical, parenteral or oral application is possible. Here, the application of inhaled compositions embodies one of the preferred application form, especially in the therapy of obstructive lung diseases or for the treatment of asthma.The pharmaceutical formulations may conveniently be presented in unit dosage form and may be prepared by any of the methods well known in the art of pharmacy. All methods include the step of bringing the active ingredient(s) into association with the carrier. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

### Combination products

A pharmaceutical composition according to the invention comprises a compound of formula (I) and a therapeutically effective amount of one or more additional therapeutic agents selected from the group consisting of a β2-adrenergic agonist, a corticosteroid, an anti-cholinergic agent, an anti-allergic agent, an anti-inflammatory agent, an immunosuppressant, a PDE4 inhibitor, a topically acting p38 inhibitor, an IKK2 inhibitor, an A_{2A} agonist, a PI3Kδγinhibitor and an anti-infective agent, for simultaneous, separate or sequential use in the treatment of the human or animal body.

Examples of suitable β2-agonists that can be combined with the compounds of the present invention are: terbutaline sulphate, eformoterol fumarate, formoterol fumarate, bambuterol, ibuterol, isoprenaline hydrochloride, dopexamine, metaprotenerol, tulobuterol, procaterol hydrochloride, sibenadet hydrochloride, mabuterol hydrochloride, albuterol sulphate, salbutamol sulphate, salmefamol, salmeterol xinafoate, carmoterol hydrochloride, (R)-albuterol hydrochloride, Levalbuterol hydrochloride; Levosalbutamol hydrochloride; (-)-Salbutamol hydrochloride, (R,R)-Formoterol tartrate; Arformoterol tartrate, sulfonterol, Bedoradrine sulphate, Indacaterol, Trantinterol hydrochloride, Milveterol hydrochloride, Olodaterol, fenoterol hydrobromide, rimoterol hydrobromide, riproterol hydrochloride, Vilanterol broxaterol, pirbuterol hydrochloride, bitolterol mesylate, clenbuterol hydrochloride, AZD-3199, GSK-159802; GSK-597901, GSK-678007, GSK-961081; 4-[2-[3-(1 H-Benzimidazol-1-yl)-1,1-dimethylpropylamino]-1-hydroxyethyl]-2-(4-methoxybenzylamino)phenol, 1-[2H-5-hydroxy-3-oxo-4H-1,4-benzoxazin-8-yl]-2-[3-(4-*N*,*N*-dimethylaminophenyl)-2-methyl-2-propylamino]ethanol, 1-[2H-5-hydroxy-3-oxo-4H-1,4-benzoxazin-8-yl]-2-[3-(4-domethoxyphenyl)-2-methyl-2-propylamino]ethanol, 1-[2H-5-hydroxy-3-oxo-4H-1,4-benzoxazin-8-yl]-2-[3-(4-n-butyloxyhenyl)-2-methyl-2-propylamino]ethanol, KUL-1248, HOKU-81, SM-110444, RP-58802B, LAS 100977 and compounds described in international patent applications Nos. WO2007/124898, WO2006/122788A1, WO2008/046598 and WO2008095720.

Examples of suitable corticosteroids and glucocorticoids that can be combined with compounds of the present invention are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RS-85095, CGP-13774, GW-250495, deltacortisone, NO-Prednisolone, etiprednol dicloacetate, QAE-397, 7beta-OH-EPIA, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11 beta-hydroxy-17alpha-[2-(methylsulfanyl)acetoxy]-4-pregnene-3,20-dione, Desisobutyrylciclesonide, hydrocortisone acetate, hydrocortisone sodium succinate, prednisolone sodium phosphate and hydrocortisone probutate, Prednisolone sodium metasulfobenzoate and clobetasol propionate.

Examples of suitable M3 antagonists (anticholinergics) that can be combined with the compounds of the present invention are tiotropium salts, oxitropium salts, flutropium salts, ipratropium salts, glycopyrronium salts, trospium salts, zamifenacin, revatropate, espatropate, darotropium bromide, Cl-923, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), *N*-[1-(6-Aminopyridin-2-ylmethyl)piperidin-4-yl]-2(R)-[3,3-difiuoro-1 (R)-cyclopentyl]-2-hydroxy-2-phenylacetamide (J-104135), 2(R)-Cyclopentyl-2-hydroxy-*N*-[1-[4(S)-methylhexyl]piperidin-4-yl]-2-phenylacetamide (J-106366), 2(R)-Cyclopentyl-2-hydroxy-*N*-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), *N*-[*N*-[2-[*N*-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, *N*-[1-(3-Hydroxybenzyl)-1-methylpiperidinium-3(S)-yl]-*N*-[*N*-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Examples of suitable anti-allergic agents that can be combined with the compounds of the present invention are anti-histamines (e.g. Methapyrilene, Mequitazine, Azelastine hydrochloride, Acrivastine, Emedastine difumarate, Emedastine fumarate, Loratadine, Cyproheptadine hydrochloride, Diphenhydramine hydrochloride, Doxepin hydrochloride, Promethazine hydrochloride, Levocabastine hydrochloride, Desloratadine, Cinnarizine, Setastine hydrochloride, Mizolastine, Ebastine, Cetirizine hydrochloride, Epinastine hydrochloride, Olopatadine hydrochloride, Bepotastine besilate,Triprolidine hydrochloride, Rupatadine fumarate, Fexofenadine hydrochloride, Levocetirizine dihydrochloride, Ketotifen, Azatadine maleate, Dimethindene maleate, Clemastine fumarate, Alcaftadine, Bilastine, Vapitadine hydrochloride, AZD-1744, GSK-1 004723D, GSK-835726, SUN-1334H).

Examples for suitable anti-inflammatory agents that can be combined with the compounds of the present invention are non-steroidal anti-inflammatory drugs (NSAID), leukotriene antagonists (e.g. Ibudilast, Pranlukast hydrate, Zafirlukast, Montelukast, Tipelukast), FLAP inhibitors, lipoxygenase inhibitors, cyclooxygenase inhibitors (e.g. diclofenac, ibuprofen, celecoxib), elastase inhibitors, CRTH2 antagonists (e.g. Ramatroban, AMG-009, OC-000459), mast cell stabilizers (e.g. nedocromil, pemirolast potassium, chromoglycate sodium, suplatast tosilate).PDE inhibitors (e.g. theophylline), JAK kinase inhibitors or another Syk kinase inhibitor.

Examples for suitable immunosupressants that can be combined with the compounds of the present invention are picremolimus, tacromilus, cyclosporine A, leflunomide, methotrexate, anti-TNF agents and compounds described in International patent applications Nos. PCT/EP2008/006573 and WO2008/077639.

Examples of suitable PDE4 inhibitors that can be combined with compounds of the present invention are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, filaminast, (R)-(+)-4-[2-(3-Cyclopentyloxy-4-methoxyphenyl)-2-phenylethyl]pyridine (CDP-840), *N*-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-*N*6-methyl-2-(trifluoromethyl)adenine (NCS-613), *N*-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(*N*,*N*-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)-naphthalen-1-yl]-1-(2-methoxyethyl)pyridin-2(1H)-one (T-440), (-)-trans-2-[3'-[3-(*N-*Cyclopropylcarbamoyl)-4-oxo-1,4-dihydro-1,8-naphthyridin-1-yl]-3-fluorobiphenyl-4-yl]-cyclopropanecarboxylic acid (MK-0873), CDC-801, UK-500001, BLX-914, 2-carbomethoxy-4-cyano-4-(3-cyclopropylmethoxy-4-difluroromethoxy-phenyl)-cyclohexan1-one, *cis* [4-cyano-4-(3-cyclopropylmethoxy-4-difluoromethoxyphenyl)-cyclohexan-1-ol, CDC-801, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the salts claimed in the PCT patent applications number WO03/097613, WO2004/058729, WO 2005/049581, WO 2005/123693 and WO 2005/123692.

Examples of suitable p38 inhibitors that can be combined with compounds of the present invention are dilmapimod, losmapimod, regorafenib, PH-797804 (from Pfizer), KC-706 (from Kemia), ARRY-614 (from Array Biopharma), TA-5493 (from Tanabe Pharma), ARRY-797 (from Array Biopharma), BMS-582949 (from Bristol-Myers Squibb), AKP-001 (from Aska Pharmaceutical), GSK-610677 (from GlaxoSmith Kline) and UR-13870 (from Palau Pharma).

Examples of suitable IKK2 inhibitors that can be combined with compounds of the present invention are IMD-0354 (from Institute of Medicinal Molecular Design), MLN-0415 (from Millennium Pharmaceuticals) and SAR-113945 (from Sanofi-Aventis).

Examples of suitable A_{2A} agonists that can be combined with the compounds of the present invention are binodenoson, apanedoson, regadenoson and sonedenoson.

Examples of suitable antagonists of the PI3Kδγ inhibitors that can be combined with the compounds of the present invention are 2-Methyl-2-[4-[3-methyl-2-oxo-8-(3-quinolinyl)-2,3-dihydro-1H-imidazo[4,5-c]quinolin-1-yl]phenyl]propanenitrile (BEZ-235 from Novartis), CAL-101 (from Calistoga Pharmaceuticals) and *N*-Ethyl-*N*'-[3-(3,4,5-trimethoxyphenylamino)pyrido[2,3-b]pyrazin-6-yl]thiourea (AEZS-126 from Aeterna Zentaris).

Examples for suitable anti-infectives that can be combined with the compounds of the present invention are mupiricin, retapamulin, clotrimazole, ketoconazole and terbinafine.

The combinations of the invention may be used in the treatment of respiratory diseases, wherein the use of bronchodilating agents is expected to have a beneficial effect, for example asthma, acute or chronic bronchitis, emphysema, or Chronic Obstructive Pulmonary Disease (COPD).

The active compounds in the combination may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The active substance compositions according to the invention are preferably administered in the form of compositions for inhalation delivered with the help of inhalers, especially dry powder inhalers, however, any other form or parenteral or oral application is possible. Here, the application of inhaled compositions embodies the preferred application form, especially in the therapy of obstructive lung diseases or for the treatment of asthma.

Additional suitable carriers for formulations of the active compounds of the present invention can be found in Remington: The Science and Practice of Pharmacy, 20th Edition, Lippincott Williams & Wilkins, Philadelphia, Pa., 2000. The following nonlimiting examples illustrate representative pharmaceutical compositions of the invention.

### FORMULATION EXAMPLES

The following preparations forms are cited as formulation examples:

| Formulation Example 1 (Oral suspension) | |
|---|---|
| **Ingredient** | **Amount** |
| Active Compound | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 mg |
| Distilled water | q.s. to 100 mL |

| Formulation Example 2 (Hard gelatine capsule for oral administration) | |
|---|---|
| **Ingredient** | **Amount** |
| Active Compound | 1 mg |
| Lactose | 150 mg |
| Magnesium stearate | 3 mg |

| Formulation Example 3 (Gelatin cartridge for inhalation) | |
|---|---|
| **Ingredient** | **Amount** |
| Active Compound (micronized) | 0,2 mg |
| Lactose | 25 mg |

| Formulation Example 4 (Formulation for inhalation with a DPI) | |
|---|---|
| **Ingredient** | **Amount** |
| Active Compound (micronized) | 15 mg |
| Lactose | 3000 mg |

| Formulation Example 5 (Formulation for a MDI) | |
|---|---|
| **Ingredient** | **Amount** |
| Active Compound (micronized) | 10 g |
| 1,1,1,2,3,3,3-heptafluoro-n-propane | q.s. to 200 ml |

Modifications, which do not affect, alter, change or modify the essential aspects of the compounds, combinations or pharmaceutical compositions described, are included within the scope of the present invention.

## Claims

1. A compound of formula (I), or a pharmaceutically acceptable salt or N-oxide or solvate or deuterated derivative thereof: 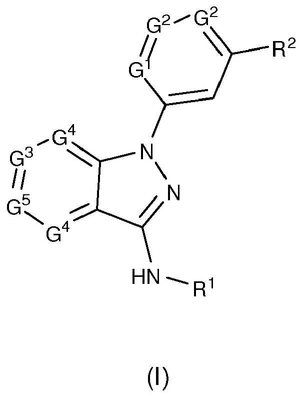 wherein:
• G¹ is selected from the group consisting of a N atom and a -CH group;
• each G² is the same or different and is independently selected from the group consisting of a N atom and a -CR³ group;
• G³ is selected from the group consisting of a N atom and a -CR⁴ group;
• each G⁴ is the same or different and is independently selected from the group consisting of a N atom and a-CH group;
• G⁵ is selected from the group consisting of a N atom and a -CR⁵ group;
• R¹ is a 5 to 6-membered monocyclic heteroaryl group comprising at least one N atom, wherein said heteroaryl group is optionally substituted with one or more substituents selected from the group consisting of a linear or branched C₁₋₆ alkyl group and a -CF₃ group;
• R² is selected from the group consisting of a -NR^{a}R^{b} group, a N-containing saturated or unsaturated 5 to 10-membered heterocyclyl group and a 5 to 6-membered monocyclic heteroaryl group comprising at least one N atom, wherein
o R^{a} represents a hydrogen atom, a linear or branched C₁₋₆ alkyl group or a linear or branched C₁₋₆ alkoxy group, and R^{b} represents a monocyclic or polycyclic C₃₋₁₀ cycloalkyl group or a N-containing saturated or unsaturated 4 to 10-membered heterocyclyl group; or
o R^{a} and R^{b} together with the nitrogen atom to which they are attached form a N-containing saturated 5 to 10-membered monocyclic or bicyclic heterocyclyl group optionally containing one additional N atom,
wherein:
the heterocyclyl, heteroaryl, and cycloalkyl groups are optionally substituted with one or more substituents selected from the group consisting of a halogen atom, a -CN group, a -CF₃ group, a hydroxy group, a C₁₋₆ alkyl group, a C₁₋₆ alkoxy-C₁₋₆ alkyl group, a C₁₋₆ hydroxyalkyl group, an amino group and a carbamoyl group;
• R³ is selected from the group consisting of a hydrogen atom, a -CF₃ group, a phenyl group, a C₁₋₆ alkyl group, a hydroxy group and a C₁₋₆ alkoxy group; wherein the alkyl and phenyl groups are optionally substituted with one or more substituents selected from a hydroxy group, a C₁₋₆ alkoxy group and a halogen atom;
• R⁴ is selected from the group consisting of a hydrogen atom, a halogen atom, a -CN group, a hydroxy group, a linear or branched C₁₋₆ alkyl group, a linear or branched C₁₋₆ alkylthio group, a linear or branched C₁₋₆ alkoxy group, a (C₁₋₆ alkoxy)-(C₁₋₆ alkoxy) group, a C₆₋₁₀ aryl group, a (C₆₋₁₀ aryl)-(C₁₋₆ alkyl) group, a C₆₋₁₀ aryl-C₁₋₆ alkoxy group, a 5 to 10-membered heteroaryl group, a C₃₋₁₀ cycloalkyl group, a -(CH₂)₀₋₁COOR^{d} group, a -CONR^{c}R^{d} group, a -NR^{c}CO_{R}^{d} group, a -NHSO₂R^{d} group and a -NR^{c}R^{d} group; wherein
o R^{c} represents a hydrogen atom or a linear or branched C₁₋₆ alkyl group, and R^{d} represents a hydrogen atom, a linear or branched C₁₋₆ alkyl group, a C₃₋₁₀ cycloalkyl group, a -(CH₂)₀₋₁C₆₋₁₀ aryl group, a 5 to 6-membered heteroaryl group containing at least one heteroatom selected from N, S and O; or
o R^{c} and R^{d} together with the nitrogen atom to which they are attached form a N-containing 4 to 6-membered heterocyclyl group;
wherein the alkyl, aryl, heteroaryl and heterocyclyl groups are optionally substituted with one or more substituents selected from a halogen atom, a hydroxy group, a linear or branched C₁₋₆ alkyl group, a linear or branched C₁₋₆ alkoxy group, a carboxy group and a benzyl group; and
• R⁵ is selected from the group consisting of a hydrogen atom, a halogen atom and a cyano group.

2. A compound according to claim 1, wherein R¹ represents a pyrazinyl group or a pyridyl group, wherein the pyridyl group is optionally substituted by a methyl group or a CF₃ group.

3. A compound according to claims 1 or 2, wherein R² represents a -NR^{a}R^{b} group, wherein R^{a} and R^{b} together with the nitrogen atom to which they are attached form a saturated 5- to 10-membered monocyclic or bicyclic heterocyclyl group containing one additional N atom, wherein the heterocyclyl is optionally substituted with one or more C₁₋₆ alkyl groups.

4. A compound according to any preceeding claim, wherein R³ is selected from the group consisting of a hydrogen atom, a C₁₋₆ alkyl group and a phenyl group, wherein the phenyl group is optionally substituted by a halogen atom.

5. A compound according to any preceeding claim, wherein R⁴ is selected from the group consisting of a hydrogen atom, a hydroxy group, an amino group, a linear or branched C₁₋₆ alkyl group, a linear or branched C₁₋₆ alkylthio group, a linear or branched C₁₋₆ alkoxy group, a (C₁₋₆ alkoxy)-(C₁₋₆ alkoxy) group, a C₃₋₁₀ cycloalkyl group, a 5 to 6-membered heteroaryl group, a C₆ aryl group, a morpohlinyl group, a -CONR^{c}R^{d} group, a -NR^{c}COR^{d} group, -NHSO₂R^{d} group; wherein
o R^{c} represents a hydrogen atom or methyl group, and R^{d} represents a linear or branched C₁₋₆ alkyl group, a C₃₋₁₀ cycloalkyl group or a C₆ aryl group, or
o R^{c} and R^{d} together with the nitrogen atom to which they are attached form a N-containing 4- to 6-membered heterocyclic group,
wherein the alkyl, aryl and heteroaryl groups are optionally substituted with a methyl group or a hydroxy group.

6. A compound according to any preceeding claim, wherein G⁵ represents a CH group.

7. A compound according to any preceeding claim, wherein
• R¹ represents a pyrazinyl group or a pyridyl group, wherein the pyridyl group is optionally substituted by a methyl group or a CF₃ group;
• R² represents a -NR^{a}R^{b} group, wherein R^{a} and R^{b} together with the nitrogen atom to which they are attached form a saturated 5- to 10-membered monocyclic or bicyclic heterocyclyl group containing one additional N atom, wherein the heterocyclyl is optionally substituted with one or more C₁₋₆ alkyl groups;
• R³ is selected from the group consisting of a hydrogen atom, a C₁₋₆ alkyl group and a phenyl group, wherein the phenyl group is optionally substituted by a halogen atom;
• R⁴ is selected from the group consisting of a hydrogen atom, a hydroxy group, an amino group, a linear or branched C₁₋₆ alkyl group, a linear or branched C₁₋₆ alkylthio group, a linear or branched C₁₋₆ alkoxy group, a (C₁₋₆ alkoxy)-(C₁₋₆ alkoxy) group, a C₃₋₁₀ cycloalkyl group, a 5 to 6-membered heteroaryl group, a C₆ aryl group, a morpohlinyl group, a -CONR^{c}R^{d} group, a -NR^{c}COR^{d} group, - NHSO₂R^{d} group; wherein
o R^{c} represents a hydrogen atom or methyl group, and R^{d} represents a linear or branched C₁₋₆ alkyl group, a C₃₋₁₀ cycloalkyl group, a C₆ aryl group, or
o R^{c} and R^{d} together with the nitrogen atom to which they are attached form a N-containing 4- to 6-membered heterocyclic group,
wherein the alkyl, aryl and heteroaryl groups are optionally substituted with a methyl group or a hydroxy group; and
• G⁵ represents a CH group.

8. A compound according to any preceeding claim, wherein
• R¹ represents a pyrazinyl group or a pyridyl group, wherein the pyridyl group is optionally substituted with a methyl group or a -CF₃ group;
• R² is selected from the group consisting of -NR^{a}R^{b}, a piperidinyl group and a tetrahydropyridinyl group, wherein
o R^{a} represents a hydrogen atom and R^{b} represents a cyclohexyl group, an azetidinyl group or an adamantyl group; or
o R^{a} and R^{b} together with the nitrogen atom to which they are attached form a piperidinyl group, a piperazinyl group, a diazepanyl group, a 2,5-diazabicyclo[2.2.1]heptanyl group, an octahydropyrrolo[3,4-c]pyrrolyl group or a 3,6-diazabicyclo[3.2.1]octanyl group,
wherein:
the piperidinyl, piperazinyl, diazepanyl, cyclohexyl and adamantyl groups are optionally substituted with one or more substituents selected from the group consisting of a fluorine atom, a -CN group, a hydroxy group, a methyl group, a methoxymethyl group, a hydroxymethyl group, a carbamoyl group and an amino group;
• R³ is selected from the group consisting of a hydrogen atom, a methyl group, an isopropyl group, a hydroxy group, a methoxy group, a hydroxymethyl group, a methoxymethyl group and a phenyl substitued with a flourine atom;
• R⁴ is selected from the group consisting of a hydrogen atom, a fluorine atom, a bromine atom, a hydroxy group, an amino group, a 2-hydroxypropan-2yl group, a -CN group, a methyl group, a butyl group, an isopentyl group, a cyclopropyl group, a phenyl group, a hydroxyphenyl group, a methoxy group, an ethoxy group, an isopropoxy group, an isobutoxy group, a butoxy group, a methoxyethoxy group, a benzyloxy group, a pyridyl group, a methylthio group, a pyrazolyl group substituted with a methyl group, a morpholinyl group, a - COOR^{d} group, a -CONR^{c}R^{d} group, a -NR^{c}COR^{d} group, and a -NHSO₂R^{d} group; wherein
o R^{c} represents a hydrogen atom, a methyl group , and R^{d} represents a hydrogen atom, a methyl group, a cyclohexyl group, a phenyl group, a benzyl group, a carboxymethyl group; or
o R^{c} and R^{d} together with the nitrogen atom to which they are attached form a piperidinyl group optionally substituted with one substituent selected from a carboxy group and a benzyl group; and
• R⁵ is selected from the group consisting of a hydrogen atom, a fluorine atom and a cyano group.

9. A compound according to claim 1 which is one of:
*N*-Pyrazin-2-yl-1-[3-(1,2,3,6-tetrahydropyridin-4-yl)phenyl]-1*H*-indazol-3-amine;
1-[3-(4-Fluoropiperidin-4-yl)phenyl]-*N*-pyrazin-2-yl-1*H*-indazol-3-amine;
1-(3-Piperazin-1-ylphenyl)-*N*-pyrazin-2-yl-1*H*-indazol-3-amine;
1-(5-Piperazin-1-ylpyridin-3-yl)-*N*-pyrazin-2-yl-1*H*-indazol-3-amine;
*N*-(4-Methylpyridin-2-yl)-1-(2-piperazin-1-ylpyridin-4-yl)-1*H*-indazol-3-amine;
1-(2-Piperazin-1-ylpyridin-4-yl)-*N*-pyrazin-2-yl-1*H*-indazol-3-amine;
6-Methoxy-1-(2-piperazin-1-ylpyridin-4-yl)-*N*-pyrazin-2-yl-1*H*-indazol-3-amine;
1-[2-(1,4-Diazepan-1-yl)pyridin-4-yl]-*N*-pyrazin-2-yl-1*H*-indazol-3-amine;
1-(2-((1S,4S)-2,5-diazabicyclo[2.2.1]heptan-2-yl)pyridin-4-yl)-*N*-(4-methylpyridin-2-yl)-1*H*-indazol-3-amine;
1-{2-[(1S,4S)-2,5-Diazabicyclo[2.2.1]hept-2-yl]pyridin-4-yl}-*N*-pyrazin-2-yl-1*H*-indazol-3-amine;
1-{2-[(1S,4S)-2,5-Diazabicyclo[2.2.1]hept-2-yl]pyridin-4-yl}-3-(pyrazin-2-ylamino)-1*H-*indazole-5-carbonitrile;
1-{2-[(1S,4S)-2,5-Diazabicyclo[2.2.1]hept-2-yl]pyridin-4-yl}-*N*³-pyrazin-2-yl-1*H*-indazole-3,6-diamine;
6-Bromo-1-{2-[(1S,4S)-2,5-diazabicyclo[2.2.1]hept-2-yl]pyridin-4-yl}-*N*-pyrazin-2-yl-1*H-*indazol-3-amine;
1-{2-[(1S,4S)-2,5-Diazabicyclo[2.2.1]hept-2-yl]pyridin-4-yl}-6-phenyl-*N*-pyrazin-2-yl-1*H-*indazol-3-amine;
Methyl 1-{2-[(1S,4S)-2,5-diazabicyclo[2.2.1]hept-2-yl]pyridin-4-yl}-3-(pyrazin-2-ylamino)-1*H*-indazole-6-carboxylate;
1-{2-[(1S,4S)-2,5-Diazabicyclo[2.2.1]hept-2-yl]pyridin-4-yl}-3-(pyrazin-2-ylamino)-1*H-*indazole-6-carboxamide;
1-{2-[(1S,4S)-2,5-Diazabicyclo[2.2.1]hept-2-yl]pyridin-4-yl}-3-(pyrazin-2-ylamino)-1*H-*indazole-6-carboxylic acid;
*N*-Cyclohexyl-1-{2-[(1S,4S)-2,5-diazabicyclo[2.2.1]hept-2-yl]pyridin-4-yl}-3-(pyrazin-2-ylamino)-1*H*-indazole-6-carboxamide;
*N*-Benzyl-1-{2-[(1S,4S)-2,5-diazabicyclo[2.2.1]hept-2-yl]pyridin-4-yl}-3-(pyrazin-2-ylamino)-1*H*-indazole-6-carboxamide;
1-{[1-{2-[(1S,4S)-2,5-Diazabicyclo[2.2.1]hept-2-yl]pyridin-4-yl}-3-(pyrazin-2-ylamino)-1*H*-indazol-6-yl]carbonyl}piperidine-4-carboxylic acid;
1-{2-[(1S,4S)-2,5-Diazabicyclo[2.2.1]hept-2-yl]pyridin-4-yl}-6-(piperidin-1-ylcarbonyl)-*N-*pyrazin-2-yl-1*H*-indazol-3-amine;
6-[(4-Benzylpiperidin-1-yl)carbonyl]-1-{2-[(1S,4S)-2,5-diazabicyclo[2.2.1]hept-2-yl]pyridin-4-yl}-*N*-pyrazin-2-yl-1*H*-indazol-3-amine;
*N*-{[1-{2-[(1S,4S)-2,5-Diazabicyclo[2.2.1]hept-2-yl]pyridin-4-yl}-3-(pyrazin-2-ylamino)-1*H*-indazol-6-yl]carbonyl}-*N*-methylglycine;
1-{2-[(1S,4S)-2,5-Diazabicyclo[2.2.1]hept-2-yl]pyridin-4-yl}-3-(pyrazin-2-ylamino)-1*H-*indazole-6-carbonitrile;
1-{2-[(1S,4S)-2,5-Diazabicyclo[2.2.1]hept-2-yl]pyridin-4-yl}-6-fluoro-*N*-pyrazin-2-yl-1*H-*indazol-3-amine;
*N-*[1-{2-[(1S,4S)-2,5-Diazabicyclo[2.2.1]hept-2-yl]pyridin-4-yl}-3-(pyrazin-2-ylamino)-1*H*-indazol-6-yl]acetamide;
*N*-[1-{2-[(1S,4S)-2,5-Diazabicyclo[2.2.1]hept-2-yl]pyridin-4-yl}-3-(pyrazin-2-ylamino)-1*H*-indazol-6-yl]benzamide;
*N*-[1-{2-[(1S,4S)-2,5-Diazabicyclo[2.2.1]hept-2-yl]pyridin-4-yl}-3-(pyrazin-2-ylamino)-1*H*-indazol-6-yl]-*N*-methylacetamide;
*N*-[1-{2-[(1S,4S)-2,5-Diazabicyclo[2.2.1]hept-2-yl]pyridin-4-yl}-3-(pyrazin-2-ylamino)-1*H*-indazol-6-yl]methanesulfonamide;
*N*-[1-{2-[(1S,4S)-2,5-Diazabicyclo[2.2.1]hept-2-yl]pyridin-4-yl}-3-(pyrazin-2-ylamino)-1*H*-indazol-6-yl]benzenesulfonamide;
1-{2-[(1S,4S)-2,5-Diazabicyclo[2.2.1]hept-2-yl]pyridin-4-yl}-6-methoxy-*N*-pyrazin-2-yl-1*H*-indazol-3-amine;
1-{2-[(1S,4S)-2,5-Diazabicyclo[2.2.1]hept-2-yl]pyridin-4-yl}-6-ethoxy-*N*-pyrazin-2-yl-1*H-*indazol-3-amine;
4-{4-[3-(Pyrazin-2-ylamino)-1*H*-indazol-1-yl]pyridin-2-yl}piperidine-4-carbonitrile;
1-{2-[(2S,5R)-2,5-dimethylpiperazin-1-yl]pyridin-4-yl}-*N*-pyrazin-2-yl-1*H*-indazol-3-amine;
(1S,2R)-*N*-{4-[3-(pyrazin-2-ylamino)-1*H*-indazol-1-yl]pyridin-2-yl}cyclohexane-1,2-diamine;
(1R,2R)-*N*-{4-[3-(pyrazin-2-ylamino)-1*H*-indazol-1-yl]pyridin-2-yl}cyclohexane-1,2-diamine;
1-{4-[3-(pyrazin-2-ylamino)-1*H*-indazol-1-yl]pyridin-2-yl}piperidine-4-carboxamide;
1-{4-[3-(Pyrazin-2-ylamino)-1*H*-indazol-1-yl]pyridin-2-yl}piperidin-4-ol;
trans-4-({4-[3-(Pyrazin-2-ylamino)-1*H*-indazol-1-yl]pyridin-2-yl}amino)cyclohexanol;
(4R,7R)-4-({4-[3-(Pyrazin-2-ylamino)-1*H*-indazol-1-yl]pyridin-2-yl}amino)adamantan-1-ol;
1-(2-Methyl-6-piperazin-1-ylpyrimidin-4-yl)-*N*-pyrazin-2-yl-1*H*-indazol-3-amine;
1-{6-[(1S,4S)-2,5-Diazabicyclo[2.2.1]hept-2-yl]-2-methylpyrimidin-4-yl}-*N*-pyrazin-2-yl-1*H*-indazol-3-amine;
1-[2-(Methoxymethyl)-6-piperazin-1-ylpyrimidin-4-yl]-*N*-pyrazin-2-yl-1*H*-indazol-3-amine;
1-{2-[(1S,4S)-2,5-Diazabicyclo[2.2.1]hept-2-yl]pyridin-4-yl}-*N*-pyrazin-2-yl-1*H-*pyrazolo[3,4-c]pyridin-3-amine;
1-{2-[(1S,4S)-2,5-Diazabicyclo[2.2.1]hept-2-yl]pyridin-4-yl}-*N*-pyrazin-2-yl-1*H-*pyrazolo[4,3-b]pyridin-3-amine;
1-{2-[(1S,4S)-2,5-Diazabicyclo[2.2.1]hept-2-yl]pyridin-4-yl}-*N*-pyrazin-2-yl-1*H-*pyrazolo[4,3-c]pyridin-3-amine;
1-{2-[(1S,4S)-2,5-Diazabicyclo[2.2.1]hept-2-yl]pyridin-4-yl}-*N*-(4-methylpyridin-2-yl)-1*H-*pyrazolo[3,4-b]pyridin-3-amine;
1-{2-[(1S,4S)-2,5-Diazabicyclo[2.2.1]hept-2-yl]pyridin-4-yl}-*N*-[4-(trifluoromethyl) pyridin-2-yl]-1*H*-pyrazolo[3,4-b]pyridin-3-amine;
1-{2-[(1S,4S)-2,5-Diazabicyclo[2.2.1]hept-2-yl]pyridin-4-yl}-*N*-pyrazin-2-yl-1*H-*pyrazolo[3,4-b]pyridin-3-amine;
1-{2-[(2S)-2-methylpiperazin-1-yl]pyridin-4-yl}-*N*-pyrazin-2-yl-1*H*-indazol-3-amine;
1-{5-[(1S,4S)-2,5-diazabicyclo[2.2.1]hept-2-yl]pyridin-3-yl}-*N*-pyrazin-2-yl-1*H*-indazol-3-amine;
{4-piperazin-1-yl-6-[3-(pyrazin-2-ylamino)-1*H*-indazol-1-yl]pyrimidin-2-yl}methanol;
1-{2-[(1S,4S)-2,5-diazabicyclo[2.2.1]hept-2-yl]pyridin-4-yl}-3-(pyrazin-2-ylamino)-1*H-*indazol-6-ol;
1-{5-[(1S,4S)-2,5-diazabicyclo[2.2.1]hept-2-yl]pyridin-3-yl}-6-methoxy-*N*-pyrazin-2-yl-1*H*-indazol-3-amine;
1-{2-[(1S,4S)-2,5-diazabicyclo[2.2.1]hept-2-yl]pyridin-4-yl}-6-methyl-*N*-pyrazin-2-yl-1*H-*indazol-3-amine;
1-{6-[(1S,4S)-2,5-diazabicyclo[2.2.1]hept-2-yl]pyrimdin-4-yl}-6-methoxy-*N*-pyrazin-2-yl-1*H*-indazol-3-amine;
1-{6-[(1S,4S)-2,5-diazabicyclo[2.2.1]hept-2-yl]pyrimidin-4-yl}-*N*-pyrazin-2-yl-1*H-*pyrazolo[3,4-b]pyridin-3-amine;
6-cyclopropyl-1-{2-[(1S,4S)-2,5-diazabicyclo[2.2.1]hept-2-yl]pyridin-4-yl}-*N*-pyrazin-2-yl-1*H*-indazol-3-amine;
1-[2-(6-methyl-1,4-diazepan-1-yl)pyridin-4-yl]-*N*-pyrazin-2-yl-1*H*-indazol-3-amine;
1-(4-piperazin-1-ylpyridin-2-yl)-*N*-pyrazin-2-yl-1*H*-indazol-3-amine;
1-{2-[(1S,4S)-2,5-diazabicyclo[2.2.1]hept-2-yl]pyridin-4-yl}-6-(2-methoxyethoxy)-*N-*pyrazin-2-yl-1*H*-indazol-3-amine;
1-[2-(1,4-diazepan-1-yl)pyridin-4-yl]-6-methoxy-*N*-pyrazin-2-yl-1*H*-indazol-3-amine;
1-{5-[(1S,4S)-2,5-Diazabicyclo[2.2.1]hept-2-yl]pyridin-3-yl}-3-(pyrazin-2-ylamino)-1*H-*indazol-6-ol;
1-{2-[(1S,4S)-2,5-Diazabicyclo[2.2.1]hept-2-yl]pyridin-4-yl}-6-methyl-*N*-pyrazin-2-yl-1*H-*pyrazolo[3,4-b]pyridin-3-amine;
1-{5-[(2S)-2-methylpiperazin-1-yl]pyridin-3-yl}-*N*-pyrazin-2-yl-1*H*-indazol-3-amine;
1-{2-[(2R)-2-methylpiperazin-1-yl]pyridin-4-yl}-*N*-pyrazin-2-yl-1*H*-indazol-3-amine;
1-[2-(3,6-diazabicyclo[3.2.1]oct-3-yl)pyridin-4-yl]-*N*-pyrazin-2-yl-1*H*-indazol-3-amine;
4-{4-[6-Methoxy-3-(pyrazin-2-ylamino)-1*H*-indazol-1-yl]pyridin-2-yl}piperidine-4-carbonitrile;
1-{4-[(1S,4S)-2,5-Diazabicyclo[2.2.1]hept-2-yl]pyridin-2-yl}-*N*-pyrazin-2-yl-1*H*-indazol-3-amine;
1-{2-[(1S,4S)-2,5-diazabicyclo[2.2.1]hept-2-yl]pyridin-4-yl}-6-isobutoxy-*N*-pyrazin-2-yl-1*H*-indazol-3-amine;
1-{5-[(1S,4S)-2,5-diazabicyclo[2.2.1]hept-2-yl]pyridin-3-yl}-6-methyl-*N*-pyrazin-2-yl-1*H-*indazol-3-amine;
6-butyl-1-{2-[(1S,4S)-2,5-diazabicyclo[2.2.1]hept-2-yl]pyridin-4-yl}-*N*-pyrazin-2-yl-1*H-*indazol-3-amine;
1-{2-[(1S,4S)-2,5-diazabicyclo[2.2.1]hept-2-yl]pyridin-4-yl}-6-(3-methylbutyl)-*N*-pyrazin-2-yl-1*H*-indazol-3-amine;
1-{2-[4-(methoxymethyl)piperidin-4-yl]pyridin-4-yl}-*N*-pyrazin-2-yl-1*H*-indazol-3-amine;
6-butoxy-1-{2-[(1S,4S)-2,5-diazabicyclo[2.2.1]hept-2-yl]pyridin-4-yl}-*N*-pyrazin-2-yl-1*H-*indazol-3-amine;
6-Methoxy-1-{2-[(2S)-2-methylpiperazin-1-yl]pyridin-4-yl}-*N*-pyrazin-2-yl-1*H*-indazol-3-amine;
1-{2-[(1S,4S)-2,5-diazabicyclo[2.2.1]hept-2-yl]pyridin-4-yl}-6-isopropoxy-*N*-pyrazin-2-yl-1*H*-indazol-3-amine;
6-(benzyloxy)-1-{2-[(2S)-2-methylpiperazin-1-yl]pyridin-4-yl}-*N*-pyrazin-2-yl-1*H*-indazol-3-amine;
6-(Benzyloxy)-1-[2-(1,4-diazepan-1-yl)pyridin-4-yl]-*N*-pyrazin-2-yl-1*H*-indazol-3-amine; 1-(5-Piperazin-1-ylpyridin-3-yl)-*N*-pyridin-2-yl-1*H*-indazol-3-amine;
1-(5-((1S,4S)-2,5-diazabicyclo[2.2.1]heptan-2-yl)pyridin-3-yl)-5-fluoro-6-methoxy-*N-*(pyrazin-2-yl)-1*H*-indazol-3-amine;
1-{5-[(1S,4S)-2,5-diazabicyclo[2.2.1]hept-2-yl]pyridin-3-yl}-5-fluoro-3-(pyrazin-2-ylamino)-1*H*-indazol-6-ol;
(4-{4-[3-(Pyrazin-2-ylamino)-1*H*-indazol-1-yl]pyridin-2-yl}piperidin-4-yl)methanol;
6-methyl-1-{2-[(2S)-2-methylpiperazin-1-yl]pyridin-4-yl}-*N*-pyrazin-2-yl-1*H*-indazol-3-amine;
1-[6-[(1S,4S)-2,5-diazabicyclo[2.2.1]hept-2-yl]-2-(3-fluorophenyl)pyrimidin-4-yl]-6-methoxy-*N*-pyrazin-2-yl-1*H*-indazol-3-amine;
1-[2-(1,4-diazepan-1-yl)pyridin-4-yl]-3-(pyrazin-2-ylamino)-1*H*-indazol-6-ol; 1-{2-[(2S)-2-methylpiperazin-1-yl]pyridin-4-yl}-3-(pyrazin-2-ylamino)-1*H*-indazol-6-ol;
1-{2-[(1S,4S)-2,5-diazabicyclo[2.2.1]hept-2-yl]pyridin-4-yl}-3-[(4-methylpyridin-2-yl)amino]-1*H*-indazol-6-ol;
1-{3-[(1S,4S)-2,5-diazabicyclo[2.2.1]hept-2-yl]phenyl}-6-methoxy-*N*-pyrazin-2-yl-1*H-*indazol-3-amine;
4-[1-{2-[(1S,4S)-2,5-diazabicyclo[2.2.1]hept-2-yl]pyridin-4-yl}-3-(pyrazin-2-ylamino)-1*H-*indazol-6-yl]phenol;
1-{2-[(1S,4S)-2,5-Diazabicyclo[2.2.1]hept-2-yl]pyridin-4-yl}-6-morpholin-4-yl-*N*-pyrazin-2-yl-1*H*-pyrazolo[3,4-b]pyridin-3-amine;
1-{2-[(1S,4S)-2,5-diazabicyclo[2.2.1]hept-2-yl]pyridin-4-yl}-3-{[4-(trifluoromethyl)pyridin-2-yl]amino}-1*H*-indazol-6-ol;
Benzyl 1-{2-[(1S,4S)-2,5-diazabicyclo[2.2.1]hept-2-yl]pyridin-4-yl}-3-(pyrazin-2-ylamino)-1*H*-indazole-6-carboxylate;
1-{5-[(1S,4S)-2,5-diazabicyclo[2.2.1]hept-2-yl]-6-methoxypyridin-3-yl}-*N*-pyrazin-2-yl-1*H*-indazol-3-amine;
1-{2-[(1S,4S)-2,5-diazabicyclo[2.2.1]hept-2-yl]pyridin-4-yl}-6-(methylthio)-*N*-pyrazin-2-yl-1*H*-indazol-3-amine;
1-{2-[(2S,5S)-2,5-Dimethylpiperazin-1-yl]pyridin-4-yl}-*N*-pyrazin-2-yl-1*H*-indazol-3-amine;
1-{6-[(1S,4S)-2,5-diazabicyclo[2.2.1]hept-2-yl]-2-isopropylpyrimidin-4-yl}-3-(pyrazin-2-ylamino)-1*H*-indazol-6-ol;
1-{2-[(1S,4S)-2,5-diazabicyclo[2.2.1]hept-2-yl]pyridin-4-yl}-*N*-pyrazin-2-yl-6-pyridin-3-yl 1*H*-indazol-3-amine;
1-{2-[(1S,4S)-2,5-diazabicyclo[2.2.1]hept-2-yl]pyridin-4-yl}-6-(1-methyl-1*H*-pyrazol-4-yl)-*N*-pyrazin-2-yl-1*H*-indazol-3-amine;
1-{5-[(2S)-2-Methylpiperazin-1-yl]pyridin-3-yl}-3-(pyrazin-2-ylamino)-1*H*-indazol-6-ol;
2-[1-{2-[(1S,4S)-2,5-Diazabicyclo[2.2.1]hept-2-yl]pyridin-4-yl}-3-(pyrazin-2-ylamino)-1*H-*indazol-6-yl]propan-2-ol;
1-[2-(Azetidin-3-ylamino)pyridin-4-yl]-*N*-pyrazin-2-yl-1*H*-indazol-3-amine;
1-{-[(3aR,6aS)-Hexahydropyrrolo[3,4-c]pyrrol-2(1*H*)-yl]pyridin-4-yl}-N-pyrazin-2-yl-1*H-*indazol-3-amine;
1-{6-[(1S,4S)-2,5-Diazabicyclo[2.2.1]hept-2-yl]-2-methylpyrimidin-4-yl}-3-(pyrazin-2-ylamino)-1*H*-indazol-6-ol;
1-{2-Methyl-6-[(2S)-2-methylpiperazin-1-yl]pyrimidin-4-yl}-3-(pyrazin-2-ylamino)-1*H-*indazol-6-ol;
3-[(1S,4S)-2,5-Diazabicyclo[2.2.1]hept-2-yl]-5-[3-(pyrazin-2-ylamino)-1*H*-indazol-1-yl]pyridin-2-ol; or
1-{3-[(2S)-2-Methylpiperazin-1-yl]phenyl}-3-(pyrazin-2-ylamino)-1*H*-indazol-6-ol.

10. A compound according to any one of claims 1 to 9 for use in the treatment of the human or animal body by therapy.

11. A compound according to any one of claims 1 to 9 for use in the treatment of a pathological condition or disease selected from asthma, chronic obstructive pulmonary disease, allergic rhinitis, allergic conjunctivitis, allergic dermatitis, atopic dermatitis, seasonal allergies, allergic and inflammatory ophthalmic diseases, rheumatoid arthritis, multiple sclerosis, systemic lupus erythematosus, type I diabetes, B cell lymphoma, chronic lymphocytic leukemia, immune thrombocytopenic purpura, macular degeneration, diabetic retinopathy, diabetic macular edema, diabetic nephropathy, glomerulonephritis, and ischemia reperfusion injury.

12. A pharmaceutical composition comprising a compound as defined in any one of claims 1 to 9 in association with a pharmaceutically acceptable diluent or carrier.

13. Use of a compound as defined in any one of claims 1 to 9 for the manufacture of a medicament for the treatment of a pathological condition or disease as defined in claim 11.

14. A method for treating a subject afflicted with a pathological condition or disease as defined in claim 11, which comprises administering to said subject an effective amount of a compound as defined in any one of claims 1 to 9.

15. A combination product comprising (i) a compound according to anyone of claims 1 to 9, and (ii) one or more active ingredients selected from the group consisting of a β2-adrenergic agonist, a corticosteroid, an anti-cholinergic agent, an anti-allergic agent, an anti-inflammatory agent, an immunosuppressant, a PDE4 inhibitor, a topically acting p38 inhibitor, an IKK2 inhibitor, an A_{2A} agonist, a PI3Kδγ inhibitor and an anti-infective agent, for simultaneous, separate or sequential use in the treatment of the human or animal body.
